(19) Europäisches Patentamt
European Patent Office
Office européen des brevets

(11) Publication number: **0 057 546**
**A2**

(12) **EUROPEAN PATENT APPLICATION**

(21) Application number: 82300353.8

(22) Date of filing: 25.01.82

(51) Int. Cl.³: C 07 D 239/42, C 07 D 239/46, C 07 D 251/22, A 01 N 47/36

(30) Priority: 26.01.81 US 228706
30.11.81 US 325121

(43) Date of publication of application: 11.08.82 Bulletin 82/32

(84) Designated Contracting States: AT BE CH DE FR GB IT LI LU NL SE

(71) Applicant: E.I. DU PONT DE NEMOURS AND COMPANY, Legal Department 1007 Market Street, Wilmington Delaware 19898 (US)

(72) Inventor: Tseng, Chi Ping, 1103 Artwin Road, Wilmington Delaware 19803 (US)

(74) Representative: Hildyard, Edward Martin et al, Frank B. Dehn & Co. European Patent Attorneys Imperial House 15-19 Kingsway, London WC2B 6UZ (GB)

(54) **Sulfonylurea N-oxides.**

(57) Compounds of the formula

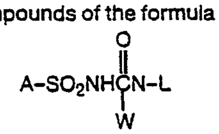

and their agriculturally suitable salts, where

A is 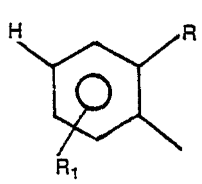 , 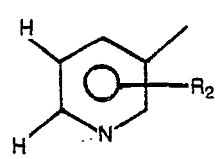 , 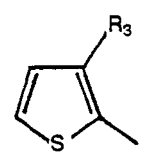 ,

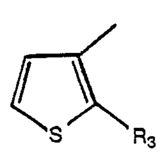 , 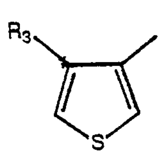 or 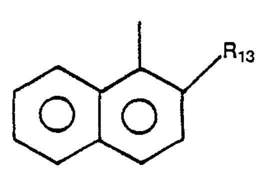 ;



EP 0 057 546 A2

where R, $R_2$ and $R_3$ are halogen, $NO_2$ or various organic groups; $R_1$ and $R_{13}$ are H, halogen, $NO_2$ or various organic groups; W is H or $CH_3$; and

L is

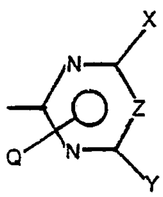
,
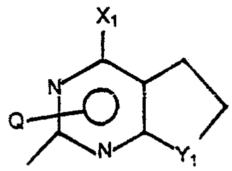
,
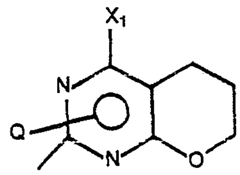
or
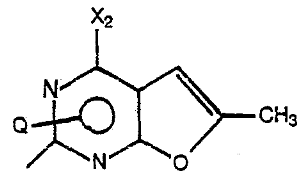

where X is $CH_3$, $C_2H_5$, $CH_3O$ or $C_2H_5O$;
Y and $X_1$ are H, Cl or various organic groups;
$Y_1$ is O or $CH_2$;
$X_2$ is $CH_3$ or $C_2H_5$;
Z is CH, CF or N; and
Q is O bonded to a nitrogen atom of L;

exhibit herbicidal and plant growth regulant effects. They may be formulated for use in conventional manner. The compounds may be made by reacting an isocyanate

$ASO_2NCO$

with an aminopyrimidine-N-oxide or aminotriazine-N-oxide

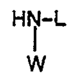

Some of the novel compounds can be made by esterifying the corresponding novel carboxylic acids wherein R or $R_3$ is $CO_2H$.

Title BA-8418-A

# SULFONYLUREA N-OXIDES

## Background of the Invention

This invention relates to ureas and isoureas and in particular their use as agricultural chemicals and particularly as herbicides.

U.S. Patent 4,127,405 teaches compounds which are useful for controlling weeds in wheat having the formula

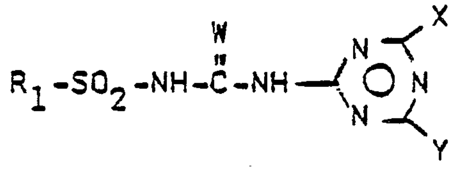

wherein

$R_1$ is

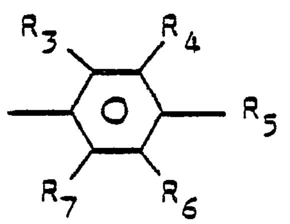 , 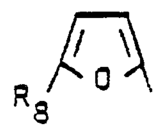 , 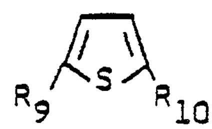

or 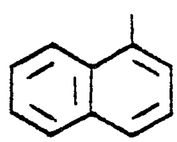 ;

$R_3$ and $R_6$ are independently hydrogen, fluorine, chlorine, bromine, iodine, alkyl of 1-4 carbon atoms, alkoxy of 1-4 carbon atoms, nitro, trifluoromethyl, cyano, $CH_3S(O)_n$- or $CH_3CH_2S(O)_n$-;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1-2 carbon atoms or alkoxy of 1-2 carbon atom;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1-3 carbon atoms, trifluoromethyl, $CH_3S$- or $CH_3OCH_2$-; and

Y is methyl or methoxy; or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

French Patent No. 1,468,747 discloses the following <u>para</u>-substituted phenylsulfonamides, useful as antidiabetic agents:

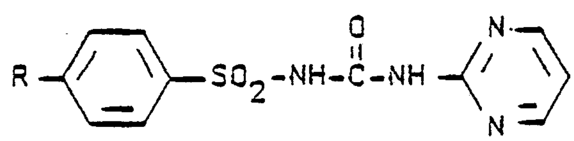

wherein

R = H, halogen, $CF_3$ or alkyl.

Logemann et al., Chem. Ab., <u>53</u>, 18052g (1959), disclose a number of sulfonamides, including uracil derivatives and those having the formula:

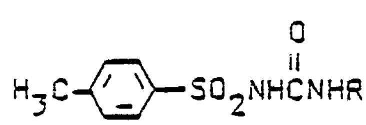

wherein

R is butyl, phenyl or 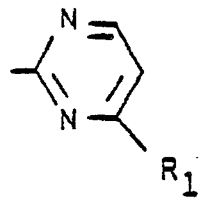 ; and

$R_1$ is hydrogen or methyl.

When tested for hypoglycemic effect in rats (oral doses of 25 mg/100 g), the compounds in which R is butyl and phenyl were most potent. The others were of low potency or inactive.

Wojciechowski, J. Acta. Polon. Pharm. 19, p. 121-5 (1962) [Chem. Ab., 59 1633 e] describes the synthesis of N-[(2,6-dimethoxypyrimidin-4-yl)aminocarbonyl]-4-methylbenzenesulfonamide:

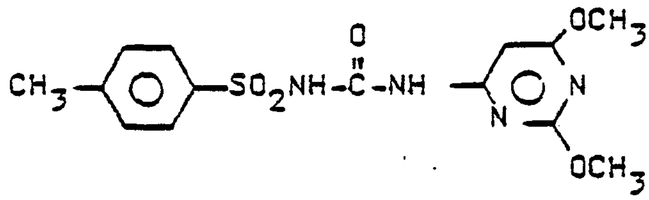

Based upon similarity to a known compound, the author predicted hypoglycemic activity for the foregoing compound.

Netherlands Patent 121,788, published September 15, 1966, teaches the preparation of compounds of Formula (i), and their use as general or selective herbicides,

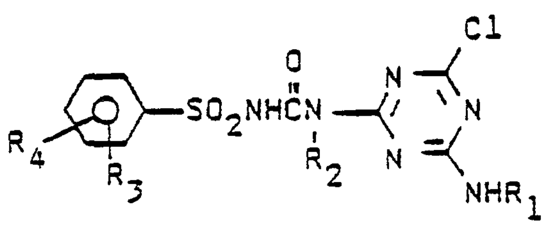 (i)

wherein

$R_1$ and $R_2$ may independently be alkyl of 1-4 carbon atoms; and

$R_3$ and $R_4$ may independently be hydrogen, chlorine or alkyl of 1-4 carbon atoms.

Compounds of Formula (ii), and their use as antidiabetic agents, are reported in J. Drug. Res. 6, 123 (1974),

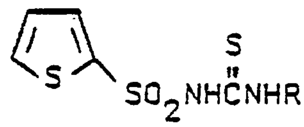 (ii)

wherein

R is pyridyl.

The presence of undesired vegetation causes substantial damage to useful crops, especially agricultural products that satisfy man's basic food needs, such as soybeans, barley, wheat, and the like. The current population explosion and concomitant world food shortage demand improvements in the efficiency of producing these crops. Prevention or minimizing the loss of a portion of valuable crops by killing, or inhibiting the growth of undesired vegetation is one way of improving this efficiency.

A wide variety of materials useful for killing, or inhibiting (controlling) the growth of undesired vegetation is available; such materials are commonly referred to as herbicides. The need exists, however, for still more effective herbicides that destroy or retard weeds without causing significant damage to useful crops.

Summary of the Invention

This invention relates to novel compounds of Formula I, to compositions containing them and to their method of use as general and selective pre- and post-emergence herbicides and as plant growth regulants.

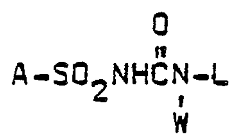

(I)

where

A is 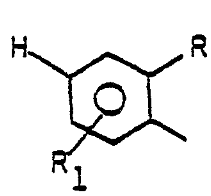, 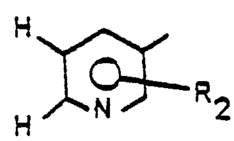, 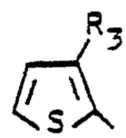,

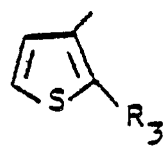, 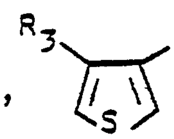 or 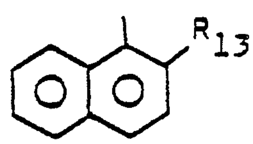;

R is $C_1$-$C_4$ alkyl, $C_1$-$C_4$ alkoxy, F, Cl, Br, $NO_2$, $CF_3$, $COR_4$, $S(O)_mR_5$, $SO_2NR_6R_7$, $SO_2OCH_2CF_3$, $SO_2OCH_2CCl_3$, $SO_2N(OCH_3)(CH_3)$, $BR_8$, $OSO_2R_9$ or $CH_2P$;

$R_1$ is H, F, Cl, Br, $CF_3$, $NO_2$, $C_1$-$C_4$ alkyl or $C_1$-$C_4$ alkoxy;

$R_4$ is $C_1$-$C_6$ alkoxy, $C_3$-$C_6$ alkenyloxy, $C_3$-$C_6$ alkynyloxy, $C_2$-$C_6$ haloalkoxy substituted with 1-3 atoms selected from F, Cl or Br, $C_5$-$C_6$ cycloalkoxy, $-O(CH_2CH_2O)_nR_{10}$, $OCH_2CH_2CH_2OR_{10}$, $OCH_2OR_5$ or $OCH_2OCH_2CH_2OR_{10}$;

n is 1 or 2;

m is 0, 1 or 2;

$R_5$ is $C_1$-$C_4$ alkyl;

$R_6$ and $R_7$ are independently $C_1$-$C_4$ alkyl, provided the total carbon atoms of $R_6$ and $R_7$ does not exceed 5;

B is O or $S(O)_m$;

$R_8$ is $CHF_2$, $CF_3$, $CH_2CF_3$ or $CF_2CHFG$ where G is F, Cl, Br or $CF_3$;

$R_9$ is $C_1$-$C_4$ alkyl or $C_1$-$C_4$ alkyl substituted with 1-3 atoms of F, Cl or Br;

P is Cl, Br, $C_1$-$C_4$ alkoxy, $C_3$-$C_4$ alkenyloxy or $S(O)_mR_5$;

$R_{10}$ is $CH_3$ or $CH_3CH_2$;

$R_2$ is F, Cl, Br, $C_1$-$C_4$ alkyl, $C_1$-$C_4$ alkoxy, $NO_2$, $CO_2R_{11}$ or $S(O)_mR_{12}$;

$R_{11}$ is $C_1$-$C_4$ alkyl;

$R_{12}$ is $C_1$-$C_3$ alkyl;

$R_3$ is $C_1$-$C_4$ alkyl, $C_1$-$C_4$ alkoxy, H, F, Cl, Br, $NO_2$, $SO_2NR_6R_7$, $SO_2N(OCH_3)(CH_3)$ or $COR_4$;

$R_{13}$ is H, Cl, $NO_2$, $S(O)_mR_5$, $SO_2NR_6R_7$, $C_1$-$C_4$ alkyl or $C_1$-$C_4$ alkoxy;

W is H or $CH_3$;

L is

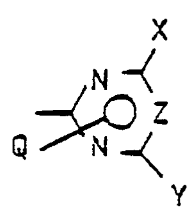
,
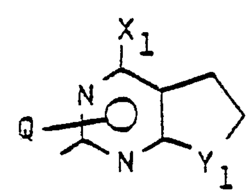
,

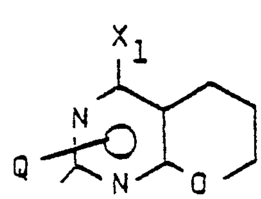
or
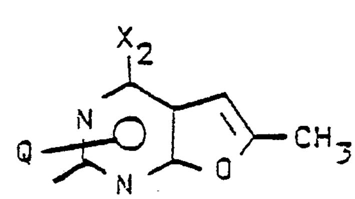

wherein

X is $CH_3$, $CH_3CH_2$, $CH_3O$ or $CH_3CH_2O$;

Y is H, Cl, $CF_3$, $C_1$-$C_4$ alkyl, $C_1$-$C_4$ alkoxy, $CH_2OCH_3$, $CH_2OCH_2CH_3$, $CH_2CH_2OCH_3$ or $CH_2CH_2OCH_2CH_3$;

$X_1$ is H, Cl, $CH_3$, $CH_3CH_2$, $CH_3O$ or $CH_3CH_2O$;

$Y_1$ is O or $CH_2$;

$X_2$ is $CH_3$ or $CH_3CH_2$;

Z is CH, CF or N; and

Q is O bonded to a nitrogen atom of L.

Preferred for reasons of high activity and/or ease of synthesis are those compounds of the generic scope in which:

1) A is 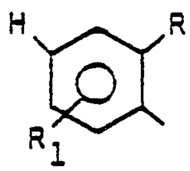 ;

2) A is 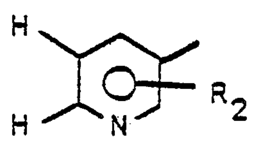 ;

3) A is 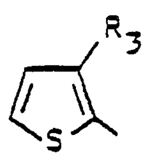 , 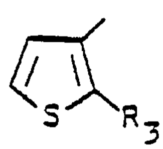 or 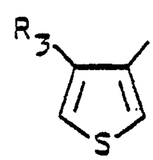 ;

4) A is 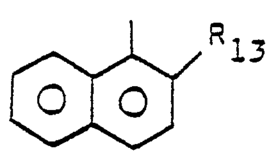 .

More preferred for reasons of higher activity and/or greater ease of synthesis are:

5) Compounds of the Preferred Group 1 in which

L is 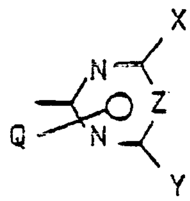 ;

6) Compounds of the Preferred Group 2 in which

L is 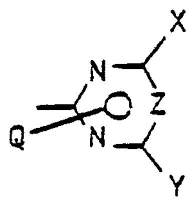 ;

7) Compounds of the Preferred Group 3 in which

L is 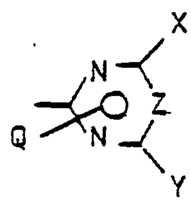 ;

8) Compounds of the Preferred Group 4 in which

L is 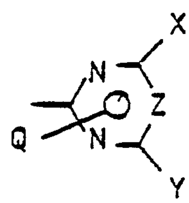 .

Even More Preferred in increasing order for higher activity and/or greater ease of synthesis are:

9) Compounds of the Preferred Group 5 in which:
R is $C_1$-$C_3$ alkyl, $OCH_3$, F, Cl, Br, $NO_2$, $CF_3$, $COR_4$, $S(O)_mR_5$, $SO_2NR_6R_7$, $SO_2N(OCH_3)(CH_3)$, $BR_8$, $OSO_2R_9$ or $CH_2P$;
$R_4$ is $C_1$-$C_4$ alkoxy, $C_3$-$C_4$ alkenyloxy or haloethoxy having 1-3 atoms of F or Cl; and
B is O.

10) Compounds of Group 9 in which:
$R_1$ is H, Cl, Br, $OCH_3$, $CH_3$ or $CF_3$;
$R_4$ is $C_1$-$C_4$ alkoxy, $C_3$-$C_4$ alkenyloxy or $OCH_2CH_2Cl$;
$R_8$ is $CF_3$, $CH_2CF_3$ or $CF_2CHF_2$;
$R_9$ is $CH_3$ or $CF_3$; and
P is $C_1$-$C_3$ alkoxy, $OCH_2CH{=}CH_2$ or $S(O)_mCH_3$.

11) Compounds of Group 10 in which:
$R_1$ is H and W is H.

Also Even More Preferred in increasing order for even higher activity and/or even greater ease of synthesis are:

12) Those compounds of the Preferred Group 6 in which $R_2$ is in the 2-position of the pyridine ring.

13) Compounds of Group 12 in which $R_2$ is Cl, Br, $CH_3$, $OCH_3$ or $S(O)_mCH_3$ and W is H.

Also More Preferred in increasing order for even higher activity and/or greater ease of synthesis are:

14) Those compounds of the Preferred Group 7 in which $R_3$ is $CH_3$, H, Cl, Br or $COR_4$.

15) Compounds of Group 14 in which $R_4$ is $C_1$-$C_4$ alkoxy, $C_3$-$C_4$ alkenyloxy or haloethoxy having 1-3 atoms of F or Cl.

16) Compounds of Group 15 in which $R_4$ is $C_1$-$C_4$ alkoxy, $C_3$-$C_4$ alkenyloxy or $OCH_2CH_2Cl$.

17) Compounds of Group 16 in which A is

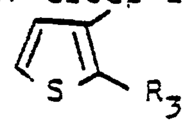

and W is H.

Also Even More Preferred for even higher activity and/or greater ease of synthesis are:

18) Those compounds of the Preferred Group 8 in which $R_{13}$ is Cl, $C_1$-$C_3$ alkyl and $C_1$-$C_3$ alkoxy.

19) Compounds of Group 18 in which $R_{13}$ is Cl and W is H.

Most Preferred in increasing order and for reasons of highest activity and/or greatest ease of synthesis are:

20) Compounds of the Even More Preferred Group 9 in which X and Y are independently $CH_3$ or $CH_3O$.

21) Compounds of the Even More Preferred Group 10 in which X and Y are independently $CH_3$ or $CH_3O$.

22) Compounds of the Even More Preferred Group 11 in which X and Y are independently $CH_3$ or $CH_3O$.

Also Most Preferred in increasing order and for highest activity and/or greatest ease of synthesis are:

23) Compounds of the Even More Preferred Group 12 in which X and Y are independently $CH_3$ or $CH_3O$.

24) Compounds of the Even More Preferred Group 13 in which X and Y are independently $CH_3$ or $CH_3O$.

Also Most Preferred in increasing order and for highest activity and/or greatest ease of synthesis are:

25) Compounds of the Even More Preferred Group 14 in which X and Y are independently $CH_3$ or $CH_3O$.

26) Compounds of the Even More Preferred Group 15 in which X and Y are independently $CH_3$ or $CH_2O$.

27) Compounds of the Even More Preferred Group 16 in which X and Y are independently $CH_3$ or $CH_3O$.

28) Compounds of the Even More Preferred Group 17 in which X and Y are independently $CH_3$ or $CH_3O$.

Also Most Preferred in increasing order and for highest activity and/or greatest ease of synthesis are:

29) Compounds of the Even More Preferred Group 18 in which X and Y are independently $CH_3$ or $CH_3O$.

30) Compounds of the Even More Preferred Group 19 in which X and Y are independently $CH_3$ or $CH_3O$.

Specifically preferred for reasons of activity and/or ease of synthesis are:

2-chloro-N-[(4,6-dimethyl-1-oxidepyrimidin-2-yl)-aminocarbonyl]benzenesulfonamide;

2-[[(4,6-dimethyl-1-oxidepyrimidin-2-yl)aminocarbonyl]aminosulfonyl]benzoic acid, methyl ester;

2-nitro-N-[(4,6-dimethyl-1-oxidepyrimidin-2-yl)aminocarbonyl]benzenesulfonamide;

2-[[(4,6-dimethyl-1-oxidepyrimidin-2-yl)aminocarbonyl]-aminosulfonyl]benzoic acid, 2-propenyl ester;

2-chloro-N-[(4-methoxy-6-methyl-1-oxidepyrimidin-2-yl)-aminocarbonyl]benzenesulfonamide;

2-[[(4-methoxy-6-methyl-1-oxidepyrimidin-2-yl)aminocarbonylaminosulfonyl]benzoic acid, methyl ester

2-nitro-N-[(4-methoxy-6-methyl-1-oxidepyrimidin-2-yl)-aminocarbonyl]benzenesulfonamide;

2-[[(4-methoxy-6-methyl-1-oxidepyrimidin-2-yl)aminocarbonyl]aminosulfonyl]benzoic acid, 1-methylethyl ester;

2-[[(4-methoxy-6-methyl-1-oxidepyrimidin-2-yl)aminocarbonyl]aminosulfonyl]benzoic acid, 2-propenyl ester;

2-[[(4-methoxy-6-methyl-1-oxidetriazin-2-yl)aminocarbonyl]aminosulfonyl]benzoic acid, methyl ester;

2-[[(4-methoxy-6-methyl-3-oxidetriazin-2-yl)aminocarbonyl]aminosulfonyl]benzoic acid, methyl ester;

2-chloro-N-[(4-methoxy-6-methyl-1-oxidetriazin-2-yl) aminocarbonyl]benzenesulfonamide;

2-chloro-N-[(4-methoxy-6-methyl-3-oxidetriazin-2-yl)-aminocarbonyl]benzenesulfonamide;

2-(propylsulfonyl)-N-[(4,6-dimethoxy-1-oxidepyrimidin-2-yl)aminocarbonyl]benzenesulfonamide;

2-(propylsulfonyl)-N-[(4-methoxy-6-methyl-1-oxidetriazin-2-yl)aminocarbonyl]benzenesulfonamide;

2-(propylsulfonyl)-N-[(4-methoxy-6-methyl-3-oxidetriazin-2-yl)aminocarbonyl]benzenesulfonamide;

2-(propoxy)-N-[(4,6-dimethoxy-1-oxidetriazin-2-yl)-aminocarbonyl]benzenesulfonamide;

2-chloro-N-[(4,6-dimethoxy-1-oxidepyrimidin-2-yl)aminocarbonyl]-1-naphthalenesulfonamide;

N',N'-dimethyl-N-[(4,6-dimethyl-1-oxidetriazin-2-yl)-aminocarbonyl]-1,2-benzenesulfonamide; and

N',N'-dimethyl-N-[(4-methoxy-6-methyl-1-oxidetriazin-2-yl)aminocarbonyl]-1,2-benzenesulfonamide.

Also, novel compounds of Formula II are valuable intermediates for the preparation of the herbicides of this invention.

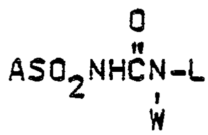

(II)

wherein

A is 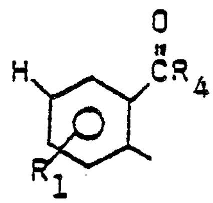 , 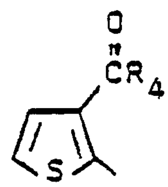 ,

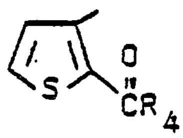 or 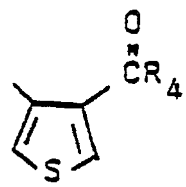

wherein

$R_1$, W and L are as previously defined; and

$R_4$ is OH.

Preferred Intermediates for reasons of ease of synthesis and/or high activity of the derived products are:

1) Compounds of Formula II in which A is

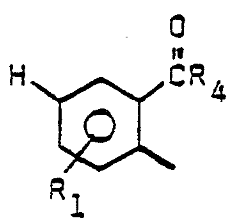 , and W is H.

2) Compounds of Formula II in which A is

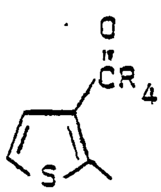 , 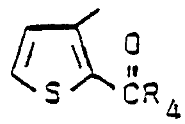 or

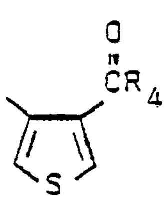 and W is H.

More Preferred Intermediates for greater ease of synthesis and/or higher activity of the derived products are:

3) Compounds of the Preferred Intermediates Group 1 in which:

L is 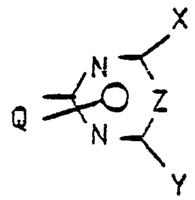 ;

wherein X, Y, Z and Q are as previously defined.

4) Compounds of the Preferred Intermediates Group 2 in which

L is 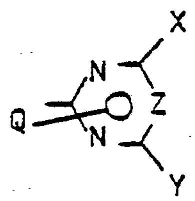 ;

Most Preferred Intermediates in increasing order for reasons of greatest ease of synthesis and/or highest activity of derived products are:

5) Compounds of More Preferred Intermediates Group 3 in which:
$R_1$ is H, Cl, Br, $OCH_3$, $CH_3$ or $CF_3$.

6) Compounds of Group 5 in which $R_1$ is H.

7) Compounds of Group 6 in which X and Y are independently $CH_3$ or $CH_3O$.

Also Most Preferred Intermediates for reasons of greatest ease of synthesis and/or highest activity of derived products are:

8) Compounds of More Preferred Intermediates Group 4 in which A is

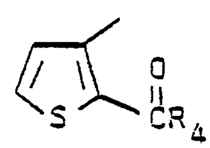

9) Compounds of Group 8 in which X and Y are independently $CH_3$ or $CH_3O$.

Specifically Preferred Intermediates for reasons of ease of synthesis and/or activity of derived products are:

2-[[(4,6-dimethyl-1-oxidepyrimidin-2-yl)aminocarbonyl]-aminosulfonyl]benzoic acid;

3-[[(4,6-dimethyl-1-oxidepyrimidin-2-yl)aminocarbonyl]-aminosulfonyl]-2-thiophenecarboxylic acid;

2-[[(4,6-dimethoxy-1-oxidepyrimidin-2-yl)aminocarbonyl]-aminosulfonyl]benzoic acid; and

3-[[(4,6-dimethoxy-1-oxidepyrimidin-2-yl)aminocarbonyl]-aminosulfonyl]-2-thiophenecarboxylic acid.

Also novel compounds of Formula III, IV, V and VI are valuable intermediates for the preparation of herbicides of this invention.

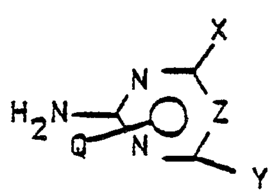

,

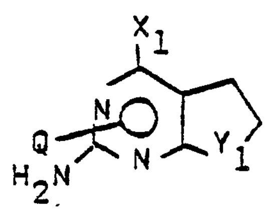

,

III

IV

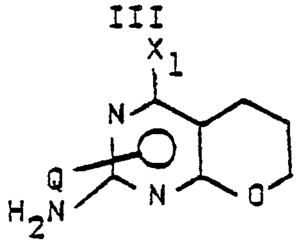

and

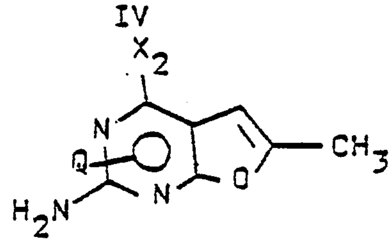

V

VI

wherein

$X$, $Y$, $X_1$, $Y_1$, $X_2$, Z and Q are as previously defined;

provided that:

(1) when Z is N, X and Y cannot both be alkyl, and

(2) when Z is CH, X and Y cannot both be methyl.

Preferred Intermediates for reasons of ease of synthesis and/or high activity of the desired products are:

1) Compounds of Formula III in which X is $CH_3$ or $CH_3O$.
2) Compounds of Formula IV in which $X_1$ is $CH_3$ or $CH_3O$; and $Y_1$ is O.
3) Compounds of Formula V in which $X_1$ is $CH_3$ or $CH_3O$.
4) Compounds of Formula VI in which $X_2$ is $CH_3$.

More Preferred Intermediates for reasons of greater ease of synthesis and/or higher activity of the derived products are:

5) Compounds of the Preferred Intermediates Group 1.

Most Preferred Intermediates for reasons of greatest ease of synthesis and/or activity of the derived products are:

6) Compounds of the More Preferred Intermediates in which X and Y are independently $CH_3$ or $CH_3O$.

Specifically preferred Intermediates for reasons of ease of synthesis and/or activity of the derived products are:

4,6-dimethoxy-1-oxide-2-pyrimidinamine;
4-methoxy-6-methyl-1-oxide-2-pyrimidinamine;
4,6-dimethoxy-1-oxide-2-(1,3,5-triazinamine); and
4-methoxy-6-methyl-1-oxide-2-(1,3,5-triazinamine).

Synthesis

Many of the compounds of Formula I can be prepared by reacting an appropriate aminopyrimidine-N-oxide or aminotriazine-N-oxide of Formula VIII with an appropriately substituted sulfonyl isocyanate of Formula VII, as shown in Equation 1.

Equation 1

$$ASO_2NCO + \underset{\displaystyle W}{HN}\text{-}L \longrightarrow ASO_2NH\overset{\displaystyle O}{\overset{\|}{C}}\underset{\displaystyle W}{N}\text{-}L$$

VII VIII I

wherein A, W and L are as previously defined. Compounds which cannot be prepared by this method are those compounds where R is $S(O)R_5$, $CH_2P$ (when P is $S(O)R_5$), $COR_4$ (when $R_4$ is OH, $OCH_2OR_5$, $OCH_2OCH_2CH_2OR_{10}$ or $C_3$-$C_6$ alkynyloxy); also those compounds where $R_2$ is $S(O)R_{12}$, $R_3$ is $COR_4$ (when $R_4$ is OH, $OCH_2OR_5$, $OCH_2OCH_2CH_2OR_{10}$ or $C_3$-$C_6$ alkynyloxy), and where $R_{13}$ is $S(O)R_5$. In those instances, the compounds may be prepared from appropriately substituted compounds of Formula I in one or more steps which will be described below in part C: Special Preparations of compounds of Formula I.

The reaction is best carried out in inert aprotic organic solvents such as methylene chloride, tetrahydrofuran or acetonitrile, at ambient pressure and temperature. The mode of addition is not critical; however, it is often convenient to add the sulfonyl isocyanate to a stirred suspension of the aminoheterocycle. Since such isocyanates usually are liquids, their addition can be easily controlled.

The reaction is generally exothermic. In some cases, the desired product is insoluble in the reaction medium and crystallizes from it in pure form. Products soluble in the reaction medium are isolated by evaporation of the solvent, trituration of the solid residue with solvents such as 1-chlorobutane or ethyl ether, and filtration.

A. Sulfonyl Isocyanate Intermediates

The intermediate aryl sulfonyl isocyanates of Formula VII can be prepared by reacting corresponding aryl sulfonamides with phosgene in the presence of *n*-butyl isocyanate at reflux in a solvent such as chlorobenzene, according to the procedure of H. Ulrich and A. A. Y. Sayigh, *Newer Methods of Preparative Organic Chemistry*, Vol. VI, p. 223-241, Academic Press, New York and London, W. Forest Ed. The intermediate pyridyl sulfonyl isocyanate of Formula II can be prepared by reacting an N-(alkylaminocarbonyl)-pyridinesulfonamide with phosgene as described in our European Patent Specification No. 0013480 to which the reader is referred for further information. The N-(alkylaminocarbonyl)pyridinesulfonamide can be prepared, as described in our said European Patent Specification, by the reaction of a pyridinesulfonamide, an alkyl isocyanate and an anhydrous base in an anhydrous solvent.

Similarly, the thiophene sulfonyl isocyanates can be prepared as shown in Equation 2.

Equation 2

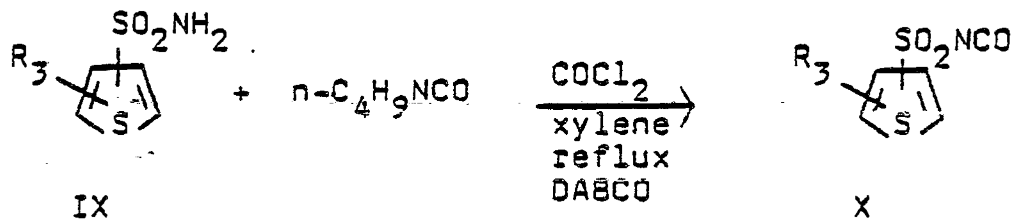


IX X

wherein $R_3$ is as previously defined.

A mixture of the appropriate sulfonamide, e.g., an 2-alkoxycarbonyl-3-thiophene sulfonamide IX such as the methyl ester, which is known in the art, an alkyl isocyanate such as butyl isocyanate and a catalytic amount of 1,4-diaza[2.2.2]bicyclooctane (DABCO) in xylene or other inert solvent of sufficiently high boiling point (e.g. 135°C) is heated to approximately 130-150°C. Phosgene is added to the mixture until an excess of phosgene is present as indicated by a drop in the boiling point. After the mixture is cooled and filtered to remove a small amount of insoluble by-products, the solvent and alkyl isocyanate are distilled off in-vacuo leaving a residue which is the crude sulfonyl isocyanate X.

The preparation of the naphthylsulfonylisocyanates proceeds in a directly analogous manner from the corresponding naphthalene-2-sulfonamides.

The preparation of sulfonamides from ammonium hydroxide and sulfonyl chloride is widely reported in the literature, e.g., Crossley et al., J. Am. Chem. Soc. 60, 2223 (1938). The preparation of pyridylsulfonamide is described in G. Machek, Monatsch 2, 84 (1939) and L. Thunus and C. L. Lapiere, Ann. Farn. 33, 663 (1975).

Certain sulfonyl chlorides are best prepared by chlorosulfonation of a substituted benzene in carbon tetrachloride according to the teaching of H. T. Clarke et al., Org. Synth. Coll. Vol. 1, 2nd Ed., 1941, p. 85. Other benzenesulfonyl chlorides are

best made by diazotization of the appropriate aniline with sodium nitrite in HCl, followed by reaction of the diazonium salt with sulfur dioxide and cuprous chloride in acetic acid according to the teaching of H. L. Yale and F. Sowinski, J. Org. Chem. 25, 1824 (1960). The preparation of pyridyl sulfonyl chlorides is described in Chem. Abs. 88, 190603 m (1978).

The naphthalene-2-sulfonamide intermediates XIII may be synthesized by several procedures. Most generally, the sulfonamides may be prepared from the sulfonyl chlorides XII as described in "Preparative Organic Chemistry", G. Hilgetag

Equation 3

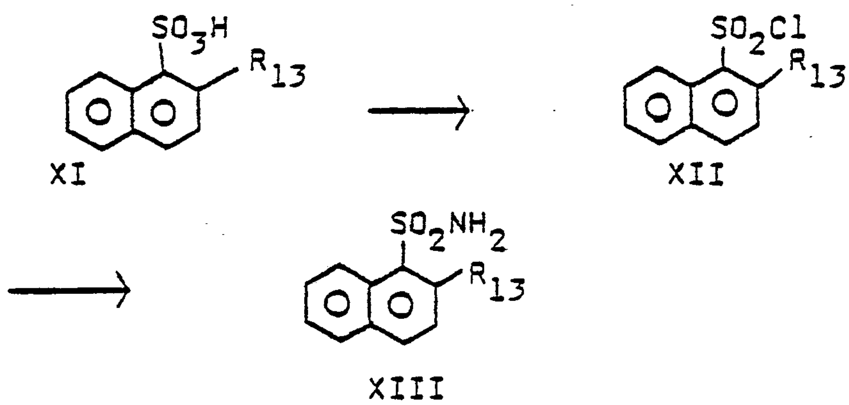

XI XII XIII

wherein $R_{13}$ is as previously defined.

and A. Martini, ed., J. Wiley and Sons, New York (1972). The sulfonyl chlorides may be prepared by chlorination of the sulfonic acids VI, by the methods described by Hilgetag and Martini, op. cit. The preparation of these compounds is known in the art (e.g., "Elsevier's Encyclopedia of Organic Chemistry" F. Radt, ed., Vol. 12B, pp. 4841-5686, Elsevier, Amsterdam, 1956). These compounds may be further modified by methods known in the art to yield other disclosed sulfonic acids.

The method used for preparing the intermediate sulfonamides when R or $R_3$ is $SO_2NR_6R_7$ is included in Equations 5a-d.

Equations 5a-d

5a

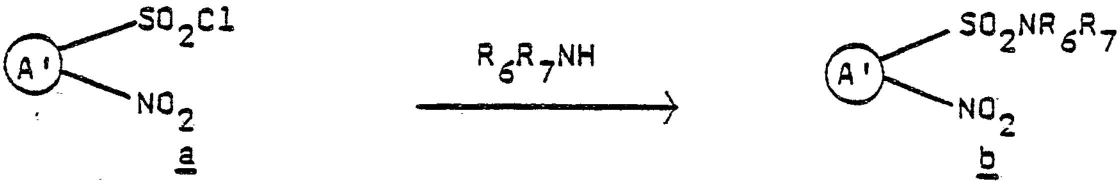


5b

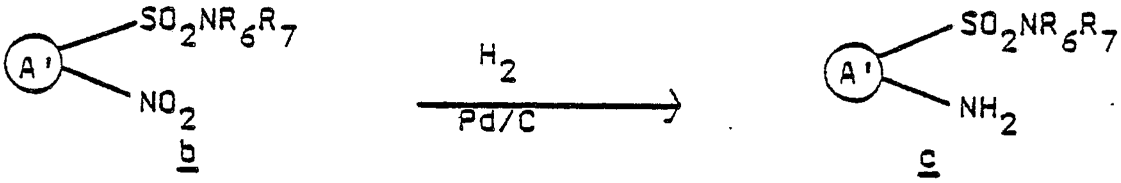


5c

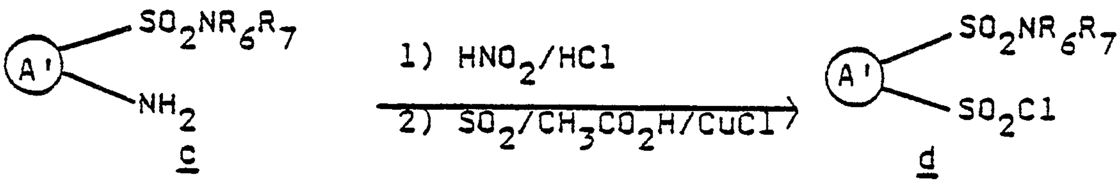


5d

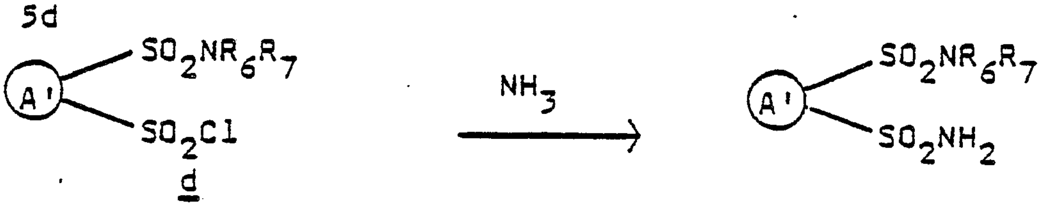


wherein 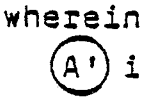 is 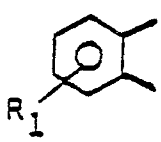 , 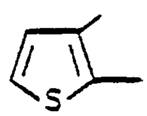 or 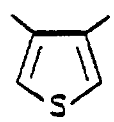 ; and

$R_1$, $R_6$ and $R_7$ are as previously defined.

In step (5a), the o-nitrobenzenesulfonyl chlorides or nitrothiophenesulfonyl chlorides in Formula a, which are well-known in the art, are treated with an amine, $R_6R_7NH$, in an inert organic solvent such as methylene chloride, ethyl ether, or tetrahydrofuran at 0-50°. The amine may be taken in excess to act as an acid acceptor; alternatively, a tertiary amine such as triethylamine or pyridine may be used as an acid acceptor. The by-product amine hydrochloride is filtered off or washed out of the solvent with water and the product isolated by evaporation of the solvent.

The reduction described in step (5b) is accomplished by treating a solution of the compounds of Formula b in a solvent such as ethanol, ethyl acetate, or DMF, in a pressure vessel with 100-1000 pounds per square inch of hydrogen at 80-150° in the presence of a hydrogenation catalyst such as 5-10% palladium adsorbed on carbon. When the theoretical amount of hydrogen has been absorbed, the solution is cooled and the catalyst is removed by filtration. The product is then isolated by evaporation of the solvent.

The diazotization and coupling with sulfur dioxide, described in step (5c), is accomplished in the following manner. A solution of the sulfamoyl compound of Formula c in a mixture of concentrated hydrochloric acid and glacial acetic acid is treated with a solution of sodium nitrite in water at -5° to 0°. After stirring for 10-15 minutes at 0° to insure complete diazotization, this solution is added to a mixture of an excess of sulfur dioxide and a catalytic amount of cuprous chloride in glacial acetic acid at 0-5°. The temperature is kept at 0-5° for 1/4 to 1 hour and is then raised to 20-25° and held at that

temperature for 2-4 hours. This solution is then poured into a large excess of ice water. The sulfonyl chloride products, d, can be isolated by filtration or by extraction into solvent such as ethyl ether or methylene chloride followed by evaporation of the solvent.

The amination described in step (5d) is conveniently carried out by treating a solution of the sulfonyl chloride of Formula d with an excess of anhydrous ammonia in a solvent such as ethyl ether or methylene chloride at 0-25°. If the product sulfonamide is insoluble it may be isolated by filtration followed by washing out the salts with water. If the product sulfonamide is soluble in the reaction solution, it may be isolated by filtering off the precipitated ammonium chloride and evaporating the solvent.

The sulfonamide precursors to intermediates of Formula VII in which R or $R_3$ is $SO_2OCH_2CF_3$, $SO_2OCH_2CCl_3$ or $SO_2N(CH_3)OCH_3$ can be prepared through a sequence analogous to that shown in Equations 5a-d. In step 5a, the amine is replaced by the appropriate alkoxide salt or N-methoxy-N-methylamine and the resulting nitro compound is treated as in Equations 5b-d.

Equation 6 describes the preparation of intermediates of Formula VII when R or $R_3$ is $S(O)_mR_5$ and where m = 0 or 2.

Equation 6

6a

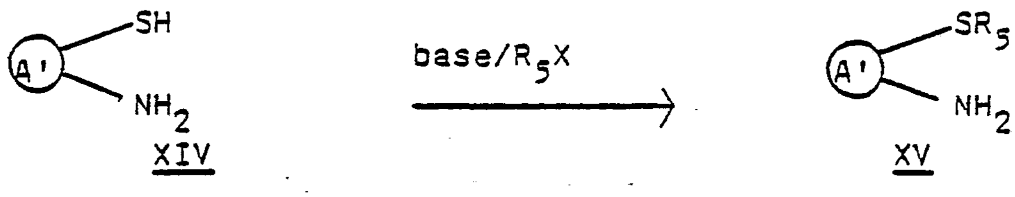


XIV XV

6b

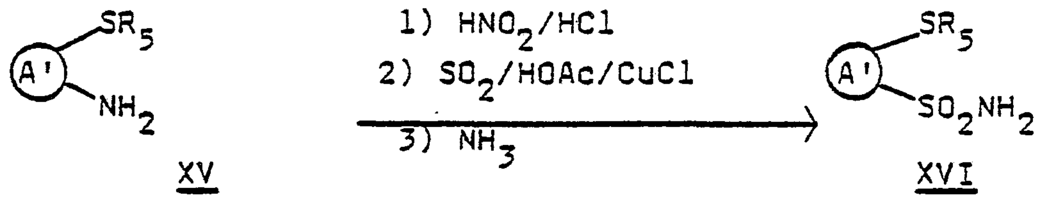


XV XVI

6c

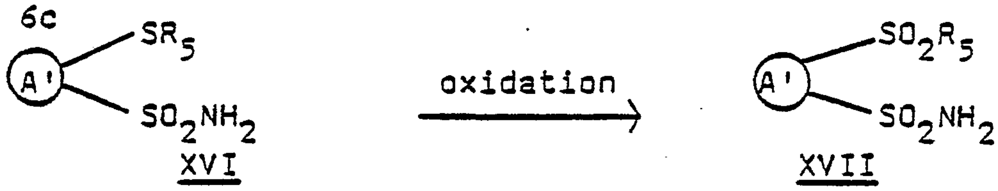


XVI XVII

wherein

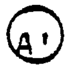 , and $R_5$ are as previously defined.

The thioether of Formula XV may be prepared from the appropriate 2-aminothiophenol or aminothiothiophene XIV and an alkyl halide as described in the literature, e.g., R. N. Prasad et al., Can. J. Chem. 44, 1247 (1966). The formation of the sulfonamide XVI is analogous to that presented in Equations 5c and 5d above.

The oxidation of XVI to the corresponding 2-alkylsulfonylbenzenesulfonamides or alkylsulfonylthiophenesulfonamides of Formula XVII may be carried out utilizing any of several of standard literature procedures with *m*-chloroperbenzoic acid (C. R. Johnson, et al., Tetrahedron, 25, 5649 (1969)), or with aqueous hydrogen peroxide in acetic acid (F. G. Bordwell, et al., J. Amer. Chem. Soc. 77, 1141, (1955).

Preparation of sulfonyl isocyanates of Formula VII wherein R is $BR_8$ (B and $R_8$ are as previously defined) is accomplished as outlined in Equation 7 below.

<u>Equation 7</u>

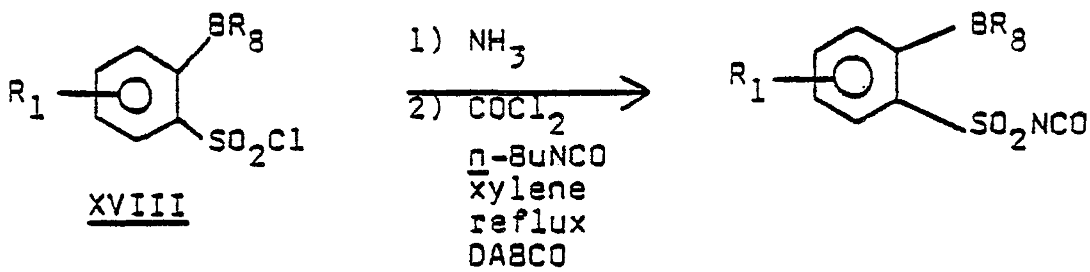


<u>XVIII</u>

wherein B is O or $S(O)_{m'}$; m' is 0 or 2, and $R_1$ and $R_8$ are as previously defined.

The (haloalkoxy, haloalkylsulfinyl, haloalkylthio and haloalkylsulfonyl)benzenesulfonyl chloride reactants XVIII are themselves prepared by the methods which are described in Equations 7a-c and 8a-e below.

(Haloalkoxy)benzene derivatives (XIX) as starting materials for preparation of I where R = $OR_8$ can be made by methods well known in the art.

(Trifluoromethoxy)benzene derivatives can be made by the method of Sheppard [J. Org. Chem. <u>29</u>, 1 (1964)], e.g.:

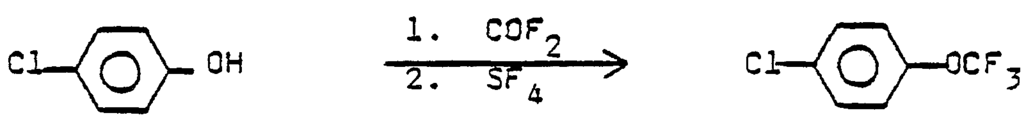


(Tetrahaloethoxy)benzene derivatives can be made by the method of England et al. [J. Am. Chem. Soc. <u>82</u>, 5116 (1960)], which also applies to the hexafluoropropoxy compounds, e.g.:

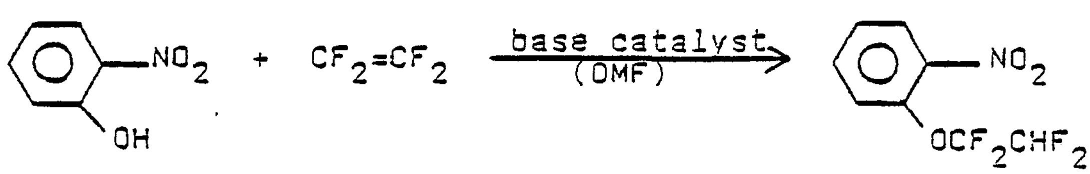

The latter compound can be purchased from Fairfield Chemical Co., Blythewood, S.C.

(Trifluoroethoxy)benzene derivatives can be made by reaction of trifluoroethanol with an activated aromatic halide, e.g.:

Equation 7a

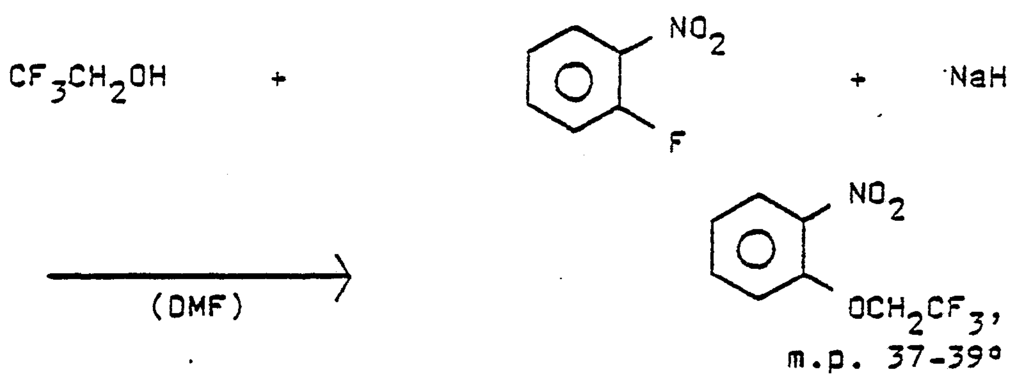


In reaction (7a) sodium hydride and trifluoroethanol are mixed in an aprotic solvent, such as dimethylformamide (DMF), dioxane or tetrahydrofuran (THF), with 2-(fluoro or chloro)-1-nitrobenzene. The reaction proceeds to completion at ambient temperature. Heat may be applied (e.g., with a steam bath) if desired to speed the reaction to completion. The product is isolated by diluting the reaction mixture with water, extracting with an organic water-immiscible solvent and evaporation of the solvent. This reaction is similar to that described in Japanese Patent 5 2057-320.

(Difluoromethoxy)benzene derivatives can be made by the method of Yagupolskii et al. [Chem. Abstr. 70, 96318d (1969)] e.g.:

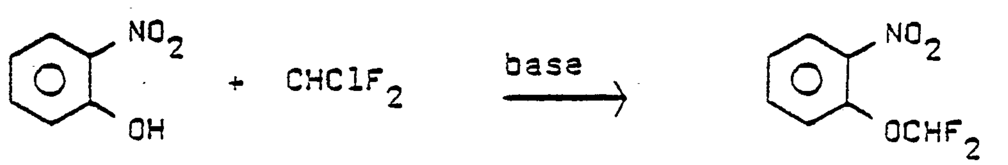

Preparation of compound XVIIIa is shown below schematically, first for the case wherein $R_8$ is $CF_3$ or $CF_3CH_2$.

Equation 7b

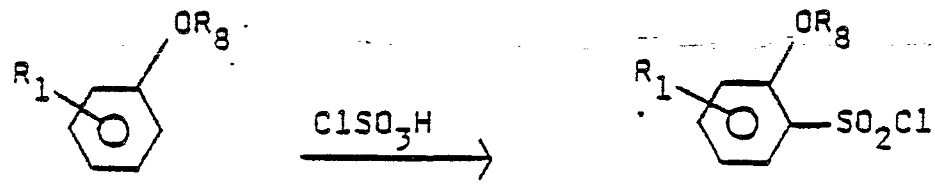


XIX XVIIIa

wherein $R_8$ is $CF_3$ or $CF_3CH_2$, and $R_1$ is as previously defined.

Chlorosulfonation of aromatic substrates is well-known (e.g., L. F. Fieser and M. Fieser, "Advanced Organic Chemistry", 696-698, Reinhold, New York, 1961). The chlorosulfonation can be accomplished by addition of the trifluoromethoxy compound to the chlorosulfonic acid or vice versa, optionally in the presence of a cosolvent, such as an alkane or chlorinated alkane (e.g., hexane, 1-chlorobutane, methylene chloride, etc.). The reaction temperature is not critical, with a range of about -5° to 50° operable and ambient temperature (e.g., 20 to 30°) preferred, for convenience. At ambient temperature some hydrolysis of the trifluoromethoxy group occurs. At lower temperatures, chlorosulfonation occurs more slowly with less of the hydrolysis, while at higher temperatures, chlorosulfonation occurs more rapidly with more accompanying hydrolysis. Reaction time at ambient temperature is about 1 to 24 hours, depending on the exact substrate being chlorosulfonated, with an overnight period (about 16 hours) satisfactory. Chlorosulfonation of the tetrahaloethoxy, hexafluoropropoxy and difluoromethoxy compounds is more difficult to control without hydrolysis of the haloalkoxy group than is chlorosulfonation of the trifluoromethoxy or trifluoroethoxy compounds.

The aromatic sulfonyl chloride is conveniently isolated from the reaction mixture by pouring the mixture into ice water, followed by extraction with a water-immiscible organic solvent in which the aromatic sulfonyl chloride is soluble. Such solvents include 1-chlorobutane, methylene chloride, 1,2-dichloroethane, ethyl acetate, toluene and diethyl ether. The solution of the sulfonyl chloride can be dried and evaporated to provide the sulfonyl chloride, which can be further purified by distillation, preferably in vacuum to suppress any thermally dependent decomposition. Alternatively, the solution of the sulfonyl chloride can be used directly in reaction with ammonia in the next step, preparation of the sulfonamide.

Chlorosulfonation of XIX can produce isomeric mixtures, e.g.:

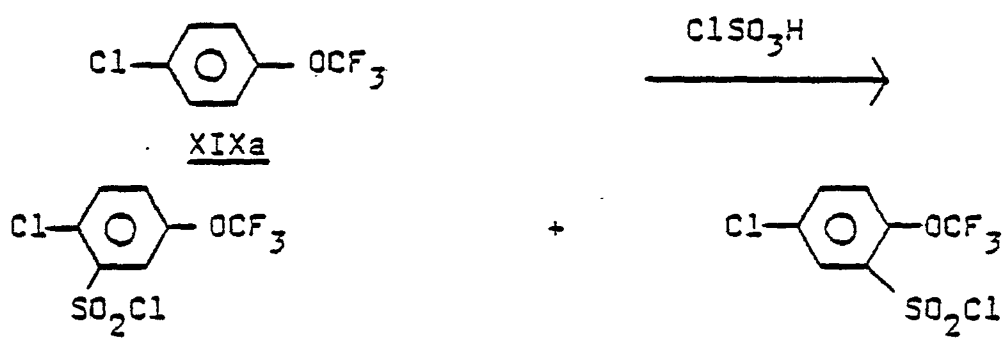


XIXa

+

Such isomeric mixtures can be separated by conventional routes (e.g., fractional distillation, chromatography) or used without separation. In the latter case, isomeric mixtures of sulfonamides, sulfonyl isocyanates and sulfonylureas are formed in the subsequent reactions. Similarly, the isomeric mixtures of intermediates formed further in the synthesis sequence can be separated or used as isomeric mixtures; isomeric mixtures of product sulfonylureas can be used as herbicides or separated and used as individual compounds.

As an alternative to reaction 7b, the sulfonyl chloride can be made from the corresponding aniline compound XX by diazotization, then treatment with sulfur dioxide and cuprous chloride. It should be emphasized that whereas reaction 7b is written as applicable to compounds where $R_8 = CF_3$ or $CF_3CH_2$ (i.e., to trifluoromethoxy or trifluoroethoxy compounds), reaction 7c is applicable to compounds where $R_8 = CF_3$, $CH_2CF_3$ <u>or</u> tetrahalo-ethyl, $CHF_2$ or hexafluoropropyl; thus reaction 7c is of more general applicability than reaction 7b.

<u>Equation 7c</u>

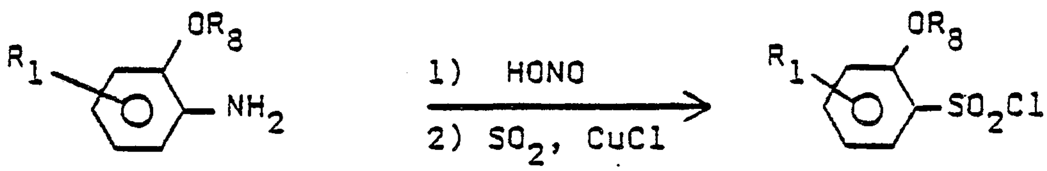


XX XVIIIb

wherein $R_1$ and $R_8$ are as previously defined.

Compound XX, where $R_8$ is $CF_3$, tetrahalo-ethyl, $HCF_2$ or hexafluoropropyl can be produced by methods described by Sheppard (loc. cit.), England (loc. cit.) and Yagupolskii (loc. cit.). The aniline compound XX is diazotized according to methods well known in the art, such as the addition of sodium nitrite to a hydrochloric acid solution of the compound XX. The intermediate diazonium compound is added, as is the well known Sandmeyer-type reaction, to a mixture of cuprous chloride, sulfur dioxide, and acetic acid at reduced temperature, e.g., 0 to 20°. The mixture is kept cold for 1/4 to 2 hours, then is allowed to warm to ambient temperature and continue to react until nitrogen evolution has substantially stopped. Dilution with water precipitates the sulfonyl chloride, generally as an oil, which is extracted into a water-immiscible organic solvent, e.g., 1-chlorobutane, diethyl ether, toluene, ethyl

acetate and the like. The organic extract can be dried and evaporated to the sulfonyl chloride or the solution can be used directly in the next step (sulfonamide preparation).

(Haloalkylthio, haloalkylsulfinyl and haloalkylsulfonyl)benzene derivatives XXIa and XXIb as starting materials for preparation of I (where R = $BR_8$ and B = $S(O)_m$), can be made by known methods [e.g., Chem. Abstr. 70, 96324c (1969); Chem. Abstr. 72, 66651f (1970); England, loc. cit.; Yagupolskii, loc. cit.].

The (trifluoroethylthio)benzenes can be made by reaction of the thiophenol compounds with a trifluoroethylating agent, such as trifluoroethyl iodide or trifluoroethyl trichloromethanesulfonate. The thiophenol compound is reacted with powdered potassium hydroxide and trifluoroethyl iodide in an aprotic solvent such as DMF, dioxane or THF. The reaction proceeds to completion at ambient temperature. Heat (e.g., with a steam bath) may be applied to increase the reaction rate. The product is isolated as described in reaction 7a.

The sulfonyl chlorides of Formula XXII may be prepared by the process described in Equation 8a.

Equation 8a

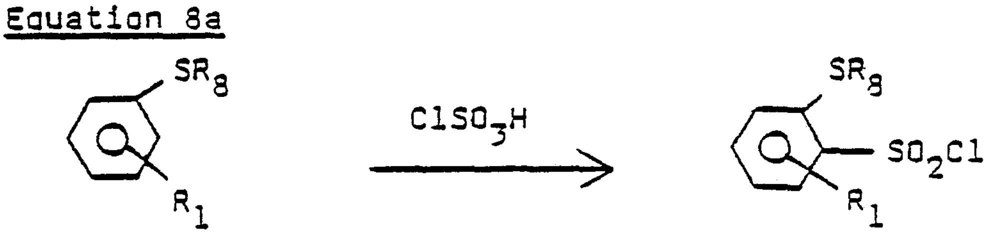


XXIa XXII

wherein $R_1$ and $R_8$ are as previously defined.

Chlorosulfonation of XXIa proceeds in the same manner as described for reaction 7b for the oxygen analog XIX.

The sulfonyl chlorides of Formula XXIIa may be prepared by the process described in Equations 8b to 8e.

Equation 8b

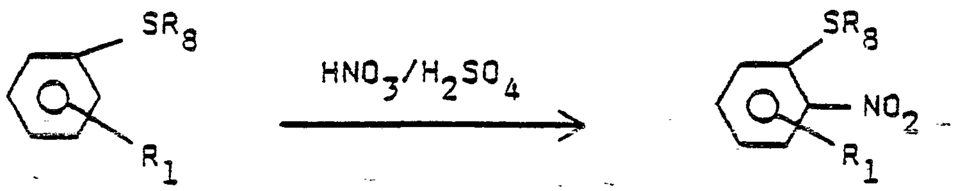


XXIb XXIII

wherein $R_1$ and $R_8$ are as previously defined.

The nitration is conveniently carried out by slow addition of slightly more than 1 equivalent of 90% nitric acid to a stirred, cooled (10-30°) mixture of the sulfide XXIb in sulfuric acid, stirring for an additional 10-45 minutes, pouring the reaction mixture into ice water, extracting the nitro compound into a water-immiscible organic solvent (e.g., 1-chlorobutane or methylene chloride), and evaporating the solution to leave residual nitro compound, which may be further purified by vacuum distillation. Thus, the reaction is a simple mononitration of a substituted benzene ring, a reaction well known in the art.

As mentioned for the oxygen analog, nitration of XXIb can lead to isomeric nitro compounds which, likewise, can be separated or used as such.

Equation 8c

XXIII

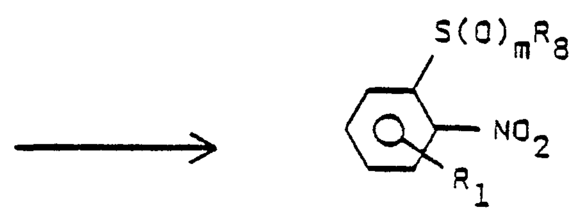

XXIV

The sulfide XXIII is oxidized to the sulfoxide (e.g., with 20-30% $H_2O_2$ in acetic acid, 1-2 hours at 90-100°); or the sulfide XXIII is oxidized to the sulfone [e.g., with chromium trioxide in acetic acid at 90-110° during 1/2-2 hours]. See Chem. Abstr. 70, 96324c (1969). If no oxidizing agent is used, m remains at zero and XXIII = XXIV.

Equation 8d

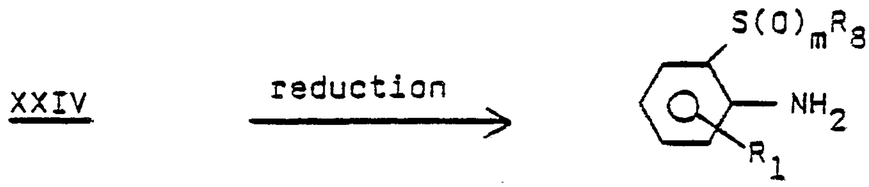


XXIV

XXV

Reduction of nitrobenzene derivatives to nitroaniline derivatives is well known in the art [e.g., W. J. Hickinbottom, "Reactions of Organic Compounds," 452-459, Longmans, London, 1959]. For example, the reduction can be accomplished by the portionwise addition of powdered iron to a mixture of the nitro compound in aqueous acetic acid at 60-110°; followed by dilution of the reaction mixture with water and filtering off, extracting or, when m = 0, steam-distilling the aniline product. Aminothiophenols can be directly tetrahaloethylated or hexafluoropropylated on the chalcogen with tetrahaloethylene or hexafluoropropene to provide directly compounds XXV with m = 0 (England et al., loc. cit.; Chem. Abstr. 73, 36584q). Also, compound XXVa is commercially available (Aldrich Chemical Co., Milwaukee, Wisc.):

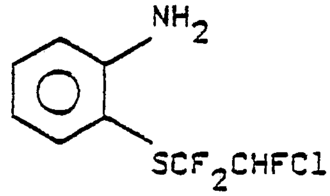

XXVa

Equation 8e

XXV

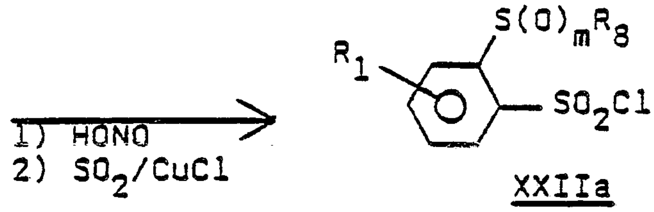


XXIIa

The aniline derivative XXV is diazotized and converted to the sulfonyl chloride as described for reaction 7c.

The compounds of this application in which R is an ortho sulfonate derivative ($OSO_2R_9$) may also be prepared via the corresponding sulfonyl isocyanate of Formula II. Sulfonamides, prepared as described in Research Disclosure, pg. 52, (1978), may conveniently be converted to the corresponding isocyanates of Formula (VII) by methods described above.

The preparation of intermediates VII in which R is $CH_2P$ (where P is Cl, Br, alkoxy, alkenyloxy, alkylthio or alkylsulfonyl) may be accomplished from the corresponding sulfonamides by the methods described above. Many of these sulfonamides are available from the corresponding nitrobenzenes by reduction, then diazotization in the presence of sulfur dioxide and cuprous chloride as shown above.

The o-alkoxymethyl- or o-thioalkoxymethylnitrobenzenes are in turn prepared via "Williamson Synthesis", according to Equations 9a or 9b.

Equation 9a

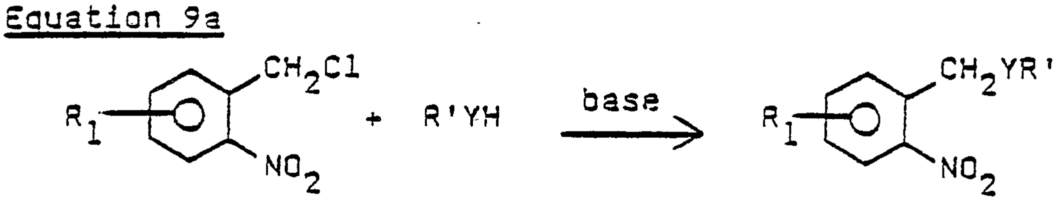

Equation 9b

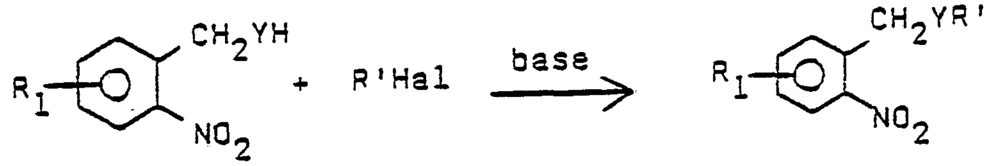


Y = O or S;

R' = $C_1$-$C_4$ alkyl or $C_3$-$C_4$ alkenyl.

"Williamson Synthesis" has been widely used for the preparation of ethers as reviewed by W. Theilheimer, Syn. Methods of Org. Chem., Vol. VII, p. 112.

Alternatively, o-alkoxymethyl or o-thioalkoxymethylbenzenesulfonyl chlorides, XXIX, can be obtained from an appropriately substituted α-hydroxy-o-toluenesulfonic acid-α-sultone, XXVII, via ring-opening reaction with an alkoxide or thioalkoxide anion as depicted in Equations 9c and 9d.

Equation 9c

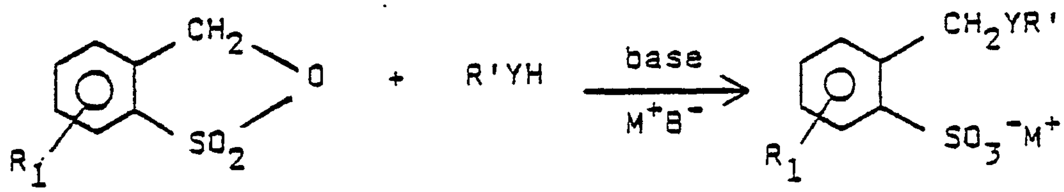


XXVII XXVIII

Equation 9d

XXVIII + $PCl_5$

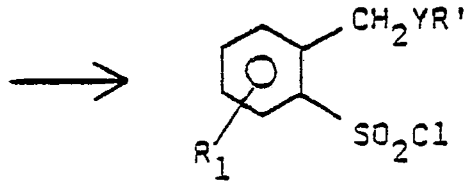

XXIX

Reaction 9c is closely related to the alkylation of acyloxides and acetamide with sultones as disclosed by J. H. Helberger et al., Ann., 565 22 (1949). Conversion of the sulfonic acid salt to the sulfonyl chloride is then carried out according to the teaching of Org. Synthesis, Coll. Vol. IV, 846, 693.

Benzenesulfonamides of Formula XXVI can also be derived from compound XXX as illustrated in Equation 9e.

Equation 9e

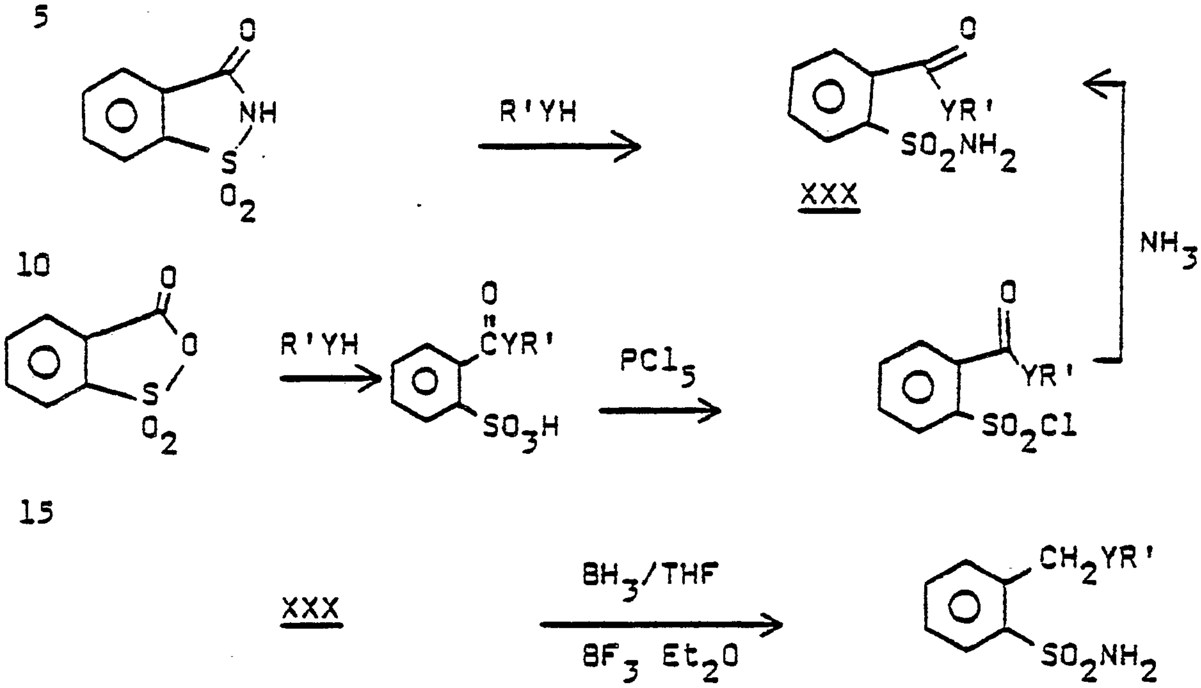


XXX

XXX

XXXI

Preparation of o-sulfamylbenzoic acid esters, XXX, from saccharin or sulfobenzoic acid anhydride is well known in the art, e.g., B. Loev and M. Kormendy, J. Org. Chem. 27, 1703 (1962). The esters, XXX, can be readily reduced to the ethers XXXI with diborane in a suitable organic solvent, e.g., tetrahydrofuran, in the presence of fifteen fold of boron trifluoride etherate under reflux for 18 hours, as described by R. P. Graber and M. B. Meyers, J. Org. Chem. 26, 4773 (1961).

B. Aminoheterocyclic-N-Oxide Intermediates

The compounds of Formulas III, IV, V, and VI may be prepared as shown below in Equation 10 by the reaction of an appropriately substituted aminoheterocycle XXXII (a-d) with an oxidizing agent such as m-chloroperoxybenzoic acid.

Equation 10

a.

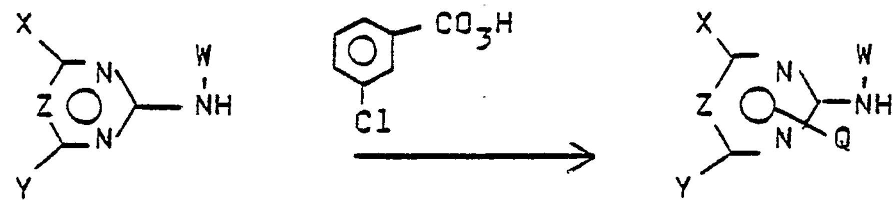

XXXIIa III

b.

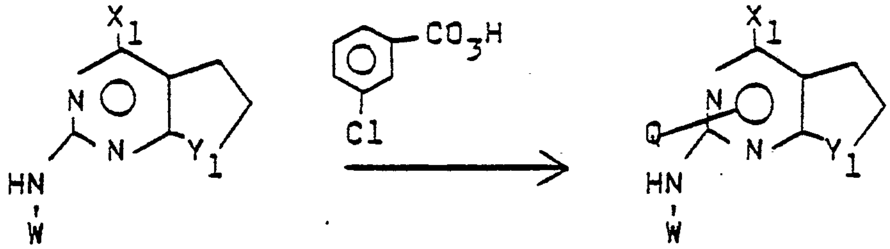

XXXIIb IV

c.

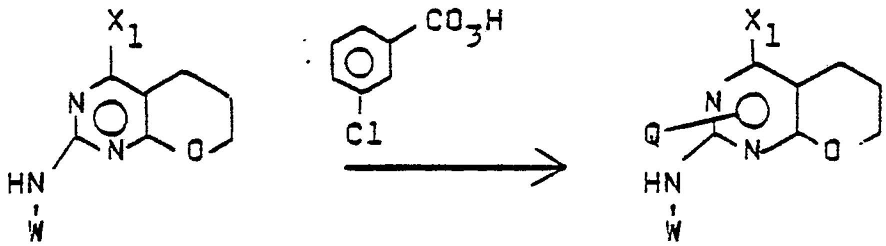

XXXIIc V

d.

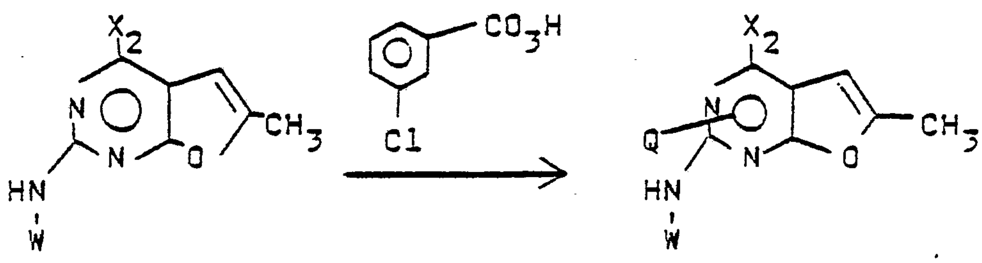

XXXIId VI

wherein

W, X, Y, $X_1$, $Y_1$, $X_2$, Z and Q are as previously defined.

As shown in Equations 10-a through 10-d; an aminoheterocycle is reacted with an oxidizing agent such as *m*-chloroperoxybenzoic acid in aprotic solvent such as acetone, methyl ethyl ketone, methylene

chloride or chloroform. Temperatures of 20-80°C over 2-15 hours are usually required to complete the reaction. The products III, IV, V, and VI are isolated by cooling and filtration. Alternatively, if the aminoheterocyclic-N-oxides III, IV, V, and VI are soluble in the solvents chosen, they may be isolated by stirring the reaction mixture with potassium carbonate or sodium carbonate, filtration and concentration of the filtrate. The crude aminoheterocyclic-N-oxides III, IV, V, and VI may be purified by washing with solvents such as acetone, ether or ethyl acetate.

Some of the compounds of Formulas III, IV, V, and VI may also be prepared from aminoheterocycles XXXIII(a-d) by the sequence of reactions described in Equation 11.

Equation 11

a.

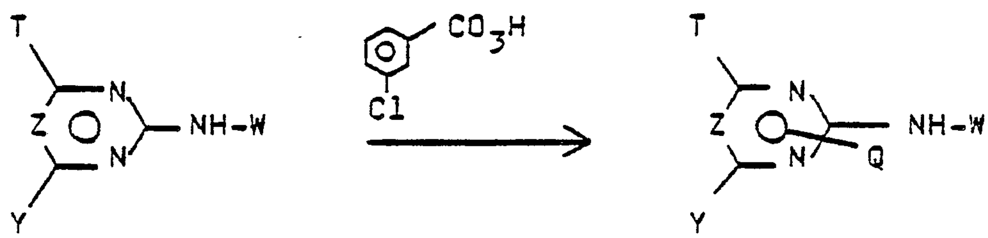

XXXIIIa XXIVa

b.

XXIVa

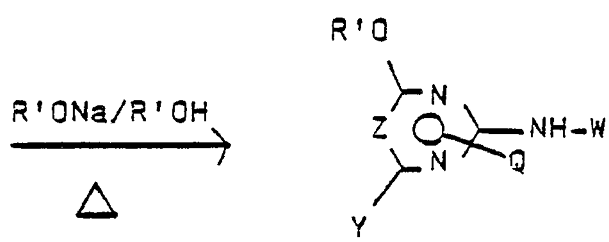


III

c.

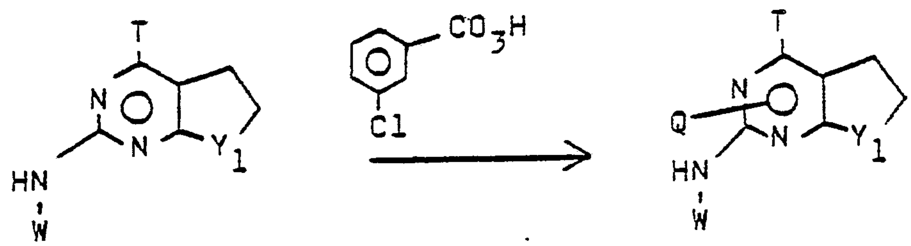

XXXIIIb XXXIVb

d.

XXXIVb

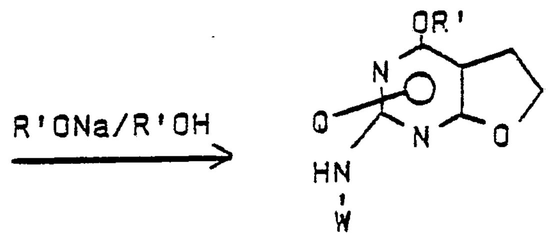


IV

e.

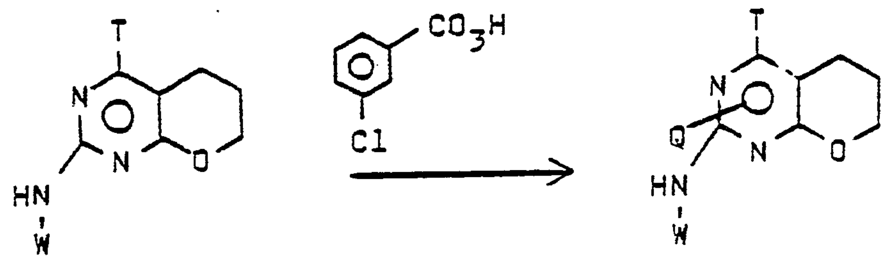

XXXIIIc XXXIVc

f.

XXXIVc

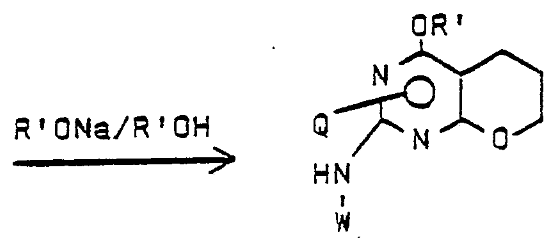


V

g.

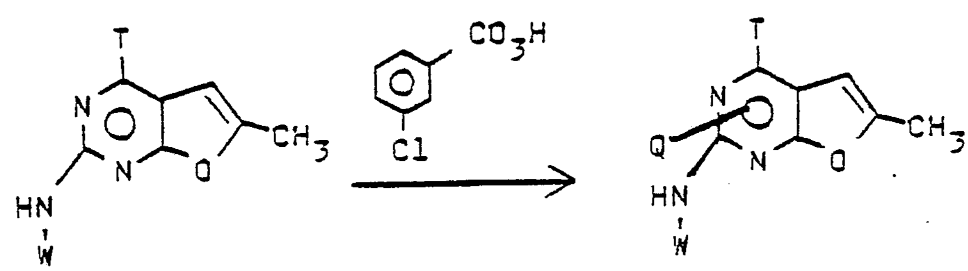

XXXIIId XXXIVd

h.

XXXIVd

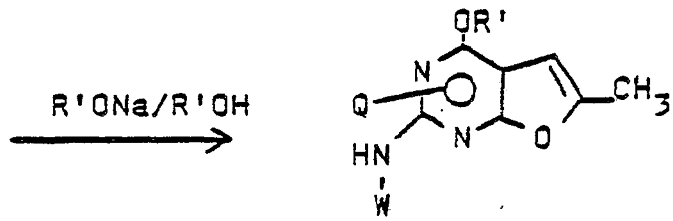


VI

wherein

W, Y, $Y_1$, Z and Q are as previously defined;

R' is $CH_3$ or $CH_3CH_2$; and

T is Cl or Br.

As shown in Equations 11a, 11c, 11e and 11g, an appropriately substituted aminoheterocycle XXXIII(a-d) is reacted with m-chloroperoxybenzoic acid in aprotic solvent such as acetone, methyl ethyl ketone, methylene chloride or chloroform. Temperatures of 20-80°C over 2-15 hours are usually required to complete the reaction. The products XXXIV(a-d) are isolated by cooling and filtration. Alternatively, if the aminoheterocyclic-N-oxides, XXXIV(a-d) are soluble in the solvent chosen, they may be isolated by stirring the reaction mixture with potassium carbonate or sodium carbonate, filtration and concentration of the filtrate. The crude aminoheterocyclic-N-oxides, XXXIV(a-d) may be purified by washing with solvents such as acetone, ether or ethyl acetate.

Displacement of the halogen T may be accomplished as shown in Equations 11b, 11d, 11f and 11h by treatment of the aminoheterocyclic-N-oxides XXXIV(a-d) with an alkali metal alkoxide such as sodium methoxide in the corresponding alcohol solvent such as methanol. Temperatures of 40-78°C over 2 to 20 hours are usually required to complete the reaction. The products III, IV, V, and VI may be isolated by filtration, acidification of the filtrate and evaporation of the solvent. Purification of these products may be

accomplished by refluxing the crude residue in solvents such as ethyl acetate or acetone followed by cooling and collecting the resulting solid products by filtration.

In either of the preparations described in equations 10 and 11 above, a mixture of isomeric N-oxides may result. In the case of pyrimidin-2-amines, two such isomers are possible; while with the aminotriazines, as many as three isomeric N-oxides may be formed.

A procedure that leads to the 5-N-oxidetriazin-2-amines has been reported by K. R. Huffman and F. C. Schaefer in U.S. 3,154,548. However, their structural assignments were based on infrared absorptions which did not provide conclusive evidence for distinguishing between the 1-(or 3-) N-oxide and the 5-N-oxide. Nuclear magnetic resonance (60 MHz) spectra of the 4,6-dimethyltriazin-2-amine-N-oxide obtained by the procedure of Huffman and Schaefer, loc. cit., in our laboratories exhibit equivalent methyl absorptions indicating the 5-N-oxide XXXVa to be the major product of this reaction, as shown in Equation 12a. Thus, the preferred method of synthesis of the intermediate 5-oxidetriazin-2-amines is through the use of the procedure of Huffman and Schaefer. Synthesis of the 1-(or 3-)oxidetriazin-2-amines may be most conveniently accomplished by the use of the oxidation procedures described above in Equations 10 and 11. As shown in Equation 12b, oxidation of 4,6-dimethyltriazin-2-amine yields 4,6-dimethyl-1-oxidetriazin-2-amine XXXVb as the major product.

Equation 12

a.

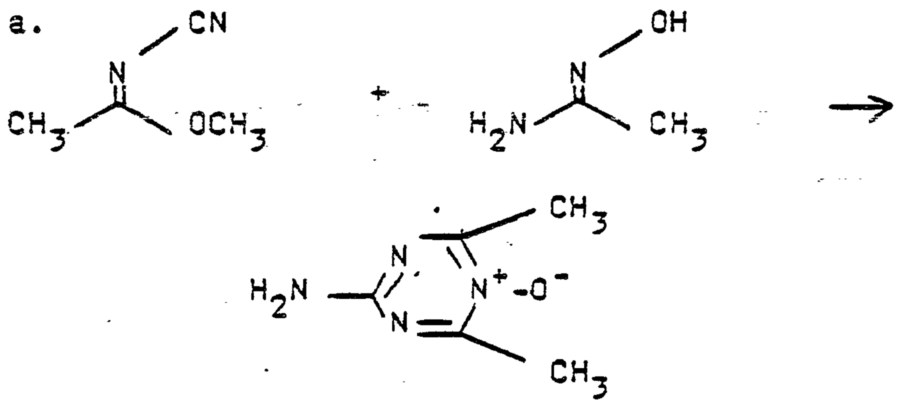

XXXVa

b.

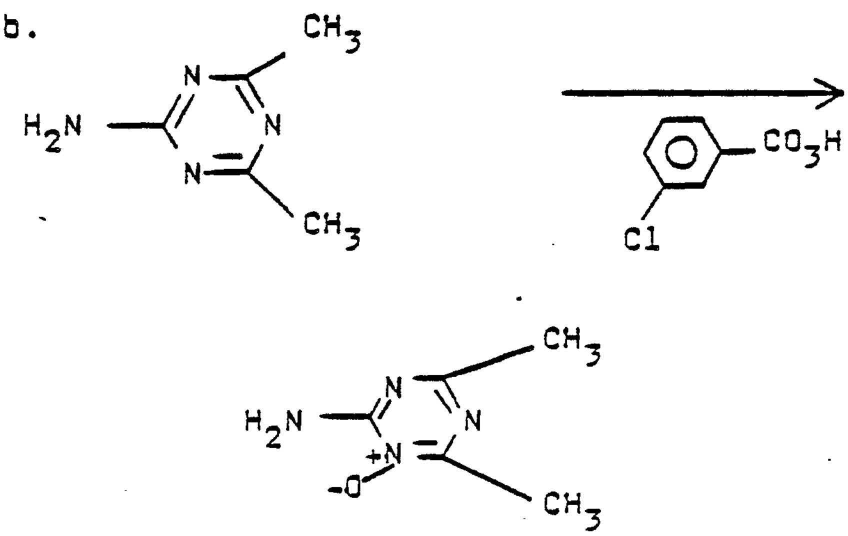

XXXVb

The position of attachment of the N-oxide was established by the appearance of two separate methyl singlets in the nuclear magnetic resonance spectrum of the product.

The oxidation procedures described above may result in isomeric N-oxide mixtures when X and Y are different for both the triazines and the pyrimidines. If X and Y have sufficiently different electronegativities, a predominance of one N-oxide isomer usually results. Structural assignment of the N-oxide in these cases is based on the chemical reactivity of the ring nitrogen atom. For example, the oxidation of 4-chloro-6-methylpyrimidin-2-amine can be expected to take place at the 1-position rather than the 3-position since the nitrogen atom at position 3 is inactivated by the close proximity of the electronegative chlorine atoms, as shown in Equation 13a. Similarly, oxidation

<u>Equation 13</u>

a.

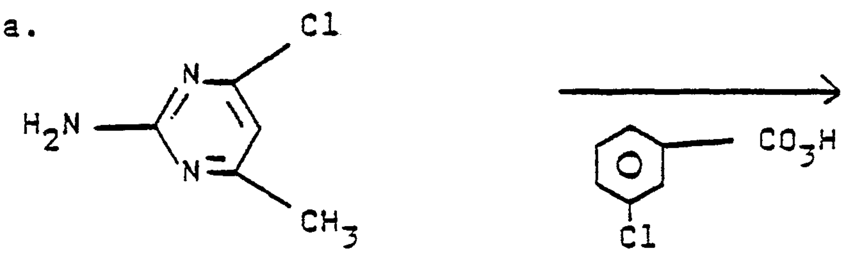

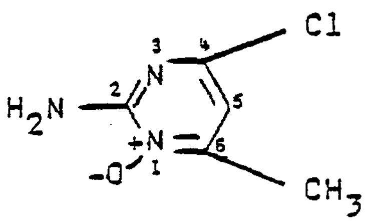

<u>XXXVI</u>

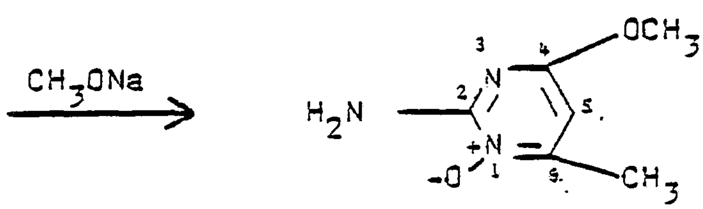


<u>XXXVII</u>

b.

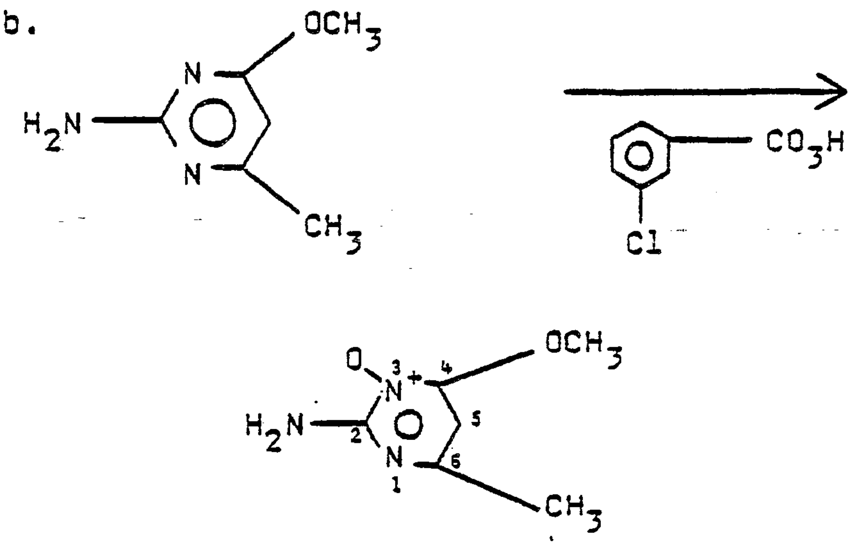

XXXVIII

of 4-methoxy-6-methylpyrimidin-2-amine can be expected to yield the 3-N-oxide XXXVIII due to the increased reactivity of the nitrogen atom in position 3 caused by the adjacent alkoxy at position 4. By further treatment of XXXVI in Equation 13a with sodium methoxide as discussed above in Equation 11, the 1-N-oxide XXXVII may be obtained. Thus, the synthetic sequences represented by Equations 13a and b are complimentary and the desired N-oxide isomer may be prepared by selection of the appropriate sequence of reactions. The same reasoning may be applied to the fused ring pyrimidines IV, V, and VI for preparation of the required positional isomers.

The synthesis of heterocyclic amines has been reviewed in "The Chemistry of Heterocyclic Compounds", published by Interscience Publ., New York and London. 2-Aminopyrimidines are described by D. J. Brown in Vol. XVI of this series. The 2-amino-1,3,5-triazines are reviewed by K. R. Huffman and in "The Triazines" of this same series. The synthesis of triazines is also described by F. C. Schaefer, U.S. Patent No. 3,154,547 and by K. R. Huffman and F. C. Schaeffer, J. Org. Chem. 28, 1816 (1963).

Braker, Sheehan, Spitzmiller and Lott, <u>J. Am. Chem. Soc. 69</u>, 3072 (1947) describe the preparation of 6,7-dihydro-4-methoxy-5H-cyclopentapyrimidin-2-amine by the following sequence of reactions.

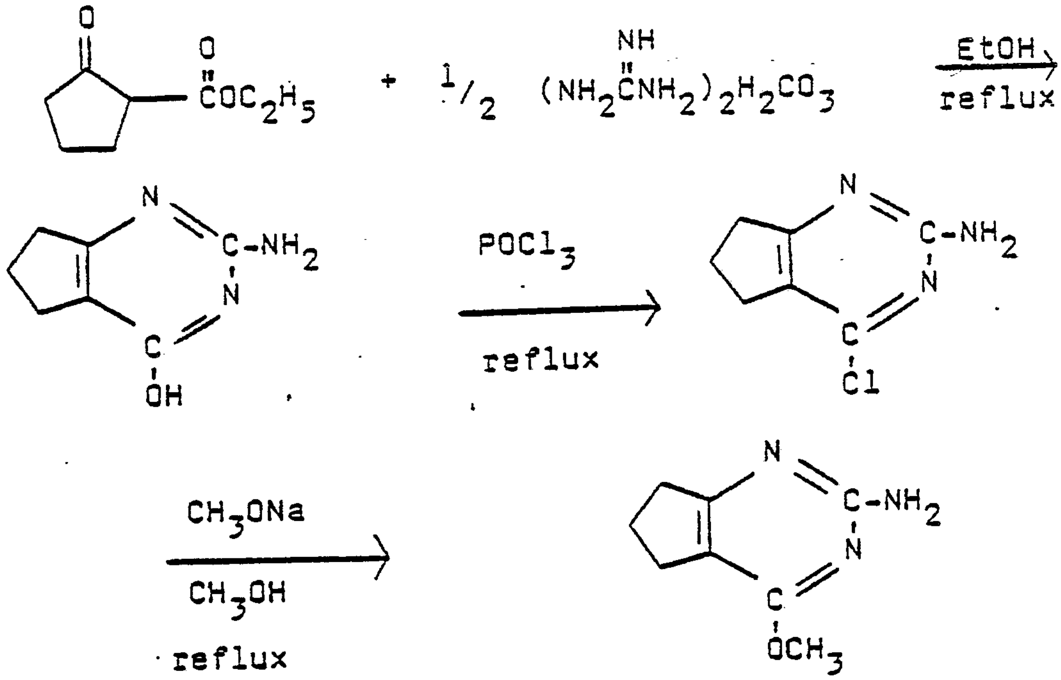


6,7-dihydro-4-methoxy-5H-cyclopentapyrimidin-2-amine.

Similarly, 6,7-dihydro-4-methyl-5H-cyclopentapyrimidin-2-amine can be prepared by the condensation of 2-acetylcyclopentanone with guanidine carbonate, but preferably by heating in dimethylsulfoxide at 135° for several hours.

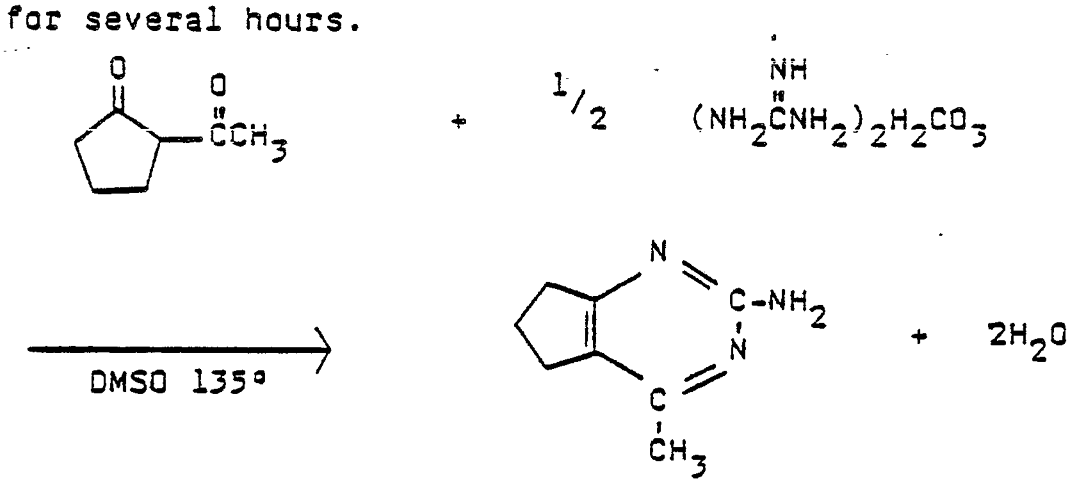


6,7-dihydro-4-methyl-5H-cyclopentapyrimidin-2-amine.

Shrage and Hitchings, J. Org. Chem. 16, 1153 (1951) describe the preparation of 5,6-dihydro-4-methylfuro[2,3-d]pyrimidin-2-amine by the following sequence of reactions:

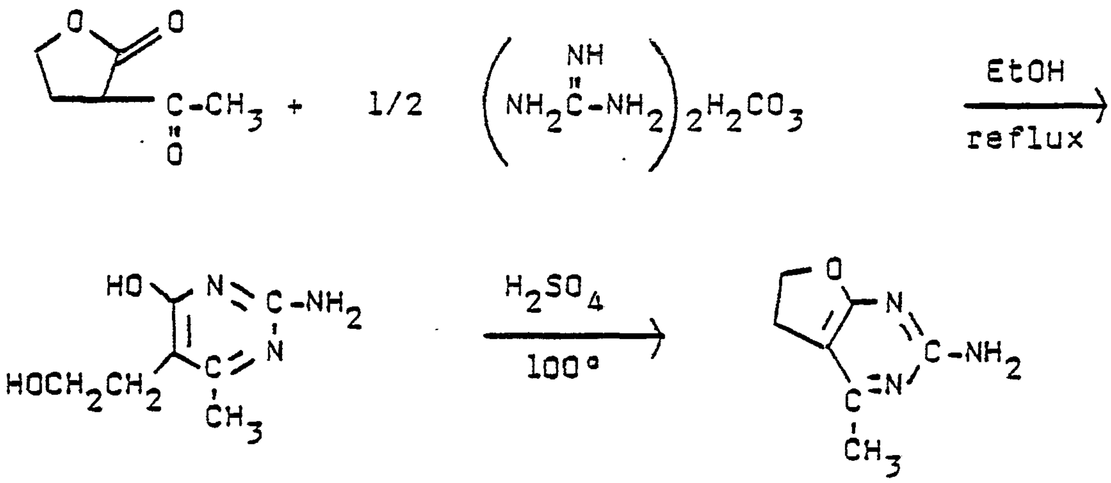


An analogous sequence of reactions can be used to prepare 6,7-dihydro-4-methyl-5H-pyrano[2,3-d]pyrimidin-2-amine starting with 2-acetyl-δ-valerolactone [Korte and Wusten, Tetrahedron 19, 1423 (1963)].

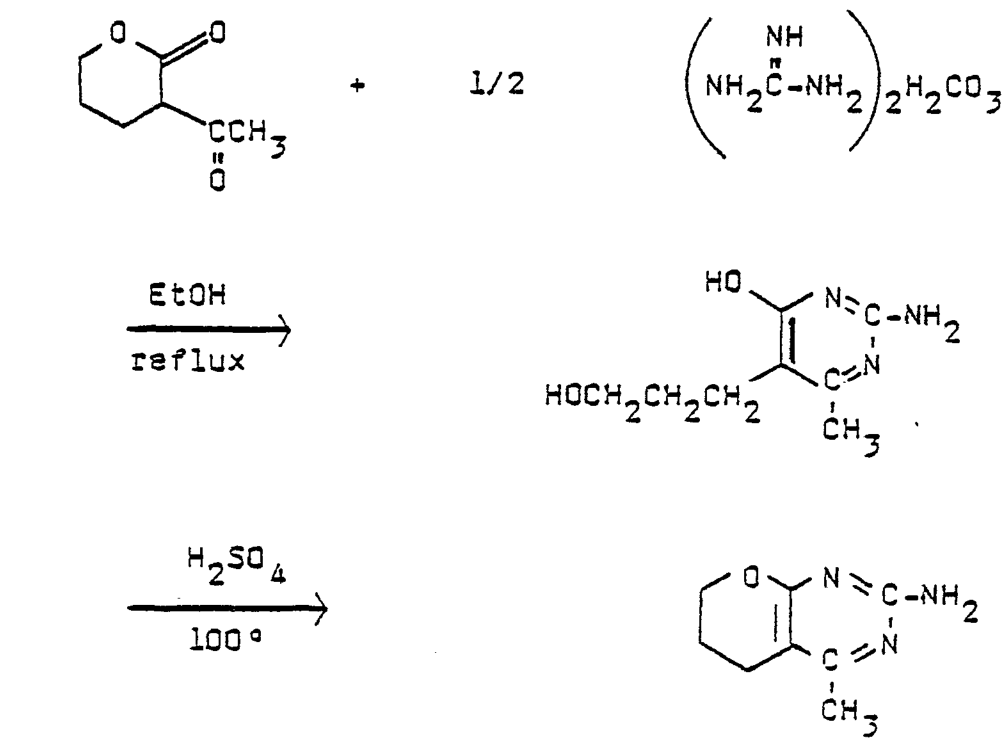

5,6-Dihydro-4-hydroxyfuro[2,3-d]pyrimidine-2-amine [Svab, Budesinski and Vavrina, Collection Czech. Chem. Commun. 32, 1582 (1967)] can be converted to 2-amino-5-(2-chloroethyl)-4,6-dichloropyrimidine by heating with phosphorus oxychloride. The product can be subsequently cyclized by treatment with two equivalents of aqueous sodium hydroxide to afford 4-chloro-5,6-dihydro[2,3-d]pyrimidin-2-amine, which is then converted to 5,6-dihydro-4-methoxyfuro[2,3-d]pyrimidin-2-amine by heating with excess sodium methoxide in methanol.

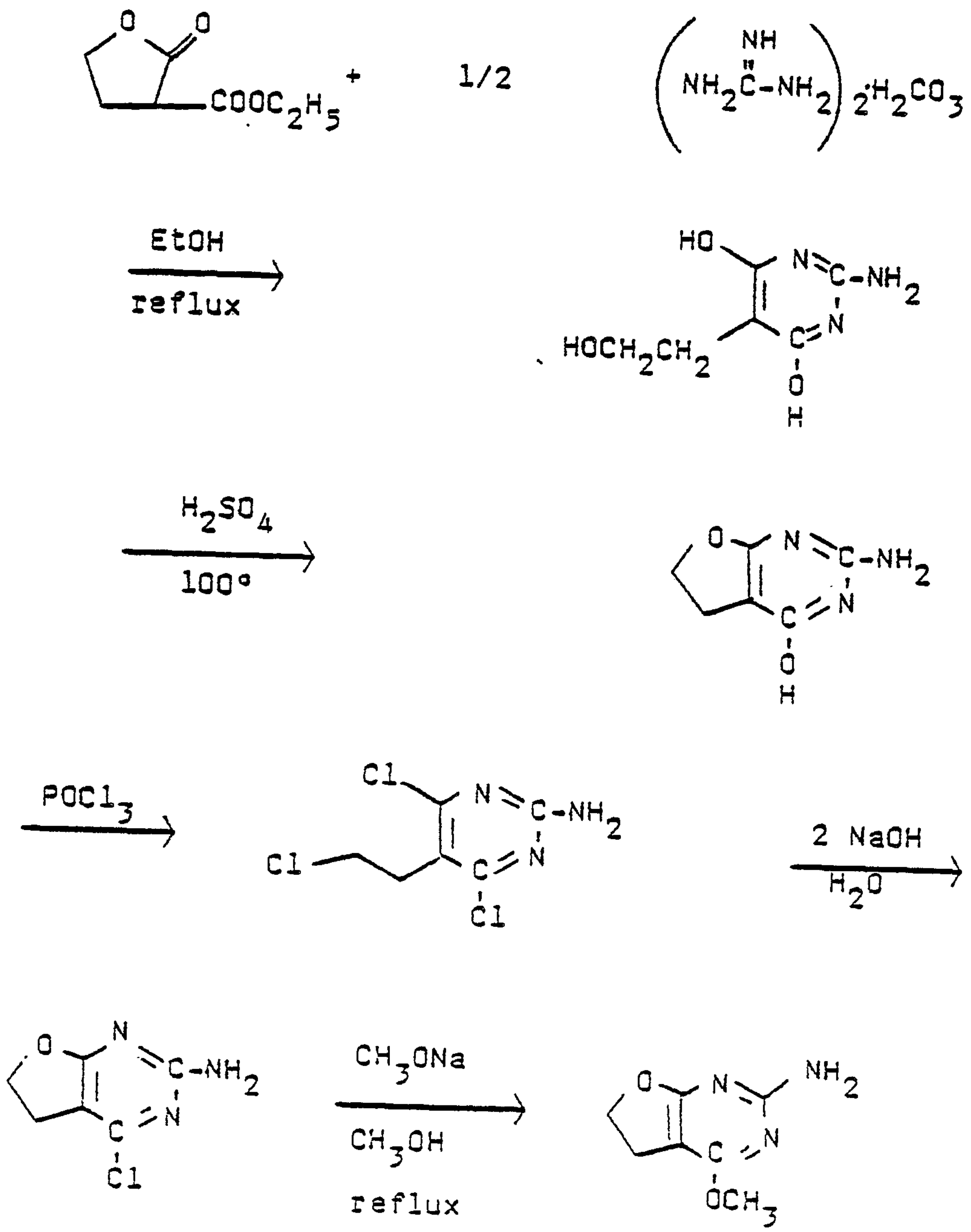

6,7-Dihydro-4-hydroxy-5H-pyrano[2,3-d]pyrimidin-2-amine can be prepared from diethyl 3-chloropropylmalonate, guanidine carbonate and sodium ethoxide in ethanol. Treatment of the product

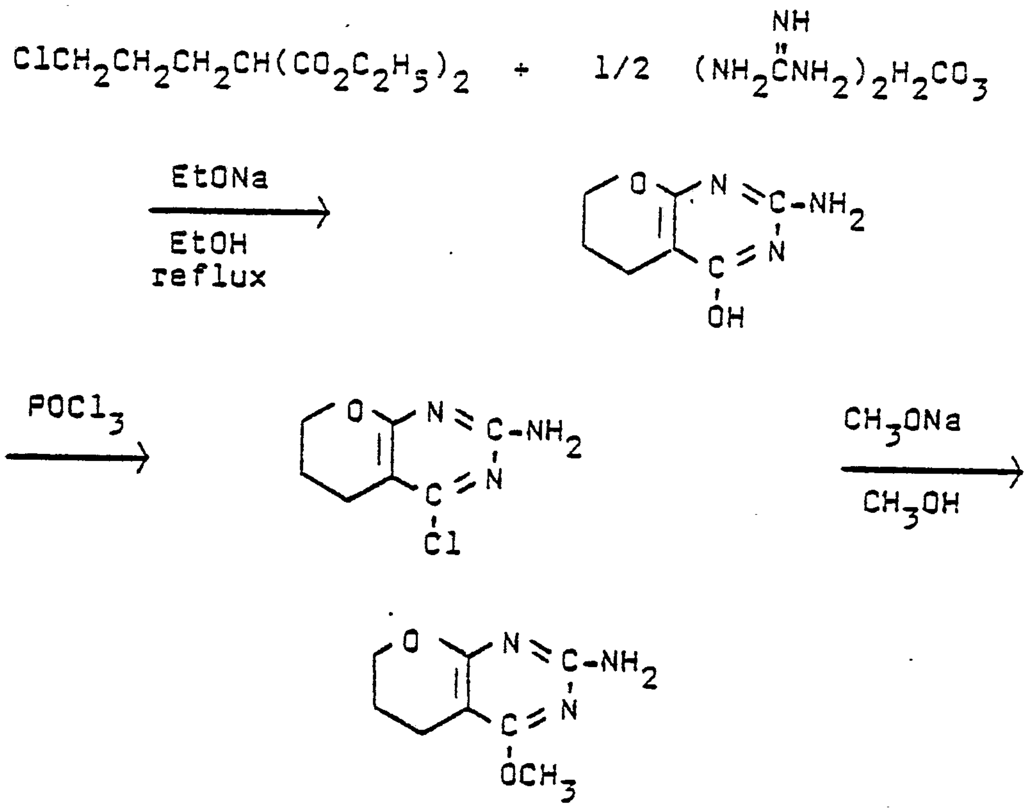


with phosphorus oxychloride gives 4-chloro-6,7-dihydro-5H-pyrano[2,3-d]pyrimidin-2-amine and subsequent reaction with sodium methoxide in refluxing methanol affords 6,7-dihydro-4-methoxy-5H-[2,3-d]pyrimidin-2-amine.

Compounds of Formulas XXVIIb and XXVIIc where $X_1$ is ethoxy can be prepared by a procedure analogous to the methoxy derivatives.

Caldwell, Kornfeld and Donnel, J. Am. Chem. Soc. 63, 2188 (1941), describe the preparation of 6,7-dihydro-5H-cyclopentapyrimidin-2-amine by the following sequence of reactions.

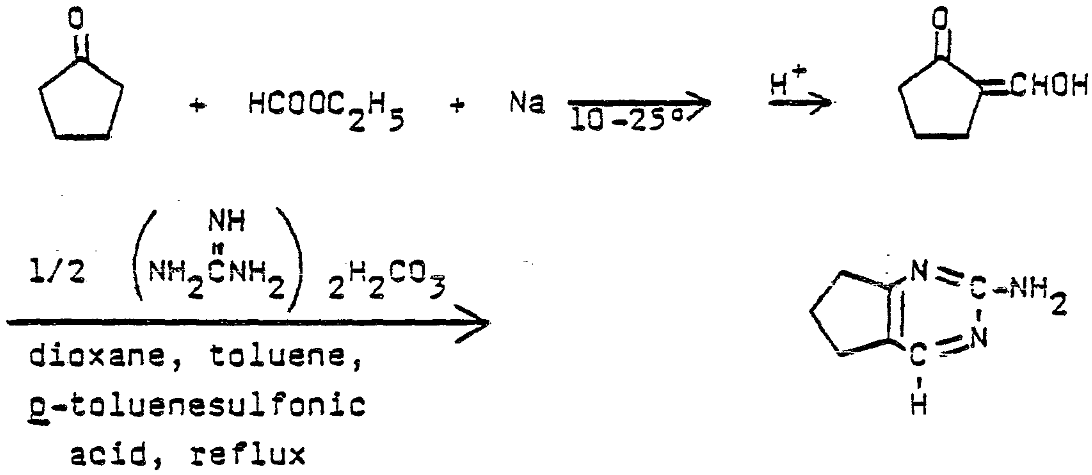


Fissekis, Myles and Brown, J. Org. Chem. 29, 2670 (1964), describe the preparation of 2-amino-4-hydroxy-5-(2-hydroxyethyl)pyrimidine which can be converted to 5,6-dihydrofuro[2,3-d]pyrimidin-2-amine by dehydration.

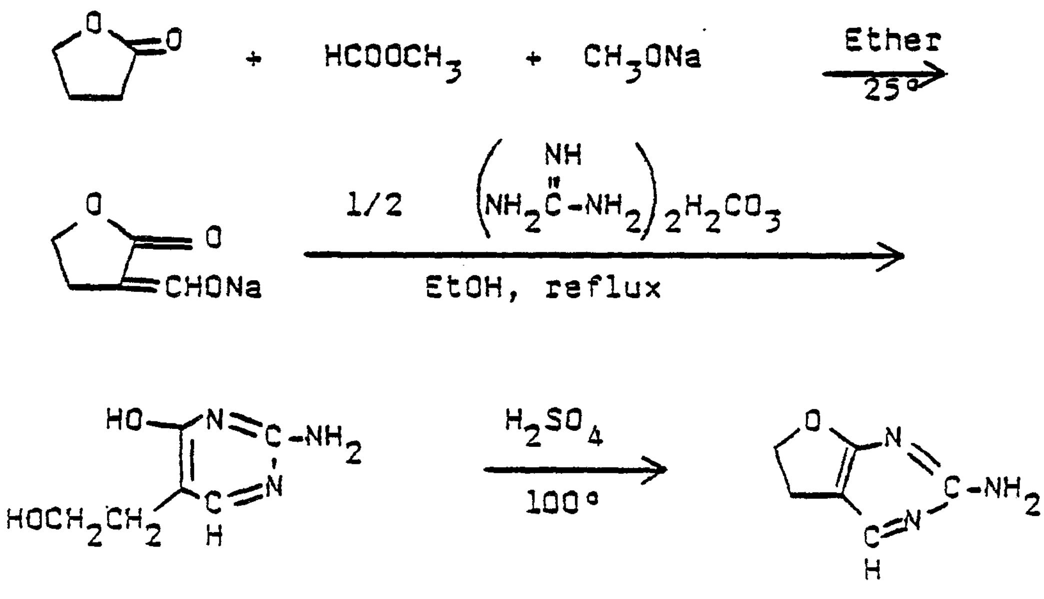


The furo[2,3-d]pyrimidine intermediates XXVIId in which W is hydrogen or methyl and $X_2$ is methyl have been reported in the literature by E. Bisagni et al., [Bul. Soc. Chim. Fr., 803 (1969)]. An apparently more efficient procedure is depicted in Equation 14 for the case in which W is hydrogen.

Equation 14

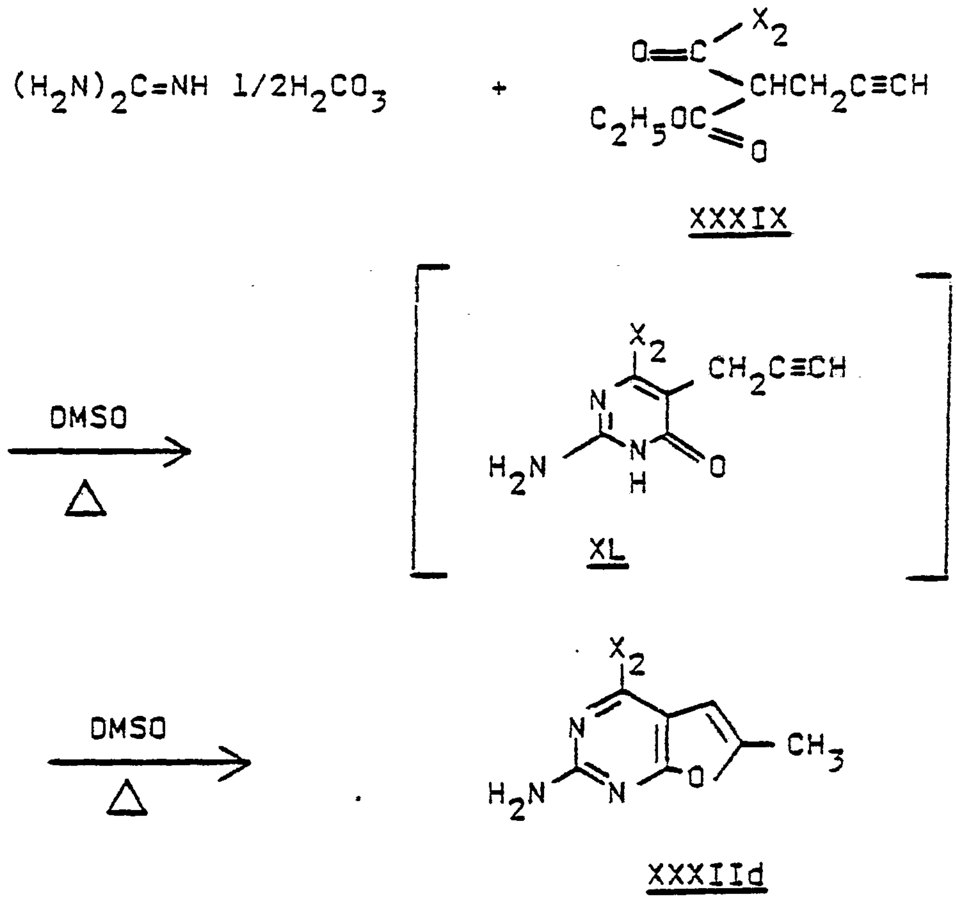


XXXIX

XL

XXXIId
(W = H)

$X_2 = CH_3$ or $C_2H_5$

The keto-ester precursors XXXIX are prepared by well known literature methods, e.g., J. F. Tinker and T. E. Whatmough, J. Amer. Chem. Soc. 74, 5235 (1952).

Treatment of XXXIX with an excess of guanidine carbonate in an organic solvent, preferably a polar aprotic solvent such as dimethylsulfoxide (DMSO), dimethylformamide (DMF), or N,N-dimethylacetamide, at a temperature of 80° to 200°, preferably 100° to 160°, ambient pressure and preferably under an inert atmosphere, yields both XXXIId and XL as products. The products are isolated upon dilution of the reaction mixture with, for example, acetone and water successively. Higher reaction temperatures and longer reaction times (e.g., in DMSO at 130°-150° for 2 to 8 hours) favor the production of the furopyrimidine XXXIId over the uncyclized pyrimidine XL.

C. Special Preparations

Several of the compounds of this invention of Formula I may also be used as starting materials for preparing different compounds of Formula I which are also agriculturally useful.

The preparation of the free acids of Formula II is accomplished by dissolving the ester XLI in an aqueous methanol or ethanol solution containing KOH. The mixture is stirred at 0-25° for 6-24 hours. The reaction yields the soluble alkali metal salt of the carboxylic acid. The salt is converted to the acid form by addition of strong mineral acids causing the carboxylic acid to precipitate. The reaction is shown in Equation 15.

Equation 15

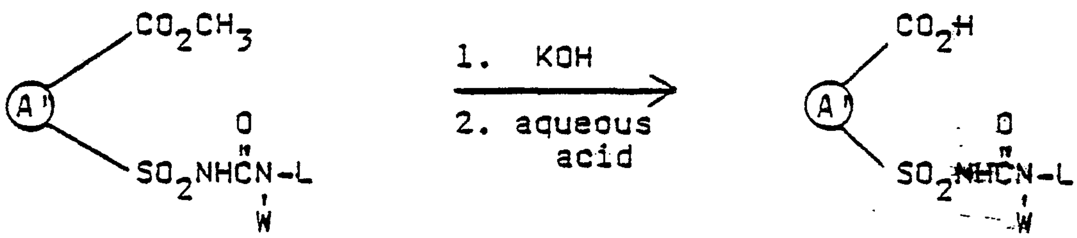


XLI II

wherein

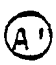 is 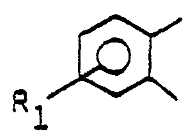, 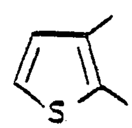 or 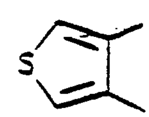; and

W and L are as previously defined.

Certain compounds of Formula I, for example XLII, can be prepared by reaction of salts of the carboxylic acid derivatives II with $R_4$-halogen as shown in Equation 16.

Equation 16

XXXIV + $R'_4$-halogen 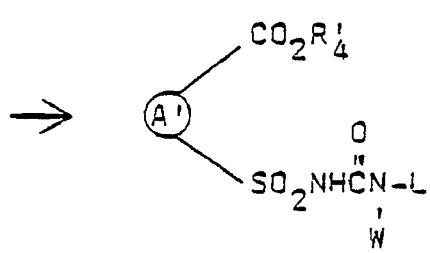

XLII

The reaction of Equation 16 is of use where the intermediate compound $R_4^{\oplus}$-halogen contains a readily replaceable halogen as is the case for substituted or unsubstituted allylic, propargyllic halides, or halomethylethers.

The procedure of Equation 16 is best carried out in inert polar solvents such as THF, acetonitrile or acetone by combining the appropriately substituted carboxylic acid and base such as triethylamine, 1,4-diaza[2.2.2]bicyclooctane or diisopropylethylamine, adding the appropriate halide and heating the mixture to reflux with stirring for 1 to 16 hours. The reaction mixture can be evaporated to dryness and the residue triturated with water, filtered and washed with water to separate the desired product from the water insoluble salt.

The procedure of Equation 16 can also be used for the synthesis of compounds where $R_4^{\oplus}$-halogen of Equation 16 is of a less reactive species than described above. In these cases, the silver salt of the carboxylic acid is used rather than the amine salt. The silver salt which is precipitated by adding silver nitrate to an aqueous solution of the sodium salt of the acid of Formula II is combined with the appropriate $R_4^{\oplus}$-halide using the same solvents and conditions as shown above for the amine salt.

The preparation of compounds of Formula I where $R = CH_2S(O)_mR_5$ and m = 1, 2 can be accomplished as shown in Equation 17.

Equation 17

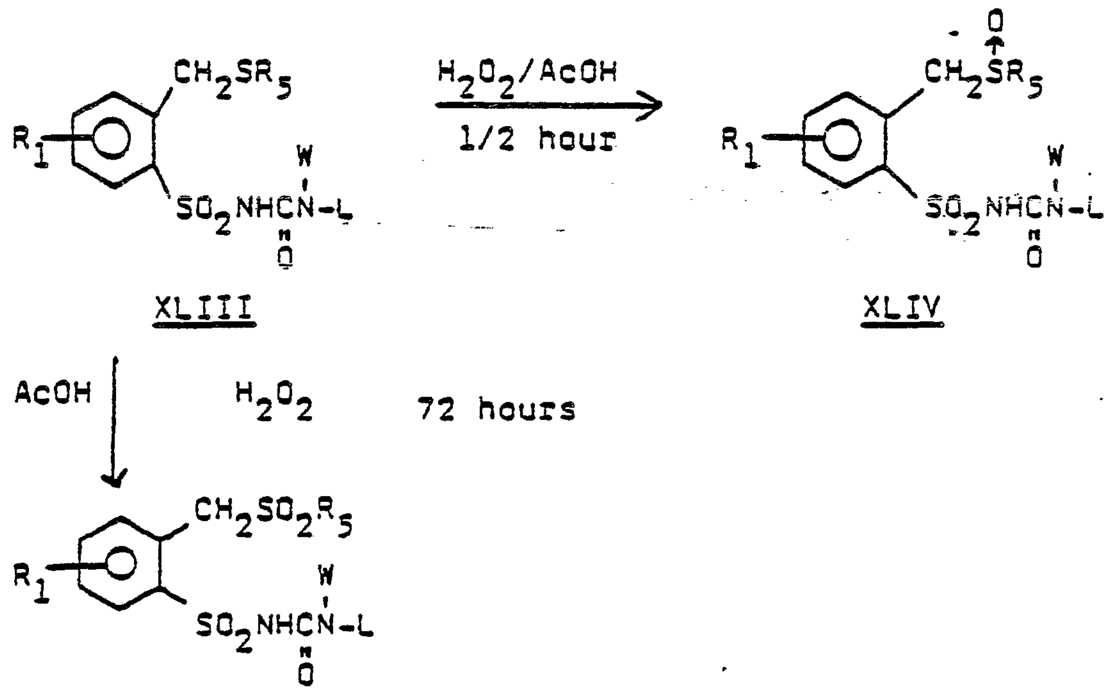


XLIII

XLIV

XLV

The o-alkylsulfinyl- and o-alkylsulfonylmethylbenzenesulfonylureas are made from their corresponding o-thioalkoxymethylbenzenesulfonylureas XLIII by means of peroxide oxidation. Reaction of the sulfidesulfonylurea with aqueous hydrogen peroxide in acetic acid at room temperature for half an hour affords exclusively the sulfoxidesulfonylurea XLIV. If the sulfide or sulfoxide is allowed to react for 72 hours under the same conditions, the sulfone XLV is obtained. Oxidation for 20 hours often results in a mixture of both sulfoxide and sulfone, which can be readily separated by column chromatography and eluted with ethyl acetate. Sulfonylureas described above are generally stable under these reaction conditions. They will however, split into heterocyclic amine and o-alkylsulfonylbenzenesulfonamide if heated. A general procedure for peroxide oxidation of sulfides to sulfones can be found in the paper by A. M. Van Arendonk and E. C. Kliderer, J. Am. Chem. Soc., 62, 3521 (1940).

Compounds of Formula I where $R = S(O)R_5$, $R_2 = S(O)R_{12}$ or $SO_2R_{12}$ and $R_{13} = S(O)R_5$ or $SO_2R_5$ are prepared in a similar manner apparent to one skilled in the art.

Several compounds of this invention of Formula I may be synthesized from the corresponding sulfonylureas XLVI(a-d) as shown in Equation 16. Oxidation of the compounds XLVI(a-d) to give the corresponding N-oxides I(a-d) may be accomplished in a similar manner to that of the preparation of aminoheterocyclic-N-oxides III, VI, V and VI as outlined in Equations 10 and 11.

Equation 16

a.

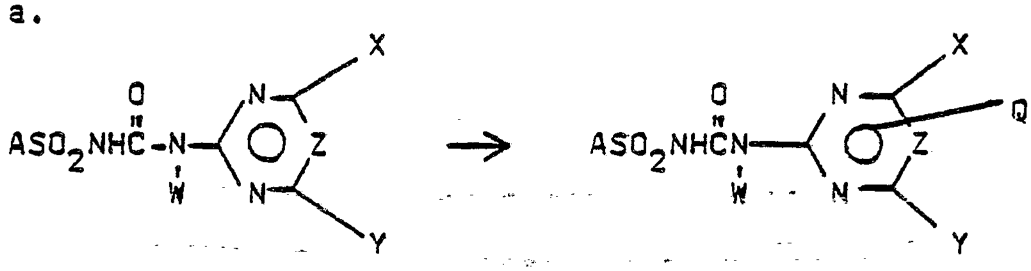

XLVIa Ia

b.

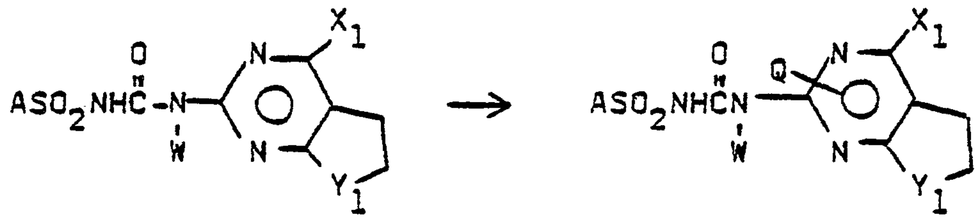

XLVIb Ib

c.

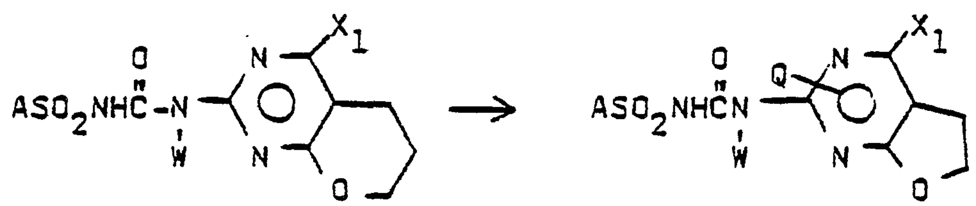

XLVIc Ic

d.

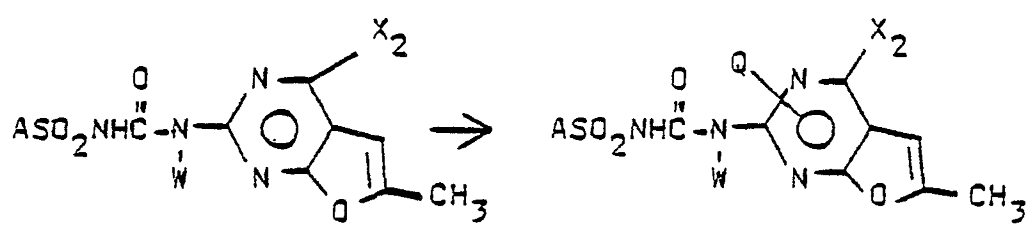

XLVId Id

Preparation of intermediates XLVI(a-d) may be accomplished by reacting a sulfonyl isocyanate with an appropriate heterocyclic amine using the methods described above and variations apparent to one skilled in the art.

Agriculturally suitable salts of compounds of Formula I are also useful herbicides and can be prepared by a number of ways known to the art. For example, metal salts can be made by treating compounds of Formula I with a solution of alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation to another. Cationic exchange can be effected by direct treatment of an aqueous solution of a salt of a compound of Formula I (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

The compounds of this invention and their preparation are further illustrated by the following examples wherein temperatures are given in degrees centigrade and all parts are by weight unless otherwise indicated.

Example 1

4,6-Dimethyl-1-oxide-2-pyrimidinamine

A solution of 24 g of m-chloroperoxybenzoic acid in 200 ml of acetone was added to a solution of 13 g of 4,6-dimethyl-2-pyrimidinamine in 300 ml of acetone with stirring. The mixture was stirred at room temperature for about two hours. The precipitate was then collected by filtration, washed with acetone and dried to yield 11 g of 4,6-dimethyl-1-oxide-2-pyrimidinamine, m.p. 252°C (dec.).

NMR (DMSO-$d_6$)$\delta$: 2.2 (s, 3H); 2.3 (s, 3H); 6.7 (s, 1H); 7.53 (b, 2H).

Example 2

2-Chloro-N-[(4,6-dimethyl-1-oxide-pyrimidin-2-yl)aminocarbonyl]benzenesulfonamide

A suspension of 1 g of 4,6-dimethyl-1-oxide-2-pyrimidinamine and a catalytic amount of DABCO® in 15 ml of anhydrous methylene chloride was stirred at room temperature for 5 minutes. With stirring, 2.1 g of 2-chlorobenzenesulfonylisocyanate was added. The mixture was heated to 40°C and was kept at 40°C for 1 hour. The mixture was then cooled to room temperature and was stirred at room temperature overnight. The reaction mixture was then filtered. The solid collected by filtration was washed with methylene chloride and was filtered. The filtrates were combined and evaporated to dryness under vacuum. The residue was triturated with n-butyl chloride. The precipitate was collected by filtration, washed with n-butyl chloride and dried to yield 1.9 g of 2-chloro-N-[(4,6-dimethyl-1-oxide-pyrimidin-2-yl)aminocarbonyl]benzenesulfonamide, m.p. 156-157°C.

NMR (DMSO-$d_6$)$\delta$: 2.33 (d, 6H);
6.8 (s, 1H);
7-9 (m, 6H);

IR (nujol)1730 $cm^{-1}$.

Example 3

4-Chloro-6-methyl-1-oxide-2-pyrimidinamine

A solution of 28 g of *m*-chloroperoxybenzoic acid in 200 ml of acetone was added to a solution of 17.3 g of 4-chloro-6-methyl-2-pyrimidinamine in 500 ml of acetone with stirring. The mixture was stirred at room temperature overnight. The precipitate was then collected by filtration, washed with acetone and dried to yield 13 g of 4-chloro-6-methyl-1-oxide-2-pyrimidinamine, m.p. 155°C (dec.).

NMR (DMSO-$d_6$) δ: 2.35 (s, 3H);
6.95 (s, 1H);
8.06 (b, 2H).

Example 4

4-Methoxy-6-methyl-1-oxide-2-pyrimidinamine

A solution of 11.2 g of 4-chloro-6-methyl-1-oxide-2-pyrimidinamine and 5.4 g of sodium methoxide in 200 ml of methanol was refluxed for 12 hours. The reaction mixture was cooled to room temperature and filtered. The filtrate was acidified with concentrated hydrogen chloride solution to pH 7 then evaporated to dryness. The residue was washed with ether, boiling ethyl acetate and then ether. The solid collected was dried to yield 10 g of 4-methoxy-6-methyl-1-oxide-2-pyrimidinamine, m.p. 199-201°C.

NMR (TFA) δ: 2.53 (s, 3H); 4.02 (s, 3H)
6.3 (s, 1H); 6.9 (b, 2H).

Example 5

2-[[(4-Methoxy-6-methyl-1-oxidepyrimidin-2-yl)aminocarbonyl]aminosulfonyl]benzoic acid, 1-methylethyl ester

A solution of 0.8 g of 4-methoxy-6-methyl-1-oxide-2-pyrimidinamine and 2.4 g of 1-methylethyl 2-(isocyanatosulfonyl)benzoate in 15 ml of anhydrous methylene chloride was stirred at room temperature overnight. The solution was then evaporated to dryness. The residue was stirred with 45 ml of n-butyl chloride at room temperature overnight. The precipitate was collected by filtration, washed with ether and dried to yield 0.4 g of 2-[[(4-methoxy-6-methyl-1-oxidepyrimidin-2-yl)aminocarbonyl]aminosulfonyl]-benzoic acid, 1-methylethyl ester, m.p. 135°C (dec.).

NMR (DMSO-$d_6$)δ: 1.33 (d, 6H); 2.33 (s, 3H); 3.83 (s, 3H); 5.2 (m, 1H); 6.33 (s, 1H); 6.6-9 (m, 6H).

IR (Nujol) $cm^{-1}$ 1710, 1735.

## Example 6

4,6-Dimethylfuro[2,3-d]pyrimidin-2-amine

A mixture of 6.0 g of 3-carbethoxy-5-hexyn-2-one and 3.6 g guanidine carbonate was heated in 9 ml dimethylsulfoxide under a nitrogen atmosphere at 140° for 4 hours. The ethanol produced in the reaction was removed as it was formed by distillation. After cooling to room temperature, acetone was added then the crystalline solid was collected then rinsed with water to yield 2.6 g of 4,6-dimethylfuro[2,3-d]pyrimidine; m.p. 262-263°. Absorptions at 2.55 (singlet), 2.82 (singlet) and 6.58 (broadened singlet) ppm in the nuclear magnetic resonance spectrum (60 MHz) indicated the title compound.

## Example 7

4,6-Dimethyl-1,3,5-triazin-2-amine-1-oxide

A solution of 1.2 g of m-chloroperoxybenzoic acid in 10 ml of acetone was added to a solution of 0.7 g of 4,6-dimethyl-1,3,5-triazin-2-amine in 40 ml of acetone with stirring. The mixture was stirred at room temperature overnight. The precipitate was then collected by filtration, washed with acetone and dried to yield 0.4 g of 4,6-dimethyl-1,3,5-triazin-2-amine-1-oxide, m.p. 260°C.

NMR (TFA): 2.77 (s, 3H); 2.97 (s, 3H) ppm.

Example 8

4,6-Dimethyl-1,3,5-triazin-2-amine-5-oxide

Preparation of the title compound was accomplished as described in U.S. 3,154,548. The nuclear magnetic resonance spectrum (60 MHz) of the product exhibited one major sharp singlet at 2.77 ppm in trifluoroacetic acid solution indicating the presence of the 5-oxide isomer as the major component.

By application of one or more of the procedures of Examples 1 through 8 and/or the general procedures described above, the compounds of Tables I through XXI can be prepared, wherein Q is as previously defined.

Table I

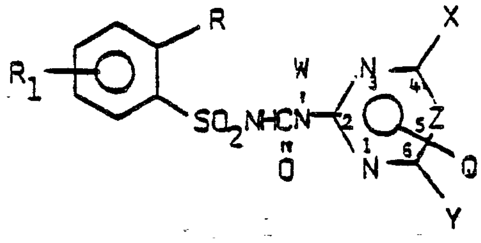

| R | $R_1$ | W | Q | X | Y | Z | m.p.(°C) |
|---|---|---|---|---|---|---|---|
| $CH_3$ | H | H | 3-O | $CH_3$ | $CH_3$ | CH | 162-164° (dec.) |
| $CH_3$ | 3-F | H | 3-O | $CH_3$ | $OCH_3$ | CH | |
| n-$C_4H_9$ | 5-Cl | H | 1-O | $OCH_3$ | $CH_3$ | CH | |
| $C_2H_5$ | H | $CH_3$ | 1-O | $OCH_3$ | $OCH_3$ | CH | |
| $OCH_3$ | 5-$NO_2$ | H | 1-O | $C_2H_5$ | $CH_3$ | CH | |
| $OC_2H_5$ | H | H | 1-O | $OC_2H_5$ | H | CH | |
| $OCH(CH_3)_2$ | H | H | 1-O | $CH_3$ | $CH_3$ | N | |
| $O(CH_2)_4H$ | 5-$O(CH_2)_4H$ | H | 3-O | $OCH_3$ | $CF_3$ | CF | |
| F | H | H | 3-O | $OCH_3$ | $CH(CH_3)C_2H_5$ | CH | |
| Br | H | H | 3-O | $CH_3$ | $OC(CH_3)_3$ | CH | |
| Cl | H | H | 1-O | $CH_3$ | $CH_3$ | CH | 156-7° (dec.) |
| Cl | H | H | 1-O | $OCH_3$ | $CH_3$ | CH | 112-115° (dec.) |
| Cl | H | H | 3-O | $OCH_3$ | $CH_3$ | CH | |
| Cl | H | H | 1-O | $OCH_3$ | $OCH_3$ | CH | |
| Cl | H | H | 5-O | $CH_3$ | $CH_3$ | N | |
| Cl | H | H | 1-O | $CH_3$ | $CH_3$ | N | 116-118° (dec.) |
| Cl | H | H | 1-O | $OCH_3$ | $CH_3$ | N | |
| Cl | H | H | 3-O | $OCH_3$ | $CH_3$ | N | |
| Cl | H | H | 5-O | $OCH_3$ | $CH_3$ | N | |
| Cl | H | H | 1-O | $OCH_3$ | $OCH_3$ | N | |
| Cl | H | H | 5-O | $OCH_3$ | $OCH_3$ | N | |
| Cl | H | H | 1-O | $OCH_3$ | H | CH | |
| Cl | H | H | 3-O | $CH_3$ | $CH_2CH_2OCH_3$ | CH | |
| $NO_2$ | H | H | 1-O | $CH_3$ | $CH_3$ | CH | 75-80° (dec.) |
| $NO_2$ | H | H | 1-O | $CH_3$ | $CH_3$ | N | 112-116° (dec.) |
| $NO_2$ | H | H | 5-O | $CH_3$ | $CH_3$ | N | |
| $NO_2$ | H | H | 3-O | $CH_3$ | $OCH_3$ | CH | 145°C (dec.) |

Table I (continued)

| R | $R_1$ | W | Q | X | Y | Z | m.p.(°C) |
|---|---|---|---|---|---|---|---|
| $NO_2$ | H | H | 1-O | $CH_3O$ | $CH_3O$ | CH | |
| $NO_2$ | H | H | 1-O | $CH_3$ | $CH_3O$ | N | |
| $NO_2$ | H | H | 3-O | $CH_3$ | $CH_3O$ | N | |
| $NO_2$ | H | H | 3-O | $CH_3$ | $CH_2CH_2OCH_2CH_3$ | CH | |
| $NO_2$ | H | $CH_3$ | 3-O | $CH_3$ | Cl | CH | |
| $CF_3$ | H | H | 1-O | $CH_3$ | $CH_3$ | CH | |
| $CF_3$ | H | H | 1-O | $OCH_3$ | $OCH_3$ | N | |
| $CF_3$ | H | H | 1-O | $OCH_3$ | $CH_3$ | N | |
| $CO_2CH_3$ | H | H | 5-O | $CH_3$ | $OCH_3$ | N | |
| $CO_2CH_3$ | H | H | 1-O | $CH_3$ | $CH_3$ | N | 118-120° (dec.) |
| $CO_2CH_3$ | H | H | 5-O | $CH_3$ | $CH_3$ | N | |
| $CO_2CH_3$ | H | H | 3-O | $CH_3$ | $OCH_3$ | N | |
| $CO_2CH_3$ | H | H | 1-O | $CH_3$ | $OCH_3$ | N | |
| $CO_2CH_3$ | H | H | 1-O | $OCH_3$ | $OCH_3$ | N | |
| $CO_2CH_3$ | H | H | 5-O | $OCH_3$ | $OCH_3$ | N | |
| $CO_2CH_3$ | H | H | 1-O | $CH_3$ | $CH_3$ | CH | 80-85°C (dec.) |
| $CO_2CH_3$ | H | H | 1-O | $CH_3$ | $OCH_3$ | CH | |
| $CO_2CH_3$ | H | H | 3-O | $CH_3$ | $OCH_3$ | CH | 101°C (dec.) |
| $CO_2CH_3$ | H | H | 1-O | $OCH_3$ | $OCH_3$ | CH | |
| $CO_2CH_3$ | H | H | 3-O | $CH_3$ | $OCH_3$ | CF | |
| $CO_2CH_3$ | H | H | 3-O | $CH_3$ | $CH_2OCH_3$ | N | |
| $CO_2CH_3$ | H | H | 3-O | $CH_3$ | $CH_2OC_2H_5$ | CH | |
| $CO_2CH_3$ | H | H | 3-O | $CH_3$ | Cl | CH | |
| $CO_2CH_3$ | H | H | 3-O | $OCH_3$ | Cl | N | |
| $CO_2CH_3$ | H | H | 1-O | $CH_3$ | H | CH | |
| $CO_2C_2H_5$ | H | H | 1-O | $CH_3$ | $CH_3$ | CH | 88-90° (dec.) |
| $CO_2CH(CH_3)_2$ | H | H | 1-O | $CH_3$ | $OCH_3$ | CH | 135° (dec.) |
| $CO_2CH(CH_3)_2$ | H | H | 1-O | $OCH_3$ | $OCH_3$ | N | |
| $CO_2(CH_2)_6H$ | H | H | 1-O | $CH_3$ | $CH_3$ | CH | |
| $CO_2CH_2CH{=}CH_2$ | H | H | 1-O | $CH_3$ | $CH_3$ | CH | 84-85° (dec.) |

Table I (continued)

| R | $R_1$ | W | Q | X | Y | Z | m.p.(°C) |
|---|---|---|---|---|---|---|---|
| $CO_2CH_2CH{=}CH_2$ | H | H | 3-O | $CH_3$ | $OCH_3$ | CH | 98°C(dec) |
| $CO_2CH_2CH{=}CH_2$ | H | H | 1-O | $OCH_3$ | $CH_3$ | N | |
| $CO_2CH(CH_3)CH{=}CH_2$ | H | H | 1-O | $OCH_3$ | $OCH_3$ | CH | |
| $CO_2(CH_2)_4CH{=}CH_2$ | H | H | 1-O | $OCH_3$ | $CH_3$ | CH | |
| $CO_2(CH_2)_4C{\equiv}CH$ | H | H | 1-O | $CH_3$ | $CH_3$ | CH | |
| $CO_2CH_2C{\equiv}CH$ | H | H | 1-O | $CH_3$ | $CH_3$ | CH | |
| $CO_2CH_2C{\equiv}CCH_3$ | 5-Br | H | 1-O | $CH_3$ | $CH_3$ | N | |
| $CO_2CH_2CF_3$ | H | H | 1-O | $OCH_3$ | $OCH_3$ | N | |
| $CO_2(CH_2)_4CHBrCH_2Br$ | H | H | 1-O | $OCH_3$ | $CH_3$ | CF | |
| $CO_2CH_2CH_2CH_2Cl$ | H | H | 1-O | $OCH_3$ | $OCH_3$ | CH | |
| 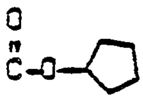 | H | H | 3-O | $OCH_3$ | $CH_3$ | N | |
| 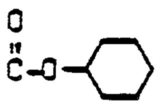 | H | H | 1-O | $CH_3$ | $CH_3$ | CH | |
| $CO_2CH_2CH_2OCH_2CH_3$ | H | H | 1-O | $OCH_3$ | $OCH_3$ | N | |
| $CO_2CH_2CH_2OCH_2CH_2OCH_3$ | H | H | 1-O | $OCH_3$ | $CH_3$ | N | |
| $CO_2CH_2OCH_3$ | H | H | 1-O | $OCH_3$ | $CH_3$ | CH | |
| $CO_2(CH_2)_3OCH_3$ | H | H | 1-O | $CH_3$ | $CH_3$ | CH | |
| $CO_2CH_2O(CH_2)_4H$ | H | H | 3-O | $CH_3$ | Cl | CH | |
| $CO_2CH_2OCH_2CH_2OCH_3$ | H | H | 3-O | $CH_3$ | $OCH_3$ | CF | |
| $SCH_3$ | H | H | 3-O | $CH_3$ | $OCH_3$ | CH | |
| $S(CH_2)_4H$ | H | H | 1-O | $OCH_3$ | $OCH_3$ | CH | |
| $S(O)CH_3$ | H | H | 5-O | $CH_3$ | $CH_3$ | N | |
| $S(O)CH_3$ | H | H | 1-O | $OCH_3$ | $CH_3$ | N | |
| $SO_2CH_3$ | H | H | 1-O | $CH_3$ | $OCH_3$ | CH | |
| $SO_2CH_3$ | H | H | 1-O | $OCH_3$ | $OCH_3$ | CH | |

Table I (continued)

| R | $R_1$ | W | Q | X | Y | Z | m.p.(°C) |
|---|---|---|---|---|---|---|---|
| $SO_2CH_3$ | H | H | 1-O | $CH_3$ | $CH_3$ | CH | 150-152° (dec.) |
| $SO_2CH_3$ | H | H | 1-O | $OCH_3$ | $OCH_3$ | N | |
| $SO_2(CH_2)_4H$ | H | H | 3-O | $CH_3$ | $OCH_3$ | N | |
| $SO_2(CH_2)_4H$ | H | H | 1-O | $OCH_3$ | $OCH_3$ | CH | |
| $SO_2(CH_2)_3H$ | H | H | 1-O | $CH_3$ | $CH_3$ | CH | |
| $SO_2N(CH_3)_2$ | H | H | 3-O | $CH_3$ | $OCH_3$ | N | |
| $SO_2N(CH_3)_2$ | H | H | 1-O | $CH_3$ | $OCH_3$ | N | |
| $SO_2N(CH_3)_2$ | H | H | 1-O | $CH_3$ | $CH_3$ | N | 138-140° |
| $SO_2N(CH_3)_2$ | H | H | 5-O | $CH_3$ | $CH_3$ | N | |
| $SO_2N(CH_3)_2$ | H | H | 1-O | $OCH_3$ | $OCH_3$ | N | |
| $SO_2N(CH_3)_2$ | H | H | 3-O | $CH_3$ | $OCH_3$ | CH | 78-84° |
| $SO_2N(CH_3)_2$ | H | H | 1-O | $CH_3$ | $OCH_3$ | CH | |
| $SO_2N(CH_3)_2$ | H | H | 1-O | $OCH_3$ | $OCH_3$ | CH | |
| $SO_2N(CH_3)_2$ | H | H | 1-O | $CH_3$ | $CH_3$ | CH | 163-164° |
| $SO_2N(CH_3)(CH_2)_4H$ | H | H | 3-O | $CH_3$ | $OCH_3$ | N | |
| $SO_2N(C_2H_5)_2$ | H | H | 1-O | $CH_3$ | $CH_3$ | CH | |
| $SO_2N(CH_3)CH(CH_3)C_2H_5$ | H | H | 3-O | $CH_3$ | $OCH_3$ | CH | |
| $SO_2N(CH_3)C_2H_5$ | H | H | 1-O | $OCH_3$ | $OCH_3$ | CH | |
| $SO_2N(CH_3)CH(CH_3)_2$ | H | H | 5-O | $CH_3$ | $CH_3$ | N | |
| $SO_2OCH_2CF_3$ | H | H | 1-O | $CH_3$ | $OCH_3$ | N | |
| $SO_2OCH_2CCl_3$ | H | H | 1-O | $CH_3$ | $CH_3$ | CH | |
| $SO_2N(OCH_3)CH_3$ | H | H | 1-O | $OCH_3$ | $OCH_3$ | CH | |
| $SO_2N(OCH_3)CH_3$ | H | H | 3-O | $CH_3$ | $OCH_3$ | N | |
| $OCHF_2$ | H | H | 1-O | $CH_3$ | $OCH_3$ | N | |
| $OCF_3$ | H | H | 1-O | $OCH_3$ | $OCH_3$ | CH | |
| $SCH_2CF_3$ | H | H | 3-O | $CH_3$ | $CH_3$ | CF | |
| $S(O)CF_2CHF_2$ | H | H | 1-O | $CH_3$ | $CH_3$ | CH | |
| $SO_2CH_3$ | H | H | 3-O | $CH_3$ | $OCH_3$ | CH | 128-132° |
| $SO_2CH_3$ | H | H | 3-O | $CH_3$ | $OC_2H_5$ | CH | 124-128° (dec.) |
| $SO_2N(CH_3)_2$ | H | H | 3-O | $CH_3$ | $OC_2H_5$ | CH | 70-73° (dec.) |
| Cl | H | H | 3-O | $CH_3$ | $OC_2H_5$ | CH | 102-105° (dec.) |

Table I (continued)

| R | $R_1$ | W | Q | X | Y | Z | m.p.(°C) |
|---|---|---|---|---|---|---|---|
| $S(O)CF_2CHFCl$ | H | H | 1-O | $OCH_3$ | $CH_3$ | N | |
| $S(O)CF_2CHFBr$ | H | H | 1-O | $OCH_3$ | $CH_3$ | CH | |
| $SO_2CF_2CHFCF_3$ | H | H | 1-O | $OCH_3$ | $OCH_3$ | N | |
| $SCF_3$ | H | H | 1-O | $OCH_3$ | $OCH_3$ | CH | |
| $S(O)CF_3$ | H | H | 1-O | $OCH_3$ | $CH_3$ | CH | |
| $SO_2CH_2CF_3$ | H | H | 1-O | $CH_3$ | $CH_3$ | CH | |
| $SO_2CF_3$ | H | H | 1-O | $CH_3$ | $OCH_3$ | N | |
| $OSO_2CH_3$ | H | H | 1-O | $CH_3$ | $CH_3$ | CH | 67-69° (dec.) |
| $OSO_2CH_3$ | H | H | 3-O | $CH_3$ | $OCH_3$ | N | |
| $OSO_2CH_3$ | H | H | 1-O | $OCH_3$ | $OCH_3$ | CH | |
| $OSO_2CH_3$ | H | H | 1-O | $OCH_3$ | $CH_3$ | CH | |
| $OSO_2(CH_2)_4H$ | H | H | 1-O | $OCH_3$ | $OCH_3$ | N | |
| $OSO_2CH_2CF_3$ | H | H | 1-O | $OCH_3$ | $CH_3$ | N | |
| $OSO_2CH_2CHBrCH_2Br$ | H | H | 3-O | $OCH_3$ | $CH_3$ | CH | |
| $OSO_2CH_2CH_2Cl$ | H | H | 1-O | $CH_3$ | $CH_3$ | N | |
| $CH_2Cl$ | H | H | 1-O | $CH_3$ | $CH_3$ | CH | |
| $CH_2Br$ | H | H | 1-O | $CH_3$ | $OCH_3$ | N | |
| $CH_2OCH_3$ | H | $CH_3$ | 1-O | $CH_3$ | $CH_3$ | N | |
| $CH_2O(CH_2)_4H$ | 6-Cl | H | 1-O | $OCH_3$ | $OCH_3$ | CH | |
| $CH_2SCH_3$ | H | H | 1-O | $OCH_3$ | $OCH_3$ | N | |
| $CH_2S(O)CH_3$ | H | H | 3-O | $OCH_3$ | $CH_3$ | CH | |
| $CH_2SO_2CH_3$ | H | H | 1-O | $OCH_3$ | $CH_3$ | N | |
| $CH_2SO_2(CH_2)_4H$ | H | H | 1-O | $CH_3$ | $CH_3$ | CH | |
| $CH_2OC_2H_5$ | H | H | 3-O | $CH_3$ | $OCH_3$ | CH | |
| $CH_2SCH(CH_3)_2$ | H | H | 1-O | $OCH_3$ | $OCH_3$ | CH | |
| $SO_2CH_3$ | H | H | 1-O | $CH_3$ | $CH_3$ | N | 168-170°(d) |
| $OSO_2CH_3$ | H | H | 3-O | $CH_3$ | $OC_2H_5$ | CH | 100-102°(d) |
| $CO_2C_2H_5$ | H | H | 3-O | $CH_3$ | $OCH_3$ | CH | 108-110°(d) |
| $CO_2C_2H_5$ | H | H | 3-O | $CH_3$ | $OC_2H_5$ | CH | 140° (d) |
| $CO_2CH(CH_3)_2$ | H | H | 1-O | $CH_3$ | $CH_3$ | N | 135-137°(d) |
| $NO_2$ | H | H | 3-O | $CH_3$ | $OC_2H_5$ | CH | 75-78°(d) |
| $CO_2CH_3$ | H | H | 3-O | $CH_3$ | $OC_2H_5$ | CH | 122-125°(d) |
| $CO_2CH_2CH=CH_2$ | H | H | 3-O | $CH_3$ | $OC_2H_5$ | CH | 105-108°(d) |

Table II

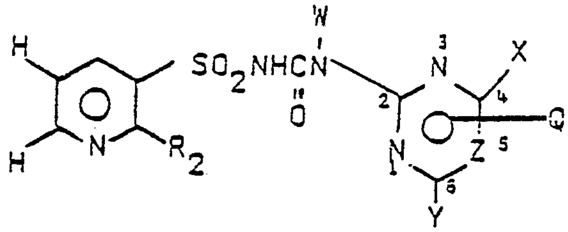

| $R_2$ | W | Q | X | Y | Z | m.p.(°C) |
|---|---|---|---|---|---|---|
| 2-F | H | 1-O | $CH_3$ | $CH_3$ | CH | |
| 2-Cl | H | 3-O | $CH_3$ | $OCH_3$ | CH | |
| 2-Cl | H | 1-O | $CH_3$ | $OCH_3$ | CH | |
| 2-Cl | H | 3-O | $CH_3$ | $OCH_3$ | N | |
| 2-Cl | H | 1-O | $CH_3$ | $OCH_3$ | N | |
| 2-Cl | H | 1-O | $CH_3$ | $CH_3$ | CH | |
| 2-Cl | H | 1-O | $OCH_3$ | $OCH_3$ | CH | |
| 2-Cl | H | 1-O | $OCH_3$ | $OCH_3$ | N | |
| 2-Cl | H | 1-O | $CH_3$ | $CH_3$ | N | |
| 2-Cl | H | 5-O | $CH_3$ | $CH_3$ | N | |
| 4-Br | H | 1-O | $CH_3$ | $CH_3$ | CF | |
| 2-$CH_3$ | H | 3-O | $CH_3$ | $OCH_3$ | N | |
| 4-$(CH_2)_4H$ | H | 1-O | $CH_3$ | $CH_3$ | CH | |
| 2-$OCH_3$ | H | 1-O | $OCH_3$ | $OCH_3$ | CH | |
| 4-$OCH_3$ | H | 1-O | $OCH_3$ | $OCH_3$ | N | |
| 2-$OCH_3$ | $CH_3$ | 3-O | $OCH_3$ | $CH_3$ | CH | |
| 2-$OC_2H_5$ | H | 3-O | $OCH_3$ | Cl | CH | |
| 4-$OC_2H_5$ | H | 1-O | $CH_3$ | $CH_3$ | CH | |
| 2-$O(CH_2)_4H$ | H | 1-O | $CH_3$ | $CH_3$ | N | |
| 4-$OCH(CH_3)_2$ | H | 1-O | $CH_3$ | $OCH_3$ | N | |
| 2-$NO_2$ | H | 1-O | $OCH_3$ | $OCH_3$ | CH | |
| 4-$NO_2$ | H | 1-O | $OCH_3$ | $OCH_3$ | N | |
| 2-$CO_2CH_3$ | H | 3-O | $CH_3$ | $OCH_3$ | CH | |

Table II (continued)

| $R_2$ | W | Q | X | Y | Z | m.p.(°C) |
|---|---|---|---|---|---|---|
| $2-CO_2CH_3$ | H | 1-O | $CH_3$ | $CH_3$ | CH | |
| $4-CO_2CH_3$ | H | 3-O | $CH_3$ | $OCH_3$ | N | |
| $4-CO_2CH_3$ | H | 1-O | $OCH_3$ | $OCH_3$ | CH | |
| $2-CO_2C_2H_5$ | H | 1-O | $OCH_3$ | $CH_3$ | CH | |
| $4-CO_2(CH_2)_4H$ | H | 1-O | $OCH_3$ | $CH_3$ | N | |
| $2-CO_2CH(CH_3)_2$ | H | 3-O | $OCH_3$ | $OCH_3$ | CH | |
| $2-SCH_3$ | H | 1-O | $CH_3$ | $CH_3$ | CH | |
| $4-SC_2H_5$ | H | 3-O | $CH_3$ | $OCH_3$ | N | |
| $2-SCH(CH_3)_2$ | H | 3-O | $CH_3$ | $OCH_3$ | CH | |
| $4-S(O)CH_3$ | H | 1-O | $OCH_3$ | $OCH_3$ | CH | |
| $2-S(O)CH_2CH_3$ | $CH_3$ | 1-O | $OCH_3$ | $OCH_3$ | N | |
| $2-SO_2C_2H_5$ | H | 1-O | $OCH_3$ | $CH_3$ | N | |
| $4-SO_2(CH_2)_3H$ | H | 1-O | $CH_3$ | $OCH_3$ | CH | |
| $2-SO_2CH_3$ | H | 1-O | $CH_3$ | $CH_3$ | CH | |

Table IIIa

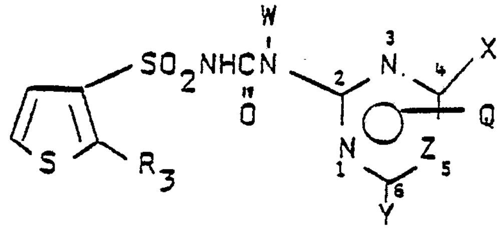

| $R_3$ | W | X | Y | Z | Q |
|---|---|---|---|---|---|
| $CH_3$ | H | $CH_3$ | $CH_3$ | N | 1-O |
| $CH_3$ | H | $CH_3$ | $OCH_3$ | CH | 3-O |
| $C_2H_5$ | H | $CH_3$ | $CH_3$ | CH | 1-O |
| Cl | H | $CH_3$ | $OCH_3$ | N | 1-O |
| Cl | H | $OCH_3$ | $OCH_3$ | CH | 1-O |
| $OC_2H_5$ | H | $OCH_3$ | $OCH_3$ | N | 1-O |
| $OCH_3$ | $CH_3$ | $OCH_3$ | $CH_3$ | CH | 1-O |
| $O(CH_2)_4H$ | H | $CH_3$ | $CH_3$ | CH | 1-O |
| $CH(CH_3)C_2H_5$ | H | $CH_3$ | $OCH_3$ | N | 3-O |
| $OCH(CH_3)_2$ | H | $CH_3$ | $OCH_3$ | CH | 1-O |
| Br | H | $OCH_3$ | $OCH_3$ | N | 1-O |
| $NO_2$ | H | $CH_3$ | $CH_3$ | N | 1-O |
| $NO_2$ | H | $OCH_3$ | $OCH_3$ | CH | 1-O |
| $SO_2N(CH_3)_2$ | H | $CH_3$ | $OCH_3$ | N | 3-O |
| $SO_2N[CH(CH_3)_2]CH_3$ | H | $CH_3$ | $CH_3$ | CH | 1-O |
| $SO_2N(CH_3)_2$ | H | $OCH_3$ | $OCH_3$ | CH | 1-O |
| $SO_2N(CH_3)C_2H_5$ | H | $CH_3$ | $OCH_3$ | CH | 3-O |
| $SO_2N(C_2H_5)_2$ | H | $OCH_3$ | $OCH_3$ | N | 1-O |
| $SO_2N(OCH_3)CH_3$ | H | $CH_3$ | $CH_3$ | N | 1-O |
| $CO_2CH_3$ | H | $CH_3$ | $CH_3$ | CH | 1-O |
| $CO_2CH_3$ | H | $CH_3$ | $OCH_3$ | N | 1-O |
| $CO_2CH_3$ | H | $CH_3$ | $OCH_3$ | N | 3-O |
| $CO_2CH_3$ | H | $OCH_3$ | $OCH_3$ | CH | 1-O |
| $CO_2CH_3$ | H | $OCH_3$ | $CH_3$ | N | 5-O |

Table IIIa (continued)

| $R_3$ | W | X | Y | Z | Q |
|---|---|---|---|---|---|
| $CO_2CH_3$ | H | $OCH_3$ | $OCH_3$ | N | 1-0 |
| $CO_2CH_3$ | H | $OCH_3$ | $OCH_3$ | N | 5-0 |
| $CO_2CH_3$ | H | $OCH_3$ | $CH_3$ | CH | 1-0 |
| $CO_2CH_3$ | H | $OCH_3$ | $CH_3$ | CH | 3-0 |
| $CO_2C_2H_5$ | H | $CH_3$ | $CH_3$ | N | 1-0 |
| $CO_2(CH_2)_6H$ | H | $CH_3$ | $CH_3$ | CH | 1-0 |
| $CO_2CH(CH_3)_2$ | H | $OCH_3$ | $CH_3$ | N | 1-0 |
| $CO_2CH(CH_3)C_2H_5$ | H | $CH_3$ | $OCH_3$ | CH | 3-0 |
| $CO_2$ 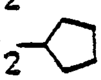 | H | $OCH_3$ | $OCH_3$ | CH | 1-0 |
| $CO_2$ 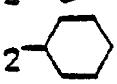 | H | $CH_3$ | $CH_3$ | CH | 1-0 |
| $CO_2CH_2CH{=}CH_2$ | H | $OCH_3$ | $CH_3$ | N | 1-0 |
| $CO_2CH(CH_3)CH{=}CH_2$ | H | $CH_3$ | $OCH_3$ | CH | 3-0 |
| $CO_2CH_2C(CH_3){=}CH_2$ | H | $OCH_3$ | $OCH_3$ | N | 1-0 |
| $CO_2(CH_2)_4CH{=}CH_2$ | H | $CH_3$ | $CH_3$ | CH | 1-0 |
| $CO_2CH_2C{\equiv}CH$ | H | $OCH_3$ | $OCH_3$ | CH | 1-0 |
| $CO_2CH_2C{\equiv}C(CH_2)_3H$ | H | $OCH_3$ | $OCH_3$ | N | 1-0 |
| $CO_2CH_2C{\equiv}C-CH_3$ | H | $CH_3$ | $CH_3$ | N | 1-0 |
| $CO_2CH_2CCl_3$ | $CH_3$ | $CH_3$ | $OCH_3$ | N | 1-0 |
| $CO_2CH_2CF_3$ | H | $CH_3$ | $OCH_3$ | CH | 1-0 |
| $CO_2CH_2CH_2Cl$ | H | $CH_3$ | $CH_3$ | CH | 1-0 |
| $CO_2CH_2CH_2CH_2Br$ | H | $OCH_3$ | $OCH_3$ | CH | 1-0 |
| $CO_2(CH_2)_6Cl$ | H | $OCH_3$ | $OCH_3$ | N | 1-0 |
| $CO_2CH_2CHFCH_2F$ | H | $CH_3$ | $OCH_3$ | CH | 3-0 |
| $CO_2CH_2CHClCHClCH_3$ | H | $CH_3$ | $OCH_3$ | N | 3-0 |
| $CO_2CH_2CH_2OCH_3$ | H | $CH_3$ | $CH_3$ | CH | 1-0 |
| $CO_2(CH_2CH_2O)_2C_2H_5$ | H | $CH_3$ | $CH_3$ | N | 1-0 |
| $OCH_2CH_2CH_2OCH_3$ | H | $OCH_3$ | $OCH_3$ | CH | 1-0 |
| $OCH_2OCH_3$ | H | $OCH_3$ | $OCH_3$ | N | 1-0 |
| $OCH_2OCH_2CH_2OCH_3$ | H | $CH_3$ | $OCH_3$ | CH | 3-0 |

Table IIIb

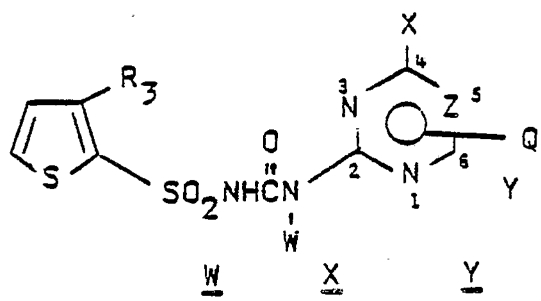

| $R_3$ | W | X | Y | Z | Q |
|---|---|---|---|---|---|
| $CH_3$ | H | $CH_3$ | $CH_3$ | N | 1-O |
| $CH_3$ | H | $CH_3$ | $OCH_3$ | CH | 3-O |
| $C_2H_5$ | H | $CH_3$ | $CH_3$ | CH | 1-O |
| Cl | H | $CH_3$ | $OCH_3$ | N | 1-O |
| Cl | H | $OCH_3$ | $OCH_3$ | CH | 1-O |
| $OC_2H_5$ | H | $OCH_3$ | $OCH_3$ | N | 1-O |
| $OCH_3$ | $CH_3$ | $OCH_3$ | $CH_3$ | CH | 1-O |
| $O(CH_2)_4H$ | H | $CH_3$ | $CH_3$ | CH | 1-O |
| $CH(CH_3)C_2H_5$ | H | $CH_3$ | $OCH_3$ | N | 3-O |
| $OCH(CH_3)_2$ | H | $CH_3$ | $OCH_3$ | CH | 1-O |
| Br | H | $OCH_3$ | $OCH_3$ | N | 1-O |
| $NO_2$ | H | $CH_3$ | $CH_3$ | N | 1-O |
| $NO_2$ | H | $OCH_3$ | $OCH_3$ | CH | 1-O |
| $SO_2N(CH_3)_2$ | H | $CH_3$ | $OCH_3$ | N | 3-O |
| $SO_2N[CH(CH_3)_2]CH_3$ | H | $CH_3$ | $CH_3$ | CH | 1-O |
| $SO_2N(CH_3)_2$ | H | $OCH_3$ | $OCH_3$ | CH | 1-O |
| $SO_2N(CH_3)C_2H_5$ | H | $CH_3$ | $OCH_3$ | CH | 3-O |
| $SO_2N(C_2H_5)_2$ | H | $OCH_3$ | $OCH_3$ | N | 1-O |
| $SO_2N(OCH_3)CH_3$ | H | $CH_3$ | $CH_3$ | N | 1-O |
| $CO_2CH_3$ | H | $CH_3$ | $CH_3$ | CH | 1-O |
| $CO_2CH_3$ | H | $CH_3$ | $OCH_3$ | N | 1-O |
| $CO_2CH_3$ | H | $CH_3$ | $OCH_3$ | N | 3-O |
| $CO_2CH_3$ | H | $OCH_3$ | $OCH_3$ | CH | 1-O |
| $CO_2CH_3$ | H | $OCH_3$ | $CH_3$ | N | 5-O |

Table IIIb (continued)

| $R_3$ | W | X | Y | Z | Q |
|---|---|---|---|---|---|
| $CO_2CH_3$ | H | $OCH_3$ | $OCH_3$ | N | 1-O |
| $CO_2CH_3$ | H | $OCH_3$ | $OCH_3$ | N | 5-O |
| $CO_2CH_3$ | H | $OCH_3$ | $CH_3$ | CH | 1-O |
| $CO_2CH_3$ | H | $OCH_3$ | $CH_3$ | CH | 3-O |
| $CO_2C_2H_5$ | H | $CH_3$ | $CH_3$ | N | 1-O |
| $CO_2(CH_2)_6H$ | H | $CH_3$ | $CH_3$ | CH | 1-O |
| $CO_2CH(CH_3)_2$ | H | $OCH_3$ | $CH_3$ | N | 1-O |
| $CO_2CH(CH_3)C_2H_5$ | H | $CH_3$ | $OCH_3$ | CH | 3-O |
| $CO_2$ 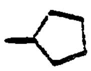 | H | $OCH_3$ | $OCH_3$ | CH | 1-O |
| $CO_2$ 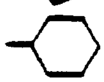 | H | $CH_3$ | $CH_3$ | CH | 1-O |
| $CO_2CH_2CH{=}CH_2$ | H | $OCH_3$ | $CH_3$ | N | 1-O |
| $CO_2CH(CH_3)CH{=}CH_2$ | H | $CH_3$ | $OCH_3$ | CH | 3-O |
| $CO_2CH_2C(CH_3){=}CH_2$ | H | $OCH_3$ | $OCH_3$ | N | 1-O |
| $CO_2(CH_2)_4CH{=}CH_2$ | H | $CH_3$ | $CH_3$ | CH | 1-O |
| $CO_2CH_2C{\equiv}CH$ | H | $OCH_3$ | $OCH_3$ | CH | 1-O |
| $CO_2CH_2C{\equiv}C(CH_2)_3H$ | H | $OCH_3$ | $OCH_3$ | N | 1-O |
| $CO_2CH_2C{\equiv}C{-}CH_3$ | H | $CH_3$ | $CH_3$ | N | 1-O |
| $CO_2CH_2CCl_3$ | $CH_3$ | $CH_3$ | $OCH_3$ | N | 1-O |
| $CO_2CH_2CF_3$ | H | $CH_3$ | $OCH_3$ | CH | 1-O |
| $CO_2CH_2CH_2Cl$ | H | $CH_3$ | $CH_3$ | CH | 1-O |
| $CO_2CH_2CH_2CH_2Br$ | H | $OCH_3$ | $OCH_3$ | CH | 1-O |
| $CO_2(CH_2)_6Cl$ | H | $OCH_3$ | $OCH_3$ | N | 1-O |
| $CO_2CH_2CHFCH_2F$ | H | $CH_3$ | $OCH_3$ | CH | 3-O |
| $CO_2CH_2CHClCHClCH_3$ | H | $CH_3$ | $OCH_3$ | N | 3-O |
| $CO_2CH_2CH_2OCH_3$ | H | $CH_3$ | $CH_3$ | CH | 1-O |
| $CO_2(CH_2CH_2O)_2C_2H_5$ | H | $CH_3$ | $CH_3$ | N | 1-O |
| $OCH_2CH_2CH_2OCH_3$ | H | $OCH_3$ | $OCH_3$ | CH | 1-O |
| $OCH_2OCH_3$ | H | $OCH_3$ | $OCH_3$ | N | 1-O |
| $OCH_2OCH_2CH_2OCH_3$ | H | $CH_3$ | $OCH_3$ | CH | 3-O |

Table IIIc

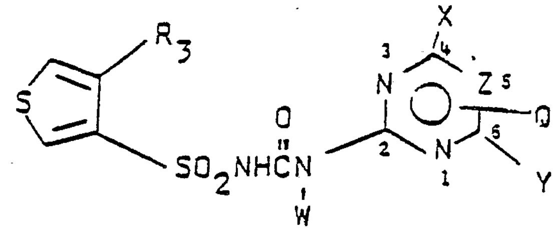

| $R_3$ | W | X | Y | Z | Q |
|---|---|---|---|---|---|
| $CH_3$ | H | $CH_3$ | $CH_3$ | N | 1-O |
| $CH_3$ | H | $CH_3$ | $OCH_3$ | CH | 3-O |
| $C_2H_5$ | H | $CH_3$ | $CH_3$ | CH | 1-O |
| Cl | H | $CH_3$ | $OCH_3$ | N | 1-O |
| Cl | H | $OCH_3$ | $OCH_3$ | CH | 1-O |
| $OC_2H_5$ | H | $OCH_3$ | $OCH_3$ | N | 1-O |
| $OCH_3$ | $CH_3$ | $OCH_3$ | $CH_3$ | CH | 1-O |
| $O(CH_2)_4H$ | H | $CH_3$ | $CH_3$ | CH | 1-O |
| $CH(CH_3)C_2H_5$ | H | $CH_3$ | $OCH_3$ | N | 3-O |
| $OCH(CH_3)_2$ | H | $CH_3$ | $OCH_3$ | CH | 1-O |
| Br | H | $OCH_3$ | $OCH_3$ | N | 1-O |
| $NO_2$ | H | $CH_3$ | $CH_3$ | N | 1-O |
| $NO_2$ | H | $OCH_3$ | $OCH_3$ | CH | 1-O |
| $SO_2N(CH_3)_2$ | H | $CH_3$ | $OCH_3$ | N | 3-O |
| $SO_2N[CH(CH_3)_2]CH_3$ | H | $CH_3$ | $CH_3$ | CH | 1-O |
| $SO_2N(CH_3)_2$ | H | $OCH_3$ | $OCH_3$ | CH | 1-O |
| $SO_2N(CH_3)C_2H_5$ | H | $CH_3$ | $OCH_3$ | CH | 3-O |
| $SO_2N(C_2H_5)_2$ | H | $OCH_3$ | $OCH_3$ | N | 1-O |
| $SO_2N(OCH_3)CH_3$ | H | $CH_3$ | $CH_3$ | N | 1-O |
| $CO_2CH_3$ | H | $CH_3$ | $CH_3$ | CH | 1-O |
| $CO_2CH_3$ | H | $CH_3$ | $OCH_3$ | N | 1-O |
| $CO_2CH_3$ | H | $CH_3$ | $OCH_3$ | N | 3-O |
| $CO_2CH_3$ | H | $OCH_3$ | $OCH_3$ | CH | 1-O |
| $CO_2CH_3$ | H | $OCH_3$ | $CH_3$ | N | 5-O |

Table IIIc (continued)

| $R_3$ | W | X | Y | Z | Q |
|---|---|---|---|---|---|
| $CO_2CH_3$ | H | $OCH_3$ | $OCH_3$ | N | 1-0 |
| $CO_2CH_3$ | H | $OCH_3$ | $OCH_3$ | N | 5-0 |
| $CO_2CH_3$ | H | $OCH_3$ | $CH_3$ | CH | 1-0 |
| $CO_2CH_3$ | H | $OCH_3$ | $CH_3$ | CH | 3-0 |
| $CO_2C_2H_5$ | H | $CH_3$ | $CH_3$ | N | 1-0 |
| $CO_2(CH_2)_6H$ | H | $CH_3$ | $CH_3$ | CH | 1-0 |
| $CO_2CH(CH_3)_2$ | H | $OCH_3$ | $CH_3$ | N | 1-0 |
| $CO_2CH(CH_3)C_2H_5$ | H | $CH_3$ | $OCH_3$ | CH | 3-0 |
| $CO_2$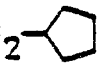 | H | $OCH_3$ | $OCH_3$ | CH | 1-0 |
| $CO_2$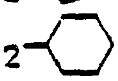 | H | $CH_3$ | $CH_3$ | CH | 1-0 |
| $CO_2CH_2CH{=}CH_2$ | H | $OCH_3$ | $CH_3$ | N | 1-0 |
| $CO_2CH(CH_3)CH{=}CH_2$ | H | $CH_3$ | $OCH_3$ | CH | 3-0 |
| $CO_2CH_2C(CH_3){=}CH_2$ | H | $OCH_3$ | $OCH_3$ | N | 1-0 |
| $CO_2(CH_2)_4CH{=}CH_2$ | H | $CH_3$ | $CH_3$ | CH | 1-0 |
| $CO_2CH_2C{\equiv}CH$ | H | $OCH_3$ | $OCH_3$ | CH | 1-0 |
| $CO_2CH_2C{\equiv}C(CH_2)_3H$ | H | $OCH_3$ | $OCH_3$ | N | 1-0 |
| $CO_2CH_2C{\equiv}C{-}CH_3$ | H | $CH_3$ | $CH_3$ | N | 1-0 |
| $CO_2CH_2CCl_3$ | $CH_3$ | $CH_3$ | $OCH_3$ | N | 1-0 |
| $CO_2CH_2CF_3$ | H | $CH_3$ | $OCH_3$ | CH | 1-0 |
| $CO_2CH_2CH_2Cl$ | H | $CH_3$ | $CH_3$ | CH | 1-0 |
| $CO_2CH_2CH_2CH_2Br$ | H | $OCH_3$ | $OCH_3$ | CH | 1-0 |
| $CO_2(CH_2)_6Cl$ | H | $OCH_3$ | $OCH_3$ | N | 1-0 |
| $CO_2CH_2CHFCH_2F$ | H | $CH_3$ | $OCH_3$ | CH | 3-0 |
| $CO_2CH_2CHClCHClCH_3$ | H | $CH_3$ | $OCH_3$ | N | 3-0 |
| $CO_2CH_2CH_2OCH_3$ | H | $CH_3$ | $CH_3$ | CH | 1-0 |
| $CO_2(CH_2CH_2O)_2C_2H_5$ | H | $CH_3$ | $CH_3$ | N | 1-0 |
| $OCH_2CH_2CH_2OCH_3$ | H | $OCH_3$ | $OCH_3$ | CH | 1-0 |
| $OCH_2OCH_3$ | H | $OCH_3$ | $OCH_3$ | N | 1-0 |
| $OCH_2OCH_2CH_2OCH_3$ | H | $CH_3$ | $OCH_3$ | CH | 3-0 |

Table IV

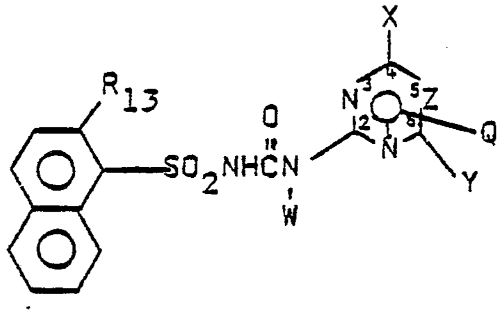

| $R_{13}$ | W | X | Y | Z | Q |
|---|---|---|---|---|---|
| $CH_3$ | H | $CH_3$ | $CH_3$ | CH | 1-O |
| $CH_3$ | H | $CH_3$ | $OCH_3$ | N | 3-O |
| $C_2H_5$ | H | $OCH_3$ | $OCH_3$ | CH | 1-O |
| H | H | $OCH_3$ | $CH_3$ | CH | 1-O |
| H | H | $CH_3$ | $OCH_3$ | N | 1-O |
| Cl | H | $CH_3$ | $CH_3$ | N | 1-O |
| Cl | H | $CH_3$ | $CH_3$ | CH | 1-O |
| Cl | H | $OCH_3$ | $OCH_3$ | N | 1-O |
| Cl | H | $OCH_3$ | $OCH_3$ | CH | 1-O |
| $NO_2$ | H | $OCH_3$ | $CH_3$ | CH | 3-O |
| $(CH_2)_4H$ | H | $OCH_3$ | $CH_3$ | N | 1-O |
| $OCH_3$ | H | $CH_3$ | $CH_3$ | CH | 1-O |
| $OC_2H_5$ | H | $CH_3$ | $CH_3$ | N | 1-O |
| $O(CH_2)_4H$ | H | $OCH_3$ | $OCH_3$ | CH | 1-O |
| $SCH_3$ | H | $OCH_3$ | $OCH_3$ | N | 1-O |
| $SO_2CH_3$ | $CH_3$ | $OCH_3$ | $CH_3$ | CH | 1-O |
| $S(O)C_2H_5$ | H | $OCH_3$ | $CH_3$ | N | 1-O |
| $S(O)CH(CH_3)_2$ | H | $CH_3$ | $CH_3$ | CH | 1-O |
| $SO_2(CH_2)_4H$ | H | $CH_3$ | $CH_3$ | N | 1-O |
| $SO_2N(CH_3)_2$ | H | $OCH_3$ | $OCH_3$ | CH | 1-O |

Table IV (continued)

| $R_{13}$ | W | X | Y | Z | Q |
|---|---|---|---|---|---|
| $SO_2N[CH(CH_3)_2]CH_3$ | H | $OCH_3$ | $OCH_3$ | N | 1-O |
| $SO_2N(C_2H_5)_2$ | H | $OCH_3$ | $CH_3$ | CH | 1-O |
| $SO_2N(CH_3)_2$ | H | $OCH_3$ | $CH_3$ | N | 1-O |
| $SO_2CH_3$ | H | $OCH_3$ | $CH_3$ | N | 3-O |

Table V

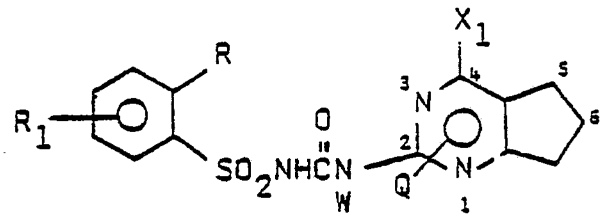

| R | $R_1$ | $X_1$ | W | Q | m.p.(°C) |
|---|---|---|---|---|---|
| H | H | $CH_3$ | H | 1-O | |
| Cl | H | $CH_3$ | H | 1-O | |
| $OC_2H_5$ | 5-$NO_2$ | $C_2H_5$ | H | 1-O | |
| $CF_3$ | H | $CH_3$ | H | 3-O | |
| $CH_3$ | H | $CH_3$ | H | 1-O | |
| $CH(CH_3)C_2H_5$ | H | $CH_3$ | $CH_3$ | 1-O | |
| $OCH_3$ | H | $C_2H_5$ | H | 3-O | |
| $O(CH_2)_3CH_3$ | H | $C_2H_5$ | H | 1-O | |
| $OCH(CH_3)_2$ | 6-Cl | $CH_3$ | H | 1-O | |
| $C_2H_5$ | 3-$CH_3$ | $CH_3$ | H | 1-O | |
| Br | H | $CH_3$ | $CH_3$ | 1-O | |
| $NO_2$ | H | $CH_3$ | H | 3-O | |
| $SCH_3$ | 5-$CH(CH_3)_2$ | $CH_3$ | H | 3-O | |
| $SC(CH_3)_3$ | H | $CH_3$ | H | 1-O | |
| $S(O)CH_3$ | H | $C_2H_5$ | H | 1-O | |
| $S(O)CH(CH_3)C_2H_5$ | 3-F | $CH_3$ | H | 1-O | |
| $SO_2CH_3$ | 5-$C_2H_5$ | $CH_3$ | H | 3-O | |
| $SO_2CH(CH_3)_2$ | 5-Br | $CH_3$ | H | 1-O | |
| $SO_2(CH_2)_3CH_3$ | 6-F | $CH_3$ | $CH_3$ | 3-O | |
| $SO_2CH_3$ | H | $CH_3$ | H | 1-O | |
| $SO_2CH_3$ | H | H | H | 1-O | |

Table V (continued)

| R | $R_1$ | $X_1$ | W | Q | m.p.(°C) |
|---|---|---|---|---|---|
| $SO_2C_2H_5$ | H | $CH_3$ | H | 1-O | |
| $SO_2OCH_2CF_3$ | H | $CH_3$ | H | 1-O | |
| $SO_2OCH_2CCl_3$ | H | $CH_3$ | H | 3-O | |
| $SO_2N(OCH_3)CH_3$ | H | $CH_3$ | H | 1-O | |
| H | H | $OCH_3$ | H | 3-O | |
| Cl | H | $OCH_3$ | H | 1-O | |
| $OC_2H_5$ | 5-$NO_2$ | $OC_2H_5$ | H | 1-O | |
| $CF_3$ | H | $OCH_3$ | H | 1-O | |
| $CH_3$ | H | $OCH_3$ | H | 1-O | |
| $CH(CH_3)C_2H_5$ | H | $OCH_3$ | $CH_3$ | 3-O | |
| $OCH_3$ | H | $OC_2H_5$ | H | 3-O | |
| $O(CH_2)_3CH_3$ | H | $OC_2H_5$ | H | 1-O | |
| $OCH(CH_3)_2$ | 6-Cl | $OCH_3$ | H | 3-O | |
| $C_2H_5$ | 3-$CH_3$ | $OCH_3$ | H | 3-O | |
| Br | H | $OCH_3$ | $CH_3$ | 3-O | |
| $NO_2$ | H | $OCH_3$ | H | 1-O | |
| $SCH_3$ | 5-$CH(CH_3)_2$ | $OCH_3$ | H | 3-O | |
| $SC(CH_3)_3$ | H | $OCH_3$ | H | 3-O | |
| $S(O)CH_3$ | H | $OC_2H_5$ | H | 1-O | |
| $S(O)CH(CH_3)C_2H_5$ | 3-F | $OCH_3$ | H | 1-O | |
| $SO_2CH_3$ | 5-$C_2H_5$ | $OCH_3$ | H | 3-O | |
| $SO_2CH(CH_3)_2$ | 5-Br | $OCH_3$ | H | 3-O | |
| $SO_2(CH_2)_3CH_3$ | 6-F | $OCH_3$ | $CH_3$ | 1-O | |
| $SO_2CH_3$ | H | $OCH_3$ | H | 1-O | |

Table V (continued)

| R | $R_1$ | $X_1$ | W | Q | m.p.(°C) |
|---|---|---|---|---|---|
| $SO_2CH_3$ | H | Cl | H | 1-0 | |
| $SO_2C_2H_5$ | H | $OCH_3$ | H | 1-0 | |
| $SO_2OCH_2CF_3$ | H | $OCH_3$ | H | 3-0 | |
| $SO_2OCH_2CCl_3$ | H | $OCH_3$ | H | 3-0 | |
| $SO_2N(OCH_3)CH_3$ | H | $OCH_3$ | H | 3-0 | |
| $S(O)CH_2CF_3$ | H | $CH_3$ | H | 3-0 | |
| $OSO_2CF_3$ | 3-$OC(CH_3)_3$ | $CH_3$ | H | 1-0 | |
| $OSO_2CH_2CH_3$ | H | $CH_3$ | H | 1-0 | |
| $OSO_2CHCl_2$ | H | $CH_3$ | H | 3-0 | |
| $OSO_2(CH_2)_3Br$ | 3-$OC_2H_5$ | $CH_3$ | H | 1-0 | |
| $OSO_2CH(CH_3)CHFCH_3$ | H | $CH_3$ | H | 1-0 | |
| $OSO_2CCl_3$ | 4-$OCH(CH_3)_2$ | $CH_3$ | H | 3-0 | |
| $OSO_2(CH_2)_3CHCl_2$ | H | $CH_3$ | H | 1-0 | |
| $OSO_2CH(CH_3)_2$ | H | $CH_3$ | H | 3-0 | |
| $CH_2Cl$ | 3-Br | $CH_3$ | H | 1-0 | |
| $CH_2Br$ | H | $CH_3$ | H | 3-0 | |
| $CH_2OCH_3$ | H | $C_2H_5$ | H | 1-0 | |
| $CH_2OCH(CH_3)C_2H_5$ | H | $CH_3$ | H | 1-0 | |
| $CH_2OCHCH{=}CH_2$ | 6-Cl | $CH_3$ | H | 1-0 | |
| $CH_2OCH(CH_3)CH{=}CH_2$ | H | $CH_3$ | H | 1-0 | |
| $CH_2SCH_2CH_3$ | H | $CH_3$ | H | 1-0 | |
| $CH_2S(O)C_2H_5$ | H | $CH_3$ | H | 3-0 | |
| $CH_2SO_2(CH_2)_3CH_3$ | H | $CH_3$ | H | 1-0 | |
| $CH_2S(O)CH_3$ | H | $CH_3$ | H | 1-0 | |
| $CH_2SO_2CH(CH_3)_2$ | H | $CH_3$ | H | 1-0 | |

Table V (continued)

| R | $R_1$ | $X_1$ | W | Q | m.p.(°C) |
|---|---|---|---|---|---|
| $SO_2N(CH_3)_2$ | H | $CH_3$ | H | 1-O | |
| $SO_2N(CH(CH_3)C_2H_5)CH_3$ | 3-$CF_3$ | $CH_3$ | H | 1-O | |
| $SO_2N(CH(CH_3)_2)CH_3$ | 5-$NO_2$ | $C_2H_5$ | H | 1-O | |
| $SO_2N(CH_3)C_2H_5$ | H | $C_2H_5$ | $CH_3$ | 1-O | |
| $SO_2N(C_2H_5)_2$ | 4-Br | $CH_3$ | H | 3-O | |
| $S(O)CH_2CF_3$ | H | $OCH_3$ | H | 3-O | |
| $OSO_2CF_3$ | 3-$OC(CH_3)_3$ | $OCH_3$ | H | 3-O | |
| $OSO_2CH_2CH_3$ | H | $OCH_3$ | H | 3-O | |
| $OSO_2CHCl_2$ | H | $OCH_3$ | H | 1-O | |
| $OSO_2(CH_2)_3Br$ | 3-$OC_2H_5$ | $OCH_3$ | H | 3-O | |
| $OSO_2CH(CH_3)CHFCH_3$ | H | $OCH_3$ | H | 1-O | |
| $OSO_2CCl_3$ | 4-$OCH(CH_3)_2$ | $OCH_3$ | H | 3-O | |
| $OSO_2(CH_2)_3CHCl_2$ | H | $OCH_3$ | H | 1-O | |
| $OSO_2CH(CH_3)_2$ | H | $OCH_3$ | H | 3-O | |
| $CH_2Cl$ | 3-Br | $OCH_3$ | H | 3-O | |
| $CH_2Br$ | H | $OCH_3$ | H | 3-O | |
| $CH_2OCH_3$ | H | $OC_2H_5$ | H | 3-O | |
| $CH_2OCH(CH_3)C_2H_5$ | H | $OCH_3$ | H | 1-O | |
| $CH_2OCHCH=CH_2$ | 6-Cl | $OCH_3$ | H | 3-O | |
| $CH_2OCH(CH_3)CH=CH_2$ | H | $OCH_3$ | H | 3-O | |
| $CH_2SCH_2CH_3$ | H | $OCH_3$ | H | 1-O | |
| $CH_2S(O)C_2H_5$ | H | $OCH_3$ | H | 3-O | |
| $CH_2SO_2(CH_2)_3CH_3$ | H | $OCH_3$ | H | 3-O | |
| $CH_2S(O)CH_3$ | H | $OCH_3$ | H | 3-O | |
| $CH_2SO_2CH(CH_3)_2$ | H | $OCH_3$ | H | 3-O | |
| $SO_2N(CH_3)_2$ | H | $OCH_3$ | H | 1-O | |

Table V (continued)

| R | $R_1$ | $X_1$ | W | Q | m.p.(°C) |
|---|---|---|---|---|---|
| $SO_2N(CH(CH_3)C_2H_5)CH_3$ | $3-CF_3$ | $OCH_3$ | H | 1-O | |
| $SO_2N(CH(CH_3)_2)CH_3$ | $5-NO_2$ | $OC_2H_5$ | H | 3-O | |
| $SO_2N(CH_3)C_2H_5$ | H | $OC_2H_5$ | $CH_3$ | 1-O | |
| $SO_2N(C_2H_5)_2$ | 4-Br | $OCH_3$ | H | 1-O | |

R = $COR_4$

| $R_4$ | $R_1$ | $X_1$ | W | Q | m.p.(°C) |
|---|---|---|---|---|---|
| $OCH_3$ | H | $CH_3$ | H | 1-O | |
| $OCH_3$ | $5-CF_3$ | $CH_3$ | H | 1-O | |
| $OCH_3$ | H | $CH_3$ | $CH_3$ | 1-O | |
| $OCH_3$ | H | $C_2H_5$ | H | 3-O | |
| $OCH_3$ | 5-Cl | $CH_3$ | H | 3-O | |
| $OC_2H_5$ | H | $CH_3$ | H | 1-O | |
| $O(CH_2)_5CH_3$ | H | $C_2H_5$ | H | 1-O | |
| $OCH(CH_3)_2$ | H | $CH_3$ | H | 1-O | |
| $OCH(CH_3)C_2H_5$ | $3-CF_3$ | $CH_3$ | H | 1-O | |
| 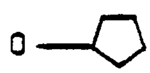 | H | $CH_3$ | H | 1-O | |
| 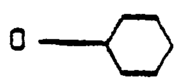 | H | $CH_3$ | H | 3-O | |
| $OCH_2CH{=}CH_2$ | H | $CH_3$ | H | 1-O | |
| $OCH(CH_3)CH{=}CH_2$ | H | $CH_3$ | H | 3-O | |
| $OCH_2C(CH_3){=}CH_2$ | H | $C_2H_5$ | H | 1-O | |
| $O(CH_2)_4CH{=}CH_2$ | H | $CH_3$ | H | 1-O | |
| $OCH_2C{\equiv}CH$ | H | $CH_3$ | H | 1-O | |
| $OCH_2C{\equiv}C(CH_2)_2CH_3$ | H | $CH_3$ | $CH_3$ | 1-O | |
| $OCH_2C{\equiv}CCH_3$ | $5-NO_2$ | $CH_3$ | H | 3-O | |

Table V (continued)

R = $COR_4$

| $R_4$ | $R_1$ | $X_1$ | W | Q | m.p.(°C) |
|---|---|---|---|---|---|
| $OCH(CH_3)C{=}CCH_3$ | 6-F | $CH_3$ | H | 1-0 | |
| $OCH_2CCl_3$ | H | $CH_3$ | H | 3-0 | |
| $OCH_2CH_2F$ | H | $C_2H_5$ | H | 1-0 | |
| $OCH_2CH_2CH_2Br$ | 3-Br | $CH_3$ | $CH_3$ | 3-0 | |
| $O(CH_2)_5CH_2Cl$ | H | $CH_3$ | H | 1-0 | |
| $OCH_2CHFCH_2F$ | H | $CH_3$ | H | 1-0 | |
| $OCH_3$ | H | $OCH_3$ | H | 1-0 | |
| $OCH_3$ | 5-$CF_3$ | $OCH_3$ | H | 3-0 | |
| $OCH_3$ | H | $OCH_3$ | $CH_3$ | 3-0 | |
| $OCH_3$ | H | $OC_2H_5$ | H | 1-0 | |
| $OCH_3$ | 5-Cl | $OCH_3$ | H | 3-0 | |
| $OC_2H_5$ | H | $OCH_3$ | H | 3-0 | |
| $O(CH_2)_5CH_3$ | H | $OC_2H_5$ | H | 1-0 | |
| $OCH(CH_3)_2$ | H | $OCH_3$ | H | 3-0 | |
| $OCH(CH_3)C_2H_5$ | 3-$CF_3$ | $OCH_3$ | H | 1-0 | |
| O 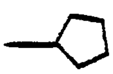 | H | $OCH_3$ | H | 3-0 | |
| O 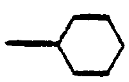 | H | $OCH_3$ | H | 3-0 | |
| $OCH_2CH{=}CH_2$ | H | $OCH_3$ | H | 3-0 | |
| $OCH(CH_3)CH{=}CH_2$ | H | $OCH_3$ | H | 3-0 | |
| $OCH_2C(CH_3){=}CH_2$ | H | $OC_2H_5$ | H | 1-0 | |
| $O(CH_2)_4CH{=}CH_2$ | H | $OCH_3$ | H | 3-0 | |
| $OCH_2C{\equiv}CH$ | H | $OCH_3$ | H | 1-0 | |
| $OCH_2C{\equiv}C(CH_2)_2CH_3$ | H | $OCH_3$ | $CH_3$ | 3-0 | |
| $OCH_2C{\equiv}CCH_3$ | 5-$NO_2$ | $OCH_3$ | H | 3-0 | |

Table V (continued)

R = $COR_4$

| $R_4$ | $R_1$ | $X_1$ | W | Q | m.p.(°C) |
|---|---|---|---|---|---|
| $OCH(CH_3)C{\equiv}CCH_3$ | 6-F | $OCH_3$ | H | 1-O | |
| $OCH_2CCl_3$ | H | $OCH_3$ | H | 1-O | |
| $OCH_2CH_2F$ | H | $OC_2H_5$ | H | 1-O | |
| $OCH_2CH_2CH_2Br$ | 3-Br | $OCH_3$ | $CH_3$ | 3-O | |
| $O(CH_2)_5CH_2Cl$ | H | $OCH_3$ | H | 1-O | |
| $OCH_2CHFCH_2F$ | H | $OCH_3$ | H | 1-O | |
| $OCH_2CHClCHClCH_3$ | 5-$OC_2H_5$ | $CH_3$ | H | 3-O | |
| $OCH_2CH_2Br$ | H | $C_2H_5$ | $CH_3$ | 1-O | |
| $OCH_2CH_2OCH_3$ | H | $CH_3$ | H | 3-O | |
| $(OCH_2CH_2)_2OC_2H_5$ | H | $CH_3$ | H | 1-O | |
| $OCH_2CH_2CH_2OCH_3$ | H | $CH_3$ | H | 1-O | |
| $OCH_2OCH_3$ | H | $CH_3$ | H | 1-O | |
| $OCH_2OCH_2CH(CH_3)_2$ | 3-$CH_3$ | $CH_3$ | H | 1-O | |
| $OCH_2OCH_2CH_2OCH_3$ | H | $CH_3$ | H | 3-O | |
| $OCH_2CHClCHClCH_3$ | 5-$OC_2H_5$ | $OCH_3$ | H | 3-O | |
| $OCH_2CH_2Br$ | H | $OC_2H_5$ | $CH_3$ | 1-O | |
| $OCH_2CH_2OCH_3$ | H | $OCH_3$ | H | 1-O | |
| $(OCH_2CH_2)_2OC_2H_5$ | H | $OCH_3$ | H | 3-O | |
| $OCH_2CH_2CH_2OCH_3$ | H | $OCH_3$ | H | 3-O | |
| $OCH_2OCH_3$ | H | $OCH_3$ | H | 1-O | |
| $OCH_2OCH_2CH(CH_3)_2$ | 3-$CH_3$ | $OCH_3$ | H | 1-O | |
| $OCH_2OCH_2CH_2OCH_3$ | H | $OCH_3$ | H | 1-O | |

Table VI

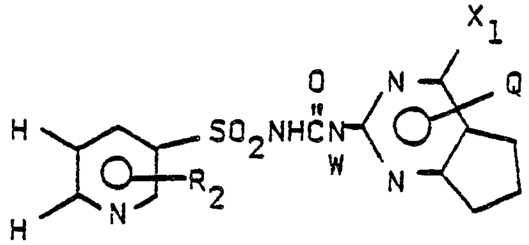

| $R_2$ | $X_1$ | W | Q | m.p.(°C) |
|---|---|---|---|---|
| 2-Cl | $CH_3$ | H | 1-O | |
| 4-F | $CH_3$ | H | 1-O | |
| 2-Br | $C_2H_5$ | H | 1-O | |
| 4-$CH_3$ | $CH_3$ | $CH_3$ | 3-O | |
| 2-$CH_2CH(CH_3)_2$ | $CH_3$ | H | 3-O | |
| 2-$OCH_3$ | $CH_3$ | H | 1-O | |
| 4-$O(CH_2)_3CH_3$ | $CH_3$ | H | 1-O | |
| 2-$NO_2$ | $CH_3$ | H | 1-O | |
| 2-$CO_2CH_3$ | $CH_3$ | H | 3-O | |
| 2-$CO_2CH_3$ | H | H | 3-O | |
| 4-$CO_2CH_2CH_3$ | $CH_3$ | H | 1-O | |
| 2-$CO_2CH(CH_3)_2$ | $C_2H_5$ | H | 1-O | |
| 2-$CO_2(CH_2)_3CH_3$ | $CH_3$ | H | 1-O | |
| 2-$SCH_3$ | $CH_3$ | H | 1-O | |
| 4-$SCH(CH_3)_2$ | $CH_3$ | H | 3-O | |
| 2-$S(O)CH_3$ | $CH_3$ | H | 1-O | |
| 4-$S(O)CH_2CH_2CH_3$ | $CH_3$ | H | 1-O | |
| 2-$SO_2CH_3$ | $CH_3$ | H | 1-O | |
| 4-$SO_2C_2H_5$ | $CH_3$ | $CH_3$ | 3-O | |
| 2-$SO_2CH(CH_3)_2$ | $CH_3$ | H | 1-O | |
| 2-$CH_3$ | $CH_3$ | H | 1-O | |
| 2-$OCH_3$ | $CH_3$ | H | 1-O | |

Table VI (continued)

| $R_2$ | $X_1$ | W | Q | m.p.(°C) |
|---|---|---|---|---|
| 2-Cl | $OCH_3$ | H | 3-O | |
| 4-F | $OCH_3$ | H | 3-O | |
| 2-Br | $OC_2H_5$ | H | 1-O | |
| $4-CH_3$ | $OCH_3$ | $CH_3$ | 3-O | |
| $2-CH_2CH(CH_3)_2$ | $OCH_3$ | H | 1-O | |
| $2-OCH_3$ | $OCH_3$ | H | 1-O | |
| $4-O(CH_2)_3CH_3$ | $OCH_3$ | H | 1-O | |
| $2-NO_2$ | $OCH_3$ | H | 3-O | |
| $2-CO_2CH_3$ | $OCH_3$ | H | 3-O | |
| $2-CO_2CH_3$ | Cl | H | 1-O | |
| $4-CO_2CH_2CH_3$ | $OCH_3$ | H | 3-O | |
| $2-CO_2CH(CH_3)_2$ | $OC_2H_5$ | H | 3-O | |
| $2-CO_2(CH_2)_3CH_3$ | $OCH_3$ | H | 1-O | |
| $2-SCH_3$ | $OCH_3$ | H | 1-O | |
| $4-SCH(CH_3)_2$ | $OCH_3$ | H | 3-O | |
| $2-S(O)CH_3$ | $OCH_3$ | H | 1-O | |
| $4-S(O)CH_2CH_2CH_3$ | $OCH_3$ | H | 3-O | |
| $2-SO_2CH_3$ | $OCH_3$ | H | 1-O | |
| $4-SO_2C_2H_5$ | $OCH_3$ | $CH_3$ | 1-O | |
| $2-SO_2CH(CH_3)_2$ | $OCH_3$ | H | 3-O | |
| $2-CH_3$ | $OCH_3$ | H | 1-O | |
| $2-OCH_3$ | $OCH_3$ | H | 3-O | |

Table VIIa

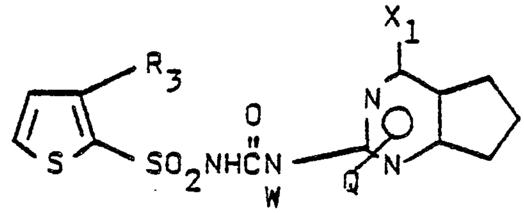

| $R_3$ | $X_1$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 3-O | |
| $CH(CH_3)C_2H_5$ | $CH_3$ | H | 1-O | |
| $OCH_3$ | $C_2H_5$ | H | 3-O | |
| $O(CH_2)_3CH_3$ | $CH_3$ | H | 1-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 1-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 3-O | |
| $SO_2N(OCH_3)CH_3$ | $CH_3$ | H | 1-O | |

$COR_4$

| $R_4$ | $X_1$ | W | Q |
|---|---|---|---|
| $OCH_3$ | $CH_3$ | H | 3-O |
| $OCH_3$ | $CH_3$ | $CH_3$ | 1-O |
| $OCH_3$ | $C_2H_5$ | H | 3-O |

Table VIIa (continued)

| $R_4$ | $X_1$ | W | Q | m.p.(°C) |
|---|---|---|---|---|
| $OCH_3$ | $CH_3$ | H | 1-0 | |
| $OCH_3$ | $C_2H_5$ | $CH_3$ | 3-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 1-0 | |
| $OCH(CH_3)C_2H_5$ | $CH_3$ | H | 3-0 | |
| 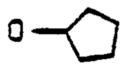 | $CH_3$ | H | 1-0 | |
| 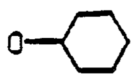 | $CH_3$ | H | 3-0 | |
| $OCH_2CH{=}CH_2$ | $CH_3$ | H | 1-0 | |
| $OCH(CH_3)CH{=}CH_2$ | $CH_3$ | H | 3-0 | |
| $OCH_2C(CH_3){=}CH_2$ | $CH_3$ | H | 1-0 | |
| $O(CH_2)_4CH{=}CH_2$ | $CH_3$ | H | 1-0 | |
| $OCH_2C{\equiv}CH$ | $CH_3$ | H | 3-0 | |
| $OCH_2C{\equiv}C(CH_2)_2CH_3$ | $CH_3$ | H | 3-0 | |
| $OCH_2C{\equiv}CCH_3$ | $CH_3$ | H | 1-0 | |
| $OCH(CH_3)C{\equiv}CCH_3$ | $CH_3$ | H | 1-0 | |
| $OCH_2CCl_3$ | $CH_3$ | H | 3-0 | |
| $OCH_2CH_2F$ | $C_2H_5$ | H | 3-0 | |
| $OCH_2CH_2CH_2Br$ | $CH_3$ | $CH_3$ | 1-0 | |
| $O(CH_2)_5CH_2Cl$ | $CH_3$ | H | 1-0 | |
| $OCH_2CHFCH_2F$ | $CH_3$ | H | 3-0 | |

Table VIIa (continued)

| $R_3$ | $X_1$ | W | Q | m.p.(°C) |
|---|---|---|---|---|
| H | $OCH_3$ | H | 3-O | |
| Cl | $OCH_3$ | H | 3-O | |
| $OC_2H_5$ | $OC_2H_5$ | H | 1-O | |
| $CH_3$ | $OCH_3$ | H | 3-O | |
| $CH(CH_3)C_2H_5$ | $OCH_3$ | H | 3-O | |
| $OCH_3$ | $OC_2H_5$ | H | 3-O | |
| $O(CH_2)_3CH_3$ | $OCH_3$ | H | 1-O | |
| $OCH(CH_3)_2$ | $OCH_3$ | H | 1-O | |
| $C_2H_5$ | $OCH_3$ | H | 3-O | |
| Br | $OCH_3$ | H | 3-O | |
| $NO_2$ | $OCH_3$ | H | 3-O | |
| $SO_2N(CH_3)_2$ | $OCH_3$ | H | 3-O | |
| $SO_2N(CH_3)_2$ | Cl | H | 1-O | |
| $SO_2N(CH(CH_3)C_2H_5)CH_3$ | $OCH_3$ | H | 3-O | |
| $SO_2N(CH(CH_3)_2)CH_3$ | $OC_2H_5$ | H | 3-O | |
| $SO_2N(CH_3)C_2H_5$ | $OC_2H_5$ | $CH_3$ | 1-O | |
| $SO_2N(C_2H_5)_2$ | $OCH_3$ | H | 1-O | |
| $SO_2N(OCH_3)CH_3$ | $OCH_3$ | H | 3-O | |

$COR_4$

| $R_4$ | $X_1$ | W | Q |
|---|---|---|---|
| $OCH_3$ | $OCH_3$ | H | 3-O |
| $OCH_3$ | $OCH_3$ | $CH_3$ | 3-O |
| $OCH_3$ | $OC_2H_5$ | H | 1-O |

Table VIIa (continued)

| R4 | X1 | W | Q | m.p.(°C) |
|---|---|---|---|---|
| $OCH_3$ | $OCH_3$ | H | 1-0 | |
| $OCH_3$ | $OC_2H_5$ | $CH_3$ | 3-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 1-0 | |
| $OCH(CH_3)C_2H_5$ | $OCH_3$ | H | 3-0 | |
| 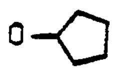 | $OCH_3$ | H | 3-0 | |
| 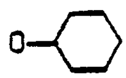 | $OCH_3$ | H | 1-0 | |
| $OCH_2CH{=}CH_2$ | $OCH_3$ | H | 1-0 | |
| $OCH(CH_3)CH{=}CH_2$ | $OCH_3$ | H | 3-0 | |
| $OCH_2C(CH_3){=}CH_2$ | $OCH_3$ | H | 3-0 | |
| $O(CH_2)_4CH{=}CH_2$ | $OCH_3$ | H | 1-0 | |
| $OCH_2C{\equiv}CH$ | $OCH_3$ | H | 3-0 | |
| $OCH_2C{\equiv}C(CH_2)_2CH_3$ | $OCH_3$ | H | 3-0 | |
| $OCH_2C{\equiv}CCH_3$ | $OCH_3$ | H | 1-0 | |
| $OCH(CH_3)C{\equiv}CCH_3$ | $OCH_3$ | H | 3-0 | |
| $OCH_2CCl_3$ | $OCH_3$ | H | 3-0 | |
| $OCH_2CH_2F$ | $OC_2H_5$ | H | 1-0 | |
| $OCH_2CH_2CH_2Br$ | $OCH_3$ | $CH_3$ | 1-0 | |
| $O(CH_2)_5CH_2Cl$ | $OCH_3$ | H | 3-0 | |
| $OCH_2CHFCH_2F$ | $OCH_3$ | H | 3-0 | |

Table VIIa (continued)

| $R_4$ | $X_1$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 3-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 1-0 | |
| $OCH_2OCH_3$ | $CH_3$ | H | 3-0 | |
| $OCH_2OCH_2CH(CH_3)_2$ | $CH_3$ | H | 3-0 | |
| $OCH_2OCH_2CH_2OCH_3$ | $CH_3$ | H | 1-0 | |
| $OCH_2CHClCHClCH_3$ | $OCH_3$ | H | 1-0 | |
| $OCH_2CH_2Br$ | $OC_2H_5$ | $CH_3$ | 3-0 | |
| $OCH_2CH_2OCH_3$ | $OCH_3$ | H | 1-0 | |
| $(OCH_2CH_2)_2OC_2H_5$ | $OCH_3$ | H | 3-0 | |
| $OCH_2CH_2CH_2OCH_3$ | $OCH_3$ | H | 3-0 | |
| $OCH_2OCH_3$ | $OCH_3$ | H | 1-0 | |
| $OCH_2OCH_2CH(CH_3)_2$ | $OCH_3$ | H | 3-0 | |
| $OCH_2OCH_2CH_2OCH_3$ | $OCH_3$ | H | 3-0 | |

Table VIIb

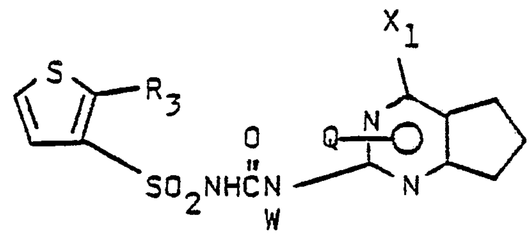

| $R_3$ | $X_1$ | W | Q | m.p.(°C) |
|---|---|---|---|---|
| H | $CH_3$ | H | 1-0 | |
| Cl | $CH_3$ | H | 1-0 | |
| $OC_2H_5$ | $C_2H_5$ | H | 1-0 | |
| $CH_3$ | $CH_3$ | H | 3-0 | |
| $CH(CH_3)C_2H_5$ | $CH_3$ | H | 1-0 | |
| $OCH_3$ | $C_2H_5$ | H | 3-0 | |
| $O(CH_2)_3CH_3$ | $CH_3$ | H | 1-0 | |
| $OCH(CH_3)_2$ | $CH_3$ | H | 1-0 | |
| $C_2H_5$ | $CH_3$ | H | 1-0 | |
| Br | $CH_3$ | H | 3-0 | |
| $NO_2$ | $CH_3$ | H | 1-0 | |
| $SO_2N(CH_3)_2$ | $CH_3$ | H | 1-0 | |
| $SO_2N(CH_3)_2$ | H | H | 1-0 | |
| $SO_2N(CH(CH_3)C_2H_5)CH_3$ | $CH_3$ | H | 1-0 | |
| $SO_2N(CH(CH_3)_2)CH_3$ | $C_2H_5$ | H | 1-0 | |
| $SO_2N(CH_3)C_2H_5$ | $C_2H_5$ | $CH_3$ | 1-0 | |
| $SO_2N(C_2H_5)_2$ | $CH_3$ | H | 3-0 | |
| $SO_2N(OCH_3)CH_3$ | $CH_3$ | H | 1-0 | |

$COR_4$

| $R_4$ | $X_1$ | W | Q |
|---|---|---|---|
| $OCH_3$ | $CH_3$ | H | 3-0 |
| $OCH_3$ | $CH_3$ | $CH_3$ | 1-0 |
| $OCH_3$ | $C_2H_5$ | H | 3-0 |

Table VIIb (continued)

| $R_4$ | $X_1$ | W | Q | m.p.(°C) |
|---|---|---|---|---|
| $OCH_3$ | $CH_3$ | H | 1-O | |
| $OCH_3$ | $C_2H_5$ | $CH_3$ | 3-O | |
| $OCH_2CH_3$ | $CH_3$ | H | 1-O | |
| $O(CH_2)_5CH_3$ | $CH_3$ | H | 1-O | |
| $OCH(CH_3)_2$ | $CH_3$ | H | 1-O | |
| $OCH(CH_3)C_2H_5$ | $CH_3$ | H | 3-O | |
| O 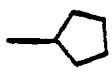 | $CH_3$ | H | 1-O | |
| O 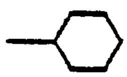 | $CH_3$ | H | 3-O | |
| $OCH_2CH{=}CH_2$ | $CH_3$ | H | 1-O | |
| $OCH(CH_3)CH{=}CH_2$ | $CH_3$ | H | 3-O | |
| $OCH_2C(CH_3){=}CH_2$ | $CH_3$ | H | 1-O | |
| $O(CH_2)_4CH{=}CH_2$ | $CH_3$ | H | 1-O | |
| $OCH_2C{\equiv}CH$ | $CH_3$ | H | 3-O | |
| $OCH_2C{\equiv}C(CH_2)_2CH_3$ | $CH_3$ | H | 3-O | |
| $OCH_2C{\equiv}CCH_3$ | $CH_3$ | H | 1-O | |
| $OCH(CH_3)C{\equiv}CCH_3$ | $CH_3$ | H | 1-O | |
| $OCH_2CCl_3$ | $CH_3$ | H | 3-O | |
| $OCH_2CH_2F$ | $C_2H_5$ | H | 3-O | |
| $OCH_2CH_2CH_2Br$ | $CH_3$ | $CH_3$ | 1-O | |
| $O(CH_2)_5CH_2Cl$ | $CH_3$ | H | 1-O | |
| $OCH_2CHFCH_2F$ | $CH_3$ | H | 3-O | |

Table VIIb (continued)

| $R_3$ | $X_1$ | W | Q | m.p.(°C) |
|---|---|---|---|---|
| H | $OCH_3$ | H | 3-O | |
| Cl | $OCH_3$ | H | 3-O | |
| $OC_2H_5$ | $OC_2H_5$ | H | 1-O | |
| $CH_3$ | $OCH_3$ | H | 3-O | |
| $CH(CH_3)C_2H_5$ | $OCH_3$ | H | 3-O | |
| $OCH_3$ | $OC_2H_5$ | H | 3-O | |
| $O(CH_2)_3CH_3$ | $OCH_3$ | H | 1-O | |
| $OCH(CH_3)_2$ | $OCH_3$ | H | 1-O | |
| $C_2H_5$ | $OCH_3$ | H | 3-O | |
| Br | $OCH_3$ | H | 3-O | |
| $NO_2$ | $OCH_3$ | H | 3-O | |
| $SO_2N(CH_3)_2$ | $OCH_3$ | H | 3-O | |
| $SO_2N(CH_3)_2$ | Cl | H | 1-O | |
| $SO_2N(CH(CH_3)C_2H_5)CH_3$ | $OCH_3$ | H | 3-O | |
| $SO_2N(CH(CH_3)_2)CH_3$ | $OC_2H_5$ | H | 3-O | |
| $SO_2N(CH_3)C_2H_5$ | $OC_2H_5$ | $CH_3$ | 1-O | |
| $SO_2N(C_2H_5)_2$ | $OCH_3$ | H | 1-O | |
| $SO_2N(OCH_3)CH_3$ | $OCH_3$ | H | 3-O | |

$COR_4$

| $R_4$ | $X_1$ | W | Q |
|---|---|---|---|
| $OCH_3$ | $OCH_3$ | H | 3-O |
| $OCH_3$ | $OCH_3$ | $CH_3$ | 3-O |
| $OCH_3$ | $OC_2H_5$ | H | 1-O |

Table VIIb (continued)

| $R_4$ | $X_1$ | W | Q | m.p.(°C) |
|---|---|---|---|---|
| $OCH_3$ | $OCH_3$ | H | 1-0 | |
| $OCH_3$ | $OC_2H_5$ | $CH_3$ | 3-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 1-0 | |
| $OCH(CH_3)C_2H_5$ | $OCH_3$ | H | 3-0 | |
| 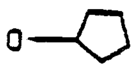 | $OCH_3$ | H | 3-0 | |
| 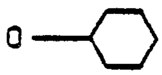 | $OCH_3$ | H | 1-0 | |
| $OCH_2CH=CH_2$ | $OCH_3$ | H | 1-0 | |
| $OCH(CH_3)CH=CH_2$ | $OCH_3$ | H | 3-0 | |
| $OCH_2C(CH_3)=CH_2$ | $OCH_3$ | H | 3-0 | |
| $O(CH_2)_4CH=CH_2$ | $OCH_3$ | H | 1-0 | |
| $OCH_2C\equiv CH$ | $OCH_3$ | H | 3-0 | |
| $OCH_2C\equiv C(CH_2)_2CH_3$ | $OCH_3$ | H | 3-0 | |
| $OCH_2C\equiv CCH_3$ | $OCH_3$ | H | 1-0 | |
| $OCH(CH_3)C\equiv CCH_3$ | $OCH_3$ | H | 3-0 | |
| $OCH_2CCl_3$ | $OCH_3$ | H | 3-0 | |
| $OCH_2CH_2F$ | $OC_2H_5$ | H | 1-0 | |
| $OCH_2CH_2CH_2Br$ | $OCH_3$ | $CH_3$ | 1-0 | |
| $O(CH_2)_5CH_2Cl$ | $OCH_3$ | H | 3-0 | |
| $OCH_2CHFCH_2F$ | $OCH_3$ | H | 3-0 | |

Table VIIb (continued)

| $R_4$ | $X_1$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 3-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 1-0 | |
| $OCH_2OCH_3$ | $CH_3$ | H | 3-0 | |
| $OCH_2OCH_2CH(CH_3)_2$ | $CH_3$ | H | 3-0 | |
| $OCH_2OCH_2CH_2OCH_3$ | $CH_3$ | H | 1-0 | |
| $OCH_2CHClCHClCH_3$ | $OCH_3$ | H | 1-0 | |
| $OCH_2CH_2Br$ | $OC_2H_5$ | $CH_3$ | 3-0 | |
| $OCH_2CH_2OCH_3$ | $OCH_3$ | H | 1-0 | |
| $(OCH_2CH_2)_2OC_2H_5$ | $OCH_3$ | H | 3-0 | |
| $OCH_2CH_2CH_2OCH_3$ | $OCH_3$ | H | 3-0 | |
| $OCH_2OCH_3$ | $OCH_3$ | H | 1-0 | |
| $OCH_2OCH_2CH(CH_3)_2$ | $OCH_3$ | H | 3-0 | |
| $OCH_2OCH_2CH_2OCH_3$ | $OCH_3$ | H | 3-0 | |

Table VIIc

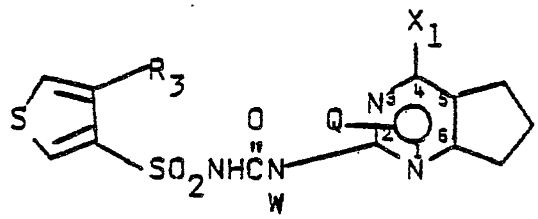

| $R_3$ | $X_1$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 3-O | |
| $CH(CH_3)C_2H_5$ | $CH_3$ | H | 1-O | |
| $OCH_3$ | $C_2H_5$ | H | 3-O | |
| $O(CH_2)_3CH_3$ | $CH_3$ | H | 1-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 1-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 3-O | |
| $SO_2N(OCH_3)CH_3$ | $CH_3$ | H | 1-O | |

$COR_4$

| $R_4$ | $X_1$ | W | Q |
|---|---|---|---|
| $OCH_3$ | $CH_3$ | H | 3-O |
| $OCH_3$ | $CH_3$ | $CH_3$ | 1-O |
| $OCH_3$ | $C_2H_5$ | H | 3-O |

Table VIIc (continued)

| $R_4$ | $X_1$ | W | Q | m.p.(°C) |
|---|---|---|---|---|
| $OCH_3$ | $CH_3$ | H | 1-0 | |
| $OCH_3$ | $C_2H_5$ | $CH_3$ | 3-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 1-0 | |
| $OCH(CH_3)C_2H_5$ | $CH_3$ | H | 3-0 | |
| 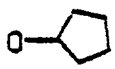 | $CH_3$ | H | 1-0 | |
| 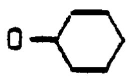 | $CH_3$ | H | 3-0 | |
| $OCH_2CH{=}CH_2$ | $CH_3$ | H | 1-0 | |
| $OCH(CH_3)CH{=}CH_2$ | $CH_3$ | H | 3-0 | |
| $OCH_2C(CH_3){=}CH_2$ | $CH_3$ | H | 1-0 | |
| $O(CH_2)_4CH{=}CH_2$ | $CH_3$ | H | 1-0 | |
| $OCH_2C{\equiv}CH$ | $CH_3$ | H | 3-0 | |
| $OCH_2C{\equiv}C(CH_2)_2CH_3$ | $CH_3$ | H | 3-0 | |
| $OCH_2C{\equiv}CCH_3$ | $CH_3$ | H | 1-0 | |
| $OCH(CH_3)C{\equiv}CCH_3$ | $CH_3$ | H | 1-0 | |
| $OCH_2CCl_3$ | $CH_3$ | H | 3-0 | |
| $OCH_2CH_2F$ | $C_2H_5$ | H | 3-0 | |
| $OCH_2CH_2CH_2Br$ | $CH_3$ | $CH_3$ | 1-0 | |
| $O(CH_2)_5CH_2Cl$ | $CH_3$ | H | 1-0 | |
| $OCH_2CHFCH_2F$ | $CH_3$ | H | 3-0 | |

Table VIIc (continued)

| $R_3$ | $X_1$ | W | Q | m.p.(°C) |
|---|---|---|---|---|
| H | $OCH_3$ | H | 3-0 | |
| Cl | $OCH_3$ | H | 3-0 | |
| $OC_2H_5$ | $OC_2H_5$ | H | 1-0 | |
| $CH_3$ | $OCH_3$ | H | 3-0 | |
| $CH(CH_3)C_2H_5$ | $OCH_3$ | H | 3-0 | |
| $OCH_3$ | $OC_2H_5$ | H | 3-0 | |
| $O(CH_2)_3CH_3$ | $OCH_3$ | H | 1-0 | |
| $OCH(CH_3)_2$ | $OCH_3$ | H | 1-0 | |
| $C_2H_5$ | $OCH_3$ | H | 3-0 | |
| Br | $OCH_3$ | H | 3-0 | |
| $NO_2$ | $OCH_3$ | H | 3-0 | |
| $SO_2N(CH_3)_2$ | $OCH_3$ | H | 3-0 | |
| $SO_2N(CH_3)_2$ | Cl | H | 1-0 | |
| $SO_2N(CH(CH_3)C_2H_5)CH_3$ | $OCH_3$ | H | 3-0 | |
| $SO_2N(CH(CH_3)_2)CH_3$ | $OC_2H_5$ | H | 3-0 | |
| $SO_2N(CH_3)C_2H_5$ | $OC_2H_5$ | $CH_3$ | 1-0 | |
| $SO_2N(C_2H_5)_2$ | $OCH_3$ | H | 1-0 | |
| $SO_2N(OCH_3)CH_3$ | $OCH_3$ | H | 3-0 | |

$COR_4$

| $R_4$ | $X_1$ | W | Q |
|---|---|---|---|
| $OCH_3$ | $OCH_3$ | H | 3-0 |
| $OCH_3$ | $OCH_3$ | $CH_3$ | 3-0 |
| $OCH_3$ | $OC_2H_5$ | H | 1-0 |

Table VIIc (continued)

| $R_4$ | $X_1$ | W | Q | m.p.(°C) |
|---|---|---|---|---|
| $OCH_3$ | $OCH_3$ | H | 1-0 | |
| $OCH_3$ | $OC_2H_5$ | $CH_3$ | 3-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 1-0 | |
| $OCH(CH_3)C_2H_5$ | $OCH_3$ | H | 3-0 | |
| 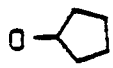 | $OCH_3$ | H | 3-0 | |
| 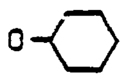 | $OCH_3$ | H | 1-0 | |
| $OCH_2CH{=}CH_2$ | $OCH_3$ | H | 1-0 | |
| $OCH(CH_3)CH{=}CH_2$ | $OCH_3$ | H | 3-0 | |
| $OCH_2C(CH_3){=}CH_2$ | $OCH_3$ | H | 3-0 | |
| $O(CH_2)_4CH{=}CH_2$ | $OCH_3$ | H | 1-0 | |
| $OCH_2C{\equiv}CH$ | $OCH_3$ | H | 3-0 | |
| $OCH_2C{\equiv}C(CH_2)_2CH_3$ | $OCH_3$ | H | 3-0 | |
| $OCH_2C{\equiv}CCH_3$ | $OCH_3$ | H | 1-0 | |
| $OCH(CH_3)C{\equiv}CCH_3$ | $OCH_3$ | H | 3-0 | |
| $OCH_2CCl_3$ | $OCH_3$ | H | 3-0 | |
| $OCH_2CH_2F$ | $OC_2H_5$ | H | 1-0 | |
| $OCH_2CH_2CH_2Br$ | $OCH_3$ | $CH_3$ | 1-0 | |
| $O(CH_2)_5CH_2Cl$ | $OCH_3$ | H | 3-0 | |
| $OCH_2CHFCH_2F$ | $OCH_3$ | H | 3-0 | |

Table VIIc (continued)

| $R_4$ | $X_1$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 3-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 1-0 | |
| $OCH_2OCH_3$ | $CH_3$ | H | 3-0 | |
| $OCH_2OCH_2CH(CH_3)_2$ | $CH_3$ | H | 3-0 | |
| $OCH_2OCH_2CH_2OCH_3$ | $CH_3$ | H | 1-0 | |
| $OCH_2CHClCHClCH_3$ | $OCH_3$ | H | 1-0 | |
| $OCH_2CH_2Br$ | $OC_2H_5$ | $CH_3$ | 3-0 | |
| $OCH_2CH_2OCH_3$ | $OCH_3$ | H | 1-0 | |
| $(OCH_2CH_2)_2OC_2H_5$ | $OCH_3$ | H | 3-0 | |
| $OCH_2CH_2CH_2OCH_3$ | $OCH_3$ | H | 3-0 | |
| $OCH_2OCH_3$ | $OCH_3$ | H | 1-0 | |
| $OCH_2OCH_2CH(CH_3)_2$ | $OCH_3$ | H | 3-0 | |
| $OCH_2OCH_2CH_2OCH_3$ | $OCH_3$ | H | 3-0 | |

Table VIII

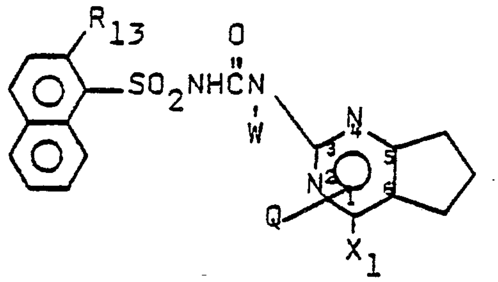

| $R_{13}$ | $X_1$ | W | Q | m.p.(°C) |
|---|---|---|---|---|
| H | $CH_3$ | H | 1-O | |
| $CH_3$ | $CH_3$ | H | 1-O | |
| $C_2H_5$ | $C_2H_5$ | H | 3-O | |
| $(CH_2)_4H$ | $CH_3$ | H | 1-O | |
| $OCH_3$ | $CH_3$ | H | 3-O | |
| $OC_2H_5$ | $CH_3$ | $CH_3$ | 1-O | |
| $O(CH_2)_4H$ | $CH_3$ | H | 3-O | |
| Cl | $CH_3$ | H | 1-O | |
| $NO_2$ | $CH_3$ | H | 3-O | |
| $SCH_3$ | $CH_3$ | H | 1-O | |
| $SC_2H_5$ | $CH_3$ | H | 1-O | |
| $SO_2N(CH_3)_2$ | $CH_3$ | H | 1-O | |
| $SO_2NCH(CH_3)C_2H_5$ | $CH_3$ | H | 3-O | |
| $SO_2N(C_2H_5)_2$ | $CH_3$ | H | 3-O | |
| $S(O)CH_3$ | $CH_3$ | H | 3-O | |
| $S(O)(CH_2)_4H$ | $CH_3$ | H | 1-O | |
| $SO_2CH_3$ | $CH_3$ | H | 1-O | |
| $SO_2(CH_2)_3H$ | $CH_3$ | H | 1-O | |
| $SO_2N(CH_3)_2$ | Cl | H | 3-O | |
| $SO_2CH_3$ | H | H | 3-O | |

Table VIII (continued)

| $R_{13}$ | $X_1$ | W | Q | m.p.(°C) |
|---|---|---|---|---|
| H | $OCH_3$ | H | 1-0 | |
| $CH_3$ | $OCH_3$ | H | 1-0 | |
| $C_2H_5$ | $OC_2H_5$ | H | 1-0 | |
| $(CH_2)_4H$ | $OCH_3$ | H | 3-0 | |
| $OCH_3$ | $OCH_3$ | H | 3-0 | |
| $OC_2H_5$ | $OCH_3$ | $CH_3$ | 3-0 | |
| $O(CH_2)_4H$ | $OCH_3$ | H | 1-0 | |
| Cl | $OCH_3$ | H | 3-0 | |
| $NO_2$ | $OCH_3$ | H | 1-0 | |
| $SCH_3$ | $OCH_3$ | H | 3-0 | |
| $SC_2H_5$ | $OCH_3$ | H | 1-0 | |
| $SO_2N(CH_3)_2$ | $OCH_3$ | H | 1-0 | |
| $SO_2NCH(CH_3)C_2H_5$ | $OCH_3$ | H | 3-0 | |
| $SO_2N(C_2H_5)_2$ | $OCH_3$ | H | 3-0 | |
| $S(O)CH_3$ | $OCH_3$ | H | 1-0 | |
| $S(O)(CH_2)_4H$ | $OCH_3$ | H | 3-0 | |
| $SO_2CH_3$ | $OCH_3$ | H | 3-0 | |
| $SO_2(CH_2)_3H$ | $OCH_3$ | H | 1-0 | |

Table IX

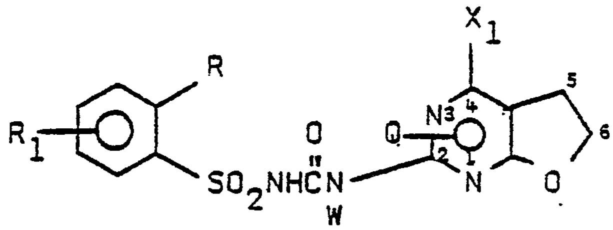

| R | $R_1$ | $X_1$ | W | Q | m.p.(°C) |
|---|---|---|---|---|---|
| H | H | $CH_3$ | H | 1-O | |
| Cl | H | $CH_3$ | H | 1-O | |
| $OC_2H_5$ | 5-$NO_2$ | $C_2H_5$ | H | 1-O | |
| $CF_3$ | H | $CH_3$ | H | 3-O | |
| $CH_3$ | H | $CH_3$ | H | 1-O | |
| $CH(CH_3)C_2H_5$ | H | $CH_3$ | $CH_3$ | 1-O | |
| $OCH_3$ | H | $C_2H_5$ | H | 3-O | |
| $O(CH_2)_3CH_3$ | H | $C_2H_5$ | H | 1-O | |
| $OCH(CH_3)_2$ | 6-Cl | $CH_3$ | H | 1-O | |
| $C_2H_5$ | 3-$CH_3$ | $CH_3$ | H | 1-O | |
| Br | H | $CH_3$ | $CH_3$ | 1-O | |
| $NO_2$ | H | $CH_3$ | H | 3-O | |
| $SCH_3$ | 5-$CH(CH_3)_2$ | $CH_3$ | H | 3-O | |
| $SC(CH_3)_3$ | H | $CH_3$ | H | 1-O | |
| $S(O)CH_3$ | H | $C_2H_5$ | H | 1-O | |
| $S(O)CH(CH_3)C_2H_5$ | 3-F | $CH_3$ | H | 1-O | |
| $SO_2CH_3$ | 5-$C_2H_5$ | $CH_3$ | H | 3-O | |
| $SO_2CH(CH_3)_2$ | 5-Br | $CH_3$ | H | 1-O | |
| $SO_2(CH_2)_3CH_3$ | 6-F | $CH_3$ | $CH_3$ | 3-O | |
| $SO_2CH_3$ | H | $CH_3$ | H | 1-O | |
| $SO_2CH_3$ | H | Cl | H | 1-O | |

Table IX (continued)

| R | $R_1$ | $X_1$ | W | Q | m.p.(°C) |
|---|---|---|---|---|---|
| $SO_2C_2H_5$ | H | $CH_3$ | H | 1-0 | |
| $SO_2OCH_2CF_3$ | H | $CH_3$ | H | 1-0 | |
| $SO_2OCH_2CCl_3$ | H | $CH_3$ | H | 3-0 | |
| $SO_2N(OCH_3)CH_3$ | H | $CH_3$ | H | 1-0 | |
| H | H | $OCH_3$ | H | 3-0 | |
| Cl | H | $OCH_3$ | H | 1-0 | |
| $OC_2H_5$ | 5-$NO_2$ | $OC_2H_5$ | H | 1-0 | |
| $CF_3$ | H | $OCH_3$ | H | 1-0 | |
| $CH_3$ | H | $OCH_3$ | H | 1-0 | |
| $CH(CH_3)C_2H_5$ | H | $OCH_3$ | $CH_3$ | 3-0 | |
| $OCH_3$ | H | $OC_2H_5$ | H | 3-0 | |
| $O(CH_2)_3CH_3$ | H | $OC_2H_5$ | H | 1-0 | |
| $OCH(CH_3)_2$ | 6-Cl | $OCH_3$ | H | 3-0 | |
| $C_2H_5$ | 3-$CH_3$ | $OCH_3$ | H | 3-0 | |
| Br | H | $OCH_3$ | $CH_3$ | 3-0 | |
| $NO_2$ | H | $OCH_3$ | H | 1-0 | |
| $SCH_3$ | 5-$CH(CH_3)_2$ | $OCH_3$ | H | 3-0 | |
| $SC(CH_3)_3$ | H | $OCH_3$ | H | 3-0 | |
| $S(O)CH_3$ | H | $OC_2H_5$ | H | 1-0 | |
| $S(O)CH(CH_3)C_2H_5$ | 3-F | $OCH_3$ | H | 1-0 | |
| $SO_2CH_3$ | 5-$C_2H_5$ | $OCH_3$ | H | 3-0 | |
| $SO_2CH(CH_3)_2$ | 5-Br | $OCH_3$ | H | 3-0 | |
| $SO_2(CH_2)_3CH_3$ | 6-F | $OCH_3$ | $CH_3$ | 1-0 | |
| $SO_2CH_3$ | H | $OCH_3$ | H | 1-0 | |

Table IX (continued)

| R | $R_1$ | $X_1$ | W | Q | m.p.(°C) |
|---|---|---|---|---|---|
| $SO_2CH_3$ | H | H | H | 1-O | |
| $SO_2C_2H_5$ | H | $OCH_3$ | H | 1-O | |
| $SO_2OCH_2CF_3$ | H | $OCH_3$ | H | 3-O | |
| $SO_2OCH_2CCl_3$ | H | $OCH_3$ | H | 3-O | |
| $SO_2N(OCH_3)CH_3$ | H | $OCH_3$ | H | 3-O | |
| $S(O)CH_2CF_3$ | H | $CH_3$ | H | 3-O | |
| $OSO_2CF_3$ | 3-$OC(CH_3)_3$ | $CH_3$ | H | 1-O | |
| $OSO_2CH_2CH_3$ | H | $CH_3$ | H | 1-O | |
| $OSO_2CHCl_2$ | H | $CH_3$ | H | 3-O | |
| $OSO_2(CH_2)_3Br$ | 3-$OC_2H_5$ | $CH_3$ | H | 1-O | |
| $OSO_2CH(CH_3)CHFCH_3$ | H | $CH_3$ | H | 1-O | |
| $OSO_2CCl_3$ | 4-$OCH(CH_3)_2$ | $CH_3$ | H | 3-O | |
| $OSO_2(CH_2)_3CHCl_2$ | H | $CH_3$ | H | 1-O | |
| $OSO_2CH(CH_3)_2$ | H | $CH_3$ | H | 3-O | |
| $CH_2Cl$ | 3-Br | $CH_3$ | H | 1-O | |
| $CH_2Br$ | H | $CH_3$ | H | 3-O | |
| $CH_2OCH_3$ | H | $C_2H_5$ | H | 1-O | |
| $CH_2OCH(CH_3)C_2H_5$ | H | $CH_3$ | H | 1-O | |
| $CH_2OCHCH=CH_2$ | 6-Cl | $CH_3$ | H | 1-O | |
| $CH_2OCH(CH_3)CH=CH_2$ | H | $CH_3$ | H | 1-O | |
| $CH_2SCH_2CH_3$ | H | $CH_3$ | H | 1-O | |
| $CH_2S(O)C_2H_5$ | H | $CH_3$ | H | 3-O | |
| $CH_2SO_2(CH_2)_3CH_3$ | H | $CH_3$ | H | 1-O | |
| $CH_2S(O)CH_3$ | H | $CH_3$ | H | 1-O | |
| $CH_2SO_2CH(CH_3)_2$ | H | $CH_3$ | H | 1-O | |

Table IX (continued)

| R | $R_1$ | $X_1$ | W | Q | m.p.(°C) |
|---|---|---|---|---|---|
| $SO_2N(CH_3)_2$ | H | $CH_3$ | H | 1-0 | |
| $SO_2N(CH(CH_3)C_2H_5)CH_3$ | $3-CF_3$ | $CH_3$ | H | 1-0 | |
| $SO_2N(CH(CH_3)_2)CH_3$ | $5-NO_2$ | $C_2H_5$ | H | 1-0 | |
| $SO_2N(CH_3)C_2H_5$ | H | $C_2H_5$ | $CH_3$ | 1-0 | |
| $SO_2N(C_2H_5)_2$ | 4-Br | $CH_3$ | H | 3-0 | |
| $S(O)CH_2CF_3$ | H | $OCH_3$ | H | 3-0 | |
| $OSO_2CF_3$ | $3-OC(CH_3)_3$ | $OCH_3$ | H | 3-0 | |
| $OSO_2CH_2CH_3$ | H | $OCH_3$ | H | 3-0 | |
| $OSO_2CHCl_2$ | H | $OCH_3$ | H | 1-0 | |
| $OSO_2(CH_2)_3Br$ | $3-OC_2H_5$ | $OCH_3$ | H | 3-0 | |
| $OSO_2CH(CH_3)CHFCH_3$ | H | $OCH_3$ | H | 1-0 | |
| $OSO_2CCl_3$ | $4-OCH(CH_3)_2$ | $OCH_3$ | H | 3-0 | |
| $OSO_2(CH_2)_3CHCl_2$ | H | $OCH_3$ | H | 1-0 | |
| $OSO_2CH(CH_3)_2$ | H | $OCH_3$ | H | 3-0 | |
| $CH_2Cl$ | 3-Br | $OCH_3$ | H | 3-0 | |
| $CH_2Br$ | H | $OCH_3$ | H | 3-0 | |
| $CH_2OCH_3$ | H | $OC_2H_5$ | H | 3-0 | |
| $CH_2OCH(CH_3)C_2H_5$ | H | $OCH_3$ | H | 1-0 | |
| $CH_2OCHCH{=}CH_2$ | 6-Cl | $OCH_3$ | H | 3-0 | |
| $CH_2OCH(CH_3)CH{=}CH_2$ | H | $OCH_3$ | H | 3-0 | |
| $CH_2SCH_2CH_3$ | H | $OCH_3$ | H | 1-0 | |
| $CH_2S(O)C_2H_5$ | H | $OCH_3$ | H | 3-0 | |
| $CH_2SO_2(CH_2)_3CH_3$ | H | $OCH_3$ | H | 3-0 | |
| $CH_2S(O)CH_3$ | H | $OCH_3$ | H | 3-0 | |
| $CH_2SO_2CH(CH_3)_2$ | H | $OCH_3$ | H | 3-0 | |
| $SO_2N(CH_3)_2$ | H | $OCH_3$ | H | 1-0 | |

Table IX (continued)

| R | $R_1$ | $X_1$ | W | Q | m.p.(°C) |
|---|---|---|---|---|---|
| $SO_2N(CH(CH_3)C_2H_5)CH_3$ | 3-$CF_3$ | $OCH_3$ | H | 1-O | |
| $SO_2N(CH(CH_3)_2)CH_3$ | 5-$NO_2$ | $OC_2H_5$ | H | 3-O | |
| $SO_2N(CH_3)C_2H_5$ | H | $OC_2H_5$ | $CH_3$ | 1-O | |
| $SO_2N(C_2H_5)_2$ | 4-Br | $OCH_3$ | H | 1-O | |

R = $COR_4$

| $R_4$ | $R_1$ | $X_1$ | W | Q | m.p.(°C) |
|---|---|---|---|---|---|
| $OCH_3$ | H | $CH_3$ | H | 1-O | |
| $OCH_3$ | 5-$CF_3$ | $CH_3$ | H | 1-O | |
| $OCH_3$ | H | $CH_3$ | $CH_3$ | 1-O | |
| $OCH_3$ | H | $C_2H_5$ | H | 3-O | |
| $OCH_3$ | 5-Cl | $CH_3$ | H | 3-O | |
| $OC_2H_5$ | H | $CH_3$ | H | 1-O | |
| $O(CH_2)_5CH_3$ | H | $C_2H_5$ | H | 1-O | |
| $OCH(CH_3)_2$ | H | $CH_3$ | H | 1-O | |
| $OCH(CH_3)C_2H_5$ | 3-$CF_3$ | $CH_3$ | H | 1-O | |
| 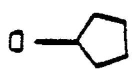 | H | $CH_3$ | H | 1-O | |
| 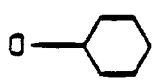 | H | $CH_3$ | H | 3-O | |
| $OCH_2CH{=}CH_2$ | H | $CH_3$ | H | 1-O | |
| $OCH(CH_3)CH{=}CH_2$ | H | $CH_3$ | H | 3-O | |
| $OCH_2C(CH_3){=}CH_2$ | H | $C_2H_5$ | H | 1-O | |
| $O(CH_2)_4CH{=}CH_2$ | H | $CH_3$ | H | 1-O | |
| $OCH_2C{\equiv}CH$ | H | $CH_3$ | H | 1-O | |
| $OCH_2C{\equiv}C(CH_2)_2CH_3$ | H | $CH_3$ | $CH_3$ | 1-O | |
| $OCH_2C{\equiv}CCH_3$ | 5-$NO_2$ | $CH_3$ | H | 3-O | |

Table IX (continued)

R = $COR_4$

| $R_4$ | $R_1$ | $X_1$ | W | Q | m.p.(°C) |
|---|---|---|---|---|---|
| $OCH(CH_3)C=CCH_3$ | 6-F | $CH_3$ | H | 1-0 | |
| $OCH_2CCl_3$ | H | $CH_3$ | H | 3-0 | |
| $OCH_2CH_2F$ | H | $C_2H_5$ | H | 1-0 | |
| $OCH_2CH_2CH_2Br$ | 3-Br | $CH_3$ | $CH_3$ | 3-0 | |
| $O(CH_2)_5CH_2Cl$ | H | $CH_3$ | H | 1-0 | |
| $OCH_2CHFCH_2F$ | H | $CH_3$ | H | 1-0 | |
| $OCH_3$ | H | $OCH_3$ | H | 1-0 | |
| $OCH_3$ | 5-$CF_3$ | $OCH_3$ | H | 3-0 | |
| $OCH_3$ | H | $OCH_3$ | $CH_3$ | 3-0 | |
| $OCH_3$ | H | $OC_2H_5$ | H | 1-0 | |
| $OCH_3$ | 5-Cl | $OCH_3$ | H | 3-0 | |
| $OC_2H_5$ | H | $OCH_3$ | H | 3-0 | |
| $O(CH_2)_5CH_3$ | H | $OC_2H_5$ | H | 1-0 | |
| $OCH(CH_3)_2$ | H | $OCH_3$ | H | 3-0 | |
| $OCH(CH_3)C_2H_5$ | 3-$CF_3$ | $OCH_3$ | H | 1-0 | |
| O 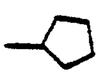 | H | $OCH_3$ | H | 3-0 | |
| O 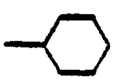 | H | $OCH_3$ | H | 3-0 | |
| $OCH_2CH=CH_2$ | H | $OCH_3$ | H | 3-0 | |
| $OCH(CH_3)CH=CH_2$ | H | $OCH_3$ | H | 3-0 | |
| $OCH_2C(CH_3)=CH_2$ | H | $OC_2H_5$ | H | 1-0 | |
| $O(CH_2)_4CH=CH_2$ | H | $OCH_3$ | H | 3-0 | |
| $OCH_2C=CH$ | H | $OCH_3$ | H | 1-0 | |
| $OCH_2C=C(CH_2)_2CH_3$ | H | $OCH_3$ | $CH_3$ | 3-0 | |
| $OCH_2C=CCH_3$ | 5-$NO_2$ | $OCH_3$ | H | 3-0 | |

Table IX (continued)

R = $COR_4$

| $R_4$ | $R_1$ | $X_1$ | W | Q | m.p.(°C) |
|---|---|---|---|---|---|
| $OCH(CH_3)C{\equiv}CCH_3$ | 6-F | $OCH_3$ | H | 1-O | |
| $OCH_2CCl_3$ | H | $OCH_3$ | H | 1-O | |
| $OCH_2CH_2F$ | H | $OC_2H_5$ | H | 1-O | |
| $OCH_2CH_2CH_2Br$ | 3-Br | $OCH_3$ | $CH_3$ | 3-O | |
| $O(CH_2)_5CH_2Cl$ | H | $OCH_3$ | H | 1-O | |
| $OCH_2CHFCH_2F$ | H | $OCH_3$ | H | 1-O | |
| $OCH_2CHClCHClCH_3$ | 5-$OC_2H_5$ | $CH_3$ | H | 3-O | |
| $OCH_2CH_2Br$ | H | $C_2H_5$ | $CH_3$ | 1-O | |
| $OCH_2CH_2OCH_3$ | H | $CH_3$ | H | 3-O | |
| $(OCH_2CH_2)_2OC_2H_5$ | H | $CH_3$ | H | 1-O | |
| $OCH_2CH_2CH_2OCH_3$ | H | $CH_3$ | H | 1-O | |
| $OCH_2OCH_3$ | H | $CH_3$ | H | 1-O | |
| $OCH_2OCH_2CH(CH_3)_2$ | 3-$CH_3$ | $CH_3$ | H | 1-O | |
| $OCH_2OCH_2CH_2OCH_3$ | H | $CH_3$ | H | 3-O | |
| $OCH_2CHClCHClCH_3$ | 5-$OC_2H_5$ | $OCH_3$ | H | 3-O | |
| $OCH_2CH_2Br$ | H | $OC_2H_5$ | $CH_3$ | 1-O | |
| $OCH_2CH_2OCH_3$ | H | $OCH_3$ | H | 1-O | |
| $(OCH_2CH_2)_2OC_2H_5$ | H | $OCH_3$ | H | 3-O | |
| $OCH_2CH_2CH_2OCH_3$ | H | $OCH_3$ | H | 3-O | |
| $OCH_2OCH_3$ | H | $OCH_3$ | H | 1-O | |
| $OCH_2OCH_2CH(CH_3)_2$ | 3-$CH_3$ | $OCH_3$ | H | 1-O | |
| $OCH_2OCH_2CH_2OCH_3$ | H | $OCH_3$ | H | 1-O | |

Table X

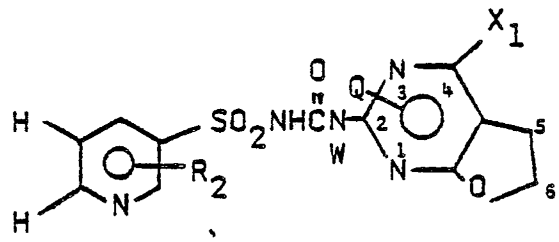

| $R_2$ | $X_1$ | W | Q | m.p.(°C) |
|---|---|---|---|---|
| 2-Cl | $CH_3$ | H | 1-O | |
| 4-F | $CH_3$ | H | 1-O | |
| 2-Br | $C_2H_5$ | H | 1-O | |
| 4-$CH_3$ | $CH_3$ | $CH_3$ | 3-O | |
| 2-$CH_2CH(CH_3)_2$ | $CH_3$ | H | 3-O | |
| 2-$OCH_3$ | $CH_3$ | H | 1-O | |
| 4-$O(CH_2)_3CH_3$ | $CH_3$ | H | 1-O | |
| 2-$NO_2$ | $CH_3$ | H | 1-O | |
| 2-$CO_2CH_3$ | $CH_3$ | H | 3-O | |
| 2-$CO_2CH_3$ | H | H | 3-O | |
| 4-$CO_2CH_2CH_3$ | $CH_3$ | H | 1-O | |
| 2-$CO_2CH(CH_3)_2$ | $C_2H_5$ | H | 1-O | |
| 2-$CO_2(CH_2)_3CH_3$ | $CH_3$ | H | 1-O | |
| 2-$SCH_3$ | $CH_3$ | H | 1-O | |
| 4-$SCH(CH_3)_2$ | $CH_3$ | H | 3-O | |
| 2-$S(O)CH_3$ | $CH_3$ | H | 1-O | |
| 4-$S(O)CH_2CH_2CH_3$ | $CH_3$ | H | 1-O | |
| 2-$SO_2CH_3$ | $CH_3$ | H | 1-O | |
| 4-$SO_2C_2H_5$ | $CH_3$ | $CH_3$ | 3-O | |
| 2-$SO_2CH(CH_3)_2$ | $CH_3$ | H | 1-O | |
| 2-$CH_3$ | $CH_3$ | H | 1-O | |
| 2-$OCH_3$ | $CH_3$ | H | 1-O | |

Table X (continued)

| $R_2$ | $X_1$ | W | Q | m.p.(°C) |
|---|---|---|---|---|
| 2-Cl | $OCH_3$ | H | 3-O | |
| 4-F | $OCH_3$ | H | 3-O | |
| 2-Br | $OC_2H_5$ | H | 1-O | |
| $4\text{-}CH_3$ | $OCH_3$ | $CH_3$ | 3-O | |
| $2\text{-}CH_2CH(CH_3)_2$ | $OCH_3$ | H | 1-O | |
| $2\text{-}OCH_3$ | $OCH_3$ | H | 1-O | |
| $4\text{-}O(CH_2)_3CH_3$ | $OCH_3$ | H | 1-O | |
| $2\text{-}NO_2$ | $OCH_3$ | H | 3-O | |
| $2\text{-}CO_2CH_3$ | $OCH_3$ | H | 3-O | |
| $2\text{-}CO_2CH_3$ | Cl | H | 1-O | |
| $4\text{-}CO_2CH_2CH_3$ | $OCH_3$ | H | 3-O | |
| $2\text{-}CO_2CH(CH_3)_2$ | $OC_2H_5$ | H | 3-O | |
| $2\text{-}CO_2(CH_2)_3CH_3$ | $OCH_3$ | H | 1-O | |
| $2\text{-}SCH_3$ | $OCH_3$ | H | 1-O | |
| $4\text{-}SCH(CH_3)_2$ | $OCH_3$ | H | 3-O | |
| $2\text{-}S(O)CH_3$ | $OCH_3$ | H | 1-O | |
| $4\text{-}S(O)CH_2CH_2CH_3$ | $OCH_3$ | H | 3-O | |
| $2\text{-}SO_2CH_3$ | $OCH_3$ | H | 1-O | |
| $4\text{-}SO_2C_2H_5$ | $OCH_3$ | $CH_3$ | 1-O | |
| $2\text{-}SO_2CH(CH_3)_2$ | $OCH_3$ | H | 3-O | |
| $2\text{-}CH_3$ | $OCH_3$ | H | 1-O | |
| $2\text{-}OCH_3$ | $OCH_3$ | H | 3-O | |

Table XIa

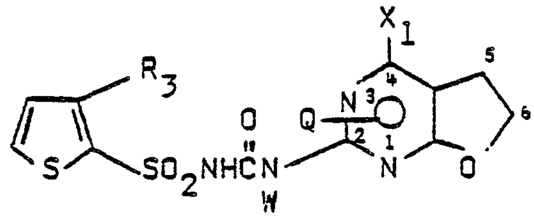

| $R_3$ | $X_1$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 3-O | |
| $CH(CH_3)C_2H_5$ | $CH_3$ | H | 1-O | |
| $OCH_3$ | $C_2H_5$ | H | 3-O | |
| $O(CH_2)_3CH_3$ | $CH_3$ | H | 1-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 1-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 3-O | |
| $SO_2N(OCH_3)CH_3$ | $CH_3$ | H | 1-O | |

$COR_4$

| $R_4$ | $X_1$ | W | Q |
|---|---|---|---|
| $OCH_3$ | $CH_3$ | H | 3-O |
| $OCH_3$ | $CH_3$ | $CH_3$ | 1-O |
| $OCH_3$ | $C_2H_5$ | H | 3-O |

Table XIa (continued)

| $R_4$ | $X_1$ | W | Q | m.p.(°C) |
|---|---|---|---|---|
| $OCH_3$ | $CH_3$ | H | 1-0 | |
| $OCH_3$ | $C_2H_5$ | $CH_3$ | 3-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 1-0 | |
| $OCH(CH_3)C_2H_5$ | $CH_3$ | H | 3-0 | |
| O 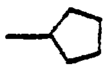 | $CH_3$ | H | 1-0 | |
| O 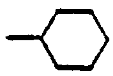 | $CH_3$ | H | 3-0 | |
| $OCH_2CH{=}CH_2$ | $CH_3$ | H | 1-0 | |
| $OCH(CH_3)CH{=}CH_2$ | $CH_3$ | H | 3-0 | |
| $OCH_2C(CH_3){=}CH_2$ | $CH_3$ | H | 1-0 | |
| $O(CH_2)_4CH{=}CH_2$ | $CH_3$ | H | 1-0 | |
| $OCH_2C{\equiv}CH$ | $CH_3$ | H | 3-0 | |
| $OCH_2C{\equiv}C(CH_2)_2CH_3$ | $CH_3$ | H | 3-0 | |
| $OCH_2C{\equiv}CCH_3$ | $CH_3$ | H | 1-0 | |
| $OCH(CH_3)C{\equiv}CCH_3$ | $CH_3$ | H | 1-0 | |
| $OCH_2CCl_3$ | $CH_3$ | H | 3-0 | |
| $OCH_2CH_2F$ | $C_2H_5$ | H | 3-0 | |
| $OCH_2CH_2CH_2Br$ | $CH_3$ | $CH_3$ | 1-0 | |
| $O(CH_2)_5CH_2Cl$ | $CH_3$ | H | 1-0 | |
| $OCH_2CHFCH_2F$ | $CH_3$ | H | 3-0 | |

Table XIa (continued)

| $R_3$ | $X_1$ | W | Q | m.p.(°C) |
|---|---|---|---|---|
| H | $OCH_3$ | H | 3-O | |
| Cl | $OCH_3$ | H | 3-O | |
| $OC_2H_5$ | $OC_2H_5$ | H | 1-O | |
| $CH_3$ | $OCH_3$ | H | 3-O | |
| $CH(CH_3)C_2H_5$ | $OCH_3$ | H | 3-O | |
| $OCH_3$ | $OC_2H_5$ | H | 3-O | |
| $O(CH_2)_3CH_3$ | $OCH_3$ | H | 1-O | |
| $OCH(CH_3)_2$ | $OCH_3$ | H | 1-O | |
| $C_2H_5$ | $OCH_3$ | H | 3-O | |
| Br | $OCH_3$ | H | 3-O | |
| $NO_2$ | $OCH_3$ | H | 3-O | |
| $SO_2N(CH_3)_2$ | $OCH_3$ | H | 3-O | |
| $SO_2N(CH_3)_2$ | Cl | H | 1-O | |
| $SO_2N(CH(CH_3)C_2H_5)CH_3$ | $OCH_3$ | H | 3-O | |
| $SO_2N(CH(CH_3)_2)CH_3$ | $OC_2H_5$ | H | 3-O | |
| $SO_2N(CH_3)C_2H_5$ | $OC_2H_5$ | $CH_3$ | 1-O | |
| $SO_2N(C_2H_5)_2$ | $OCH_3$ | H | 1-O | |
| $SO_2N(OCH_3)CH_3$ | $OCH_3$ | H | 3-O | |

$COR_4$

| $R_4$ | $X_1$ | W | Q |
|---|---|---|---|
| $OCH_3$ | $OCH_3$ | H | 3-O |
| $OCH_3$ | $OCH_3$ | $CH_3$ | 3-O |
| $OCH_3$ | $OC_2H_5$ | H | 1-O |

Table XIa (continued)

| $R_4$ | $X_1$ | W | Q | m.p.(°C) |
|---|---|---|---|---|
| $OCH_3$ | $OCH_3$ | H | 1-0 | |
| $OCH_3$ | $OC_2H_5$ | $CH_3$ | 3-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 1-0 | |
| $OCH(CH_3)C_2H_5$ | $OCH_3$ | H | 3-0 | |
| O 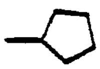 | $OCH_3$ | H | 3-0 | |
| O 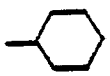 | $OCH_3$ | H | 1-0 | |
| $OCH_2CH=CH_2$ | $OCH_3$ | H | 1-0 | |
| $OCH(CH_3)CH=CH_2$ | $OCH_3$ | H | 3-0 | |
| $OCH_2C(CH_3)=CH_2$ | $OCH_3$ | H | 3-0 | |
| $O(CH_2)_4CH=CH_2$ | $OCH_3$ | H | 1-0 | |
| $OCH_2C\equiv CH$ | $OCH_3$ | H | 3-0 | |
| $OCH_2C\equiv C(CH_2)_2CH_3$ | $OCH_3$ | H | 3-0 | |
| $OCH_2C\equiv CCH_3$ | $OCH_3$ | H | 1-0 | |
| $OCH(CH_3)C\equiv CCH_3$ | $OCH_3$ | H | 3-0 | |
| $OCH_2CCl_3$ | $OCH_3$ | H | 3-0 | |
| $OCH_2CH_2F$ | $OC_2H_5$ | H | 1-0 | |
| $OCH_2CH_2CH_2Br$ | $OCH_3$ | $CH_3$ | 1-0 | |
| $O(CH_2)_5CH_2Cl$ | $OCH_3$ | H | 3-0 | |
| $OCH_2CHFCH_2F$ | $OCH_3$ | H | 3-0 | |

0057546

Table XIa (continued)

| $R_4$ | $X_1$ | W | Q | m.p.(°C) |
|---|---|---|---|---|
| $OCH_2CHClCHClCH_3$ | $CH_3$ | H | 1-O | |
| $OCH_2CH_2Br$ | $C_2H_5$ | $CH_3$ | 3-O | |
| $OCH_2CH_2OCH_3$ | $CH_3$ | H | 1-O | |
| $(OCH_2CH_2)_2OC_2H_5$ | $CH_3$ | H | 3-O | |
| $OCH_2CH_2CH_2OCH_3$ | $CH_3$ | H | 1-O | |
| $OCH_2OCH_3$ | $CH_3$ | H | 3-O | |
| $OCH_2OCH_2CH(CH_3)_2$ | $CH_3$ | H | 3-O | |
| $OCH_2OCH_2CH_2OCH_3$ | $CH_3$ | H | 1-O | |
| $OCH_2CHClCHClCH_3$ | $OCH_3$ | H | 1-O | |
| $OCH_2CH_2Br$ | $OC_2H_5$ | $CH_3$ | 3-O | |
| $OCH_2CH_2OCH_3$ | $OCH_3$ | H | 1-O | |
| $(OCH_2CH_2)_2OC_2H_5$ | $OCH_3$ | H | 3-O | |
| $OCH_2CH_2CH_2OCH_3$ | $OCH_3$ | H | 3-O | |
| $OCH_2OCH_3$ | $OCH_3$ | H | 1-O | |
| $OCH_2OCH_2CH(CH_3)_2$ | $OCH_3$ | H | 3-O | |
| $OCH_2OCH_2CH_2OCH_3$ | $OCH_3$ | H | 3-O | |

Table XIb

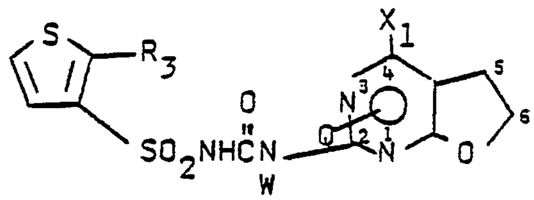

| $R_3$ | $X_1$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 3-O | |
| $CH(CH_3)C_2H_5$ | $CH_3$ | H | 1-O | |
| $OCH_3$ | $C_2H_5$ | H | 3-O | |
| $O(CH_2)_3CH_3$ | $CH_3$ | H | 1-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 1-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 3-O | |
| $SO_2N(OCH_3)CH_3$ | $CH_3$ | H | 1-O | |

$COR_4$

| $R_4$ | $X_1$ | W | Q |
|---|---|---|---|
| $OCH_3$ | $CH_3$ | H | 3-O |
| $OCH_3$ | $CH_3$ | $CH_3$ | 1-O |
| $OCH_3$ | $C_2H_5$ | H | 3-O |

Table XIb (continued)

| $R_4$ | $X_1$ | W | Q | m.p.(°C) |
|---|---|---|---|---|
| $OCH_3$ | $CH_3$ | H | 1-O | |
| $OCH_3$ | $C_2H_5$ | $CH_3$ | 3-O | |
| $OCH_2CH_3$ | $CH_3$ | H | 1-O | |
| $O(CH_2)_5CH_3$ | $CH_3$ | H | 1-O | |
| $OCH(CH_3)_2$ | $CH_3$ | H | 1-O | |
| $OCH(CH_3)C_2H_5$ | $CH_3$ | H | 3-O | |
| 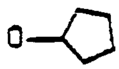 | $CH_3$ | H | 1-O | |
| 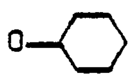 | $CH_3$ | H | 3-O | |
| $OCH_2CH{=}CH_2$ | $CH_3$ | H | 1-O | |
| $OCH(CH_3)CH{=}CH_2$ | $CH_3$ | H | 3-O | |
| $OCH_2C(CH_3){=}CH_2$ | $CH_3$ | H | 1-O | |
| $O(CH_2)_4CH{=}CH_2$ | $CH_3$ | H | 1-O | |
| $OCH_2C{\equiv}CH$ | $CH_3$ | H | 3-O | |
| $OCH_2C{\equiv}C(CH_2)_2CH_3$ | $CH_3$ | H | 3-O | |
| $OCH_2C{\equiv}CCH_3$ | $CH_3$ | H | 1-O | |
| $OCH(CH_3)C{\equiv}CCH_3$ | $CH_3$ | H | 1-O | |
| $OCH_2CCl_3$ | $CH_3$ | H | 3-O | |
| $OCH_2CH_2F$ | $C_2H_5$ | H | 3-O | |
| $OCH_2CH_2CH_2Br$ | $CH_3$ | $CH_3$ | 1-O | |
| $O(CH_2)_5CH_2Cl$ | $CH_3$ | H | 1-O | |
| $OCH_2CHFCH_2F$ | $CH_3$ | H | 3-O | |

Table XIb (continued)

| $R_3$ | $X_1$ | W | Q | m.p.(°C) |
|---|---|---|---|---|
| H | $OCH_3$ | H | 3-O | |
| Cl | $OCH_3$ | H | 3-O | |
| $OC_2H_5$ | $OC_2H_5$ | H | 1-O | |
| $CH_3$ | $OCH_3$ | H | 3-O | |
| $CH(CH_3)C_2H_5$ | $OCH_3$ | H | 3-O | |
| $OCH_3$ | $OC_2H_5$ | H | 3-O | |
| $O(CH_2)_3CH_3$ | $OCH_3$ | H | 1-O | |
| $OCH(CH_3)_2$ | $OCH_3$ | H | 1-O | |
| $C_2H_5$ | $OCH_3$ | H | 3-O | |
| Br | $OCH_3$ | H | 3-O | |
| $NO_2$ | $OCH_3$ | H | 3-O | |
| $SO_2N(CH_3)_2$ | $OCH_3$ | H | 3-O | |
| $SO_2N(CH_3)_2$ | Cl | H | 1-O | |
| $SO_2N(CH(CH_3)C_2H_5)CH_3$ | $OCH_3$ | H | 3-O | |
| $SO_2N(CH(CH_3)_2)CH_3$ | $OC_2H_5$ | H | 3-O | |
| $SO_2N(CH_3)C_2H_5$ | $OC_2H_5$ | $CH_3$ | 1-O | |
| $SO_2N(C_2H_5)_2$ | $OCH_3$ | H | 1-O | |
| $SO_2N(OCH_3)CH_3$ | $OCH_3$ | H | 3-O | |

$COR_4$

| $R_4$ | $X_1$ | W | Q |
|---|---|---|---|
| $OCH_3$ | $OCH_3$ | H | 3-O |
| $OCH_3$ | $OCH_3$ | $CH_3$ | 3-O |
| $OCH_3$ | $OC_2H_5$ | H | 1-O |

Table XIb (continued)

| $R_4$ | $X_1$ | W | Q | m.p.(°C) |
|---|---|---|---|---|
| $OCH_3$ | $OCH_3$ | H | 1-0 | |
| $OCH_3$ | $OC_2H_5$ | $CH_3$ | 3-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 1-0 | |
| $OCH(CH_3)C_2H_5$ | $OCH_3$ | H | 3-0 | |
| 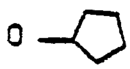 | $OCH_3$ | H | 3-0 | |
| 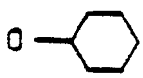 | $OCH_3$ | H | 1-0 | |
| $OCH_2CH{=}CH_2$ | $OCH_3$ | H | 1-0 | |
| $OCH(CH_3)CH{=}CH_2$ | $OCH_3$ | H | 3-0 | |
| $OCH_2C(CH_3){=}CH_2$ | $OCH_3$ | H | 3-0 | |
| $O(CH_2)_4CH{=}CH_2$ | $OCH_3$ | H | 1-0 | |
| $OCH_2C{\equiv}CH$ | $OCH_3$ | H | 3-0 | |
| $OCH_2C{\equiv}C(CH_2)_2CH_3$ | $OCH_3$ | H | 3-0 | |
| $OCH_2C{\equiv}CCH_3$ | $OCH_3$ | H | 1-0 | |
| $OCH(CH_3)C{\equiv}CCH_3$ | $OCH_3$ | H | 3-0 | |
| $OCH_2CCl_3$ | $OCH_3$ | H | 3-0 | |
| $OCH_2CH_2F$ | $OC_2H_5$ | H | 1-0 | |
| $OCH_2CH_2CH_2Br$ | $OCH_3$ | $CH_3$ | 1-0 | |
| $O(CH_2)_5CH_2Cl$ | $OCH_3$ | H | 3-0 | |
| $OCH_2CHFCH_2F$ | $OCH_3$ | H | 3-0 | |

Table XIb (continued)

| $R_4$ | $X_1$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 3-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 1-0 | |
| $OCH_2OCH_3$ | $CH_3$ | H | 3-0 | |
| $OCH_2OCH_2CH(CH_3)_2$ | $CH_3$ | H | 3-0 | |
| $OCH_2OCH_2CH_2OCH_3$ | $CH_3$ | H | 1-0 | |
| $OCH_2CHClCHClCH_3$ | $OCH_3$ | H | 1-0 | |
| $OCH_2CH_2Br$ | $OC_2H_5$ | $CH_3$ | 3-0 | |
| $OCH_2CH_2OCH_3$ | $OCH_3$ | H | 1-0 | |
| $(OCH_2CH_2)_2OC_2H_5$ | $OCH_3$ | H | 3-0 | |
| $OCH_2CH_2CH_2OCH_3$ | $OCH_3$ | H | 3-0 | |
| $OCH_2OCH_3$ | $OCH_3$ | H | 1-0 | |
| $OCH_2OCH_2CH(CH_3)_2$ | $OCH_3$ | H | 3-0 | |
| $OCH_2OCH_2CH_2OCH_3$ | $OCH_3$ | H | 3-0 | |

Table XIc

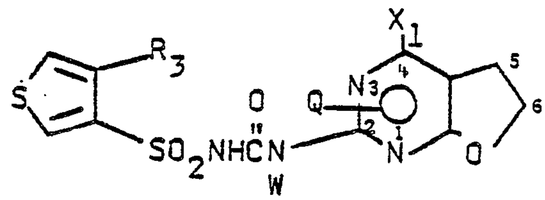

| $R_3$ | $X_1$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 3-O | |
| $CH(CH_3)C_2H_5$ | $CH_3$ | H | 1-O | |
| $OCH_3$ | $C_2H_5$ | H | 3-O | |
| $O(CH_2)_3CH_3$ | $CH_3$ | H | 1-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 1-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 3-O | |
| $SO_2N(OCH_3)CH_3$ | $CH_3$ | H | 1-O | |

$COR_4$

| $R_4$ | $X_1$ | W | Q |
|---|---|---|---|
| $OCH_3$ | $CH_3$ | H | 3-O |
| $OCH_3$ | $CH_3$ | $CH_3$ | 1-O |
| $OCH_3$ | $C_2H_5$ | H | 3-O |

Table XIc (continued)

| $R_4$ | $X_1$ | W | Q | m.p.(°C) |
|---|---|---|---|---|
| $OCH_3$ | $CH_3$ | H | 1-0 | |
| $OCH_3$ | $C_2H_5$ | $CH_3$ | 3-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 1-0 | |
| $OCH(CH_3)C_2H_5$ | $CH_3$ | H | 3-0 | |
| O 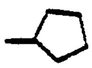 | $CH_3$ | H | 1-0 | |
| O 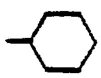 | $CH_3$ | H | 3-0 | |
| $OCH_2CH=CH_2$ | $CH_3$ | H | 1-0 | |
| $OCH(CH_3)CH=CH_2$ | $CH_3$ | H | 3-0 | |
| $OCH_2C(CH_3)=CH_2$ | $CH_3$ | H | 1-0 | |
| $O(CH_2)_4CH=CH_2$ | $CH_3$ | H | 1-0 | |
| $OCH_2C \equiv CH$ | $CH_3$ | H | 3-0 | |
| $OCH_2C \equiv C(CH_2)_2CH_3$ | $CH_3$ | H | 3-0 | |
| $OCH_2C \equiv CCH_3$ | $CH_3$ | H | 1-0 | |
| $OCH(CH_3)C \equiv CCH_3$ | $CH_3$ | H | 1-0 | |
| $OCH_2CCl_3$ | $CH_3$ | H | 3-0 | |
| $OCH_2CH_2F$ | $C_2H_5$ | H | 3-0 | |
| $OCH_2CH_2CH_2Br$ | $CH_3$ | $CH_3$ | 1-0 | |
| $O(CH_2)_5CH_2Cl$ | $CH_3$ | H | 1-0 | |
| $OCH_2CHFCH_2F$ | $CH_3$ | H | 3-0 | |

Table XIc (continued)

| $R_3$ | $X_1$ | W | Q | m.p.(°C) |
|---|---|---|---|---|
| H | $OCH_3$ | H | 3-O | |
| Cl | $OCH_3$ | H | 3-O | |
| $OC_2H_5$ | $OC_2H_5$ | H | 1-O | |
| $CH_3$ | $OCH_3$ | H | 3-O | |
| $CH(CH_3)C_2H_5$ | $OCH_3$ | H | 3-O | |
| $OCH_3$ | $OC_2H_5$ | H | 3-O | |
| $O(CH_2)_3CH_3$ | $OCH_3$ | H | 1-O | |
| $OCH(CH_3)_2$ | $OCH_3$ | H | 1-O | |
| $C_2H_5$ | $OCH_3$ | H | 3-O | |
| Br | $OCH_3$ | H | 3-O | |
| $NO_2$ | $OCH_3$ | H | 3-O | |
| $SO_2N(CH_3)_2$ | $OCH_3$ | H | 3-O | |
| $SO_2N(CH_3)_2$ | Cl | H | 1-O | |
| $SO_2N(CH(CH_3)C_2H_5)CH_3$ | $OCH_3$ | H | 3-O | |
| $SO_2N(CH(CH_3)_2)CH_3$ | $OC_2H_5$ | H | 3-O | |
| $SO_2N(CH_3)C_2H_5$ | $OC_2H_5$ | $CH_3$ | 1-O | |
| $SO_2N(C_2H_5)_2$ | $OCH_3$ | H | 1-O | |
| $SO_2N(OCH_3)CH_3$ | $OCH_3$ | H | 3-O | |

$COR_4$

| $R_4$ | $X_1$ | W | Q |
|---|---|---|---|
| $OCH_3$ | $OCH_3$ | H | 3-O |
| $OCH_3$ | $OCH_3$ | $CH_3$ | 3-O |
| $OCH_3$ | $OC_2H_5$ | H | 1-O |

Table XIc (continued)

| $R_4$ | $X_1$ | W | Q | m.p.(°C) |
|---|---|---|---|---|
| $OCH_3$ | $OCH_3$ | H | 1-0 | |
| $OCH_3$ | $OC_2H_5$ | $CH_3$ | 3-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 1-0 | |
| $OCH(CH_3)C_2H_5$ | $OCH_3$ | H | 3-0 | |
| O 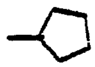 | $OCH_3$ | H | 3-0 | |
| O 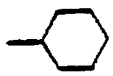 | $OCH_3$ | H | 1-0 | |
| $OCH_2CH{=}CH_2$ | $OCH_3$ | H | 1-0 | |
| $OCH(CH_3)CH{=}CH_2$ | $OCH_3$ | H | 3-0 | |
| $OCH_2C(CH_3){=}CH_2$ | $OCH_3$ | H | 3-0 | |
| $O(CH_2)_4CH{=}CH_2$ | $OCH_3$ | H | 1-0 | |
| $OCH_2C{\equiv}CH$ | $OCH_3$ | H | 3-0 | |
| $OCH_2C{\equiv}C(CH_2)_2CH_3$ | $OCH_3$ | H | 3-0 | |
| $OCH_2C{\equiv}CCH_3$ | $OCH_3$ | H | 1-0 | |
| $OCH(CH_3)C{\equiv}CCH_3$ | $OCH_3$ | H | 3-0 | |
| $OCH_2CCl_3$ | $OCH_3$ | H | 3-0 | |
| $OCH_2CH_2F$ | $OC_2H_5$ | H | 1-0 | |
| $OCH_2CH_2CH_2Br$ | $OCH_3$ | $CH_3$ | 1-0 | |
| $O(CH_2)_5CH_2Cl$ | $OCH_3$ | H | 3-0 | |
| $OCH_2CHFCH_2F$ | $OCH_3$ | H | 3-0 | |

Table XIc (continued)

| $R_4$ | $X_1$ | W | Q | m.p.(°C) |
|---|---|---|---|---|
| $OCH_2CHClCHClCH_3$ | $CH_3$ | H | 1-O | |
| $OCH_2CH_2Br$ | $C_2H_5$ | $CH_3$ | 3-O | |
| $OCH_2CH_2OCH_3$ | $CH_3$ | H | 1-O | |
| $(OCH_2CH_2)_2OC_2H_5$ | $CH_3$ | H | 3-O | |
| $OCH_2CH_2CH_2OCH_3$ | $CH_3$ | H | 1-O | |
| $OCH_2OCH_3$ | $CH_3$ | H | 3-O | |
| $OCH_2OCH_2CH(CH_3)_2$ | $CH_3$ | H | 3-O | |
| $OCH_2OCH_2CH_2OCH_3$ | $CH_3$ | H | 1-O | |
| $OCH_2CHClCHClCH_3$ | $OCH_3$ | H | 1-O | |
| $OCH_2CH_2Br$ | $OC_2H_5$ | $CH_3$ | 3-O | |
| $OCH_2CH_2OCH_3$ | $OCH_3$ | H | 1-O | |
| $(OCH_2CH_2)_2OC_2H_5$ | $OCH_3$ | H | 3-O | |
| $OCH_2CH_2CH_2OCH_3$ | $OCH_3$ | H | 3-O | |
| $OCH_2OCH_3$ | $OCH_3$ | H | 1-O | |
| $OCH_2OCH_2CH(CH_3)_2$ | $OCH_3$ | H | 3-O | |
| $OCH_2OCH_2CH_2OCH_3$ | $OCH_3$ | H | 3-O | |

Table XII

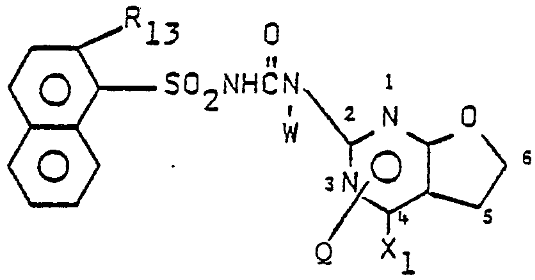

| $R_{13}$ | $X_1$ | W | Q | m.p.(°C) |
|---|---|---|---|---|
| H | $CH_3$ | H | 1-0 | |
| $CH_3$ | $CH_3$ | H | 1-0 | |
| $C_2H_5$ | $C_2H_5$ | H | 3-0 | |
| $(CH_2)_4H$ | $CH_3$ | H | 1-0 | |
| $OCH_3$ | $CH_3$ | H | 3-0 | |
| $OC_2H_5$ | $CH_3$ | $CH_3$ | 1-0 | |
| $O(CH_2)_4H$ | $CH_3$ | H | 1-0 | |
| Cl | $CH_3$ | H | 1-0 | |
| $NO_2$ | $CH_3$ | H | 1-0 | |
| $SCH_3$ | $CH_3$ | H | 1-0 | |
| $SC_2H_5$ | $CH_3$ | H | 1-0 | |
| $SO_2N(CH_3)_2$ | $CH_3$ | H | 1-0 | |
| $SO_2NCH(CH_3)C_2H_5$ | $CH_3$ | H | 1-0 | |
| $SO_2N(C_2H_5)_2$ | $CH_3$ | H | 1-0 | |
| $S(O)CH_3$ | $CH_3$ | H | 1-0 | |
| $S(O)(CH_2)_4H$ | $CH_3$ | H | 1-0 | |
| $SO_2CH_3$ | $CH_3$ | H | 1-0 | |
| $SO_2(CH_2)_3H$ | $CH_3$ | H | 1-0 | |
| $SO_2N(CH_3)_2$ | Cl | H | 1-0 | |
| $SO_2CH_3$ | H | H | 1-0 | |

Table XII (continued)

| $R_{13}$ | $X_1$ | W | Q | m.p.(°C) |
|---|---|---|---|---|
| H | $OCH_3$ | H | 3-O | |
| $CH_3$ | $OCH_3$ | H | 1-O | |
| $C_2H_5$ | $OC_2H_5$ | H | 3-O | |
| $(CH_2)_4H$ | $OCH_3$ | H | 1-O | |
| $OCH_3$ | $OCH_3$ | H | 1-O | |
| $OC_2H_5$ | $OCH_3$ | $CH_3$ | 3-O | |
| $O(CH_2)_4H$ | $OCH_3$ | H | 3-O | |
| Cl | $OCH_3$ | H | 1-O | |
| $NO_2$ | $OCH_3$ | H | 1-O | |
| $SCH_3$ | $OCH_3$ | H | 3-O | |
| $SC_2H_5$ | $OCH_3$ | H | 1-O | |
| $SO_2N(CH_3)_2$ | $OCH_3$ | H | 3-O | |
| $SO_2NCH(CH_3)C_2H_5$ | $OCH_3$ | H | 1-O | |
| $SO_2N(C_2H_5)_2$ | $OCH_3$ | H | 1-O | |
| $S(O)CH_3$ | $OCH_3$ | H | 1-O | |
| $S(O)(CH_2)_4H$ | $OCH_3$ | H | 3-O | |
| $SO_2CH_3$ | $OCH_3$ | H | 3-O | |
| $SO_2(CH_2)_3H$ | $OCH_3$ | H | 3-O | |

Table XIII

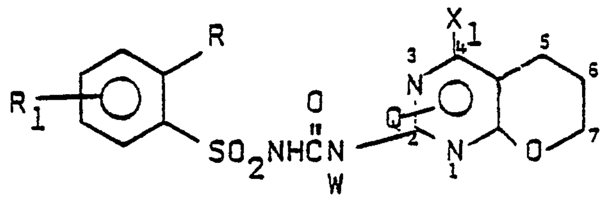

| R | $R_1$ | $X_1$ | W | Q | m.p.(°C) |
|---|---|---|---|---|---|
| H | H | $CH_3$ | H | 1-O | |
| Cl | H | $CH_3$ | H | 1-O | |
| $OC_2H_5$ | 5-$NO_2$ | $C_2H_5$ | H | 1-O | |
| $CF_3$ | H | $CH_3$ | H | 1-O | |
| $CH_3$ | H | $CH_3$ | H | 1-O | |
| $CH(CH_3)C_2H_5$ | H | $CH_3$ | $CH_3$ | 3-O | |
| $OCH_3$ | H | $C_2H_5$ | H | 1-O | |
| $O(CH_2)_3CH_3$ | H | $C_2H_5$ | H | 1-O | |
| $OCH(CH_3)_2$ | 6-Cl | $CH_3$ | H | 3-O | |
| $C_2H_5$ | 3-$CH_3$ | $CH_3$ | H | 1-O | |
| Br | H | $CH_3$ | $CH_3$ | 3-O | |
| $NO_2$ | H | $CH_3$ | H | 1-O | |
| $SCH_3$ | 5-$CH(CH_3)_2$ | $CH_3$ | H | 1-O | |
| $SC(CH_3)_3$ | H | $CH_3$ | H | 1-O | |
| $S(O)CH_3$ | H | $C_2H_5$ | H | 1-O | |
| $S(O)CH(CH_3)C_2H_5$ | 3-F | $CH_3$ | H | 1-O | |
| $SO_2CH_3$ | 5-$C_2H_5$ | $CH_3$ | H | 1-O | |
| $SO_2CH(CH_3)_2$ | 5-Br | $CH_3$ | H | 1-O | |
| $SO_2(CH_2)_3CH_3$ | 6-F | $CH_3$ | $CH_3$ | 3-O | |
| $SO_2CH_3$ | H | $CH_3$ | H | 1-O | |
| $SO_2CH_3$ | H | H | H | 3-O | |

Table XIII (continued)

| R | $R_1$ | $X_1$ | W | Q | m.p.(°C) |
|---|---|---|---|---|---|
| $SO_2C_2H_5$ | H | $CH_3$ | H | 1-O | |
| $SO_2OCH_2CF_3$ | H | $CH_3$ | H | 1-O | |
| $SO_2OCH_2CCl_3$ | H | $CH_3$ | H | 1-O | |
| $SO_2N(OCH_3)CH_3$ | H | $CH_3$ | H | 1-O | |
| H | H | $OCH_3$ | H | 3-O | |
| Cl | H | $OCH_3$ | H | 1-O | |
| $OC_2H_5$ | $5-NO_2$ | $OC_2H_5$ | H | 3-O | |
| $CF_3$ | H | $OCH_3$ | H | 1-O | |
| $CH_3$ | H | $OCH_3$ | H | 3-O | |
| $CH(CH_3)C_2H_5$ | H | $OCH_3$ | $CH_3$ | 3-O | |
| $OCH_3$ | H | $OC_2H_5$ | H | 1-O | |
| $O(CH_2)_3CH_3$ | H | $OC_2H_5$ | H | 1-O | |
| $OCH(CH_3)_2$ | 6-Cl | $OCH_3$ | H | 3-O | |
| $C_2H_5$ | $3-CH_3$ | $OCH_3$ | H | 3-O | |
| Br | H | $OCH_3$ | $CH_3$ | 1-O | |
| $NO_2$ | H | $OCH_3$ | H | 1-O | |
| $SCH_3$ | $5-CH(CH_3)_2$ | $OCH_3$ | H | 3-O | |
| $SC(CH_3)_3$ | H | $OCH_3$ | H | 1-O | |
| $S(O)CH_3$ | H | $OC_2H_5$ | H | 1-O | |
| $S(O)CH(CH_3)C_2H_5$ | 3-F | $OCH_3$ | H | 3-O | |
| $SO_2CH_3$ | $5-C_2H_5$ | $OCH_3$ | H | 1-O | |
| $SO_2CH(CH_3)_2$ | 5-Br | $OCH_3$ | H | 1-O | |
| $SO_2(CH_2)_3CH_3$ | 6-F | $OCH_3$ | $CH_3$ | 1-O | |
| $SO_2CH_3$ | H | $OCH_3$ | H | 3-O | |

Table XIII (continued)

| R | $R_1$ | $X_1$ | W | Q | m.p.(°C) |
|---|---|---|---|---|---|
| $SO_2CH_3$ | H | Cl | H | 1-0 | |
| $SO_2C_2H_5$ | H | $OCH_3$ | H | 3-0 | |
| $SO_2OCH_2CF_3$ | H | $OCH_3$ | H | 1-0 | |
| $SO_2OCH_2CCl_3$ | H | $OCH_3$ | H | 3-0 | |
| $SO_2N(OCH_3)CH_3$ | H | $OCH_3$ | H | 1-0 | |
| $S(O)CH_2CF_3$ | H | $CH_3$ | H | 1-0 | |
| $OSO_2CF_3$ | 3-$OC(CH_3)_3$ | $CH_3$ | H | 1-0 | |
| $OSO_2CH_2CH_3$ | H | $CH_3$ | H | 1-0 | |
| $OSO_2CHCl_2$ | H | $CH_3$ | H | 1-0 | |
| $OSO_2(CH_2)_3Br$ | 3-$OC_2H_5$ | $CH_3$ | H | 1-0 | |
| $OSO_2CH(CH_3)CHFCH_3$ | H | $CH_3$ | H | 1-0 | |
| $OSO_2CCl_3$ | 4-$OCH(CH_3)_2$ | $CH_3$ | H | 1-0 | |
| $OSO_2(CH_2)_3CHCl_2$ | H | $CH_3$ | H | 1-0 | |
| $OSO_2CH(CH_3)_2$ | H | $CH_3$ | H | 1-0 | |
| $CH_2Cl$ | 3-Br | $CH_3$ | H | 1-0 | |
| $CH_2Br$ | H | $CH_3$ | H | 1-0 | |
| $CH_2OCH_3$ | H | $C_2H_5$ | H | 1-0 | |
| $CH_2OCH(CH_3)C_2H_5$ | H | $CH_3$ | H | 1-0 | |
| $CH_2OCHCH{=}CH_2$ | 6-Cl | $CH_3$ | H | 3-0 | |
| $CH_2OCH(CH_3)CH{=}CH_2$ | H | $CH_3$ | H | 1-0 | |
| $CH_2SCH_2CH_3$ | H | $CH_3$ | H | 1-0 | |
| $CH_2S(O)C_2H_5$ | H | $CH_3$ | H | 3-0 | |
| $CH_2SO_2(CH_2)_3CH_3$ | H | $CH_3$ | H | 1-0 | |
| $CH_2S(O)CH_3$ | H | $CH_3$ | H | 1-0 | |
| $CH_2SO_2CH(CH_3)_2$ | H | $CH_3$ | H | 1-0 | |

Table XIII (continued)

| R | $R_1$ | $X_1$ | W | Q | m.p.(°C) |
|---|---|---|---|---|---|
| $SO_2N(CH_3)_2$ | H | $CH_3$ | H | 1-O | |
| $SO_2N(CH(CH_3)C_2H_5)CH_3$ | 3-$CF_3$ | $CH_3$ | H | 1-O | |
| $SO_2N(CH(CH_3)_2)CH_3$ | 5-$NO_2$ | $C_2H_5$ | H | 1-O | |
| $SO_2N(CH_3)C_2H_5$ | H | $C_2H_5$ | $CH_3$ | 1-O | |
| $SO_2N(C_2H_5)_2$ | 4-Br | $CH_3$ | H | 1-O | |
| $S(O)CH_2CF_3$ | H | $OCH_3$ | H | 3-O | |
| $OSO_2CF_3$ | 3-$OC(CH_3)_3$ | $OCH_3$ | H | 3-O | |
| $OSO_2CH_2CH_3$ | H | $OCH_3$ | H | 1-O | |
| $OSO_2CHCl_2$ | H | $OCH_3$ | H | 1-O | |
| $OSO_2(CH_2)_3Br$ | 3-$OC_2H_5$ | $OCH_3$ | H | 3-O | |
| $OSO_2CH(CH_3)CHFCH_3$ | H | $OCH_3$ | H | 3-O | |
| $OSO_2CCl_3$ | 4-$OCH(CH_3)_2$ | $OCH_3$ | H | 1-O | |
| $OSO_2(CH_2)_3CHCl_2$ | H | $OCH_3$ | H | 1-O | |
| $OSO_2CH(CH_3)_2$ | H | $OCH_3$ | H | 1-O | |
| $CH_2Cl$ | 3-Br | $OCH_3$ | H | 1-O | |
| $CH_2Br$ | H | $OCH_3$ | H | 3-O | |
| $CH_2OCH_3$ | H | $OC_2H_5$ | H | 3-O | |
| $CH_2OCH(CH_3)C_2H_5$ | H | $OCH_3$ | H | 3-O | |
| $CH_2OCHCH=CH_2$ | 6-Cl | $OCH_3$ | H | 1-O | |
| $CH_2OCH(CH_3)CH=CH_2$ | H | $OCH_3$ | H | 1-O | |
| $CH_2SCH_2CH_3$ | H | $OCH_3$ | H | 1-O | |
| $CH_2S(O)C_2H_5$ | H | $OCH_3$ | H | 3-O | |
| $CH_2SO_2(CH_2)_3CH_3$ | H | $OCH_3$ | H | 1-O | |
| $CH_2S(O)CH_3$ | H | $OCH_3$ | H | 3-O | |
| $CH_2SO_2CH(CH_3)_2$ | H | $OCH_3$ | H | 1-O | |
| $SO_2N(CH_3)_2$ | H | $OCH_3$ | H | 1-O | |

Table XIII (continued)

| R | $R_1$ | $X_1$ | W | Q | m.p.(°C) |
|---|---|---|---|---|---|
| $SO_2N(CH(CH_3)C_2H_5)CH_3$ | 3-$CF_3$ | $OCH_3$ | H | 3-O | |
| $SO_2N(CH(CH_3)_2)CH_3$ | 5-$NO_2$ | $OC_2H_5$ | H | 3-O | |
| $SO_2N(CH_3)C_2H_5$ | H | $OC_2H_5$ | $CH_3$ | 1-O | |
| $SO_2N(C_2H_5)_2$ | 4-Br | $OCH_3$ | H | 3-O | |

R = $COR_4$

| $R_4$ | $R_1$ | $X_1$ | W | Q | m.p.(°C) |
|---|---|---|---|---|---|
| $OCH_3$ | H | $CH_3$ | H | 1-O | |
| $OCH_3$ | H | $CH_3$ | H | 3-O | |
| $OCH_3$ | 5-$CF_3$ | $CH_3$ | H | 1-O | |
| $OCH_3$ | 5-$CF_3$ | $CH_3$ | H | 3-O | |
| $OCH_3$ | H | $CH_3$ | $CH_3$ | 1-O | |
| $OCH_3$ | H | $CH_3$ | $CH_3$ | 3-O | |
| $OCH_3$ | H | $C_2H_5$ | H | 1-O | |
| $OCH_3$ | H | $C_2H_5$ | H | 3-O | |
| $OCH_3$ | 5-Cl | $CH_3$ | H | 1-O | |
| $OCH_3$ | 5-Cl | $CH_3$ | H | 3-O | |
| $OC_2H_5$ | H | $CH_3$ | H | 1-O | |
| $OC_2H_5$ | H | $CH_3$ | H | 3-O | |
| $O(CH_2)_5CH_3$ | H | $C_2H_5$ | H | 1-O | |
| $OCH(CH_3)_2$ | H | $CH_3$ | H | 1-O | |
| $OCH(CH_3)C_2H_5$ | 3-$CF_3$ | $CH_3$ | H | 1-O | |
| O 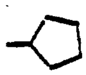 | H | $CH_3$ | H | 1-O | |
| O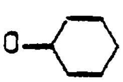 | H | $CH_3$ | H | 3-O | |
| $OCH_2CH{=}CH_2$ | H | $CH_3$ | H | 1-O | |
| $OCH(CH_3)CH{=}CH_2$ | H | $CH_3$ | H | 1-O | |
| $OCH_2C(CH_3){=}CH_2$ | H | $C_2H_5$ | H | 3-O | |
| $O(CH_2)_4CH{=}CH_2$ | H | $CH_3$ | H | 1-O | |
| $OCH_2C{\equiv}CH$ | H | $CH_3$ | H | 1-O | |
| $OCH_2C{\equiv}C(CH_2)_2CH_3$ | H | $CH_3$ | $CH_3$ | 1-O | |
| $OCH_2C{\equiv}CCH_3$ | 5-$NO_2$ | $CH_3$ | H | 1-O | |

Table XIII (continued)

R = $COR_4$

| $R_4$ | $R_1$ | $X_1$ | W | Q | m.p.(°C) |
|---|---|---|---|---|---|
| $OCH(CH_3)C{=}CCH_3$ | 6-F | $CH_3$ | H | 1-0 | |
| $OCH_2CCl_3$ | H | $CH_3$ | H | 1-0 | |
| $OCH_2CH_2F$ | H | $C_2H_5$ | H | 1-0 | |
| $OCH_2CH_2CH_2Br$ | 3-Br | $CH_3$ | $CH_3$ | 1-0 | |
| $O(CH_2)_5CH_2Cl$ | H | $CH_3$ | H | 3-0 | |
| $OCH_2CHFCH_2F$ | H | $CH_3$ | H | 1-0 | |
| $OCH_3$ | H | $OCH_3$ | H | 1-0 | |
| $OCH_3$ | H | $OCH_3$ | H | 3-0 | |
| $OCH_3$ | 5-$CF_3$ | $OCH_3$ | H | 1-0 | |
| $OCH_3$ | 5-$CF_3$ | $OCH_3$ | H | 3-0 | |
| $OCH_3$ | H | $OCH_3$ | $CH_3$ | 1-0 | |
| $OCH_3$ | H | $OCH_3$ | $CH_3$ | 3-0 | |
| $OCH_3$ | H | $OC_2H_5$ | H | 1-0 | |
| $OCH_3$ | 5-Cl | $OCH_3$ | H | 1-0 | |
| $OCH_3$ | 5-Cl | $OCH_3$ | H | 3-0 | |
| $OC_2H_5$ | H | $OCH_3$ | H | 1-0 | |
| $OC_2H_5$ | H | $OCH_3$ | H | 3-0 | |
| $O(CH_2)_5CH_3$ | H | $OC_2H_5$ | H | 1-0 | |
| $OCH(CH_3)_2$ | H | $OCH_3$ | H | 3-0 | |
| $OCH(CH_3)C_2H_5$ | 3-$CF_3$ | $OCH_3$ | H | 1-0 | |
| O 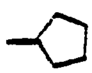 | H | $OCH_3$ | H | 3-0 | |
| O 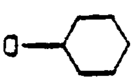 | H | $OCH_3$ | H | 1-0 | |
| $OCH_2CH{=}CH_2$ | H | $OCH_3$ | H | 3-0 | |
| $OCH(CH_3)CH{=}CH_2$ | H | $OCH_3$ | H | 1-0 | |
| $OCH_2C(CH_3){=}CH_2$ | H | $OC_2H_5$ | H | 3-0 | |
| $O(CH_2)_4CH{=}CH_2$ | H | $OCH_3$ | H | 1-0 | |
| $OCH_2C{\equiv}CH$ | H | $OCH_3$ | H | 3-0 | |
| $OCH_2C{\equiv}C(CH_2)_2CH_3$ | H | $OCH_3$ | $CH_3$ | 1-0 | |
| $OCH_2C{\equiv}CCH_3$ | 5-$NO_2$ | $OCH_3$ | H | 3-0 | |

Table XIII (continued)

R = $COR_4$

| $R_4$ | $R_1$ | $X_1$ | W | Q | m.p.(°C) |
|---|---|---|---|---|---|
| $OCH(CH_3)C{=}CCH_3$ | 6-F | $OCH_3$ | H | 1-0 | |
| $OCH_2CCl_3$ | H | $OCH_3$ | H | 3-0 | |
| $OCH_2CH_2F$ | H | $OC_2H_5$ | H | 1-0 | |
| $OCH_2CH_2CH_2Br$ | 3-Br | $OCH_3$ | $CH_3$ | 3-0 | |
| $O(CH_2)_5CH_2Cl$ | H | $OCH_3$ | H | 1-0 | |
| $OCH_2CHFCH_2F$ | H | $OCH_3$ | H | 3-0 | |
| $OCH_2CHClCHClCH_3$ | 5-$OC_2H_5$ | $CH_3$ | H | 1-0 | |
| $OCH_2CH_2Br$ | H | $C_2H_5$ | $CH_3$ | 3-0 | |
| $OCH_2CH_2OCH_3$ | H | $CH_3$ | H | 1-0 | |
| $(OCH_2CH_2)_2OC_2H_5$ | H | $CH_3$ | H | 3-0 | |
| $OCH_2CH_2CH_2OCH_3$ | H | $CH_3$ | H | 1-0 | |
| $OCH_2OCH_3$ | H | $CH_3$ | H | 3-0 | |
| $OCH_2OCH_2CH(CH_3)_2$ | 3-$CH_3$ | $CH_3$ | H | 1-0 | |
| $OCH_2OCH_2CH_2OCH_3$ | H | $CH_3$ | H | 3-0 | |
| $OCH_2CHClCHClCH_3$ | 5-$OC_2H_5$ | $OCH_3$ | H | 1-0 | |
| $OCH_2CH_2Br$ | H | $OC_2H_5$ | $CH_3$ | 3-0 | |
| $OCH_2CH_2OCH_3$ | H | $OCH_3$ | H | 1-0 | |
| $(OCH_2CH_2)_2OC_2H_5$ | H | $OCH_3$ | H | 3-0 | |
| $OCH_2CH_2CH_2OCH_3$ | H | $OCH_3$ | H | 1-0 | |
| $OCH_2OCH_3$ | H | $OCH_3$ | H | 3-0 | |
| $OCH_2OCH_2CH(CH_3)_2$ | 3-$CH_3$ | $OCH_3$ | H | 1-0 | |
| $OCH_2OCH_2CH_2OCH_3$ | H | $OCH_3$ | H | 3-0 | |

Table XIV

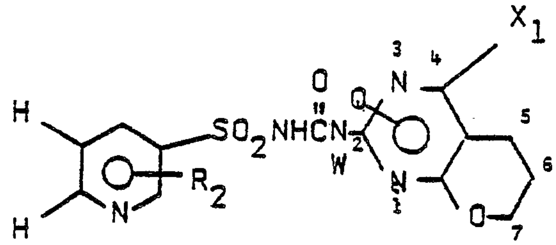

| $R_2$ | $X_1$ | W | Q | m.p.(°C) |
|---|---|---|---|---|
| 2-Cl | $CH_3$ | H | 1-O | |
| 4-F | $CH_3$ | H | 1-O | |
| 2-Br | $C_2H_5$ | H | 1-O | |
| 4-$CH_3$ | $CH_3$ | $CH_3$ | 1-O | |
| 2-$CH_2CH(CH_3)_2$ | $CH_3$ | H | 3-O | |
| 2-$OCH_3$ | $CH_3$ | H | 1-O | |
| 4-$O(CH_2)_3CH_3$ | $CH_3$ | H | 1-O | |
| 2-$NO_2$ | $CH_3$ | H | 1-O | |
| 2-$CO_2CH_3$ | $CH_3$ | H | 1-O | |
| 2-$CO_2CH_3$ | $CH_3$ | H | 3-O | |
| 2-$CO_2CH_3$ | H | H | 1-O | |
| 2-$CO_2CH_3$ | H | H | 3-O | |
| 4-$CO_2CH_2CH_3$ | $CH_3$ | H | 1-O | |
| 4-$CO_2CH_2CH_3$ | $CH_3$ | H | 3-O | |
| 2-$CO_2CH(CH_3)_2$ | $C_2H_5$ | H | 1-O | |
| 2-$CO_2(CH_2)_3CH_3$ | $CH_3$ | H | 1-O | |
| 2-$SCH_3$ | $CH_3$ | H | 1-O | |
| 4-$SCH(CH_3)_2$ | $CH_3$ | H | 1-O | |
| 2-$S(O)CH_3$ | $CH_3$ | H | 1-O | |
| 4-$S(O)CH_2CH_2CH_3$ | $CH_3$ | H | 1-O | |
| 2-$SO_2CH_3$ | $CH_3$ | H | 1-O | |
| 4-$SO_2C_2H_5$ | $CH_3$ | $CH_3$ | 1-O | |
| 2-$SO_2CH(CH_3)_2$ | $CH_3$ | H | 3-O | |
| 2-$CH_3$ | $CH_3$ | H | 1-O | |
| 2-$OCH_3$ | $CH_3$ | H | 1-O | |

Table XIV (continued)

| $R_2$ | $X_1$ | W | Q | m.p.(°C) |
|---|---|---|---|---|
| 2-Cl | $OCH_3$ | H | 1-0 | |
| 4-F | $OCH_3$ | H | 3-0 | |
| 2-Br | $OC_2H_5$ | H | 1-0 | |
| 4-$CH_3$ | $OCH_3$ | $CH_3$ | 3-0 | |
| 2-$CH_2CH(CH_3)_2$ | $OCH_3$ | H | 1-0 | |
| 2-$OCH_3$ | $OCH_3$ | H | 3-0 | |
| 4-$O(CH_2)_3CH_3$ | $OCH_3$ | H | 1-0 | |
| 2-$NO_2$ | $OCH_3$ | H | 3-0 | |
| 2-$CO_2CH_3$ | $OCH_3$ | H | 1-0 | |
| 2-$CO_2CH_3$ | $OCH_3$ | H | 3-0 | |
| 2-$CO_2CH_3$ | Cl | H | 1-0 | |
| 4-$CO_2CH_2CH_3$ | $OCH_3$ | H | 1-0 | |
| 4-$CO_2CH_2CH_3$ | $OCH_3$ | H | 3-0 | |
| 2-$CO_2CH(CH_3)_2$ | $OC_2H_5$ | H | 1-0 | |
| 2-$CO_2(CH_2)_3CH_3$ | $OCH_3$ | H | 1-0 | |
| 2-$SCH_3$ | $OCH_3$ | H | 3-0 | |
| 4-$SCH(CH_3)_2$ | $OCH_3$ | H | 1-0 | |
| 2-$S(O)CH_3$ | $OCH_3$ | H | 1-0 | |
| 4-$S(O)CH_2CH_2CH_3$ | $OCH_3$ | H | 3-0 | |
| 2-$SO_2CH_3$ | $OCH_3$ | H | 3-0 | |
| 4-$SO_2C_2H_5$ | $OCH_3$ | $CH_3$ | 1-0 | |
| 2-$SO_2CH(CH_3)_2$ | $OCH_3$ | H | 1-0 | |
| 2-$CH_3$ | $OCH_3$ | H | 3-0 | |
| 2-$OCH_3$ | $OCH_3$ | H | 3-0 | |

Table XVa

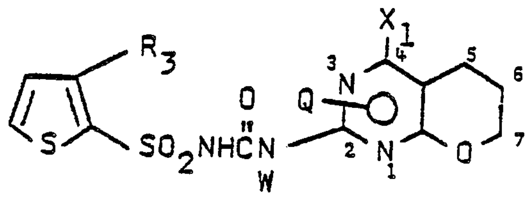

| $R_3$ | $X_1$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 1-O | |
| $CH(CH_3)C_2H_5$ | $CH_3$ | H | 1-O | |
| $OCH_3$ | $C_2H_5$ | H | 1-O | |
| $O(CH_2)_3CH_3$ | $CH_3$ | H | 3-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 3-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 1-O | |
| $SO_2N(OCH_3)CH_3$ | $CH_3$ | H | 1-O | |

$COR_4$

| $R_4$ | $X_1$ | W | Q |
|---|---|---|---|
| $OCH_3$ | $CH_3$ | H | 1-O |
| $OCH_3$ | $CH_3$ | $CH_3$ | 1-O |
| $OCH_3$ | $CH_3$ | $CH_3$ | 3-O |
| $OCH_3$ | $C_2H_5$ | H | 1-O |

Table XVa (continued)

| $R_4$ | $X_1$ | W | Q | m.p.(°C) |
|---|---|---|---|---|
| $OCH_3$ | $CH_3$ | H | 3-0 | |
| $OCH_3$ | $C_2H_5$ | $CH_3$ | 1-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 3-0 | |
| $OCH(CH_3)C_2H_5$ | $CH_3$ | H | 1-0 | |
| O 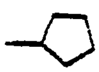 | $CH_3$ | H | 1-0 | |
| O 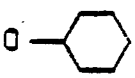 | $CH_3$ | H | 1-0 | |
| $OCH_2CH{=}CH_2$ | $CH_3$ | H | 1-0 | |
| $OCH(CH_3)CH{=}CH_2$ | $CH_3$ | H | 1-0 | |
| $OCH_2C(CH_3){=}CH_2$ | $CH_3$ | H | 1-0 | |
| $O(CH_2)_4CH{=}CH_2$ | $CH_3$ | H | 1-0 | |
| $OCH_2C{\equiv}CH$ | $CH_3$ | H | 3-0 | |
| $OCH_2C{\equiv}C(CH_2)_2CH_3$ | $CH_3$ | H | 1-0 | |
| $OCH_2C{\equiv}CCH_3$ | $CH_3$ | H | 1-0 | |
| $OCH(CH_3)C{\equiv}CCH_3$ | $CH_3$ | H | 3-0 | |
| $OCH_2CCl_3$ | $CH_3$ | H | 1-0 | |
| $OCH_2CH_2F$ | $C_2H_5$ | H | 1-0 | |
| $OCH_2CH_2CH_2Br$ | $CH_3$ | $CH_3$ | 1-0 | |
| $O(CH_2)_5CH_2Cl$ | $CH_3$ | H | 3-0 | |
| $OCH_2CHFCH_2F$ | $CH_3$ | H | 1-0 | |

Table XVa (continued)

| $R_3$ | $X_1$ | W | Q | m.p.(°C) |
|---|---|---|---|---|
| H | $OCH_3$ | H | 1-0 | |
| Cl | $OCH_3$ | H | 1-0 | |
| $OC_2H_5$ | $OC_2H_5$ | H | 3-0 | |
| $CH_3$ | $OCH_3$ | H | 1-0 | |
| $CH(CH_3)C_2H_5$ | $OCH_3$ | H | 3-0 | |
| $OCH_3$ | $OC_2H_5$ | H | 1-0 | |
| $O(CH_2)_3CH_3$ | $OCH_3$ | H | 3-0 | |
| $OCH(CH_3)_2$ | $OCH_3$ | H | 1-0 | |
| $C_2H_5$ | $OCH_3$ | H | 3-0 | |
| Br | $OCH_3$ | H | 1-0 | |
| $NO_2$ | $OCH_3$ | H | 3-0 | |
| $SO_2N(CH_3)_2$ | $OCH_3$ | H | 1-0 | |
| $SO_2N(CH_3)_2$ | Cl | H | 3-0 | |
| $SO_2N(CH(CH_3)C_2H_5)CH_3$ | $OCH_3$ | H | 1-0 | |
| $SO_2N(CH(CH_3)_2)CH_3$ | $OC_2H_5$ | H | 3-0 | |
| $SO_2N(CH_3)C_2H_5$ | $OC_2H_5$ | $CH_3$ | 1-0 | |
| $SO_2N(C_2H_5)_2$ | $OCH_3$ | H | 3-0 | |
| $SO_2N(OCH_3)CH_3$ | $OCH_3$ | H | 3-0 | |

$COR_4$

| $R_4$ | $X_1$ | W | Q |
|---|---|---|---|
| $OCH_3$ | $OCH_3$ | H | 1-0 |
| $OCH_3$ | $OCH_3$ | $CH_3$ | 1-0 |
| $OCH_3$ | $OCH_3$ | $CH_3$ | 3-0 |
| $OCH_3$ | $OC_2H_5$ | H | 1-0 |
| $OCH_3$ | $OC_2H_5$ | H | 3-0 |

Table XVa (continued)

| $R_4$ | $X_1$ | W | Q | m.p.(°C) |
|---|---|---|---|---|
| $OCH_3$ | $OCH_3$ | H | 3-0 | |
| $OCH_3$ | $OC_2H_5$ | $CH_3$ | 1-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 3-0 | |
| $OCH(CH_3)C_2H_5$ | $OCH_3$ | H | 1-0 | |
| 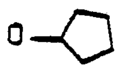 | $OCH_3$ | H | 3-0 | |
| 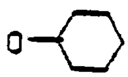 | $OCH_3$ | H | 1-0 | |
| $OCH_2CH{=}CH_2$ | $OCH_3$ | H | 1-0 | |
| $OCH(CH_3)CH{=}CH_2$ | $OCH_3$ | H | 3-0 | |
| $OCH_2C(CH_3){=}CH_2$ | $OCH_3$ | H | 3-0 | |
| $O(CH_2)_4CH{=}CH_2$ | $OCH_3$ | H | 1-0 | |
| $OCH_2C{\equiv}CH$ | $OCH_3$ | H | 1-0 | |
| $OCH_2C{\equiv}C(CH_2)_2CH_3$ | $OCH_3$ | H | 3-0 | |
| $OCH_2C{\equiv}CCH_3$ | $OCH_3$ | H | 3-0 | |
| $OCH(CH_3)C{\equiv}CCH_3$ | $OCH_3$ | H | 1-0 | |
| $OCH_2CCl_3$ | $OCH_3$ | H | 1-0 | |
| $OCH_2CH_2F$ | $OC_2H_5$ | H | 1-0 | |
| $OCH_2CH_2CH_2Br$ | $OCH_3$ | $CH_3$ | 3-0 | |
| $O(CH_2)_5CH_2Cl$ | $OCH_3$ | H | 3-0 | |
| $OCH_2CHFCH_2F$ | $OCH_3$ | H | 1-0 | |

Table XVa (continued)

| $R_4$ | $X_1$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 1-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 3-0 | |
| $OCH_2OCH_3$ | $CH_3$ | H | 1-0 | |
| $OCH_2OCH_2CH(CH_3)_2$ | $CH_3$ | H | 1-0 | |
| $OCH_2OCH_2CH_2OCH_3$ | $CH_3$ | H | 1-0 | |
| $OCH_2CHClCHClCH_3$ | $OCH_3$ | H | 3-0 | |
| $OCH_2CH_2Br$ | $OC_2H_5$ | $CH_3$ | 1-0 | |
| $OCH_2CH_2OCH_3$ | $OCH_3$ | H | 1-0 | |
| $(OCH_2CH_2)_2OC_2H_5$ | $OCH_3$ | H | 3-0 | |
| $OCH_2CH_2CH_2OCH_3$ | $OCH_3$ | H | 3-0 | |
| $OCH_2OCH_3$ | $OCH_3$ | H | 1-0 | |
| $OCH_2OCH_2CH(CH_3)_2$ | $OCH_3$ | H | 3-0 | |
| $OCH_2OCH_2CH_2OCH_3$ | $OCH_3$ | H | 3-0 | |

Table XVb

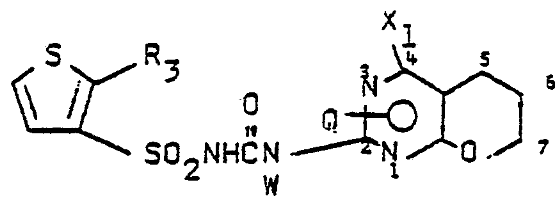

| $R_3$ | $X_1$ | W | Q | m.p.(°C) |
|---|---|---|---|---|
| H | $CH_3$ | H | 1-0 | |
| Cl | $CH_3$ | H | 1-0 | |
| $OC_2H_5$ | $C_2H_5$ | H | 1-0 | |
| $CH_3$ | $CH_3$ | H | 1-0 | |
| $CH(CH_3)C_2H_5$ | $CH_3$ | H | 1-0 | |
| $OCH_3$ | $C_2H_5$ | H | 1-0 | |
| $O(CH_2)_3CH_3$ | $CH_3$ | H | 3-0 | |
| $OCH(CH_3)_2$ | $CH_3$ | H | 1-0 | |
| $C_2H_5$ | $CH_3$ | H | 1-0 | |
| Br | $CH_3$ | H | 3-0 | |
| $NO_2$ | $CH_3$ | H | 1-0 | |
| $SO_2N(CH_3)_2$ | $CH_3$ | H | 1-0 | |
| $SO_2N(CH_3)_2$ | H | H | 1-0 | |
| $SO_2N(CH(CH_3)C_2H_5)CH_3$ | $CH_3$ | H | 3-0 | |
| $SO_2N(CH(CH_3)_2)CH_3$ | $C_2H_5$ | H | 1-0 | |
| $SO_2N(CH_3)C_2H_5$ | $C_2H_5$ | $CH_3$ | 1-0 | |
| $SO_2N(C_2H_5)_2$ | $CH_3$ | H | 1-0 | |
| $SO_2N(OCH_3)CH_3$ | $CH_3$ | H· | 1-0 | |

$COR_4$

| $R_4$ | $X_1$ | W | Q |
|---|---|---|---|
| $OCH_3$ | $CH_3$ | H | 1-0 |
| $OCH_3$ | $CH_3$ | $CH_3$ | 1-0 |
| $OCH_3$ | $C_2H_5$ | H | 1-0 |

Table XVb (continued)

| $R_4$ | $X_1$ | W | Q | m.p.(°C) |
|---|---|---|---|---|
| $OCH_3$ | $CH_3$ | H | 3-O | |
| $OCH_3$ | $C_2H_5$ | $CH_3$ | 1-O | |
| $OCH_2CH_3$ | $CH_3$ | H | 1-O | |
| $O(CH_2)_5CH_3$ | $CH_3$ | H | 1-O | |
| $OCH(CH_3)_2$ | $CH_3$ | H | 3-O | |
| $OCH(CH_3)C_2H_5$ | $CH_3$ | H | 1-O | |
| 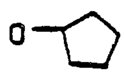 | $CH_3$ | H | 1-O | |
| 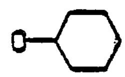 | $CH_3$ | H | 1-O | |
| $OCH_2CH{=}CH_2$ | $CH_3$ | H | 1-O | |
| $OCH(CH_3)CH{=}CH_2$ | $CH_3$ | H | 1-O | |
| $OCH_2C(CH_3){=}CH_2$ | $CH_3$ | H | 1-O | |
| $O(CH_2)_4CH{=}CH_2$ | $CH_3$ | H | 1-O | |
| $OCH_2C{\equiv}CH$ | $CH_3$ | H | 3-O | |
| $OCH_2C{\equiv}C(CH_2)_2CH_3$ | $CH_3$ | H | 1-O | |
| $OCH_2C{\equiv}CCH_3$ | $CH_3$ | H | 1-O | |
| $OCH(CH_3)C{\equiv}CCH_3$ | $CH_3$ | H | 3-O | |
| $OCH_2CCl_3$ | $CH_3$ | H | 1-O | |
| $OCH_2CH_2F$ | $C_2H_5$ | H | 1-O | |
| $OCH_2CH_2CH_2Br$ | $CH_3$ | $CH_3$ | 1-O | |
| $O(CH_2)_5CH_2Cl$ | $CH_3$ | H | 3-O | |
| $OCH_2CHFCH_2F$ | $CH_3$ | H | 1-O | |

Table XVb (continued)

| $R_3$ | $X_1$ | W | Q | m.p.(°C) |
|---|---|---|---|---|
| H | $OCH_3$ | H | 1-0 | |
| Cl | $OCH_3$ | H | 1-0 | |
| $OC_2H_5$ | $OC_2H_5$ | H | 3-0 | |
| $CH_3$ | $OCH_3$ | H | 1-0 | |
| $CH(CH_3)C_2H_5$ | $OCH_3$ | H | 3-0 | |
| $OCH_3$ | $OC_2H_5$ | H | 1-0 | |
| $O(CH_2)_3CH_3$ | $OCH_3$ | H | 3-0 | |
| $OCH(CH_3)_2$ | $OCH_3$ | H | 1-0 | |
| $C_2H_5$ | $OCH_3$ | H | 3-0 | |
| Br | $OCH_3$ | H | 1-0 | |
| $NO_2$ | $OCH_3$ | H | 3-0 | |
| $SO_2N(CH_3)_2$ | $OCH_3$ | H | 1-0 | |
| $SO_2N(CH_3)_2$ | Cl | H | 3-0 | |
| $SO_2N(CH(CH_3)C_2H_5)CH_3$ | $OCH_3$ | H | 1-0 | |
| $SO_2N(CH(CH_3)_2)CH_3$ | $OC_2H_5$ | H | 3-0 | |
| $SO_2N(CH_3)C_2H_5$ | $OC_2H_5$ | $CH_3$ | 1-0 | |
| $SO_2N(C_2H_5)_2$ | $OCH_3$ | H | 3-0 | |
| $SO_2N(OCH_3)CH_3$ | $OCH_3$ | H | 3-0 | |

$COR_4$

| $R_4$ | $X_1$ | W | Q |
|---|---|---|---|
| $OCH_3$ | $OCH_3$ | H | 1-0 |
| $OCH_3$ | $OCH_3$ | $CH_3$ | 1-0 |
| $OCH_3$ | $OC_2H_5$ | H | 1-0 |

Table XVb (continued)

| $R_4$ | $X_1$ | W | Q | m.p.(°C) |
|---|---|---|---|---|
| $OCH_3$ | $OCH_3$ | H | 3-O | |
| $OCH_3$ | $OC_2H_5$ | $CH_3$ | 1-O | |
| $OCH_2CH_3$ | $OCH_3$ | H | 3-O | |
| $O(CH_2)_5CH_3$ | $OCH_3$ | H | 1-O | |
| $OCH(CH_3)_2$ | $OCH_3$ | H | 3-O | |
| $OCH(CH_3)C_2H_5$ | $OCH_3$ | H | 1-O | |
| 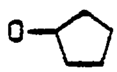 | $OCH_3$ | H | 3-O | |
| 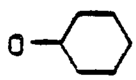 | $OCH_3$ | H | 1-O | |
| $OCH_2CH{=}CH_2$ | $OCH_3$ | H | 1-O | |
| $OCH(CH_3)CH{=}CH_2$ | $OCH_3$ | H | 3-O | |
| $OCH_2C(CH_3){=}CH_2$ | $OCH_3$ | H | 3-O | |
| $O(CH_2)_4CH{=}CH_2$ | $OCH_3$ | H | 1-O | |
| $OCH_2C{\equiv}CH$ | $OCH_3$ | H | 1-O | |
| $OCH_2C{\equiv}C(CH_2)_2CH_3$ | $OCH_3$ | H | 3-O | |
| $OCH_2C{\equiv}CCH_3$ | $OCH_3$ | H | 3-O | |
| $OCH(CH_3)C{\equiv}CCH_3$ | $OCH_3$ | H | 1-O | |
| $OCH_2CCl_3$ | $OCH_3$ | H | 1-O | |
| $OCH_2CH_2F$ | $OC_2H_5$ | H | 1-O | |
| $OCH_2CH_2CH_2Br$ | $OCH_3$ | $CH_3$ | 3-O | |
| $O(CH_2)_5CH_2Cl$ | $OCH_3$ | H | 3-O | |
| $OCH_2CHFCH_2F$ | $OCH_3$ | H | 1-O | |

Table XVb (continued)

| $R_4$ | $X_1$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 1-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 3-0 | |
| $OCH_2OCH_3$ | $CH_3$ | H | 1-0 | |
| $OCH_2OCH_2CH(CH_3)_2$ | $CH_3$ | H | 1-0 | |
| $OCH_2OCH_2CH_2OCH_3$ | $CH_3$ | H | 1-0 | |
| $OCH_2CHClCHClCH_3$ | $OCH_3$ | H | 3-0 | |
| $OCH_2CH_2Br$ | $OC_2H_5$ | $CH_3$ | 1-0 | |
| $OCH_2CH_2OCH_3$ | $OCH_3$ | H | 1-0 | |
| $(OCH_2CH_2)_2OC_2H_5$ | $OCH_3$ | H | 3-0 | |
| $OCH_2CH_2CH_2OCH_3$ | $OCH_3$ | H | 3-0 | |
| $OCH_2OCH_3$ | $OCH_3$ | H | 1-0 | |
| $OCH_2OCH_2CH(CH_3)_2$ | $OCH_3$ | H | 3-0 | |
| $OCH_2OCH_2CH_2OCH_3$ | $OCH_3$ | H | 3-0 | |

Table XVc

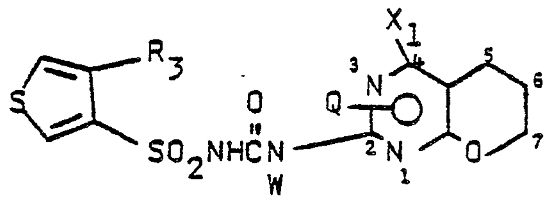

| $R_3$ | $X_1$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 1-O | |
| $CH(CH_3)C_2H_5$ | $CH_3$ | H | 1-O | |
| $OCH_3$ | $C_2H_5$ | H | 1-O | |
| $O(CH_2)_3CH_3$ | $CH_3$ | H | 3-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 3-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 1-O | |
| $SO_2N(OCH_3)CH_3$ | $CH_3$ | H | 1-O | |

$COR_4$

| $R_4$ | $X_1$ | W | Q |
|---|---|---|---|
| $OCH_3$ | $CH_3$ | H | 1-O |
| $OCH_3$ | $CH_3$ | $CH_3$ | 1-O |
| $OCH_3$ | $C_2H_5$ | H | 1-O |

Table XVc (continued)

| $R_4$ | $X_1$ | W | Q | m.p.(°C) |
|---|---|---|---|---|
| $OCH_3$ | $CH_3$ | H | 3-0 | |
| $OCH_3$ | $C_2H_5$ | $CH_3$ | 1-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 3-0 | |
| $OCH(CH_3)C_2H_5$ | $CH_3$ | H | 1-0 | |
| 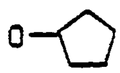 | $CH_3$ | H | 1-0 | |
| 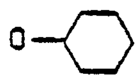 | $CH_3$ | H | 1-0 | |
| $OCH_2CH=CH_2$ | $CH_3$ | H | 1-0 | |
| $OCH(CH_3)CH=CH_2$ | $CH_3$ | H | 1-0 | |
| $OCH_2C(CH_3)=CH_2$ | $CH_3$ | H | 1-0 | |
| $O(CH_2)_4CH=CH_2$ | $CH_3$ | H | 1-0 | |
| $OCH_2C\equiv CH$ | $CH_3$ | H | 3-0 | |
| $OCH_2C\equiv C(CH_2)_2CH_3$ | $CH_3$ | H | 1-0 | |
| $OCH_2C\equiv CCH_3$ | $CH_3$ | H | 1-0 | |
| $OCH(CH_3)C\equiv CCH_3$ | $CH_3$ | H | 3-0 | |
| $OCH_2CCl_3$ | $CH_3$ | H | 1-0 | |
| $OCH_2CH_2F$ | $C_2H_5$ | H | 1-0 | |
| $OCH_2CH_2CH_2Br$ | $CH_3$ | $CH_3$ | 1-0 | |
| $O(CH_2)_5CH_2Cl$ | $CH_3$ | H | 3-0 | |
| $OCH_2CHFCH_2F$ | $CH_3$ | H | 1-0 | |

## Table XVc (continued)

| $R_3$ | $X_1$ | W | Q | m.p.(°C) |
|---|---|---|---|---|
| H | $OCH_3$ | H | 1-O | |
| Cl | $OCH_3$ | H | 1-O | |
| $OC_2H_5$ | $OC_2H_5$ | H | 3-O | |
| $CH_3$ | $OCH_3$ | H | 1-O | |
| $CH(CH_3)C_2H_5$ | $OCH_3$ | H | 3-O | |
| $OCH_3$ | $OC_2H_5$ | H | 1-O | |
| $O(CH_2)_3CH_3$ | $OCH_3$ | H | 3-O | |
| $OCH(CH_3)_2$ | $OCH_3$ | H | 1-O | |
| $C_2H_5$ | $OCH_3$ | H | 3-O | |
| Br | $OCH_3$ | H | 1-O | |
| $NO_2$ | $OCH_3$ | H | 3-O | |
| $SO_2N(CH_3)_2$ | $OCH_3$ | H | 1-O | |
| $SO_2N(CH_3)_2$ | Cl | H | 3-O | |
| $SO_2N(CH(CH_3)C_2H_5)CH_3$ | $OCH_3$ | H | 1-O | |
| $SO_2N(CH(CH_3)_2)CH_3$ | $OC_2H_5$ | H | 3-O | |
| $SO_2N(CH_3)C_2H_5$ | $OC_2H_5$ | $CH_3$ | 1-O | |
| $SO_2N(C_2H_5)_2$ | $OCH_3$ | H | 3-O | |
| $SO_2N(OCH_3)CH_3$ | $OCH_3$ | H | 3-O | |

$COR_4$

| $R_4$ | $X_1$ | W | Q |
|---|---|---|---|
| $OCH_3$ | $OCH_3$ | H | 1-O |
| $OCH_3$ | $OCH_3$ | $CH_3$ | 1-O |
| $OCH_3$ | $OC_2H_5$ | H | 1-O |

Table XVc (continued)

| $R_4$ | $X_1$ | W | Q | m.p.(°C) |
|---|---|---|---|---|
| $OCH_3$ | $OCH_3$ | H | 3-0 | |
| $OCH_3$ | $OC_2H_5$ | $CH_3$ | 1-0 | |
| $OCH_2CH_3$ | $OCH_3$ | H | 3-0 | |
| $O(CH_2)_5CH_3$ | $OCH_3$ | H | 1-0 | |
| $OCH(CH_3)_2$ | $OCH_3$ | H | 3-0 | |
| $OCH(CH_3)C_2H_5$ | $OCH_3$ | H | 1-0 | |
| O 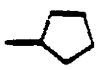 | $OCH_3$ | H | 3-0 | |
| O 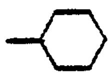 | $OCH_3$ | H | 1-0 | |
| $OCH_2CH=CH_2$ | $OCH_3$ | H | 1-0 | |
| $OCH(CH_3)CH=CH_2$ | $OCH_3$ | H | 3-0 | |
| $OCH_2C(CH_3)=CH_2$ | $OCH_3$ | H | 3-0 | |
| $O(CH_2)_4CH=CH_2$ | $OCH_3$ | H | 1-0 | |
| $OCH_2C\equiv CH$ | $OCH_3$ | H | 1-0 | |
| $OCH_2C\equiv C(CH_2)_2CH_3$ | $OCH_3$ | H | 3-0 | |
| $OCH_2C\equiv CCH_3$ | $OCH_3$ | H | 3-0 | |
| $OCH(CH_3)C\equiv CCH_3$ | $OCH_3$ | H | 1-0 | |
| $OCH_2CCl_3$ | $OCH_3$ | H | 1-0 | |
| $OCH_2CH_2F$ | $OC_2H_5$ | H | 1-0 | |
| $OCH_2CH_2CH_2Br$ | $OCH_3$ | $CH_3$ | 3-0 | |
| $O(CH_2)_5CH_2Cl$ | $OCH_3$ | H | 3-0 | |
| $OCH_2CHFCH_2F$ | $OCH_3$ | H | 1-0 | |

Table XVc (continued)

| $R_4$ | $X_1$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 1-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 3-0 | |
| $OCH_2OCH_3$ | $CH_3$ | H | 1-0 | |
| $OCH_2OCH_2CH(CH_3)_2$ | $CH_3$ | H | 1-0 | |
| $OCH_2OCH_2CH_2OCH_3$ | $CH_3$ | H | 1-0 | |
| $OCH_2CHClCHClCH_3$ | $OCH_3$ | H | 3-0 | |
| $OCH_2CH_2Br$ | $OC_2H_5$ | $CH_3$ | 1-0 | |
| $OCH_2CH_2OCH_3$ | $OCH_3$ | H | 1-0 | |
| $(OCH_2CH_2)_2OC_2H_5$ | $OCH_3$ | H | 3-0 | |
| $OCH_2CH_2CH_2OCH_3$ | $OCH_3$ | H | 3-0 | |
| $OCH_2OCH_3$ | $OCH_3$ | H | 1-0 | |
| $OCH_2OCH_2CH(CH_3)_2$ | $OCH_3$ | H | 3-0 | |
| $OCH_2OCH_2CH_2OCH_3$ | $OCH_3$ | H | 3-0 | |

Table XVI

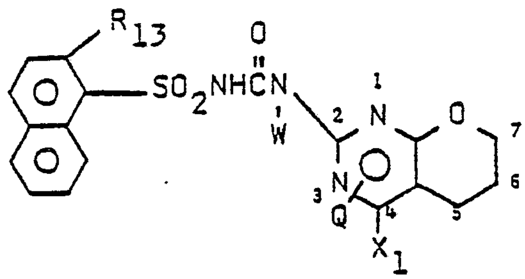

| $R_{13}$ | $X_1$ | W | Q | m.p.(°C) |
|---|---|---|---|---|
| H | $CH_3$ | H | 1-O | |
| $CH_3$ | $CH_3$ | H | 1-O | |
| $C_2H_5$ | $C_2H_5$ | H | 3-O | |
| $(CH_2)_4H$ | $CH_3$ | H | 1-O | |
| $OCH_3$ | $CH_3$ | H | 3-O | |
| $OC_2H_5$ | $CH_3$ | $CH_3$ | 1-O | |
| $O(CH_2)_4H$ | $CH_3$ | H | 1-O | |
| Cl | $CH_3$ | H | 1-O | |
| $NO_2$ | $CH_3$ | H | 1-O | |
| $SCH_3$ | $CH_3$ | H | 1-O | |
| $SC_2H_5$ | $CH_3$ | H | 1-O | |
| $SO_2N(CH_3)_2$ | $CH_3$ | H | 1-O | |
| $SO_2NCH(CH_3)C_2H_5$ | $CH_3$ | H | 1-O | |
| $SO_2N(C_2H_5)_2$ | $CH_3$ | H | 1-O | |
| $S(O)CH_3$ | $CH_3$ | H | 1-O | |
| $S(O)(CH_2)_4H$ | $CH_3$ | H | 1-O | |
| $SO_2CH_3$ | $CH_3$ | H | 1-O | |
| $SO_2(CH_2)_3H$ | $CH_3$ | H | 1-O | |
| $SO_2N(CH_3)_2$ | Cl | H | 1-O | |
| $SO_2CH_3$ | H | H | 1-O | |

Table XVI (continued)

| $R_{13}$ | $X_1$ | W | Q | m.p.(°C) |
|---|---|---|---|---|
| H | $OCH_3$ | H | 3-O | |
| $CH_3$ | $OCH_3$ | H | 1-O | |
| $C_2H_5$ | $OC_2H_5$ | H | 3-O | |
| $(CH_2)_4H$ | $OCH_3$ | H | 1-O | |
| $OCH_3$ | $OCH_3$ | H | 1-O | |
| $OC_2H_5$ | $OCH_3$ | $CH_3$ | 3-O | |
| $O(CH_2)_4H$ | $OCH_3$ | H | 3-O | |
| Cl | $OCH_3$ | H | 1-O | |
| $NO_2$ | $OCH_3$ | H | 1-O | |
| $SCH_3$ | $OCH_3$ | H | 3-O | |
| $SC_2H_5$ | $OCH_3$ | H | 1-O | |
| $SO_2N(CH_3)_2$ | $OCH_3$ | H | 3-O | |
| $SO_2NCH(CH_3)C_2H_5$ | $OCH_3$ | H | 1-O | |
| $SO_2N(C_2H_5)_2$ | $OCH_3$ | H | 1-O | |
| $S(O)CH_3$ | $OCH_3$ | H | 1-O | |
| $S(O)(CH_2)_4H$ | $OCH_3$ | H | 3-O | |
| $SO_2CH_3$ | $OCH_3$ | H | 3-O | |
| $SO_2(CH_2)_3H$ | $OCH_3$ | H | 3-O | |

Table XVII

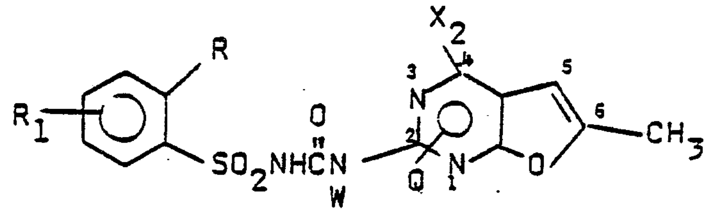

| R | $R_1$ | $X_2$ | W | Q | m.p.(°C) |
|---|---|---|---|---|---|
| H | H | $CH_3$ | H | 1-O | |
| Cl | H | $CH_3$ | H | 1-O | |
| $OC_2H_5$ | 5-$NO_2$ | $C_2H_5$ | H | 1-O | |
| $CF_3$ | H | $CH_3$ | H | 1-O | |
| $CH_3$ | H | $CH_3$ | H | 1-O | |
| $CH(CH_3)C_2H_5$ | H | $CH_3$ | $CH_3$ | 3-O | |
| $OCH_3$ | H | $C_2H_5$ | H | 1-O | |
| $O(CH_2)_3CH_3$ | H | $C_2H_5$ | H | 1-O | |
| $OCH(CH_3)_2$ | 6-Cl | $CH_3$ | H | 3-O | |
| $C_2H_5$ | 3-$CH_3$ | $CH_3$ | H | 1-O | |
| Br | H | $CH_3$ | $CH_3$ | 3-O | |
| $NO_2$ | H | $CH_3$ | H | 1-O | |
| $SCH_3$ | 5-$CH(CH_3)_2$ | $CH_3$ | H | 1-O | |
| $SC(CH_3)_3$ | H | $CH_3$ | H | 1-O | |
| $S(O)CH_3$ | H | $C_2H_5$ | H | 1-O | |
| $S(O)CH(CH_3)C_2H_5$ | 3-F | $CH_3$ | H | 1-O | |
| $SO_2CH_3$ | 5-$C_2H_5$ | $CH_3$ | H | 1-O | |
| $SO_2CH(CH_3)_2$ | 5-Br | $CH_3$ | H | 1-O | |
| $SO_2(CH_2)_3CH_3$ | 6-F | $CH_3$ | $CH_3$ | 3-O | |
| $SO_2CH_3$ | H | $CH_3$ | H | 1-O | |
| $SO_2C_2H_5$ | H | $CH_3$ | H | 3-O | |

Table XVII (continued)

| R | $R_1$ | $X_2$ | W | Q | m.p.(°C) |
|---|---|---|---|---|---|
| $SO_2OCH_2CF_3$ | H | $CH_3$ | H | 1-0 | |
| $SO_2OCH_2CCl_3$ | H | $CH_3$ | H | 1-0 | |
| $SO_2N(OCH_3)CH_3$ | H | $CH_3$ | H | 1-0 | |
| $S(O)CH_2CF_3$ | H | $CH_3$ | H | 1-0 | |
| $OSO_2CF_3$ | 3-$OC(CH_3)_3$ | $CH_3$ | H | 1-0 | |
| $OSO_2CH_2CH_3$ | H | $CH_3$ | H | 3-0 | |
| $OSO_2CHCl_2$ | H | $CH_3$ | H | 1-0 | |
| $OSO_2(CH_2)_3Br$ | 3-$OC_2H_5$ | $CH_3$ | H | 1-0 | |
| $OSO_2CH(CH_3)CHFCH_3$ | H | $CH_3$ | H | 1-0 | |
| $OSO_2CCl_3$ | 4-$OCH(CH_3)_2$ | $CH_3$ | H | 3-0 | |
| $OSO_2(CH_2)_3CHCl_2$ | H | $CH_3$ | H | 1-0 | |
| $OSO_2CH(CH_3)_2$ | H | $CH_3$ | H | 1-0 | |
| $CH_2Cl$ | 3-Br | $CH_3$ | H | 1-0 | |
| $CH_2Br$ | H | $CH_3$ | H | 1-0 | |
| $CH_2OCH_3$ | H | $C_2H_5$ | H | 1-0 | |
| $CH_2OCH(CH_3)C_2H_5$ | H | $CH_3$ | H | 3-0 | |
| $CH_2OCHCH=CH_2$ | 6-Cl | $CH_3$ | H | 1-0 | |
| $CH_2OCH(CH_3)CH=CH_2$ | H | $CH_3$ | H | 1-0 | |
| $CH_2SCH_2CH_3$ | H | $CH_3$ | H | 1-0 | |
| $CH_2S(O)C_2H_5$ | H | $CH_3$ | H | 3-0 | |
| $CH_2SO_2(CH_2)_3CH_3$ | H | $CH_3$ | H | 1-0 | |
| $CH_2S(O)CH_3$ | H | $CH_3$ | H | 1-0 | |
| $CH_2SO_2CH(CH_3)_2$ | H | $CH_3$ | H | 1-0 | |

Table XVII (continued)

| R | $R_1$ | $X_2$ | W | Q | m.p.(°C) |
|---|---|---|---|---|---|
| $SO_2N(CH_3)_2$ | H | $CH_3$ | H | 1-O | |
| $SO_2N(CH(CH_3)C_2H_5)CH_3$ | 3-$CF_3$ | $CH_3$ | H | 3-O | |
| $SO_2N(CH(CH_3)_2)CH_3$ | 5-$NO_2$ | $C_2H_5$ | H | 3-O | |
| $SO_2N(CH_3)C_2H_5$ | H | $C_2H_5$ | $CH_3$ | 1-O | |
| $SO_2N(C_2H_5)_2$ | 4-Br | $CH_3$ | H | 3-O | |

R = $COR_4$

| $R_4$ | $R_1$ | $X_2$ | W | Q | m.p.(°C) |
|---|---|---|---|---|---|
| $OCH_3$ | H | $CH_3$ | H | 1-O | |
| $OCH_3$ | 5-$CF_3$ | $CH_3$ | H | 1-O | |
| $OCH_3$ | H | $CH_3$ | $CH_3$ | 1-O | |
| $OCH_3$ | H | $C_2H_5$ | H | 1-O | |
| $OCH_3$ | 5-Cl | $CH_3$ | H | 1-O | |
| $OC_2H_5$ | H | $CH_3$ | H | 1-O | |
| $O(CH_2)_5CH_3$ | H | $C_2H_5$ | H | 1-O | |
| $OCH(CH_3)_2$ | H | $CH_3$ | H | 1-O | |
| $OCH(CH_3)C_2H_5$ | 3-$CF_3$ | $CH_3$ | H | 1-O | |
| 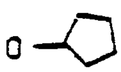 | H | $CH_3$ | H | 1-O | |
| 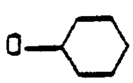 | H | $CH_3$ | H | 3-O | |
| $OCH_2CH=CH_2$ | H | $CH_3$ | H | 1-O | |
| $OCH(CH_3)CH=CH_2$ | H | $CH_3$ | H | 1-O | |
| $OCH_2C(CH_3)=CH_2$ | H | $C_2H_5$ | H | 3-O | |
| $O(CH_2)_4CH=CH_2$ | H | $CH_3$ | H | 1-O | |
| $OCH_2C\equiv CH$ | H | $CH_3$ | H | 1-O | |
| $OCH_2C\equiv C(CH_2)_2CH_3$ | H | $CH_3$ | $CH_3$ | 1-O | |
| $OCH_2C\equiv CCH_3$ | 5-$NO_2$ | $CH_3$ | H | 1-O | |

Table XVII (continued)

R = $COR_4$

| $R_4$ | $R_1$ | $X_2$ | W | Q | m.p.(°C) |
|---|---|---|---|---|---|
| $OCH(CH_3)C{\equiv}CCH_3$ | 6-F | $CH_3$ | H | 3-O | |
| $OCH_2CCl_3$ | H | $CH_3$ | H | 1-O | |
| $OCH_2CH_2F$ | H | $C_2H_5$ | H | 1-O | |
| $OCH_2CH_2CH_2Br$ | 3-Br | $CH_3$ | $CH_3$ | 1-O | |
| $O(CH_2)_5CH_2Cl$ | H | $CH_3$ | H | 1-O | |
| $OCH_2CHFCH_2F$ | H | $CH_3$ | H | 1-O | |
| $OCH_2CHClCHClCH_3$ | 5-$OC_2H_5$ | $CH_3$ | H | 1-O | |
| $OCH_2CH_2Br$ | H | $C_2H_5$ | $CH_3$ | 3-O | |
| $OCH_2CH_2OCH_3$ | H | $CH_3$ | H | 1-O | |
| $(OCH_2CH_2)_2OC_2H_5$ | H | $CH_3$ | H | 1-O | |
| $OCH_2CH_2CH_2OCH_3$ | H | $CH_3$ | H | 1-O | |
| $OCH_2OCH_3$ | H | $CH_3$ | H | 1-O | |
| $OCH_2OCH_2CH(CH_3)_2$ | 3-$CH_3$ | $CH_3$ | H | 1-O | |
| $OCH_2OCH_2CH_2OCH_3$ | H | $CH_3$ | H | 1-O | |

Table XVIII

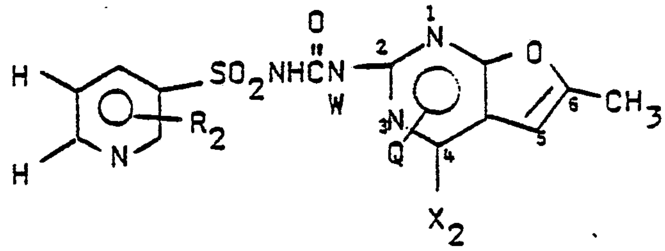

| $R_2$ | $X_2$ | W | Q | m.p.(°C) |
|---|---|---|---|---|
| 2-Cl | $CH_3$ | H | 1-0 | |
| 4-F | $CH_3$ | H | 1-0 | |
| 2-Br | $C_2H_5$ | H | 1-0 | |
| 4-$CH_3$ | $CH_3$ | $CH_3$ | 1-0 | |
| 2-$CH_2CH(CH_3)_2$ | $CH_3$ | H | 3-0 | |
| 2-$OCH_3$ | $CH_3$ | H | 1-0 | |
| 4-$O(CH_2)_3CH_3$ | $CH_3$ | H | 1-0 | |
| 2-$NO_2$ | $CH_3$ | H | 1-0 | |
| 2-$CO_2CH_3$ | $CH_3$ | H | 1-0 | |
| 2-$CO_2CH_3$ | H | H | 1-0 | |
| 4-$CO_2CH_2CH_3$ | $CH_3$ | H | 1-0 | |
| 2-$CO_2CH(CH_3)_2$ | $C_2H_5$ | H | 1-0 | |
| 2-$CO_2(CH_2)_3CH_3$ | $CH_3$ | H | 1-0 | |
| 2-$SCH_3$ | $CH_3$ | H | 1-0 | |
| 4-$SCH(CH_3)_2$ | $CH_3$ | H | 1-0 | |
| 2-$S(O)CH_3$ | $CH_3$ | H | 1-0 | |
| 4-$S(O)CH_2CH_2CH_3$ | $CH_3$ | H | 1-0 | |
| 2-$SO_2CH_3$ | $CH_3$ | H | 1-0 | |
| 4-$SO_2C_2H_5$ | $CH_3$ | $CH_3$ | 1-0 | |
| 2-$SO_2CH(CH_3)_2$ | $CH_3$ | H | 3-0 | |
| 2-$CH_3$ | $CH_3$ | H | 1-0 | |
| 2-$OCH_3$ | $CH_3$ | H | 1-0 | |

Table XIXa

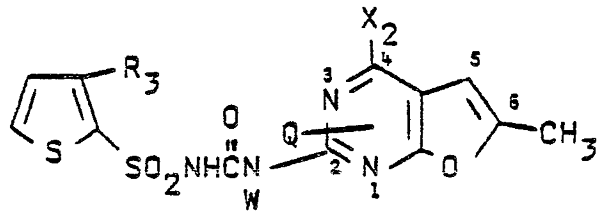

| $R_3$ | $X_2$ | W | Q | m.p.(°C) |
|---|---|---|---|---|
| H | $CH_3$ | H | 1-0 | |
| Cl | $CH_3$ | H | 1-0 | |
| $OC_2H_5$ | $C_2H_5$ | H | 1-0 | |
| $CH_3$ | $CH_3$ | H | 1-0 | |
| $CH(CH_3)C_2H_5$ | $CH_3$ | H | 1-0 | |
| $OCH_3$ | $C_2H_5$ | H | 1-0 | |
| $O(CH_2)_3CH_3$ | $CH_3$ | H | 3-0 | |
| $OCH(CH_3)_2$ | $CH_3$ | H | 1-0 | |
| $C_2H_5$ | $CH_3$ | H | 1-0 | |
| Br | $CH_3$ | H | 3-0 | |
| $NO_2$ | $CH_3$ | H | 1-0 | |
| $SO_2N(CH_3)_2$ | $CH_3$ | H | 1-0 | |
| $SO_2N(CH_3)_2$ | H | H | 1-0 | |
| $SO_2N(CH(CH_3)C_2H_5)CH_3$ | $CH_3$ | H | 3-0 | |
| $SO_2N(CH(CH_3)_2)CH_3$ | $C_2H_5$ | H | 1-0 | |
| $SO_2N(CH_3)C_2H_5$ | $C_2H_5$ | $CH_3$ | 1-0 | |
| $SO_2N(C_2H_5)_2$ | $CH_3$ | H | 1-0 | |
| $SO_2N(OCH_3)CH_3$ | $CH_3$ | H | 1-0 | |

$COR_4$

| $R_4$ | $X_2$ | W | Q |
|---|---|---|---|
| $OCH_3$ | $CH_3$ | H | 1-0 |
| $OCH_3$ | $CH_3$ | $CH_3$ | 1-0 |
| $OCH_3$ | $C_2H_5$ | H | 1-0 |

Table XIXa (continued)

| $R_4$ | $X_2$ | W | Q | m.p.(°C) |
|---|---|---|---|---|
| $OCH_3$ | $CH_3$ | H | 3-0 | |
| $OCH_3$ | $C_2H_5$ | $CH_3$ | 1-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 3-0 | |
| $OCH(CH_3)C_2H_5$ | $CH_3$ | H | 1-0 | |
| 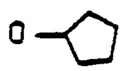 | $CH_3$ | H | 1-0 | |
| 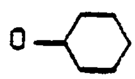 | $CH_3$ | H | 1-0 | |
| $OCH_2CH=CH_2$ | $CH_3$ | H | 1-0 | |
| $OCH(CH_3)CH=CH_2$ | $CH_3$ | H | 1-0 | |
| $OCH_2C(CH_3)=CH_2$ | $CH_3$ | H | 1-0 | |
| $O(CH_2)_4CH=CH_2$ | $CH_3$ | H | 1-0 | |
| $OCH_2C≡CH$ | $CH_3$ | H | 3-0 | |
| $OCH_2C≡C(CH_2)_2CH_3$ | $CH_3$ | H | 1-0 | |
| $OCH_2C≡CCH_3$ | $CH_3$ | H | 1-0 | |
| $OCH(CH_3)C≡CCH_3$ | $CH_3$ | H | 3-0 | |
| $OCH_2CCl_3$ | $CH_3$ | H | 1-0 | |
| $OCH_2CH_2F$ | $C_2H_5$ | H | 1-0 | |
| $OCH_2CH_2CH_2Br$ | $CH_3$ | $CH_3$ | 1-0 | |
| $O(CH_2)_5CH_2Cl$ | $CH_3$ | H | 3-0 | |
| $OCH_2CHFCH_2F$ | $CH_3$ | H | 1-0 | |

Table XIXa (continued)

| $R_4$ | $X_2$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 1-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 1-0 | |
| $OCH_2OCH_3$ | $CH_3$ | H | 1-0 | |
| $OCH_2OCH_2CH(CH_3)_2$ | $CH_3$ | H | 1-0 | |
| $OCH_2OCH_2CH_2OCH_3$ | $CH_3$ | H | 1-0 | |

Table XIXb

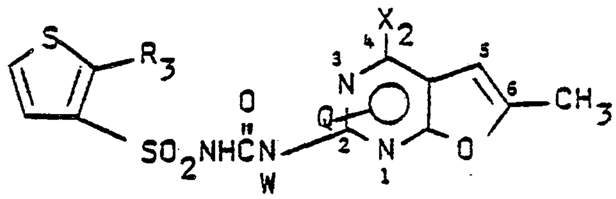

| $R_3$ | $X_2$ | W | Q | m.p.(°C) |
|---|---|---|---|---|
| H | $CH_3$ | H | 1-0 | |
| Cl | $CH_3$ | H | 1-0 | |
| $OC_2H_5$ | $C_2H_5$ | H | 1-0 | |
| $CH_3$ | $CH_3$ | H | 1-0 | |
| $CH(CH_3)C_2H_5$ | $CH_3$ | H | 1-0 | |
| $OCH_3$ | $C_2H_5$ | H | 1-0 | |
| $O(CH_2)_3CH_3$ | $CH_3$ | H | 3-0 | |
| $OCH(CH_3)_2$ | $CH_3$ | H | 1-0 | |
| $C_2H_5$ | $CH_3$ | H | 1-0 | |
| Br | $CH_3$ | H | 3-0 | |
| $NO_2$ | $CH_3$ | H | 1-0 | |
| $SO_2N(CH_3)_2$ | $CH_3$ | H | 1-0 | |
| $SO_2N(CH_3)_2$ | H | H | 1-0 | |
| $SO_2N(CH(CH_3)C_2H_5)CH_3$ | $CH_3$ | H | 3-0 | |
| $SO_2N(CH(CH_3)_2)CH_3$ | $C_2H_5$ | H | 1-0 | |
| $SO_2N(CH_3)C_2H_5$ | $C_2H_5$ | $CH_3$ | 1-0 | |
| $SO_2N(C_2H_5)_2$ | $CH_3$ | H | 1-0 | |
| $SO_2N(OCH_3)CH_3$ | $CH_3$ | H | 1-0 | |

$COR_4$

| $R_4$ | $X_2$ | W | Q |
|---|---|---|---|
| $OCH_3$ | $CH_3$ | H | 1-0 |
| $OCH_3$ | $CH_3$ | $CH_3$ | 1-0 |
| $OCH_3$ | $C_2H_5$ | H | 1-0 |

Table XIXb (continued)

| $R_4$ | $X_2$ | W | Q | m.p.(°C) |
|---|---|---|---|---|
| $OCH_3$ | $CH_3$ | H | 3-0 | |
| $OCH_3$ | $C_2H_5$ | $CH_3$ | 1-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 3-0 | |
| $OCH(CH_3)C_2H_5$ | $CH_3$ | H | 1-0 | |
| 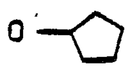 | $CH_3$ | H | 1-0 | |
| 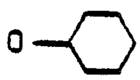 | $CH_3$ | H | 1-0 | |
| $OCH_2CH{=}CH_2$ | $CH_3$ | H | 1-0 | |
| $OCH(CH_3)CH{=}CH_2$ | $CH_3$ | H | 1-0 | |
| $OCH_2C(CH_3){=}CH_2$ | $CH_3$ | H | 1-0 | |
| $O(CH_2)_4CH{=}CH_2$ | $CH_3$ | H | 1-0 | |
| $OCH_2C{\equiv}CH$ | $CH_3$ | H | 3-0 | |
| $OCH_2C{\equiv}C(CH_2)_2CH_3$ | $CH_3$ | H | 1-0 | |
| $OCH_2C{\equiv}CCH_3$ | $CH_3$ | H | 1-0 | |
| $OCH(CH_3)C{\equiv}CCH_3$ | $CH_3$ | H | 3-0 | |
| $OCH_2CCl_3$ | $CH_3$ | H | 1-0 | |
| $OCH_2CH_2F$ | $C_2H_5$ | H | 1-0 | |
| $OCH_2CH_2CH_2Br$ | $CH_3$ | $CH_3$ | 1-0 | |
| $O(CH_2)_5CH_2Cl$ | $CH_3$ | H | 3-0 | |
| $OCH_2CHFCH_2F$ | $CH_3$ | H | 1-0 | |

Table XIXb (continued)

| $R_4$ | $X_2$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 1-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 1-0 | |
| $OCH_2OCH_3$ | $CH_3$ | H | 1-0 | |
| $OCH_2OCH_2CH(CH_3)_2$ | $CH_3$ | H | 1-0 | |
| $OCH_2OCH_2CH_2OCH_3$ | $CH_3$ | H | 1-0 | |

Table XIXc

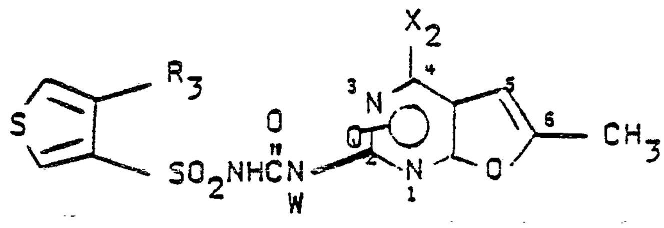

| $R_3$ | $X_2$ | W | Q | m.p.(°C) |
|---|---|---|---|---|
| H | $CH_3$ | H | 1-O | |
| Cl | $CH_3$ | H | 1-O | |
| $OC_2H_5$ | $C_2H_5$ | H | 1-O | |
| $CH_3$ | $CH_3$ | H | 1-O | |
| $CH(CH_3)C_2H_5$ | $CH_3$ | H | 1-O | |
| $OCH_3$ | $C_2H_5$ | H | 1-O | |
| $O(CH_2)_3CH_3$ | $CH_3$ | H | 3-O | |
| $OCH(CH_3)_2$ | $CH_3$ | H | 1-O | |
| $C_2H_5$ | $CH_3$ | H | 1-O | |
| Br | $CH_3$ | H | 3-O | |
| $NO_2$ | $CH_3$ | H | 1-O | |
| $SO_2N(CH_3)_2$ | $CH_3$ | H | 1-O | |
| $SO_2N(CH_3)_2$ | H | H | 1-O | |
| $SO_2N(CH(CH_3)C_2H_5)CH_3$ | $CH_3$ | H | 3-O | |
| $SO_2N(CH(CH_3)_2)CH_3$ | $C_2H_5$ | H | 1-O | |
| $SO_2N(CH_3)C_2H_5$ | $C_2H_5$ | $CH_3$ | 1-O | |
| $SO_2N(C_2H_5)_2$ | $CH_3$ | H | 1-O | |
| $SO_2N(OCH_3)CH_3$ | $CH_3$ | H | 1-O | |

$COR_4$

| $R_4$ | $X_2$ | W | Q |
|---|---|---|---|
| $OCH_3$ | $CH_3$ | H | 1-O |
| $OCH_3$ | $CH_3$ | $CH_3$ | 1-O |
| $OCH_3$ | $C_2H_5$ | H | 1-O |

Table XIXc (continued)

| $R_4$ | $X_2$ | W | Q | m.p.(°C) |
|---|---|---|---|---|
| $OCH_3$ | $CH_3$ | H | 3-0 | |
| $OCH_3$ | $C_2H_5$ | $CH_3$ | 1-0 | |
| $OCH_2CH_3$ | $CH_3$ | H | 1-0 | |
| $O(CH_2)_5CH_3$ | $CH_3$ | H | 1-0 | |
| $OCH(CH_3)_2$ | $CH_3$ | H | 3-0 | |
| $OCH(CH_3)C_2H_5$ | $CH_3$ | H | 1-0 | |
| 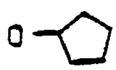 | $CH_3$ | H | 1-0 | |
| 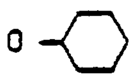 | $CH_3$ | H | 1-0 | |
| $OCH_2CH=CH_2$ | $CH_3$ | H | 1-0 | |
| $OCH(CH_3)CH=CH_2$ | $CH_3$ | H | 1-0 | |
| $OCH_2C(CH_3)=CH_2$ | $CH_3$ | H | 1-0 | |
| $O(CH_2)_4CH=CH_2$ | $CH_3$ | H | 1-0 | |
| $OCH_2C\equiv CH$ | $CH_3$ | H | 3-0 | |
| $OCH_2C\equiv C(CH_2)_2CH_3$ | $CH_3$ | H | 1-0 | |
| $OCH_2C\equiv CCH_3$ | $CH_3$ | H | 1-0 | |
| $OCH(CH_3)C\equiv CCH_3$ | $CH_3$ | H | 3-0 | |
| $OCH_2CCl_3$ | $CH_3$ | H | 1-0 | |
| $OCH_2CH_2F$ | $C_2H_5$ | H | 1-0 | |
| $OCH_2CH_2CH_2Br$ | $CH_3$ | $CH_3$ | 1-0 | |
| $O(CH_2)_5CH_2Cl$ | $CH_3$ | H | 3-0 | |
| $OCH_2CHFCH_2F$ | $CH_3$ | H | 1-0 | |

Table XIXc (continued)

| $R_4$ | $X_2$ | W | Q | m.p.(°C) |
|---|---|---|---|---|
| $OCH_2CHClCHClCH_3$ | $CH_3$ | H | 1-0 | |
| $OCH_2CH_2Br$ | $C_2H_5$ | $CH_3$ | 1-0 | |
| $OCH_2CH_2OCH_3$ | $CH_3$ | H | 1-0 | |
| $(OCH_2CH_2)_2OC_2H_5$ | $CH_3$ | H | 3-0 | |
| $OCH_2CH_2CH_2OCH_3$ | $CH_3$ | H | 1-0 | |
| $OCH_2OCH_3$ | $CH_3$ | H | 1-0 | |
| $OCH_2OCH_2CH(CH_3)_2$ | $CH_3$ | H | 1-0 | |
| $OCH_2OCH_2CH_2OCH_3$ | $CH_3$ | H | 1-0 | |

Table XX

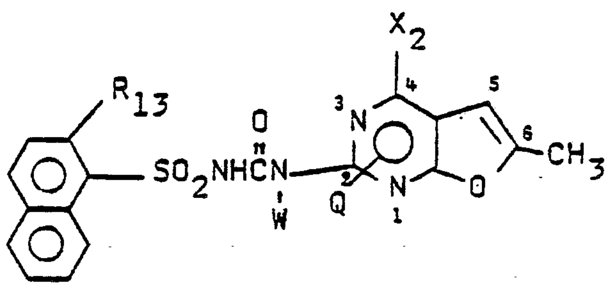

| $R_{13}$ | $X_2$ | W | Q | m.p.(°C) |
|---|---|---|---|---|
| H | $CH_3$ | H | 1-O | |
| $CH_3$ | $CH_3$ | H | 1-O | |
| $C_2H_5$ | $CH_3$ | H | 1-O | |
| $(CH_2)_4H$ | $CH_3$ | H | 1-O | |
| $OCH_3$ | $CH_3$ | H | 1-O | |
| $OC_2H_5$ | $CH_3$ | $CH_3$ | 3-O | |
| $O(CH_2)_4H$ | $CH_3$ | H | 1-O | |
| Cl | $CH_3$ | H | 1-O | |
| $SCH_3$ | $CH_3$ | H | 1-O | |
| $SC_2H_5$ | $C_2H_5$ | H | 3-O | |
| $NO_2$ | $CH_3$ | H | 1-O | |
| $SO_2N(CH_3)_2$ | $CH_3$ | H | 1-O | |
| $SO_2NCH(CH_3)C_2H_5$ | $CH_3$ | H | 1-O | |
| $SO_2N[CH(CH_3)_2]C_2H_5$ | $CH_3$ | H | 1-O | |
| $S(O)CH_3$ | $CH_3$ | H | 1-O | |
| $S(O)(CH_2)_4H$ | $CH_3$ | H | 1-O | |
| $SO_2CH_3$ | $CH_3$ | H | 1-O | |
| $SO_2(CH_2)_3H$ | $CH_3$ | H | 1-O | |

The carboxylic acids of Tables XXI(a-p) are useful as intermediates for the preparation of compounds of Formula I:

Table XXIa

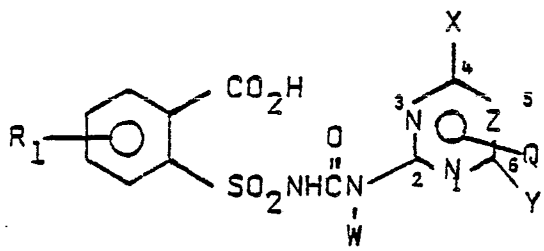

| $R_1$ | W | X | Y | Z | Q | m.p.(°C) |
|---|---|---|---|---|---|---|
| H | H | $CH_3$ | $CH_3$ | N | 5-O | |
| H | H | $CH_3$ | $OCH_3$ | N | 3-O | |
| H | H | $OCH_3$ | $OCH_3$ | N | 1-O | |
| H | H | $CH_3$ | $CH_3$ | CH | 1-O | |
| H | H | $CH_3$ | $OCH_3$ | CH | 3-O | |
| H | H | $OCH_3$ | $OCH_3$ | CH | 1-O | |
| H | H | $OCH_3$ | $CH_3$ | CF | 1-O | |
| 5-Cl | H | $CH_3$ | $CH_3$ | N | 1-O | |
| H | $CH_3$ | $OCH_3$ | $OCH_3$ | CH | 1-O | |

Table XXIb

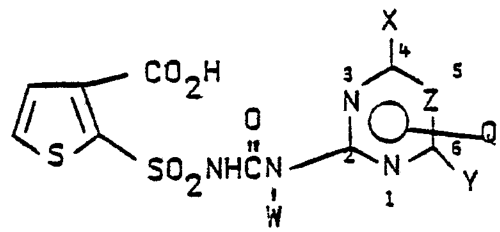

| W | X | Y | Z | Q |
|---|---|---|---|---|
| H | $CH_3$ | $CH_3$ | CH | 1-O |
| H | $CH_3$ | $OCH_3$ | CH | 3-O |
| H | $OCH_3$ | $OCH_3$ | CH | 1-O |
| H | $CH_3$ | $OCH_3$ | N | 3-O |
| H | $CH_3$ | $CH_3$ | N | 1-O |
| H | $OCH_3$ | $OCH_3$ | N | 1-O |
| $CH_3$ | $CH_3$ | $CH_3$ | N | 5-O |

Table XXIc

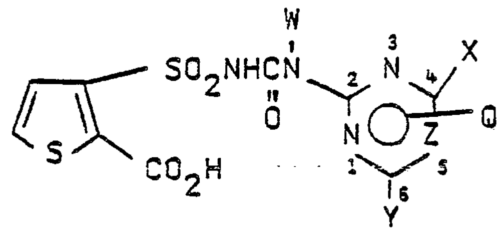

| W | X | Y | Z | Q |
|---|---|---|---|---|
| H | $CH_3$ | $CH_3$ | CH | 1-O |
| H | $CH_3$ | $OCH_3$ | CH | 3-O |
| H | $OCH_3$ | $OCH_3$ | CH | 1-O |
| H | $CH_3$ | $OCH_3$ | N | 3-O |
| H | $CH_3$ | $CH_3$ | N | 1-O |
| H | $OCH_3$ | $OCH_3$ | N | 1-O |
| $CH_3$ | $CH_3$ | $CH_3$ | N | 5-O |

Table XXId

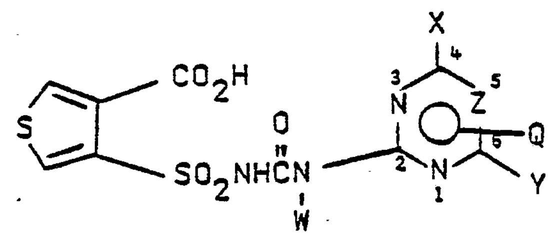

| W | X | Y | Z | Q |
|---|---|---|---|---|
| H | $CH_3$ | $CH_3$ | CH | 1-O |
| H | $CH_3$ | $OCH_3$ | CH | 3-O |
| H | $OCH_3$ | $OCH_3$ | CH | 1-O |
| H | $CH_3$ | $OCH_3$ | N | 3-O |
| H | $CH_3$ | $CH_3$ | N | 1-O |
| H | $OCH_3$ | $OCH_3$ | N | 1-O |
| $CH_3$ | $CH_3$ | $CH_3$ | N | 5-O |

Table XXIe

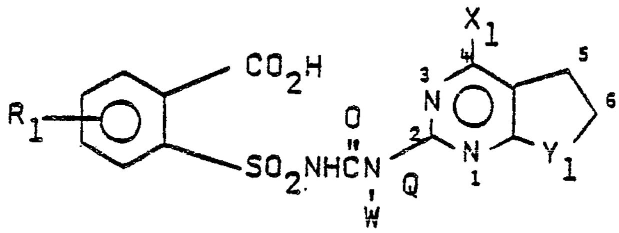

| $R_1$ | W | $X_1$ | $Y_1$ | Q | m.p.(°C) |
|---|---|---|---|---|---|
| H | H | $CH_3$ | $CH_2$ | 3-0 | |
| H | H | $CH_3$ | 0 | 1-0 | |
| H | H | $OCH_3$ | 0 | 1-0 | |
| H | H | $OCH_3$ | $CH_2$ | 3-0 | |
| H | H | Cl | 0 | 1-0 | |
| H | $CH_3$ | $CH_3$ | 0 | 1-0 | |
| 5-$NO_2$ | H | $OC_2H_5$ | 0 | 3-0 | |

Table XXIf

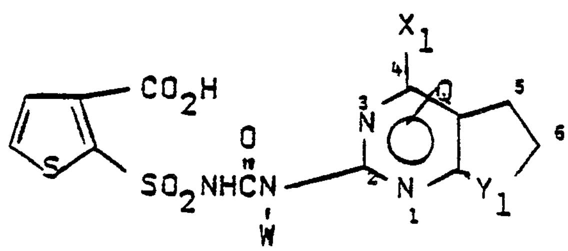

| W | $X_1$ | $Y_1$ | Q | m.p.(°C) |
|---|---|---|---|---|
| H | $CH_3$ | 0 | 1-0 | |
| H | $CH_3$ | $CH_2$ | 3-0 | |
| H | $OCH_3$ | 0 | 1-0 | |
| H | $OCH_3$ | $CH_2$ | 3-0 | |
| $CH_3$ | $CH_3$ | 0 | 1-0 | |

Table XXIg

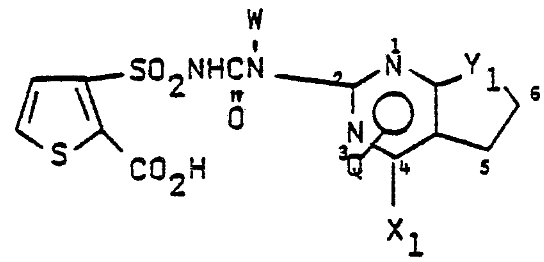

| W | $X_1$ | $Y_1$ | Q | m.p.(°C) |
|---|---|---|---|---|
| H | $CH_3$ | O | 1-O | |
| H | $CH_3$ | $CH_2$ | 3-O | |
| H | $OCH_3$ | O | 1-O | |
| H | $OCH_3$ | $CH_2$ | 3-O | |
| $CH_3$ | $CH_3$ | O | 1-O | |

Table XXIh

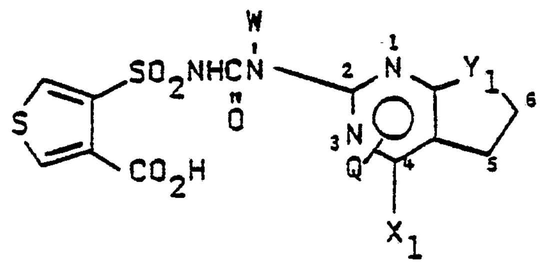

| W | $X_1$ | $Y_1$ | Q | m.p.(°C) |
|---|---|---|---|---|
| H | $CH_3$ | O | 1-O | |
| H | $CH_3$ | $CH_2$ | 3-O | |
| H | $OCH_3$ | O | 1-O | |
| H | $OCH_3$ | $CH_2$ | 3-O | |
| $CH_3$ | $CH_3$ | O | 1-O | |

Table XXIi

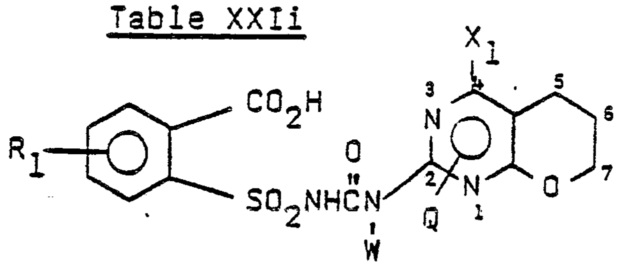

| R1 | W | X1 | Q | m.p.(°C) |
|---|---|---|---|---|
| H | H | $CH_3$ | 1-O | |
| H | H | $OCH_3$ | 3-O | |
| H | H | Cl | 1-O | |
| 6-Cl | H | $CH_3$ | 1-O | |
| H | $CH_3$ | $OCH_3$ | 3-O | |

Table XXIj

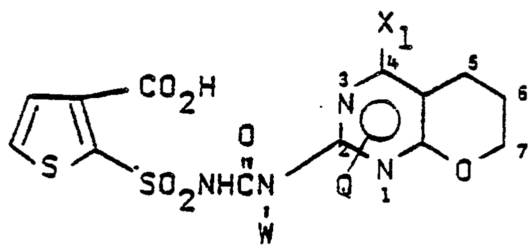

| W | X1 | Q | m.p.(°C) |
|---|---|---|---|
| H | $OCH_3$ | 3-O | |
| H | $CH_3$ | 1-O | |
| H | H | 1-O | |
| $CH_3$ | Cl | 1-O | |

Table XXIk

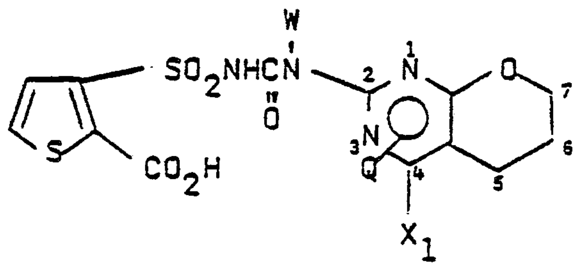

| W | $X_1$ | Q | m.p.(°C) |
|---|---|---|---|
| H | $OCH_3$ | 3-O | |
| H | $CH_3$ | 1-O | |
| H | H | 1-O | |
| $CH_3$ | Cl | 1-O | |

Table XXIl

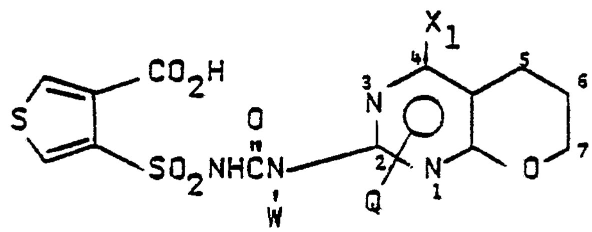

| W | $X_1$ | Q | m.p.(°C) |
|---|---|---|---|
| H | $OCH_3$ | 3-O | |
| H | $CH_3$ | 1-O | |
| H | H | 1-O | |
| $CH_3$ | Cl | 1-O | |

Table XXIm

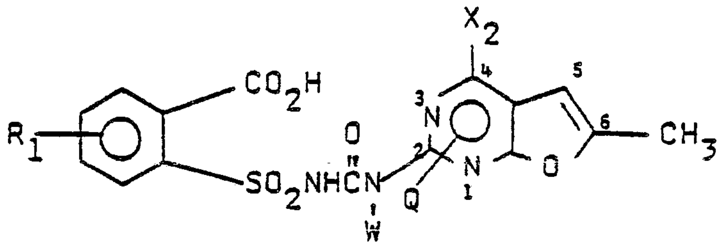

| $R_1$ | W | $X_2$ | Q | m.p.(°C) |
|---|---|---|---|---|
| H | H | $CH_3$ | 1-O | |
| H | H | $C_2H_5$ | 1-O | |
| 5-Br | H | $CH_3$ | 1-O | |
| H | $CH_3$ | $CH_3$ | 3-O | |

Table XXIn

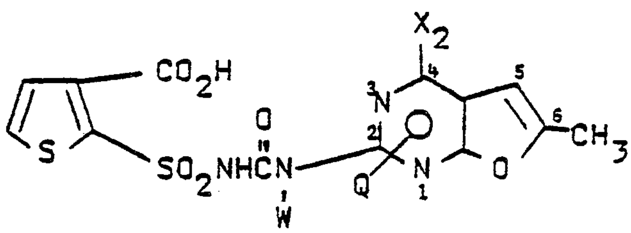

| W | $X_2$ | Q | m.p.(°C) |
|---|---|---|---|
| H | $CH_3$ | 1-O | |
| H | $C_2H_5$ | 1-O | |
| $CH_3$ | $CH_3$ | 3-O | |

Table XXIo

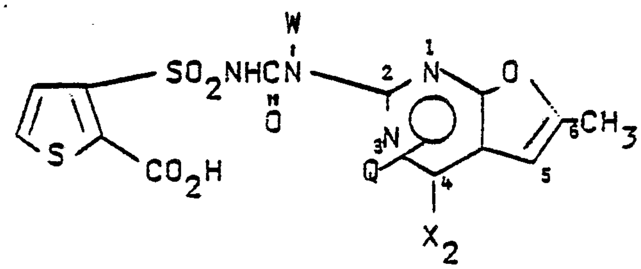

| W | $X_2$ | Q | m.p.(°C) |
|---|---|---|---|
| H | $CH_3$ | 1-O | |
| H | $C_2H_5$ | 1-O | |
| $CH_3$ | $CH_3$ | 3-O | |

Table XXIo

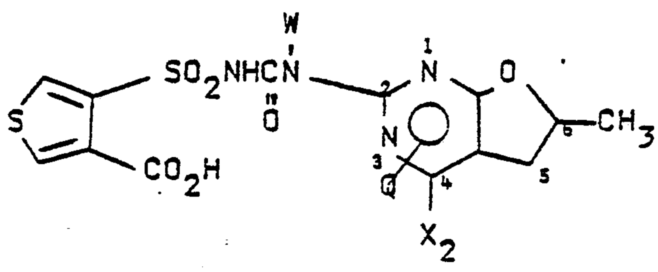

| W | $X_2$ | Q | m.p.(°C) |
|---|---|---|---|
| H | $CH_3$ | 1-O | |
| H | $C_2H_5$ | 1-O | |
| $CH_3$ | $CH_3$ | 3-O | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

TABLE XXII

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley & Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

## Example 9

**Wettable Powder**

| | |
|---|---|
| 2-chloro-N-[(4,6-dimethyl-1-oxidepyrimidin-2-yl)-aminocarbonyl]benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 10

Wettable Powder

| | |
|---|---|
| 2-chloro-N-[(4-methoxy-6-methyl-1-oxidepyrimidin-2-yl)-aminocarbonyl]benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 11

Granule

| | |
|---|---|
| Wettable Powder of Example 10 | 5% |
| attapulgite granules (U.S.S. 20-40 mesh; 0.84-0.42 mm) | 95% |

A slurry of wettable powder containing ≈ 25% solids is sprayed on the surface of attapulgite granules in a double-cone blender. The granules are dried and packaged.

Example 12

Extruded Pellet

| | |
|---|---|
| 2-[[(4,6-dimethyl-1-oxidepyrimidin-2-yl)aminocarbonyl]-aminosulfonyl]benzoic acid, methyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed

to pass a U.S.S. No. 20 sieve (0.84 mm openings). The granules held on a U.S.S. No. 40 sieve (0.42 mm openings) may be packaged for use and the fines recycled.

Example 13

Oil Suspension

| | |
|---|---|
| 2-nitro-N-[(4-methoxy-6-methyl-1-oxidepyrimidin-2-yl)-aminocarbonyl]benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 14

Wettable Powder

| | |
|---|---|
| 2-nitro-N-[(4,6-dimethyl-1-oxidepyrimidin-2-yl)-aminocarbonyl]benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 15

Low Strength Granule

| | |
|---|---|
| 2-[[(4,6-dimethyl-1-oxidepyrimidin-2-yl)aminocarbonyl]-aminosulfonyl]benzoic acid, methyl ester | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 16

Aqueous Suspension

| | |
|---|---|
| 2-[[(4-methoxy-6-methyl-1-oxidepyrimidin-2-yl)aminocarbonyl]aminosulfonyl]benzoic acid, methyl ester | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 17

Solution

| | |
|---|---|
| 2-[[(4-methoxy-6-methyl-1-oxidepyrimidin-2-yl)aminocarbonyl]aminosulfonyl]benzoic acid, 1-methylethyl ester, sodium salt | 5% |
| water | 95% |

The salt is added directly to the water with stirring to produce the solution, which may then be packaged for use.

Example 18

Low Strength Granule

2-[[(4-methoxy-6-methyl-1-oxidepyrimidin-2-yl)aminocarbonyl]aminosulfonyl]benzoic acid, 2-propenyl ester 0.1%

attapulgite granules (U.S.S. 20-40 mesh) 99.9%

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 19

Granule

2-chloro-N-[(4-methoxy-6-methyl-1-oxidepyrimidin-2-yl)-aminocarbonyl]benzenesulfonamide 80%

wetting agent 1%

crude ligninsulfonate salt (containing 5-20% of the natural sugars) 10%

attapulgite clay 9%

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 20

High Strength Concentrate

| | |
|---|---|
| 2-chloro-N-[(4,6-dimethyl-1-oxidepyrimidin-2-yl)-aminocarbonyl]benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 21

Wettable Powder

| | |
|---|---|
| 2-chloro-N-[(4-methoxy-6-methyl-1-oxidepyrimidin-2-yl)-aminocarbonyl]benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 22

Wettable Powder

| | |
|---|---|
| 2-nitro-N-[(4-methoxy-6-methyl-1-oxidepyrimidin-2-yl)-aminocarbonyl]benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

## Example 23

Oil Suspension

| | |
|---|---|
| 2-chloro-N-[(4,6-dimethyl-1-oxidepyrimidin-2-yl)-aminocarbonyl]benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

## Example 24

Dust

| | |
|---|---|
| 2-[[(4-methoxy-6-methyl-1-oxidepyrimidin-2-yl)aminocarbonyl]aminosulfonyl]benzoic acid, 1-methylethyl ester | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

UTILITY

The compounds of the present invention are active herbicides. They have utility for broad-spectrum pre- and/or post-emergence weed control in areas where complete control of all vegetation is desired, such as under fences, around fuel storage tanks, ammunition depots, industrial storage areas, parking lots, drive-in theaters, around billboards, highway and railroad structures. Alternatively, the subject compounds are useful as growth regulants. For such use the compounds are applied in an amount which is effective but substantially non-phytotoxic to the crop plants.

The rates of application for the compounds of the invention are determined by a number of factors, including their use as growth regulants or herbicides, the crop species involved, the types of weeds to be controlled, weather and climate, formulations selected, mode of application, amount of foliage present, etc. In general terms, the subject compounds should be applied at levels of around 0.125 to 10 kg/ha, the lower rates being suggested for use on lighter soils and/or those having a low organic matter content, for plant growth regulation or for situations where only short-term persistence is required.

The compounds of the invention may be used in combination with any other commercial herbicide examples of which are those of the triazine, triazole, uracil, urea, amide, diphenylether, carbamate and bipyridylium types.

The herbicidal and plant growth regulating properties of the subject compounds were discovered in a number of greenhouse tests. The test procedures and results follow.

Test A

Seeds of crabgrass (Digitaria spp.), barnyardgrass (Echinochloa crusgalli), wild oats (Avena fatua), cassia (Cassia tora), morningglory (Ipomoea spp.), cocklebur (Xanthium spp.), sorghum, corn, soybean, rice, wheat and nutsedge tubers (Cyperus rotundus) were planted in a growth medium and treated pre-emergence with the chemicals dissolved in a non-phytotoxic solvent. At the same time, cotton having five leaves (including cotyledonary ones), bush beans with the second trifoliate leaf expanding, crabgrass with two leaves, barnyardgrass with two leaves, wild oats with one leaf, cassia with three leaves (including cotyledonary ones), morningglory and cocklebur with four leaves (including the cotyledonary ones), sorghum and corn with three leaves, soybean with two cotyledonary leaves, rice with two leaves, wheat with two leaves, and nutsedge with three-five leaves were sprayed. Treated plants and controls were maintained in a greenhouse for sixteen days, then all treated plants were compared with their respective controls and rated visually for response to treatment.

The ratings are based on a numerical scale extending from 0 = no injury, to 10 = complete kill. The accompanying descriptive symbols have the following meanings:

C = chlorosis or necrosis;
D = defoliation;
E = emergence inhibition;
G = growth retardation;
H = formative effects;
U = unusual pigmentation;
X = axillary stimulation; and
6Y = abscised buds or flowers.

Compound 1

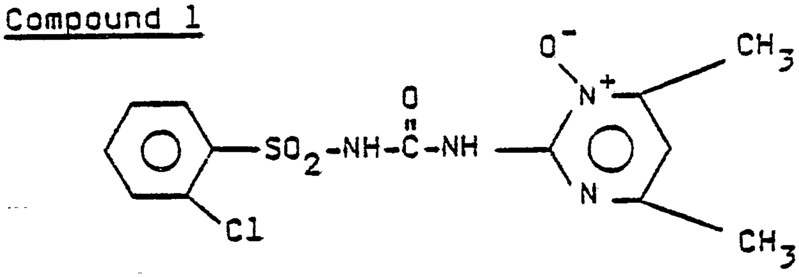

Compound 2

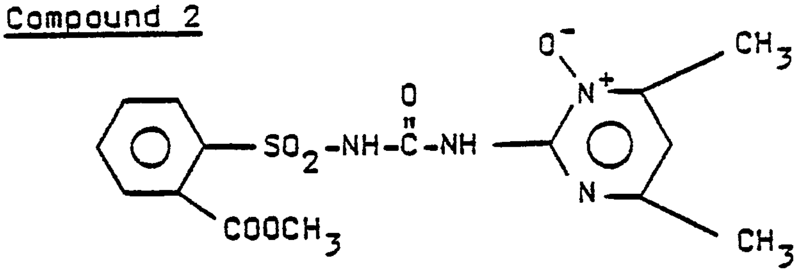

Compound 3

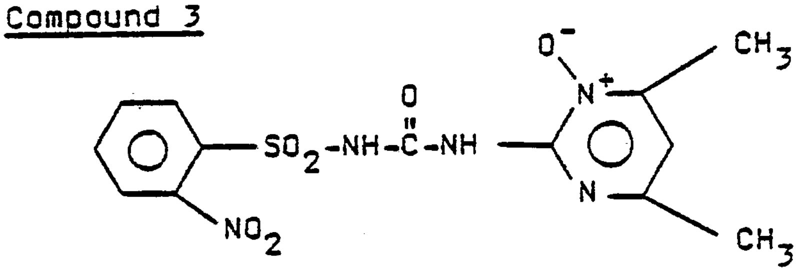

Compound 4

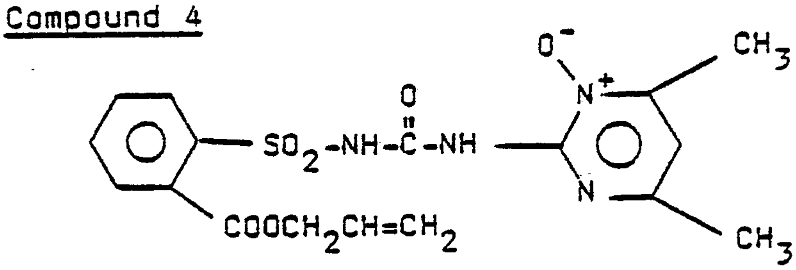

Compound 5

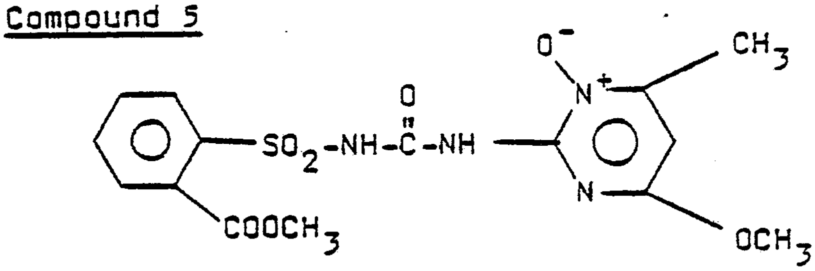

0057546



Compound 6

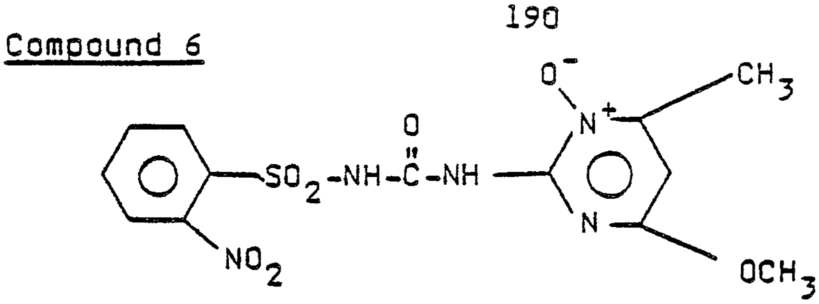

Compound 7

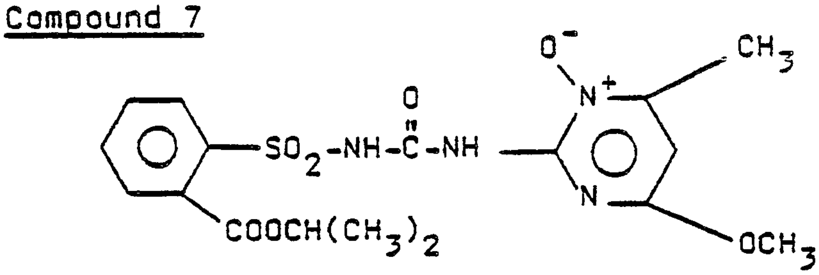

Compound 8

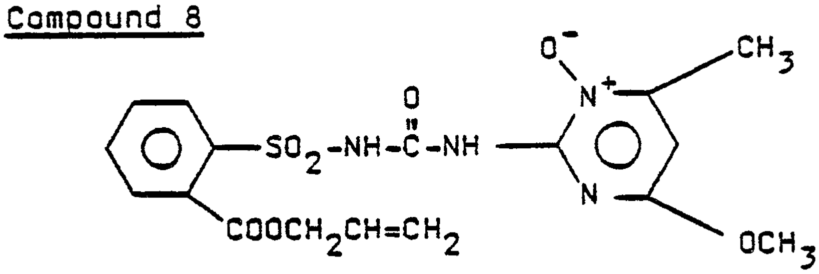

Compound 9

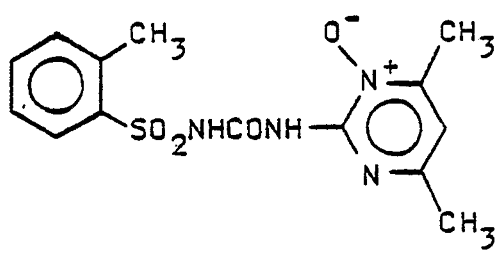

Compound 10

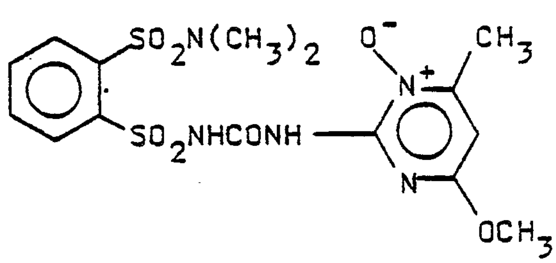

Compound 11

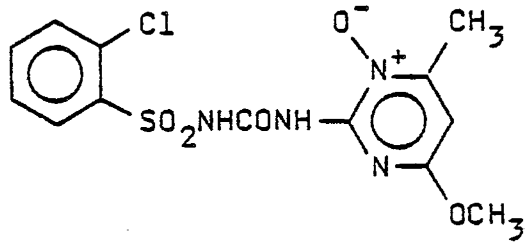

Compound 12

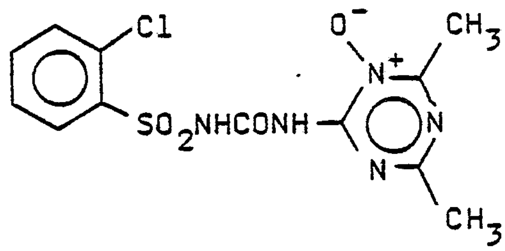

Compound 13

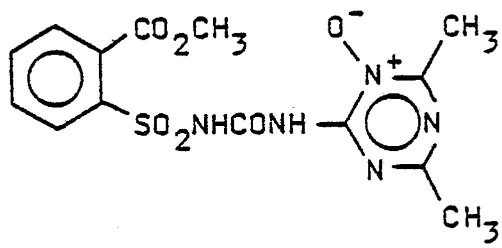

Compound 14

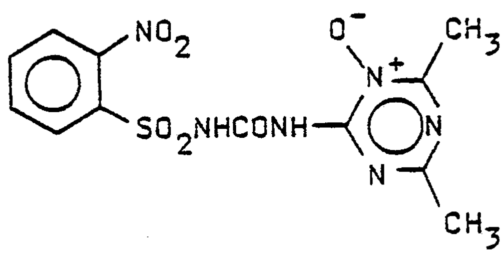

Compound 15

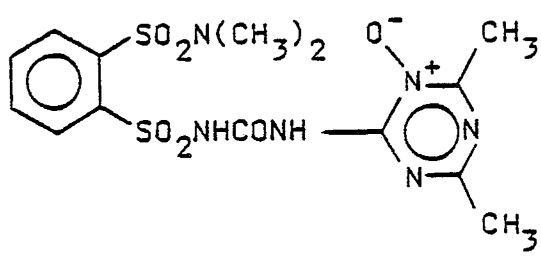

Compound 16

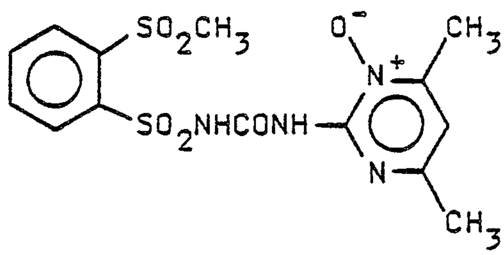

Compound 17

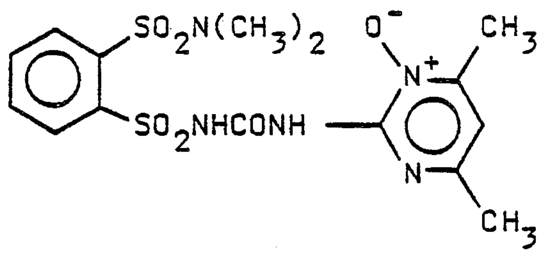

Compound 18

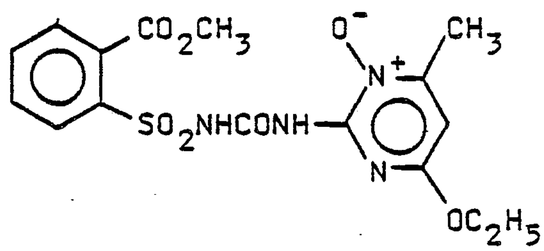

Compound 19

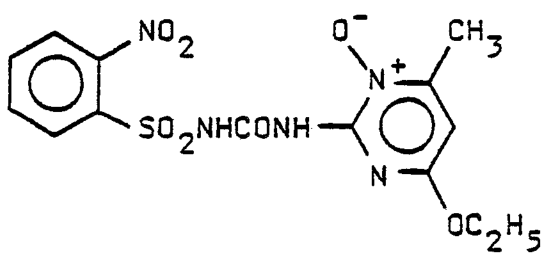

Compound 20

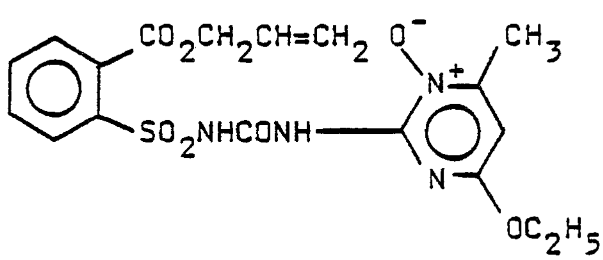

Compound 21

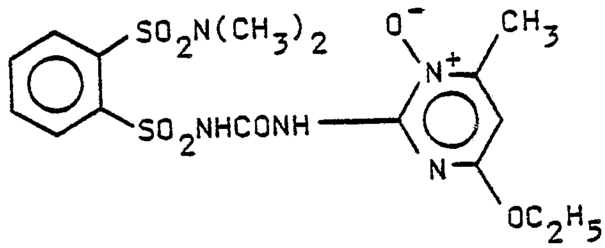

Compound 22

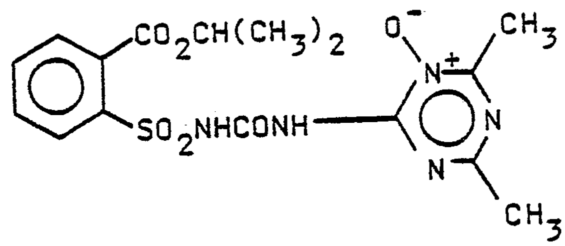

Compound 23

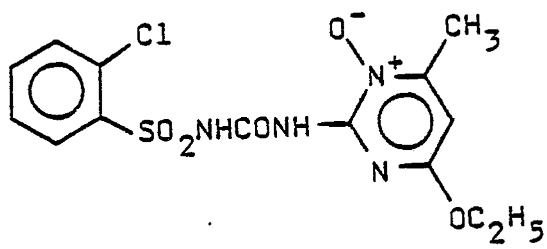

Compound 24

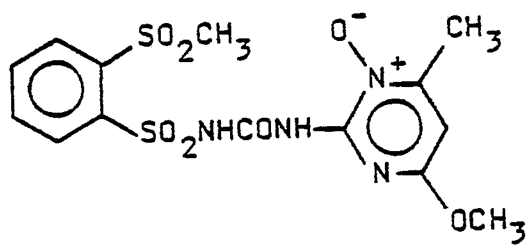

Compound 25

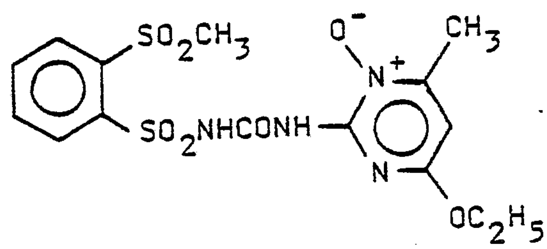

Compound 26

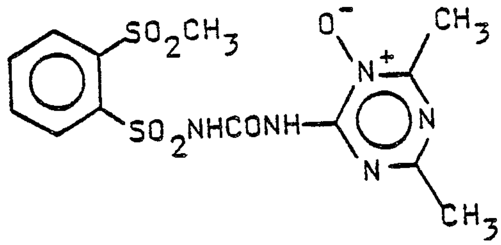

Compound 27

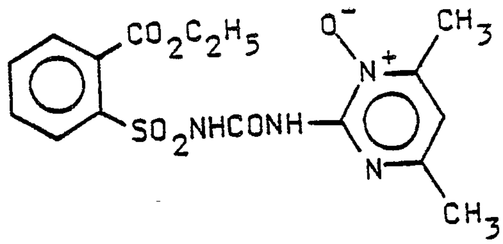

Compound 28

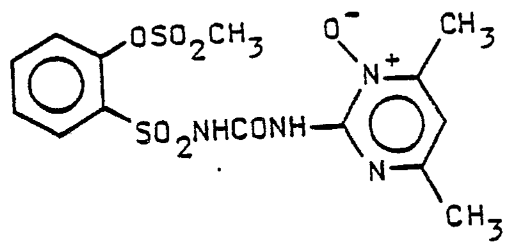

Compound 29

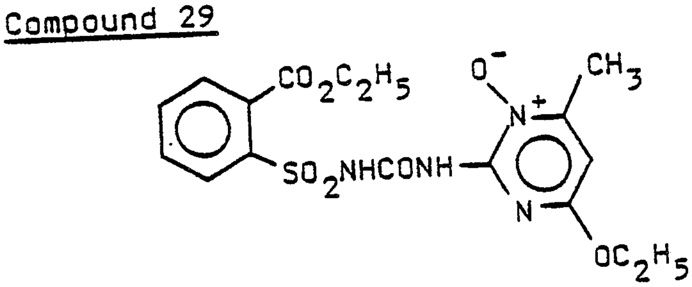

Compound 30

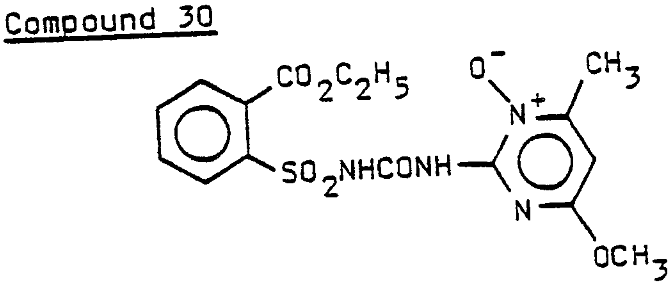

Compound 31

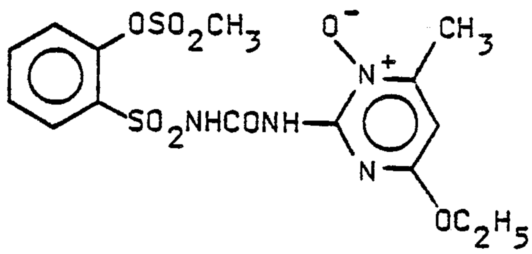

The ratings made 16 days after treatment are summarized in Table A. It may be seen that the compounds are active herbicides and that several of them are useful as plant growth regulants.

Table A

| | Compound No. | | |
|---|---|---|---|
| | 1 | 2 | 3 |
| kg/ha | 0.4 | 0.4 | 0.4 |
| Post-Emergence | | | |
| Bushbean | 6C,9G,6Y | 9C | 9C |
| Cotton | 4C,8G | 9C | 9C |
| Morningglory | 10C | 6C,9G | 9C |
| Cocklebur | 5C,9G | 9C | 6C,9G |
| Cassia | 4C,8G | 9C | 9C |
| Nutsedge | 9C | 10C | 5C,9G |
| Crabgrass | 1C,3G | 9C | 3C,9G |
| Barnyardgrass | 9C | 10C | 9C |
| Wild Oats | 3C | 6C,9G | 2C,9H |
| Wheat | 0 | 5C,9G | 1C,6G |
| Corn | 9C | 10C | 4C,9H |
| Soybean | 2C,9G | 9C | 3C,9G |
| Rice | 4C,9G | 9C | 5C,9G |
| Sorghum | 6C,9G | 10C | 3C,9G |
| Pre-Emergence | | | |
| Morningglory | 9G | 9G | 9G |
| Cocklebur | 5C,9G | 9H | 9H |
| Cassia | 3C,9G | 9G | 9C |
| Nutsedge | 2C,9G | 10E | 10E |
| Crabgrass | 2C | 3C,9G | 5C,8G |
| Barnyardgrass | 4C,9H | 2C,9H | 4C,9H |
| Wild Oats | 3C,9G | 5C,9H | 1C,9G |
| Wheat | 9G | 10E | 8G |
| Corn | 3C,9G | 10E | 9H |
| Soybean | 3H,7G | 9H | 8H |
| Rice | 10E | 10E | 10E |
| Sorghum | 3C,9H | 10H | 2C,8H |

Table A (continued)

| | Compound No. | | |
|---|---|---|---|
| | 4 | 5 | 6 |
| kg/ha | 0.4 | 0.05 | 0.05 |
| Post-Emergence | | | |
| Bushbean | 6C,9G,6Y | 4C,9G,6Y | 9C |
| Cotton | 5C,9G | 1C,6G | 6C,8G |
| Morningglory | 2C,7G | 3C,8G | 9C |
| Cocklebur | 5C,9G | 2G | 9C |
| Cassia | 3C,8G | 2C | 4C,8G |
| Nutsedge | 7C,9G | 1C,5G | 2C,9G |
| Crabgrass | 2G | 0 | 2C,5G |
| Barnyardgrass | 5C,9H | 3C,9H | 9C |
| Wild Oats | 6C,9H | 1C,5G | 2C,9H |
| Wheat | 3C,8G | 7G,5X | 7G |
| Corn | 5C,9H | 2C,9H | 3U,9G |
| Soybean | 2C,9G,5X | 3C,8G,5X | 6C,9G |
| Rice | 5C,9G | 2C,9G | 9C |
| Sorghum | 2C,9G | 2C,9H | 5C,9G |
| Pre-Emergence | | | |
| Morningglory | 7G | 2C,8G | 9G |
| Cocklebur | 9H | 9H | 9H |
| Cassia | 7G | 6G | 2C,8G |
| Nutsedge | 4G | 7G | 8G |
| Crabgrass | 0 | 2G | 2C,6G |
| Barnyardgrass | 2C,8H | 3C,8H | 1C,6G |
| Wild Oats | 1C,9G | 1C,5G | 8G |
| Wheat | 1C,9G | 5G | 2C,9H |
| Corn | 2C,9G | 2C,9G | 3C,9H |
| Soybean | 1C,3G | 2C | 2C,6H |
| Rice | 3C,7H | 9H | 9H |
| Sorghum | 2C,8H | 1C,8G | 3C,9H |

Table A (continued)

| | Compound No. 7 | Compound No. 8 |
|---|---|---|
| kg/ha | 0.05 | 0.05 |
| Post-Emergence | | |
| Bushbean | 9C | 10D,9G,6Y |
| Cotton | 6C,9G | 3C,3H,8G |
| Morningglory | 6C,9G | 6C,9G |
| Cocklebur | 6C,9G | 9C |
| Cassia | 6C,9G | 4C,6H |
| Nutsedge | 3C,9G | 2C,9G,7X |
| Crabgrass | 1C,5G | 2G |
| Barnyardgrass | 10C | 6C,9H |
| Wild Oats | 1C,6G | 1C |
| Wheat | 2C,8G | 5G |
| Corn | 5U,9G | 2C,8H |
| Soybean | 9C | 2G,8H,5X |
| Rice | 9C | 2C,8G |
| Sorghum | 10C | 2C,9G |
| Pre-Emergence | | |
| Morningglory | 9G | 9G |
| Cocklebur | 9H | 2C,9H |
| Cassia | 3C,9G | 5C,8G |
| Nutsedge | 10E | 9G |
| Crabgrass | 2C,7G | 2C |
| Barnyardgrass | 5C,9H | 2C,8H |
| Wild Oats | 3C,9H | 1C,6G |
| Wheat | 1C,9H | 1C,6G |
| Corn | 5C,9G | 3C,9G |
| Soybean | 2C,8H | 2C,5G |
| Rice | 10E | 9H |
| Sorghum | 9C | 3C,9H |

Table A (continued)

| | Compound No. 9 | 10 | 11 |
|---|---|---|---|
| kg/ha | 0.4 | 0.4 | 0.4 |
| **Post-Emergence** | | | |
| Bushbean | 5C,6G,6Y | 5C,9G,6Y | 1C,6Y |
| Cotton | 5C,8G | 6C,9G | 1C |
| Morningglory | 5C,8G | 10C | 2G |
| Cocklebur | 4C,8G | 10C | 0 |
| Cassia | 3C,5G | 5C,8H | 0 |
| Nutsedge | 2C,8G | 1C,9G | 0 |
| Crabgrass | 2C,4G | 4C,8G | 0 |
| Barnyardgrass | 2C,9H | 9C | 1C,6H |
| Wild Oats | 1C,7G | 8C | 0 |
| Wheat | 5G | 10C | 0 |
| Corn | 2U,9G | 5U,9C | 1C,7H |
| Soybean | 4C,8G | 9C | 1C,5G |
| Rice | 4C,9G | 5C,9G | 1C,7G |
| Sorghum | 2C,9H | 3U,9G | 2C,7H |
| **Pre-Emergence** | | | |
| Morningglory | 2C,7G | 9G | 8G |
| Cocklebur | 2C,7H | 9G | 9G |
| Cassia | 3C,5G | 8G | 5G |
| Nutsedge | 2C | 2C,9G | 3G |
| Crabgrass | 2C | 5C,9G | 2G |
| Barnyardgrass | 4C,8G | 6C,9H | 2C,8H |
| Wild Oats | 2C,7G | 4C,9G | 5G |
| Wheat | 2C,7G | 1C,9G | 2G |
| Corn | 2C,8G | 3U,9G | 2C,6G |
| Soybean | 2C,3H | 4C,9H | 1C |
| Rice | 9H | 10E | 1C,8H |
| Sorghum | 3C,8G | 3C,9H | 2C,8G |

Table A (continued)

| | Compound No. 12 | 13 | 14 |
|---|---|---|---|
| kg/ha | 0.4 | 0.4 | 0.4 |
| Post-Emergence | | | |
| Bushbean | 6C,8G,6Y | 9C | 10D,9G |
| Cotton | 4C,8G | 5C,9G | 4C,8G |
| Morningglory | 4C,9G | 4C,9G | 10C |
| Cocklebur | 2C,6G | 10C | 1C,3H |
| Cassia | 5C | 4C,8G | 3C,4G |
| Nutsedge | 0 | 2G | 0 |
| Crabgrass | 1C,3G | 2C,5G | 1C,3G |
| Barnyardgrass | 5C,9H | 5C,9H | 5C,9H |
| Wild Oats | 0 | 3C,9G | 0 |
| Wheat | 0 | 3G | 0 |
| Corn | 7U,9G | 3U,9G | 8U,9G |
| Soybean | 2C,8G | 3C,8G | 6C,9G |
| Rice | 3C,9G | 5C,9G | 9C |
| Sorghum | 9G,5I | 10C | 2C,9H |
| Pre-Emergence | | | |
| Morningglory | 2C,6G | 8G | 8G |
| Cocklebur | 5G | 9H | 8H |
| Cassia | 4C,5G | 4C,7G | 3C,7G |
| Nutsedge | 0 | 5G | 2C,5G |
| Crabgrass | 2G | 2C | 2C,5G |
| Barnyardgrass | 3C,8G | 2C,5G | 5C,9H |
| Wild Oats | 1C | 2C,8G | 2C,7G |
| Wheat | 2G | 0 | 7G |
| Corn | 2C,7G | 2C,7G | 5U,9G |
| Soybean | 2C | 2C,4H | 4C,5H |
| Rice | 9H | 9H | 10E |
| Sorghum | 2C,9G | 2C,8G | 2C,9G |

Table A (continued)

| | Compound No. 15 | 16 | 16 |
|---|---|---|---|
| kg/ha | 0.4 | 0.4 | 0.05 |
| Post-Emergence | | | |
| Bushbean | 9C | 6C,9G,6Y | 5C,9G,6Y |
| Cotton | 5C,9G | 5C,9G | 5C,9G |
| Morningglory | 10C | 6C,9G | 3C,9G |
| Cocklebur | 10C | 6C,9G | 4C,9G |
| Cassia | 4C,6G | 4C,9G | 4C,9G |
| Nutsedge | 2G | 2C,9G | 8G |
| Crabgrass | 2C,9G | 5C,9G | 2C,9G |
| Barnyardgrass | 9C | 9C | 6C,9H |
| Wild Oats | 6C,9G | 6C,9G | 2C,9H |
| Wheat | 6C,9G | 3C,9G | 2C,8G |
| Corn | 9C | 5C,9G | 5C,9H |
| Soybean | 9C | 5C,9G | 2C,9G |
| Rice | 6C,9G | 6C,9G | 6C,9G |
| Sorghum | 9C | 5U,9G | 2U,9G |
| Pre-Emergence | | | |
| Morningglory | 9G | 9G | 8H |
| Cocklebur | 9G | 9H | 8H |
| Cassia | 2C,5G | 2C,8G | 3C,5G |
| Nutsedge | 0 | 8G | 2G |
| Crabgrass | 2C,5G | 2C,6G | 1C |
| Barnyardgrass | 4C,9H | 4C,9H | 2C,8H |
| Wild Oats | 3C,8G | 3C,8G | 2C,8G |
| Wheat | 2C,8G | 3C,9G | 8G |
| Corn | 2U,9G | 3C,9G | 2C,8G |
| Soybean | 3C,6H | 2C,8G | 1C,1H |
| Rice | 10E | 10E | 5C,9H |
| Sorghum | 2C,9H | 5C,9H | 4C,9G |

Table A (continued)

| | Compound No. | | |
|---|---|---|---|
| | 17 | 18 | 19 |
| kg/ha | 0.4 | 0.4 | 0.4 |
| Post-Emergence | | | |
| Bushbean | 9C | 6C,9G | 9C |
| Cotton | 2U,6C,9G | 5C,4H,9G | 6C,9G |
| Morningglory | 9C | 4C,8H | 9C |
| Cocklebur | 9C | 2C,4G | 1C,5G |
| Cassia | 9C | 2C | 1C |
| Nutsedge | 9G | 1C,9G | 5G |
| Crabgrass | 6C,9G | 2C,5G | 2C,6G |
| Barnyardgrass | 10C | 6C,9H | 3C,9H |
| Wild Oats | 6C,9G | 1C,9G | 1C,8G |
| Wheat | 3U,6C,9G | 2C,9G | 2C,9G |
| Corn | 8U,9G | 9G | 2C,9G |
| Soybean | 9C | 6C,9G | 6C,9G |
| Rice | 9C | 4C,9G | 3C,9G |
| Sorghum | 10C | 4C,9G | 2C,7G |
| Pre-Emergence | | | |
| Morningglory | 5C,9G | 9G | 9G |
| Cocklebur | 9H | 8H | 8H |
| Cassia | 3C,9G | 4G | 6G |
| Nutsedge | 10E | 4G | 7G |
| Crabgrass | 5C,8G | 4G,1C | 1C,4G |
| Barnyardgrass | 5C,9H | 3C,9H | 3C,7G |
| Wild Oats | 5C,9H | 2C,7G | 3C,7G |
| Wheat | 3C,9H | 1C,9G | 2C,9H |
| Corn | 9G | 2C,9G | 5C,9H |
| Soybean | 9H | 2C,5H | 3C,7H |
| Rice | 10E | 10E | 10E |
| Sorghum | 8C,9H | 3C,9H | 4C,9H |

Table A (continued)

| | Compound No. | | |
|---|---|---|---|
| | 20 | 21 | 22 |
| kg/ha | 0.4 | 0.4 | 0.4 |
| Post-Emergence | | | |
| Bushbean | 9C | 5C,8G,6Y | 2C,4G,6Y |
| Cotton | 5C,9G | 6C,9G | 5C,8G |
| Morningglory | 3C,5H | 6C,9G | 4C,8G |
| Cocklebur | 5C,9G | 4C,8G | 6C,9G |
| Cassia | 2C,8H | 5C | 5C |
| Nutsedge | 2C,9G | 5G | 1C |
| Crabgrass | 2C,4G | 2C,7G | 0 |
| Barnyardgrass | 3C,9H | 5C,9H | 1C |
| Wild Oats | 1C,5G | 2C,8G | 1C,3G |
| Wheat | 2C,8G | 1C,7G | 0 |
| Corn | 2C,9G | 2C,8H | 3C,7H |
| Soybean | 6C,9G | 6C,9G | 4C,8G |
| Rice | 5C,9G | 6C,9G | 4C,8G |
| Sorghum | 4C,9G | 1U,9G | 3C,8H |
| Pre-Emergence | | | |
| Morningglory | 9G | 9G | 3C,5H |
| Cocklebur | 2C,9H | 9G | 2C |
| Cassia | 5C,8G | 5G | 3C |
| Nutsedge | 4G | 5G | 1C |
| Crabgrass | 2C,5G | 9G,2C | 1C |
| Barnyardgrass | 2C,7G | 5C,9H | 1C |
| Wild Oats | 3C,6G | 2C,8G | 1C |
| Wheat | 3C,9G | 1C,9G | 0 |
| Corn | 1C,9G | 1C,8G | 2C |
| Soybean | 2C,7H | 1C | 1C |
| Rice | 2C,9H | 10E | 3C |
| Sorghum | 3C,9H | 2C,9H | 2C |

Table A (continued)

| | Compound No. | | |
|---|---|---|---|
| | 23 | 24 | 24 |
| kg/ha | 0.4 | 0.4 | 0.05 |
| **Post-Emergence** | | | |
| Bushbean | 4S,7G,6Y | 5C,9G,6Y | 4C,7H,6Y |
| Cotton | 2C,2H | 5C,9G | 4C,9G |
| Morningglory | 0 | 4C,9G | 3C,9G |
| Cocklebur | 0 | 4C,9G | 3C,9H |
| Cassia | 0 | 4C,9G | 3C,7H |
| Nutsedge | 0 | 3C,9G | 8G |
| Crabgrass | 2G | 4C,9G | 8H |
| Barnyardgrass | 1H | 5C,9G | 2C,8H |
| Wild Oats | 0 | 4C,9G | 2C,8H |
| Wheat | 0 | 3C,9G | 9G |
| Corn | 7H | 6U,9C | 2U,9G |
| Soybean | 1C,4G | 6C,9G | 4C,9G |
| Rice | 5G | 9C | 6C,9G |
| Sorghum | 2G | 5U,9G | 2C,9G |
| **Pre-Emergence** | | | |
| Morningglory | 7G | 9C | 9G |
| Cocklebur | 8H | 9H | 9H |
| Cassia | 7G | 2C,9G | 2C,5G |
| Nutsedge | 0 | 9G | 4G |
| Crabgrass | 0 | 5C,9G | 3G |
| Barnyardgrass | 3C,7H | 6C,9G | 2C,9H |
| Wild Oats | 5G | 4C,9G | 3C,8G |
| Wheat | 2G | 2C,9G | 9G |
| Corn | 2C,7H | 2C,9G | 1C,9G |
| Soybean | 1H | 3C,7H | 2C,2H |
| Rice | 3C,8H | 10E | 5C,9H |
| Sorghum | 2C,7G | 5C,9H | 3C,9G |

Table A (continued)

| | Compound No. | | |
|---|---|---|---|
| | 25 | 25 | 26 |
| kg/ha | 0.4 | 0.05 | 0.4 |
| Post-Emergence | | | |
| Bushbean | 5C,8G,6Y | 4C,7G,6Y | 9C |
| Cotton | 4C,5H,9G | 2G | 6C,9G |
| Morningglory | 4C,9G | 2C,5H | 6C,9G |
| Cocklebur | 1H | 0 | 6C,9G |
| Cassia | 4C,7G | 3C | 4C,8H |
| Nutsedge | 2C,5G | 0 | 2C,5G |
| Crabgrass | 2C,9G | 5G | 6C,9G |
| Barnyardgrass | 5C,9H | 8H | 9C |
| Wild Oats | 2C,9H | 2G | 6C,9G |
| Wheat | 2C,9G | 0 | 4U,9G |
| Corn | 2C,9G | 2C,7H | 7U,9C |
| Soybean | 4C,9G | 2C,8G | 4C,9G |
| Rice | 5C,9G | 7G | 6C,9G |
| Sorghum | 2C,9H | 2C,9H | 6U,9G |
| Pre-Emergence | | | |
| Morningglory | 9G | 8H | 2C,9G |
| Cocklebur | 9H | 9H | 9H |
| Cassia | 7G,3C | 2C | 3C,9G |
| Nutsedge | 2C | 0 | 7G |
| Crabgrass | 2C,5G | 3G | 5C,9G |
| Barnyardgrass | 5C,9H | 2C,7H | 6C,9H |
| Wild Oats | 4C,9G | 3C,8G | 4C,9G |
| Wheat | 3C,9G | 2C | 5C,9G |
| Corn | 2C,9G | 3C,6G | 4C,9G |
| Soybean | 2C,5H | 2C | 3C,7H |
| Rice | 10E | 2C,7H | 10E |
| Sorghum | 3C,9G | 2C,6G | 6C,9G |

Table A (continued)

| | Compound No. 26 | 27 | 28 |
|---|---|---|---|
| kg/ha | 0.05 | 0.4 | 0.4 |
| Post-Emergence | | | |
| Bushbean | 9G,6Y,4C | 9C | 6C,9G,6Y |
| Cotton | 4C,5H,9G | 5C,9G | 6C,9G |
| Morningglory | 2C,4H | 6C,9G | 9C |
| Cocklebur | 2H,3G | 9C | 4C,9G |
| Cassia | 4C,4H | 9C | 6C,9G |
| Nutsedge | 0 | 9C | 2C,8G |
| Crabgrass | 2C,8H | 4C,9G | 7G |
| Barnyardgrass | 6C,9G | 9C | 4C,8H |
| Wild Oats | 9G,5X | 5C,9G | 9G |
| Wheat | 9G | 5U,9G | 3C,8G |
| Corn | 3U,9G | 5U,9C | 2U,9G |
| Soybean | 3C,9G,5X | 4C,9G | 2C,9G |
| Rice | 6C,9G | 4C,9G | 6C,9G |
| Sorghum | 2U,9H | 9C | 4C,9G |
| Pre-Emergence | | | |
| Morningglory | 1C,2H | 8H | 9G |
| Cocklebur | 2C | 9H | 9H |
| Cassia | 1C | 3C,9G | 2C,9G |
| Nutsedge | 0 | 10E | 5G |
| Crabgrass | 1C | 2C,7G | 1C |
| Barnyardgrass | 2C,7G | 3C,9H | 4C,8G |
| Wild Oats | 2C,6G | 3C,9H | 9G |
| Wheat | 2C,6G | 9H | 7G |
| Corn | 2C,7G | 3C,9H | 9G |
| Soybean | 1C | 9H | 3H |
| Rice | 6C,9H | 10E | 9H |
| Sorghum | 3C,8G | 3C,9H | 6C,9H |

Table A (continued)

| | Compound No. 29 | 30 | 31 |
|---|---|---|---|
| kg/ha | 0.4 | 0.4 | 0.4 |
| Post-Emergence | | | |
| Bushbean | 9C | 9C | 6C,9G,6Y |
| Cotton | 9C | 6C,9G | 5C,8G |
| Morningglory | 6C,9G | 9C | 5C,8G |
| Cocklebur | 10C | 9C | 5G |
| Cassia | 9C | 10C | 4C,8H |
| Nutsedge | 4C,9G | 6C,9G | 6G |
| Crabgrass | 9C | 6C,9G | 2C,5G |
| Barnyardgrass | 9C | 9C | 3C,9H |
| Wild Oats | 10C | 6C,9G | 5G |
| Wheat | 5U,9G | 4U,9G | 1G |
| Corn | 3U,9C | 10C | 3H,9G |
| Soybean | 4C,9G | 9C | 3C,8G |
| Rice | 5C,9G | 5C,9G | 8G |
| Sorghum | 3U,9G | 10C | 2C,9H |
| Pre-Emergence | | | |
| Morningglory | 9G | 9G | 9G |
| Cocklebur | - | 9H | 8H |
| Cassia | 3C,9G | 9G | 3C,8G |
| Nutsedge | 8G | 10E | 3C,6G |
| Crabgrass | 4C,8G | 3C,8G | 2C |
| Barnyardgrass | 5C,9H | 5C,9H | 3C,6G |
| Wild Oats | 5C,9H | 5C,9H | 1C,5G |
| Wheat | 9H | 5C,9H | 2C |
| Corn | 10E | 10H | 9G |
| Soybean | 9H | 9H | 2C,5H |
| Rice | 10E | 10E | 3G |
| Sorghum | 7C,9H | 6C,9H | 2C,9H |

Test B

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (*Cyperus rotundus*), and several broadleaf weeds. The following grassy and broadleaf weeds were planted: crabgrass (*Digitaria sanguinalis*), barnyardgrass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinus*), mustard (*Brassica arvensis*), cocklebur (*Xanthium pensylvanicum*), pigweed (*Amaranthus retroflexus*), morningglory (*Ipomoea hederacea*), sicklepod (*Cassia obtusifolia*), teaweed (*Sida spinosa*), velvetleaf (*Abutilon theophrasti*), and jimsonweed (*Datura stramonium*). A 12.5 cm diameter plastic pot was also filled with prepared soil and planted with rice and wheat. Another 12.5 cm pot was planted with sugarbeets. The above four containers were treated preemergence with several test compounds within the scope of the invention.

Twenty-eight days after treatment, the plants were evaluated and visually rated for response to the chemical treatments utilizing the rating system described previously for Test A. The data are summarized in Table B. Note that when applied at low rates to soil, the compounds exhibit good herbicidal activity.

Table B

PRE-EMERGENCE ON FALLSINGTON SILT LOAM

| | Compound No. | | | |
|---|---|---|---|---|
| | 1 | | 2 | |
| Rate kg/ha | 0.06 | 0.25 | 0.06 | 0.25 |
| Crabgrass | 0 | 0 | 3G | 5G |
| Barnyardgrass | 2G | 4G | 4G,2C | 7G,3C |
| Sorghum | 4G | 6G,3H | 10C | 10C |
| Wild Oats | 0 | 3G | 5G,3H | 7G,7C |
| Johnsongrass | 0 | 2G | 6G,3H | 8G,5H |
| Dallisgrass | 0 | 0 | 5G | 6G |
| Giant foxtail | 0 | 2G | 0 | 4G |
| Ky. Bluegrass | 0 | 0 | 7G | 7G |
| Cheatgrass | 6G,8E | 6G,8E | 7G,8C | 10C |
| Sugarbeets | 0 | 5G,3C | 7G,6C | 9G,9C |
| Corn | 0 | 3G | 5G,3H | 10E |
| Mustard | 0 | 5G,5E | 7G,8C | 9G,9C |
| Cocklebur | 0 | 0 | 7G | 7G |
| Pigweed | 0 | 9G,9C | 7G | 10E |
| Nutsedge | 0 | 0 | 9G | 8G |
| Cotton | 0 | 3G | 7G | 8G |
| Morningglory | 0 | 3G | 5G | 8G,3H |
| Cassia | 0 | 0 | 5G | 7G,3C |
| Teaweed | 0 | 3G | 5G | 5G |
| Velvetleaf | 0 | 7G,5H | 5G | 9G,5H |
| Jimsonweed | 0 | 0 | 3G | 5G |
| Soybean | 0 | 3G | 4G,3H | 7G,5H |
| Rice | 4G | 7G,3H | 8G,8C | 10E |
| Wheat | 0 | 3G | 5G | 6G |

Table B (continued)

PRE-EMERGENCE ON FALLSINGTON SILT LOAM

| | Compound No. | | | |
|---|---|---|---|---|
| | 3 | | 4 | |
| Rate kg/ha | 0.06 | 0.25 | 0.06 | 0.25 |
| Crabgrass | 2G | 6G | 0 | 0 |
| Barnyardgrass | 4G | 4G | 0 | 0 |
| Sorghum | 4G | 5G | 0 | 3G |
| Wild Oats | 0 | 5G | 0 | 0 |
| Johnsongrass | 3G | 4G | 0 | 0 |
| Dallisgrass | 3G | 4G | 0 | 0 |
| Giant foxtail | 0 | 3G | 0 | 0 |
| Ky. Bluegrass | 0 | 3G | 0 | 0 |
| Cheatgrass | 4G | 8G,8E | 0 | 0 |
| Sugarbeets | 5G | 7G,5C | 0 | 3G |
| Corn | 0 | 5G | 0 | 0 |
| Mustard | 6G,3C | 6G,3C | 0 | 3G |
| Cocklebur | 4G | 6G | 0 | 0 |
| Pigweed | 5G | 7G,8E | 3G | 5G |
| Nutsedge | 5G | 4G | 0 | 0 |
| Cotton | 3G | 5G | 0 | 0 |
| Morningglory | 2G | 5G | 0 | 2G |
| Cassia | 3G | 5G,2C | 0 | 0 |
| Teaweed | 0 | 3G | 0 | 0 |
| Velvetleaf | 3G | 4G,3H | 0 | 0 |
| Jimsonweed | 0 | 3G | 0 | 0 |
| Soybean | 0 | 0 | 0 | 0 |
| Rice | 5G | 7G,5C | 0 | 3G |
| Wheat | 0 | 3G | 0 | 2G |

Test C

Twenty-five cm diameter plastic pots filled with Fallsington silt loam were planted with soybeans, cotton, alfalfa, corn, rice, wheat, sorghum, velvetleaf (*Abutilon theophrasti*), sesbania (*Sesbania exaltata*), sicklepod (*Cassia obtusifolia*), morningglory (*Ipomoea hederacea*), jimsonweed (*Datura stramonium*), cocklebur (*Xanthium pensylvanicum*), crabgrass (*Digitaria* spp.), nutsedge (*Cyperus rotundus*), barnyardgrass (*Echinochloa crusgalli*), giant foxtail (*Setaria faberii*) and wild oats (*Avena fatua*). Approximately two weeks after planting, the young plants and the soil around them were sprayed overall with the test chemicals dissolved in a non-phytotoxic solvent. Two weeks after treatment, all species were compared to untreated controls and visually rated for response to treatment. The rating system was as described previously for Test A. The data are presented in Table C. The compounds possess high herbicidal activity in low-rate soil/foliage applications.

Table C

| | Compound No. | | | |
|---|---|---|---|---|
| | 1 | | 2 | |
| Rate kg/ha | 0.250 | 0.063 | 0.250 | 0.063 |
| Soybeans | 8G,2C | - | 9G,9C | 9G,9C |
| Velvetleaf | 7G,5C | 4C | 7G,2C | 6G |
| Sesbania | 7G,5C | 6G,3C | 9G,4C | 5G,1C |
| Cassia | - | 3C | 5G,2C | 5G |
| Cotton | 7G,5C | 4G,3C | 7G,3C | 6G,2C |
| Morningglory | 7G,1C | 4G,1C | 5G | 4G |
| Alfalfa | 3G,2C | 1C | 2G | 1G |
| Jimsonweed | 2C | - | 1G | - |
| Cocklebur | 10C | 3G,4C | 8G,4C | 6G,1C |
| Corn | 6G,4U | 6G,3U | 8G,7U | 5G,2U |
| Crabgrass | 1G | 1G | 1G | 1G |
| Rice | 6G,7C | 6G | 7G,3C | 6G,1C |
| Nutsedge | 8G,4C | 7G,3C | 10C | 8G,4C |
| Barnyardgrass | 5G,1C | 5G,1C | 9C | 5G,1C |
| Wheat | 4G | 2G | 5G | 5G |
| Giant Foxtail | 4G | 4C | 5G | - |
| Wild Oats | 5G,2C | 5G | 6G,3C | 7G |
| Sorghum | 6G,1U | 7G,1C | 7G,4U | 7G,3U |

Table C (continued)

| | Compound No. | | | |
|---|---|---|---|---|
| | 3 | | 4 | |
| Rate kg/ha | 0.250 | 0.063 | 0.250 | 0.063 |
| Soybeans | 10C | 9G,9C | 0 | 0 |
| Velvetleaf | 7G | 8G | 3C | 0 |
| Sesbania | 6G,5C | 4G | 5G | 1G |
| Cassia | 3G | 4G | 0 | 3G,1C |
| Cotton | 6G,1C | 5G | 0 | 1G |
| Morningglory | 5G | 4G | 0 | 0 |
| Alfalfa | 2G,2C | 0 | 0 | 0 |
| Jimsonweed | - | - | - | - |
| Cocklebur | 5G | 1G | 0 | 2G |
| Corn | 8G,8U | 6G,3U | 4G,1U | 3G,2C |
| Crabgrass | 3G | 0 | 0 | 0 |
| Rice | 7G | 6G | 5G | 5G |
| Nutsedge | 5G | 6G | 5G | 5G |
| Barnyardgrass | 5G,2C | 1C | 5G,2C | 5G,2C |
| Wheat | 4G | 0 | 3G | 3G |
| Giant Foxtail | 2C | 0 | - | 0 |
| Wild Oats | 4G | 2G | 3G | 3G |
| Sorghum | 5G,1C | 3G | 5G,3C | 4G |

Austria
BA-8418-A

Claims:

1. A process for the preparation of a compound of the formula:

$$A{-}SO_2NH\overset{O}{\overset{\|}{C}}N{-}L$$

or an agriculturally suitable salt thereof, where

A is 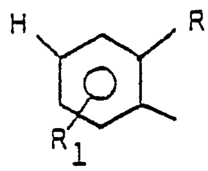, 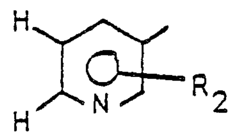, 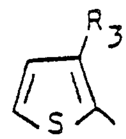,

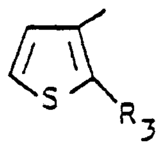, 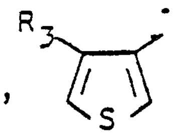 or 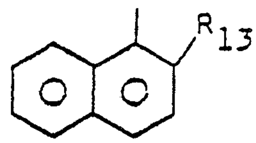;

R is $C_1$-$C_4$ alkyl, $C_1$-$C_4$ alkoxy, F, Cl, Br, $NO_2$, $CF_3$, $COR_4$, $S(O)_mR_5$, $SO_2NR_6R_7$, $SO_2OCH_2CF_3$, $SO_2OCH_2CCl_3$, $SO_2N(OCH_3)(CH_3)$, $BR_8$, $OSO_2R_9$ or $CH_2P$;

$R_1$ is H, F, Cl, Br, $CF_3$, $NO_2$, $C_1$-$C_4$ alkyl or $C_1$-$C_4$ alkoxy;

$R_4$ is $C_1$-$C_6$ alkoxy, $C_3$-$C_6$ alkenyloxy, $C_3$-$C_6$ alkynyloxy, $C_2$-$C_6$ haloalkoxy substituted with 1-3 atoms selected from F, Cl or Br, $C_5$-$C_6$ cycloalkoxy, $-O(CH_2CH_2O)_nR_{10}$, $OCH_2CH_2CH_2OR_{10}$, $OCH_2OR_5$ or $OCH_2OCH_2CH_2OR_{10}$;

n is 1 or 2;

m is 0, 1 or 2;

$R_5$ is $C_1$-$C_4$ alkyl;

$R_6$ and $R_7$ are independently $C_1$-$C_4$ alkyl, provided the total carbon atoms of $R_6$ and $R_7$ does not exceed 5;

B is O or $S(O)_m$;

$R_8$ is $CHF_2$, $CF_3$, $CH_2CF_3$ or $CF_2CHFG$ where G is F, Cl, Br or $CF_3$;

$R_9$ is $C_1$-$C_4$ alkyl or $C_1$-$C_4$ alkyl substituted with 1-3 atoms of F, Cl or Br;

P is Cl, Br, $C_1$-$C_4$ alkoxy, $C_3$-$C_4$ alkenyloxy or $S(O)_mR_5$;

$R_{10}$ is $CH_3$ or $CH_3CH_2$;

$R_2$ is F, Cl, Br, $C_1$-$C_4$ alkyl, $C_1$-$C_4$ alkoxy, $NO_2$, $CO_2R_{11}$ or $S(O)_mR_{12}$;

$R_{11}$ is $C_1$-$C_4$ alkyl;

$R_{12}$ is $C_1$-$C_3$ alkyl;

$R_3$ is $C_1$-$C_4$ alkyl, $C_1$-$C_4$ alkoxy, H, F, Cl, Br, $NO_2$, $SO_2NR_6R_7$, $SO_2N(OCH_3)(CH_3)$ or $COR_4$;

$R_{13}$ is H, Cl, $NO_2$, $S(O)_mR_5$, $SO_2NR_6R_7$, $C_1$-$C_4$ alkyl or $C_1$-$C_4$ alkoxy;

W is H or $CH_3$;

L is

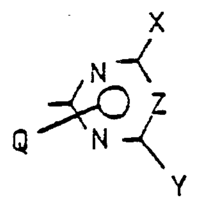 , 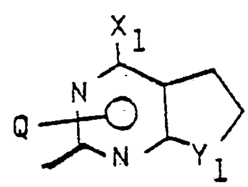 ,

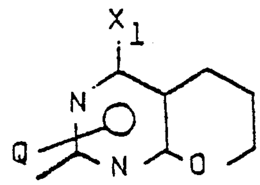 or 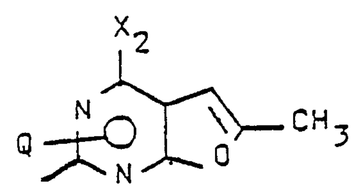

wherein

X is $CH_3$, $CH_3CH_2$, $CH_3O$ or $CH_3CH_2O$;

Y is H, Cl, $CF_3$, $C_1$-$C_4$ alkyl, $C_1$-$C_4$ alkoxy, $CH_2OCH_3$, $CH_2OCH_2CH_3$, $CH_2CH_2OCH_3$ or $CH_2CH_2OCH_2CH_3$;

$X_1$ is H, Cl, $CH_3$, $CH_3CH_2$, $CH_3O$ or $CH_3CH_2O$;

0057546

$Y_1$ is O or $CH_2$;
$X_2$ is $CH_3$ or $CH_3CH_2$;
Z is CH, CF or N; and
Q is O bonded to a nitrogen atom of L,
which comprises

(a) reacting an isocyanate of general formula

$ASO_2NCO$

with an aminopyrimidine-N-oxide or aminotriazine-N-oxide of general formula

HN-L
W

wherein A, W and L are as defined above except that R is not $S(O)R_5$, $CH_2P$ (when P is $S(O)R_5$), $COR_4$ (when $R_4$ is $OCH_2OR_5$, $OCH_2OCH_2CH_2OR_{10}$ or $C_3$-$C_6$ alkynyloxy);

$R_2$ is not $S(O)R_{12}$

$R_3$ is not $COR_4$ (when $R_4$ is $OCH_2OR_5$, $OCH_2OCH_2CH_2OR_{10}$ or $C_3$-$C_6$ alkynyloxy; and

$R_{13}$ is not $S(O)R_{13}$; or

(b) reacting a salt of a corresponding carboxylic acid of general formula

O
$ASO_2NHCN$-L (II)
W

wherein A is

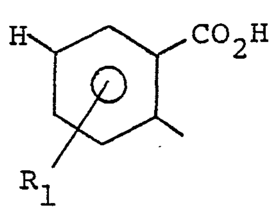

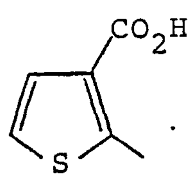

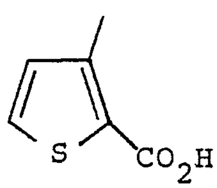

or

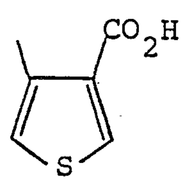

and W and L are as defined above, with an appropriate halide of general formula

$R_4$-halogen; or

(c) oxidising a compound of general formula I wherein m is 0 or 1 to a desired compound wherein m is 1 or 2; or

(d) introducing the N-oxide substituent Q by oxidising the pyrimidinyl or triazinyl moiety of a corresponding sulfonylurea lacking said substituent.

2. A process of claim 1 wherein

A is 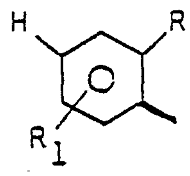 .

3. A process of claim 1 wherein

A is 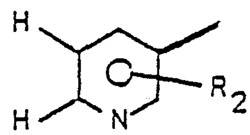 .

4. A process of claim 1 wherein

A is 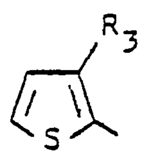 , 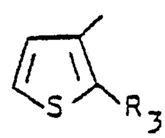 or 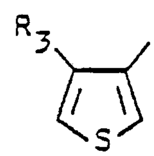 .

5. A process of claim 1 wherein

A is 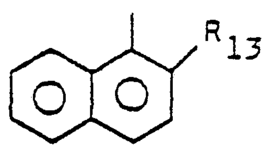 .

6. The process of any of claims 2 to 5 in which

L is 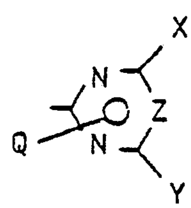 .

7. The process of claim 6 in which:

R is $C_1$-$C_3$ alkyl, $OCH_3$, F, Cl, Br, $NO_2$, $CF_3$, $COR_4$, $S(O)_mR_5$, $SO_2NR_6R_7$, $SO_2N(OCH_3)(CH_3)$, $BR_8$, $OSO_2R_9$ or $CH_2P$;

$R_4$ is $C_1$-$C_4$ alkoxy, $C_3$-$C_4$ alkenyloxy or haloethoxy having 1-3 atoms of F or Cl; and

B is O.

8. The process of claim 7 in which:

$R_1$ is H, Cl, Br, $OCH_3$, $CH_3$ or $CF_3$;

$R_4$ is $C_1$-$C_4$ alkoxy, $C_3$-$C_4$ alkenyloxy or $OCH_2CH_2Cl$;

$R_8$ is $CF_3$, $CH_2CF_3$ or $CF_2CHF_2$;

$R_9$ is $CH_3$ or $CF_3$; and

P is $C_1$-$C_3$ alkoxy, $OCH_2CH=CH_2$ or $S(O)_mCH_3$.

9. The process of claim 8 in which $R_1$ is H and W is H

10. The process of claims 5 and 6 in which $R_{13}$ is Cl, $C_1$-$C_3$ alkyl or $C_1$-$C_3$ alkoxy and W is H.

11. The process of any of claims 7-10 in which X and Y are independently $CH_3$ or $CH_3O$.

12. The process of claim 1 wherein the product is a compound selected from 2-chloro-N-[(4,6-dimethyl-1-oxide-pyrimidin-2-yl)aminocarbonyl]benzenesulfonamide;

2-[[(4,6-dimethyl-1-oxidepyrimidin-2-yl)aminocarbonyl]-aminosulfonyl]benzoic acid, methyl ester;

2-nitro-N-[(4,6-dimethyl-1-oxidepyrimidin-2-yl)aminocarbonyl]benzenesulfonamide;

2-[[(4,6-dimethyl-1-oxidepyrimidin-2-yl)aminocarbonyl]-aminosulfonyl]benzoic acid, 2-propenyl ester;

2-chloro-N-[(4-methoxy-6-methyl-1-oxidepyrimidin-2-yl)-aminocarbonyl]benzenesulfonamide;

2-[[(3-methoxy-6-methyl-1-oxidepyrimidin-2-yl)aminocarbonyl]aminosulfonyl]benzoic acid, methyl ester;

2-nitro-N-[(4-methoxy-6-methyl-1-oxidepyrimidin-2-yl)-aminocarbonyl]benzenesulfonamide;

2-[[(4-methoxy-6-methyl-1-oxidepyrimidin-2-yl)aminocarbonyl]aminosulfonyl]benzoic acid, 1-methylethyl ester;

2-[[(4-methoxy-6-methyl-1-oxidepyrimidin-2-yl)aminocarbonyl]aminosulfonyl]benzoic acid, 2-propenyl ester;

2-[[(4-methoxy-6-methyl-1-oxidetriazin-2-yl)aminocarbonyl]aminosulfonyl]benzoic acid, methyl ester;

2-[[(4-methoxy-6-methyl-3-oxidetriazin-2-yl)aminocarbonyl]aminosulfonyl]benzoic acid, methyl ester;

2-chloro-N-[(4-methoxy-6-methyl-1-oxidetriazin-2-yl)-aminocarbonyl]benzenesulfonamide;

2-chloro-N-[(4-methoxy-6-methyl-3-oxidetriazin-2-yl)-aminocarbonyl]benzenesulfonamide;

2-(propylsulfonyl)-N-[(4,6-dimethoxy-1-oxidepyrimidin-2-yl)aminocarbonyl]benzenesulfonamide;

2-(propylsulfonyl)-N-[(4-methoxy-6-methyl-1-oxidetriazin-2-yl)aminocarbonyl]benzenesulfonamide,:

2-(propylsulfonyl)-N-[(4-methoxy-6-methyl-3-oxidetriazin-2-yl)aminocarbonyl]benzenesulfonamide;

2-(propoxy)-N-[(4,6-dimethoxy-1-oxidetriazin-2-yl)-aminocarbonyl]benzenesulfonamide;

2-chloro-N-[(4,6-dimethoxy-1-oxidepyrimidin-2-yl)-aminocarbonyl]-1-naphthalenesulfonamide;

N',N'-dimethyl-N-[(4,6-dimethyl-1-oxidetriazin-2-yl)-aminocarbonyl]-1,2-benzenesulfonamide; and

N',N'-dimethyl-N-[(4-methoxy-6-methyl-1-oxidetriazin-2-yl)aminocarbonyl]-1,2-benzenesulfonamide.

13. A method for controlling the growth of undesired vegetation by applying to the locus to be protected an effective amount of a herbicidal compound, characterised in that said herbicidal compound comprises a compound of general formula I as defined in any of claims 1 - 12.

14. A method for regulating the growth of plants by applying to the locus of such plants an effective but substantially non-phytotoxic amount of a plant growth regulant,

following: surfactant, solid or liquid diluent, characterised in

that said herbicidal compound comprises a compound of any of claims 1 to 31.

34. A method for controlling the growth of undesired vegetation by applying to the locus to be protected an effective amount of a herbicidal compound, characterised in

that said herbicidal compound comprises a compound of any of claims 1 to 31.

35. A method for regulating the growth of plants by applying to the locus of such plants an effective but substantially non-phytotoxic amount of a plant growth regulant, characterised in

that said plant growth regulant comprises a compound of any of claims 1 to 31.

36. A compound selected from:

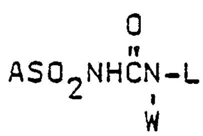

wherein

A is 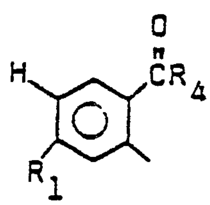 , 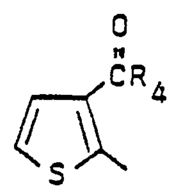 ,

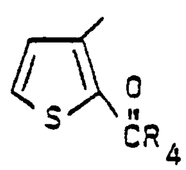 or 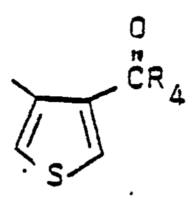

wherein

$R_1$, W and L are as defined in claim 1 and $R_4$ is OH.

37. A compound selected from:

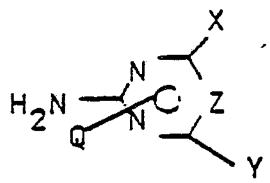 , 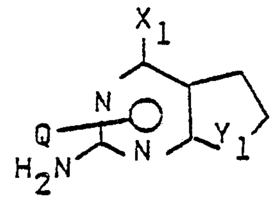 ,

(a) (b)

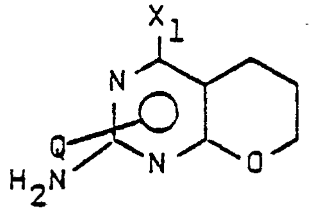 or 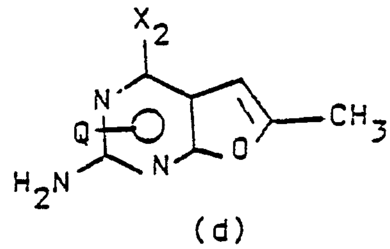

(c) (d)

wherein

X, Y, $X_1$, $Y_1$, $X_2$, Z and Q are as defined in claim 1, provided that:

(1) when Z is N, X and Y cannot both be alkyl; and

(2) when Z is CH, X and Y cannot both be methyl.

Claims: BA-8418-A

1. A compound of the formula:

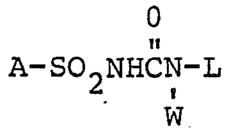

and its agriculturally suitable salts, where

A is 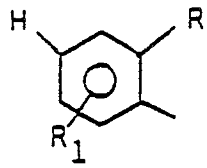, 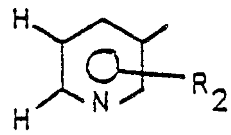, 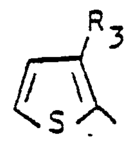,

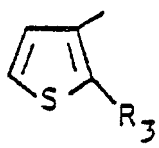, 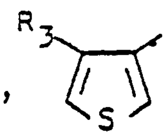 or 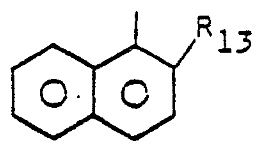;

R is $C_1$-$C_4$ alkyl, $C_1$-$C_4$ alkoxy, F, Cl, Br, $NO_2$, $CF_3$, $COR_4$, $S(O)_mR_5$, $SO_2NR_6R_7$, $SO_2OCH_2CF_3$, $SO_2OCH_2CCl_3$, $SO_2N(OCH_3)(CH_3)$, $BR_8$, $OSO_2R_9$ or $CH_2P$;

$R_1$ is H, F, Cl, Br, $CF_3$, $NO_2$, $C_1$-$C_4$ alkyl or $C_1$-$C_4$ alkoxy;

$R_4$ is $C_1$-$C_6$ alkoxy, $C_3$-$C_6$ alkenyloxy, $C_3$-$C_6$ alkynyloxy, $C_2$-$C_6$ haloalkoxy substituted with 1-3 atoms selected from F, Cl or Br, $C_5$-$C_6$ cycloalkoxy, $-O(CH_2CH_2O)_nR_{10}$, $OCH_2CH_2CH_2OR_{10}$, $OCH_2OR_5$ or $OCH_2OCH_2CH_2OR_{10}$;

n is 1 or 2;

m is 0, 1 or 2;

$R_5$ is $C_1$-$C_4$ alkyl;

$R_6$ and $R_7$ are independently $C_1$-$C_4$ alkyl, provided the total carbon atoms of $R_6$ and $R_7$ does not exceed 5;

B is O or $S(O)_m$;

$R_8$ is $CHF_2$, $CF_3$, $CH_2CF_3$ or $CF_2CHFG$ where G is F, Cl, Br or $CF_3$;

$R_9$ is $C_1$-$C_4$ alkyl or $C_1$-$C_4$ alkyl substituted with 1-3 atoms of F, Cl or Br;

P is Cl, Br, $C_1$-$C_4$ alkoxy, $C_3$-$C_4$ alkenyloxy or $S(O)_mR_5$;

$R_{10}$ is $CH_3$ or $CH_3CH_2$;

$R_2$ is F, Cl, Br, $C_1$-$C_4$ alkyl, $C_1$-$C_4$ alkoxy, $NO_2$, $CO_2R_{11}$ or $S(O)_mR_{12}$;

$R_{11}$ is $C_1$-$C_4$ alkyl;

$R_{12}$ is $C_1$-$C_3$ alkyl;

$R_3$ is $C_1$-$C_4$ alkyl, $C_1$-$C_4$ alkoxy, H, F, Cl, Br, $NO_2$, $SO_2NR_6R_7$, $SO_2N(OCH_3)(CH_3)$ or $COR_4$;

$R_{13}$ is H, Cl, $NO_2$, $S(O)_mR_5$, $SO_2NR_6R_7$, $C_1$-$C_4$ alkyl or $C_1$-$C_4$ alkoxy;

W is H or $CH_3$;

L is

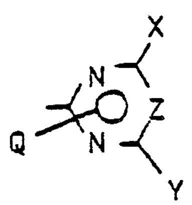 , 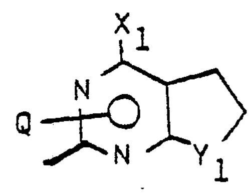 ,

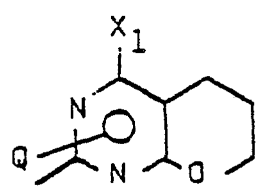 or 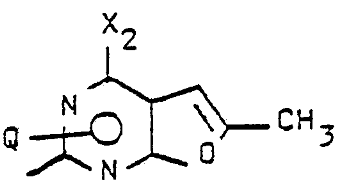

wherein

X is $CH_3$, $CH_3CH_2$, $CH_3O$ or $CH_3CH_2O$;

Y is H, Cl, $CF_3$, $C_1$-$C_4$ alkyl, $C_1$-$C_4$ alkoxy, $CH_2OCH_3$, $CH_2OCH_2CH_3$, $CH_2CH_2OCH_3$ or $CH_2CH_2OCH_2CH_3$;

$X_1$ is H, Cl, $CH_3$, $CH_3CH_2$, $CH_3O$ or $CH_3CH_2O$;

$Y_1$ is O or $CH_2$;

$X_2$ is $CH_3$ or $CH_3CH_2$;

Z is CH, CF or N; and

Q is O bonded to a nitrogen atom of L.

2. A compound of Claim 1 wherein

A is 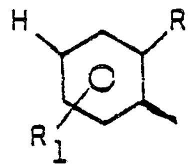 .

3. A compound of Claim 1 wherein

A is 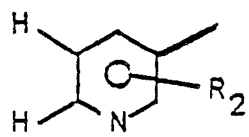 .

4. A compound of Claim 1 wherein

A is 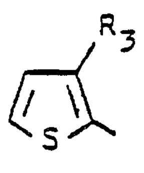 , 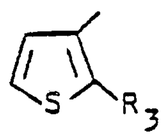 or 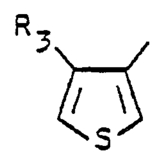 .

5. A compound of Claim 1 wherein

A is 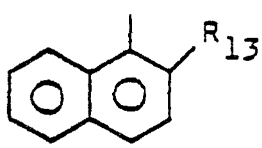 .

6. The compounds of any of claims 2 to 5 in which

L is 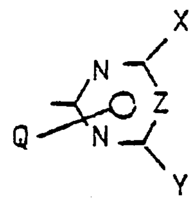 .

7. The compounds of Claim 6 in which:

R is $C_1$-$C_3$ alkyl, $OCH_3$, F, Cl, Br, $NO_2$, $CF_3$, $COR_4$, $S(O)_mR_5$, $SO_2NR_6R_7$, $SO_2N(OCH_3)(CH_3)$, $BR_8$, $OSO_2R_9$ or $CH_2P$;

$R_4$ is $C_1$-$C_4$ alkoxy, $C_3$-$C_4$ alkenyloxy or haloethoxy having 1-3 atoms of F or Cl; and

B is O.

8. The compounds of Claim 7 in which:

$R_1$ is H, Cl, Br, $OCH_3$, $CH_3$ or $CF_3$;

$R_4$ is $C_1$-$C_4$ alkoxy, $C_3$-$C_4$ alkenyloxy or $OCH_2CH_2Cl$;

$R_8$ is $CF_3$, $CH_2CF_3$ or $CF_2CHF_2$;

$R_9$ is $CH_3$ or $CF_3$; and

P is $C_1$-$C_3$ alkoxy, $OCH_2CH{=}CH_2$ or $S(O)_mCH_3$.

9. The compounds of Claim 8 in which $R_1$ is H and W is H.

10. The compounds of claims 5 and 6 in which $R_{13}$ is Cl, $C_1$-$C_3$ alkyl or $C_1$-$C_3$ alkoxy and W is H.

11. The compounds of any of claims 7-10 in which X and Y are independently $CH_3$ or $CH_3O$.

12. The compound of Claim 1, 2-chloro-N-[(4,6-dimethyl-1-oxidepyrimidin-2-yl)aminocarbonyl]benzenesulfonamide.

13. The compound of Claim 1, 2-[[(4,6-dimethyl-1-oxidepyrimidin-2-yl)aminocarbonyl]aminosulfonyl]benzoic acid, methyl ester.

14. The compound of Claim 1, 2-nitro-N-[(4,6-dimethyl-1-oxidepyrimidin-2-yl)aminocarbonyl]benzenesulfonamide.

15. The compound of Claim 1, 2-[[(4,6-dimethyl-1-oxidepyrimidin-2-yl)aminocarbonyl]aminosulfonyl]benzoic acid, 2-propenyl ester.

16. The compound of Claim 1, 2-chloro-N-[(4-methoxy-6-methyl-1-oxidepyrimidin-2-yl)aminocarbonyl]-benzenesulfonamide.

17. The compound of Claim 1, 2-[[(4-methoxy-6-methyl-1-oxidepyrimidin-2-yl)aminocarbonyl]aminosulfonyl]benzoic acid, methyl ester.

18. The compound of Claim 1, 2-nitro-N-[(4-methoxy-6-methyl-1-oxidepyrimidin-2-yl)aminocarbonyl]-benzenesulfonamide.

19. The compound of Claim 1, 2-[[(4-methoxy-6-methyl-1-oxidepyrimidin-2-yl)aminocarbonyl]aminosulfonyl]benzoic acid, 1-methylethyl ester.

20. The compound of Claim 1, 2-[[(4-methoxy-6-methyl-1-oxidepyrimidin-2-yl)aminocarbonyl]aminosulfonyl]benzoic acid, 2-propenyl ester.

21. The compound of Claim 1, 2-[[(4-methoxy-6-methyl-1-oxidetriazin-2-yl)aminocarbonyl]aminosulfonyl]benzoic acid, methyl ester.

22. The compound of Claim 1, 2-[[(4-methoxy-6-methyl-3-oxidetriazin-2-yl)aminocarbonyl]aminosulfonyl]benzoic acid, methyl ester.

23. The compound of Claim 1, 2-chloro-N-[(4-methoxy-6-methyl-1-oxidetriazin-2-yl)aminocarbonyl]-benzenesulfonamide.

24. The compound of Claim 1, 2-chloro-N-[(4-methoxy-6-methyl-3-oxidetriazin-2-yl)aminocarbonyl]-benzenesulfonamide.

25. The compound of Claim 1, 2-(propylsulfonyl)-N-[(4,6-dimethoxy-1-oxidepyrimidin2-yl)aminocarbonyl]-benzenesulfonamide.

26. The compound of Claim 1, 2-(propylsulfonyl)-N-[(4-methoxy-6-methyl-1-oxidetriazin-2-yl)aminocarbonyl]benzenesulfonamide.

27. The compound of Claim 1, 2-(propylsulfonyl)-N-[(4-methoxy-6-methyl-3-oxidetriazin-2-yl)aminocarbonyl]benzenesulfonamide.

28. The compound of Claim 1, 2-(propoxy)-N-[(4,6-dimethoxy-1-oxidetriazin-2-yl)aminocarbonyl]benzenesulfonamide.

29. The compound of Claim 1, 2-chloro-N-[(4,6-dimethoxy-1-oxidepyrimidin-2-yl)aminocarbonyl]-1-naphthalenesulfonamide.

30. The compound of Claim 1, N',N'-dimethyl-N-[(4,6-dimethyl-1-oxidetriazin-2-yl)aminocarbonyl]-1,2-benzenesulfonamide.

31. The compound of Claim 1, N',N'-dimethyl-N-[(4-methoxy-6-methyl-1-oxidetriazin-2-yl)aminocarbonyl]-1,2-benzenesulfonamide.

32. A process for preparing a compound of claim 1 which comprises reacting an isocyanate of general formula

$$ASO_2NCO$$

with an aminopyrimidine-N-oxide or aminotriazine-N-oxide of general formula

$$\begin{array}{c}HN-L\\ |\\ W\end{array}$$

wherein A, W and L are as defined in claim 1 except that R is not $S(O)R_5$, $CH_2P$ (when P is $S(O)R_5$), $COR_4$ (when $R_4$ is $OCH_2OR_5$, $OCH_2OCH_2CH_2OR_{10}$ or $C_3$-$C_6$ alkynyloxy);

$R_2$ is not $S(O)R_{12}$;

$R_3$ is not $COR_4$ (when $R_4$ is as defined above); and

$R_{13}$ is not $S(O)R_5$.

33. A composition suitable for controlling the growth of undesired vegetation comprising an effective amount of a herbicidal compound and at least one of the

following: surfactant, solid or liquid diluent, characterised in

that said herbicidal compound comprises a compound of any of claims 1 to 31.

34. A method for controlling the growth of undesired vegetation by applying to the locus to be protected an effective amount of a herbicidal compound, characterised in

that said herbicidal compound comprises a compound of any of claims 1 to 31.

35. A method for regulating the growth of plants by applying to the locus of such plants an effective but substantially non-phytotoxic amount of a plant growth regulant, characterised in

that said plant growth regulant comprises a compound of any of claims 1 to 31.

36. A compound selected from:

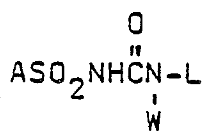

wherein

A is 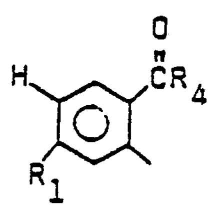 , 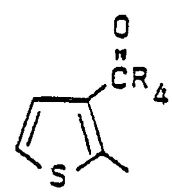 ,

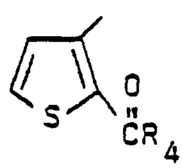 or 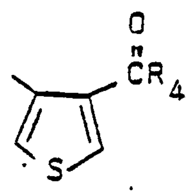

wherein

$R_1$, W and L are as defined in claim 1 and $R_4$ is OH.

37. A compound selected from:

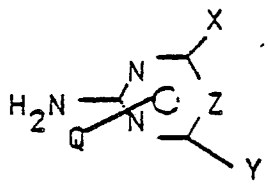
,
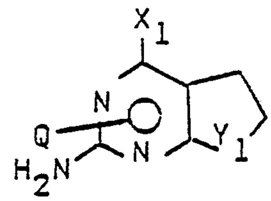
,

(a) (b)

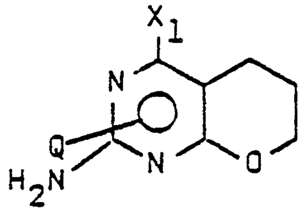
or
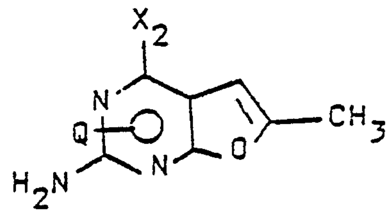

(c) (d)

wherein

X, Y, $X_1$, $Y_1$, $X_2$, Z and Q are as defined in claim 1, provided that:

(1) when Z is N, X and Y cannot both be alkyl; and

(2) when Z is CH, X and Y cannot both be methyl.

Claims:

1. A process for the preparation of a compound of the formula:

$$A-SO_2NH\overset{O}{\overset{\|}{C}}N-L$$

or an agriculturally suitable salt thereof, where

A is 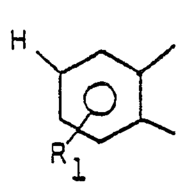, 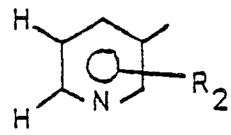, 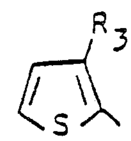,

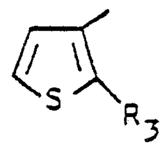, 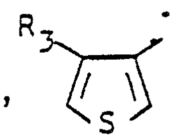 or 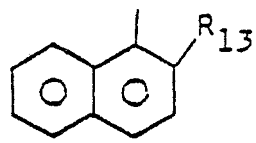;

R is $C_1$-$C_4$ alkyl, $C_1$-$C_4$ alkoxy, F, Cl, Br, $NO_2$, $CF_3$, $COR_4$, $S(O)_mR_5$, $SO_2NR_6R_7$, $SO_2OCH_2CF_3$, $SO_2OCH_2CCl_3$, $SO_2N(OCH_3)(CH_3)$, $BR_8$, $OSO_2R_9$ or $CH_2P$;

$R_1$ is H, F, Cl, Br, $CF_3$, $NO_2$, $C_1$-$C_4$ alkyl or $C_1$-$C_4$ alkoxy;

$R_4$ is $C_1$-$C_6$ alkoxy, $C_3$-$C_6$ alkenyloxy, $C_3$-$C_6$ alkynyloxy, $C_2$-$C_6$ haloalkoxy substituted with 1-3 atoms selected from F, Cl or Br, $C_5$-$C_6$ cycloalkoxy, $-O(CH_2CH_2O)_nR_{10}$, $OCH_2CH_2CH_2OR_{10}$, $OCH_2OR_5$ or $OCH_2OCH_2CH_2OR_{10}$;

n is 1 or 2;

m is 0, 1 or 2;

$R_5$ is $C_1$-$C_4$ alkyl;

$R_6$ and $R_7$ are independently $C_1$-$C_4$ alkyl, provided the total carbon atoms of $R_6$ and $R_7$ does not exceed 5;

B is O or $S(O)_m$;

$R_8$ is $CHF_2$, $CF_3$, $CH_2CF_3$ or $CF_2CHFG$ where G is F, Cl, Br or $CF_3$;

$R_9$ is $C_1$-$C_4$ alkyl or $C_1$-$C_4$ alkyl substituted with 1-3 atoms of F, Cl or Br;

P is Cl, Br, $C_1$-$C_4$ alkoxy, $C_3$-$C_4$ alkenyloxy or $S(O)_mR_5$;

$R_{10}$ is $CH_3$ or $CH_3CH_2$;

$R_2$ is F, Cl, Br, $C_1$-$C_4$ alkyl, $C_1$-$C_4$ alkoxy, $NO_2$, $CO_2R_{11}$ or $S(O)_mR_{12}$;

$R_{11}$ is $C_1$-$C_4$ alkyl;

$R_{12}$ is $C_1$-$C_3$ alkyl;

$R_3$ is $C_1$-$C_4$ alkyl, $C_1$-$C_4$ alkoxy, H, F, Cl, Br, $NO_2$, $SO_2NR_6R_7$, $SO_2N(OCH_3)(CH_3)$ or $COR_4$;

$R_{13}$ is H, Cl, $NO_2$, $S(O)_mR_5$, $SO_2NR_6R_7$, $C_1$-$C_4$ alkyl or $C_1$-$C_4$ alkoxy;

W is H or $CH_3$;

L is

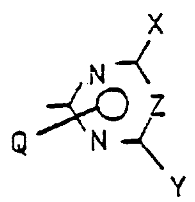

,

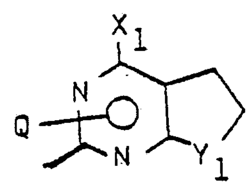

,

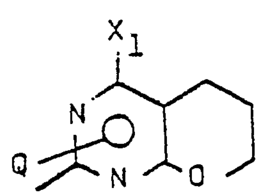

or

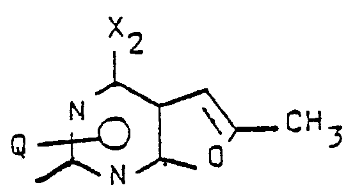

wherein

X is $CH_3$, $CH_3CH_2$, $CH_3O$ or $CH_3CH_2O$;

Y is H, Cl, $CF_3$, $C_1$-$C_4$ alkyl, $C_1$-$C_4$ alkoxy, $CH_2OCH_3$, $CH_2OCH_2CH_3$, $CH_2CH_2OCH_3$ or $CH_2CH_2OCH_2CH_3$;

$X_1$ is H, Cl, $CH_3$, $CH_3CH_2$, $CH_3O$ or $CH_3CH_2O$;

$Y_1$ is O or $CH_2$;
$X_2$ is $CH_3$ or $CH_3CH_2$;
Z is CH, CF or N; and
Q is O bonded to a nitrogen atom of L;
which comprises

(a) reacting an isocyanate of general formula

$ASO_2NCO$

with an aminopyrimidine-N-oxide or aminotriazine-N-oxide of general formula

HN-L
 ¦
 W

wherein A, W and L are as defined above except that R is not $S(O)R_5$, $CH_2P$ (when P is $S(O)R_5$), $COR_4$ (when $R_4$ is $OCH_2OR_5$, $OCH_2OCH_2CH_2OR_{10}$ or $C_3$-$C_6$ alkynyloxy);
$R_2$ is not $S(O)R_{12}$
$R_3$ is not $COR_4$ (when $R_4$ is $OCH_2OR_5$, $OCH_2OCH_2CH_2OR_{10}$ or $C_3$-$C_6$ alkynyloxy; and
$R_{13}$ is not $S(O)R_{13}$; or

(b) reacting a salt of a corresponding carboxylic acid of general formula

$$ASO_2NHC(=O)N(W)-L \quad (II)$$

wherein A is

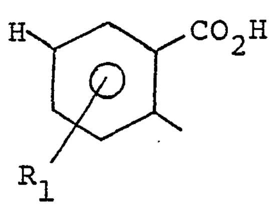

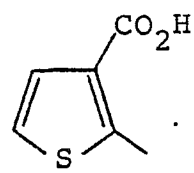

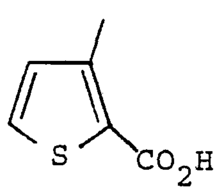

or

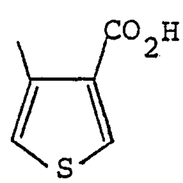

and W and L are as defined above, with an appropriate halide of general formula

$R_4$-halogen; or

(c) oxidising a compound of general formula I wherein m is 0 or 1 to a desired compound wherein m is 1 or 2; or

(d) introducing the N-oxide substituent Q by oxidising the pyrimidinyl or triazinyl moiety of a corresponding sulfonylurea lacking said substituent.

2. A process of claim 1 wherein

A is 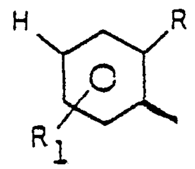 .

3. A process of claim 1 wherein

A is 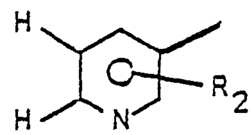 .

4. A process of claim 1 wherein

A is 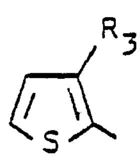 , 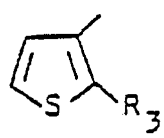 or 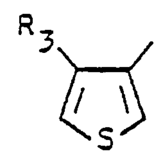 .

5. A process of claim 1 wherein

A is 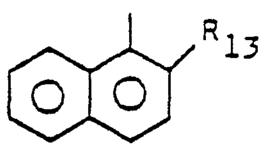 .

6. The process of any of claims 2 to 5 in which

L is 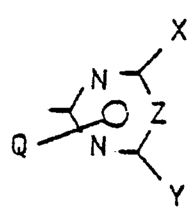 .

7. The process of claim 6 in which:

R is $C_1$-$C_3$ alkyl, $OCH_3$, F, Cl, Br, $NO_2$, $CF_3$, $COR_4$, $S(O)_mR_5$, $SO_2NR_6R_7$, $SO_2N(OCH_3)(CH_3)$, $BR_8$, $OSO_2R_9$ or $CH_2P$;

$R_4$ is $C_1$-$C_4$ alkoxy, $C_3$-$C_4$ alkenyloxy or haloethoxy having 1-3 atoms of F or Cl; and

B is O.

8. The process of claim 7 in which:

$R_1$ is H, Cl, Br, $OCH_3$, $CH_3$ or $CF_3$;

$R_4$ is $C_1$-$C_4$ alkoxy, $C_3$-$C_4$ alkenyloxy or $OCH_2CH_2Cl$;

$R_8$ is $CF_3$, $CH_2CF_3$ or $CF_2CHF_2$;

$R_9$ is $CH_3$ or $CF_3$; and

P is $C_1$-$C_3$ alkoxy, $OCH_2CH=CH_2$ or $S(O)_mCH_3$.

9. The process of claim 8 in which $R_1$ is H and W is H

10. The process of claims 5 and 6 in which $R_{13}$ is Cl, $C_1$-$C_3$ alkyl or $C_1$-$C_3$ alkoxy and W is H.

11. The process of any of claims 7-10 in which X and Y are independently $CH_3$ or $CH_3O$.

12. The process of claim 1 wherein the product is a compound selected from 2-chloro-N-[(4,6-dimethyl-1-oxide-pyrimidin-2-yl)aminocarbonyl]benzenesulfonamide;

2-[[(4,6-dimethyl-1-oxidepyrimidin-2-yl)aminocarbonyl]-aminosulfonyl]benzoic acid, methyl ester;

2-nitro-N-[(4,6-dimethyl-1-oxidepyrimidin-2-yl)aminocarbonyl]benzenesulfonamide;

2-[[(4,6-dimethyl-1-oxidepyrimidin-2-yl)aminocarbonyl]-aminosulfonyl]benzoic acid, 2-propenyl ester;

2-chloro-N-[(4-methoxy-6-methyl-1-oxidepyrimidin-2-yl)-aminocarbonyl]benzenesulfonamide;

2-[[(3-methoxy-6-methyl-1-oxidepyrimidin-2-yl)aminocarbonyl]aminosulfonyl]benzoic acid, methyl ester;

2-nitro-N-[(4-methoxy-6-methyl-1-oxidepyrimidin-2-yl)-aminocarbonyl]benzenesulfonamide;

2-[[(4-methoxy-6-methyl-1-oxidepyrimidin-2-yl)aminocarbonyl]aminosulfonyl]benzoic acid, 1-methylethyl ester;

2-[[(4-methoxy-6-methyl-1-oxidepyrimidin-2-yl)aminocarbonyl]aminosulfonyl]benzoic acid, 2-propenyl ester;

2-[[(4-methoxy-6-methyl-1-oxidetriazin-2-yl)aminocarbonyl]aminosulfonyl]benzoic acid, methyl ester;

2-[[(4-methoxy-6-methyl-3-oxidetriazin-2-yl)aminocarbonyl]aminosulfonyl]benzoic acid, methyl ester;

2-chloro-N-[(4-methoxy-6-methyl-1-oxidetriazin-2-yl)-aminocarbonyl]benzenesulfonamide;

2-chloro-N-[(4-methoxy-6-methyl-3-oxidetriazin-2-yl)-aminocarbonyl]benzenesulfonamide;

2-(propylsulfonyl)-N-[(4,6-dimethoxy-1-oxidepyrimidin-2-yl)aminocarbonyl]benzenesulfonamide;

2-(propylsulfonyl)-N-[(4-methoxy-6-methyl-1-oxide-triazin-2-yl)aminocarbonyl]benzenesulfonamide,:

2-(propylsulfonyl)-N-[(4-methoxy-6-methyl-3-oxide-triazin-2-yl)aminocarbonyl]benzenesulfonamide;

2-(propoxy)-N-[(4,6-dimethoxy-1-oxidetriazin-2-yl)-aminocarbonyl]benzenesulfonamide;

2-chloro-N-[(4,6-dimethoxy-1-oxidepyrimidin-2-yl)-aminocarbonyl]-1-naphthalenesulfonamide;

N',N'-dimethyl-N-[(4,6-dimethyl-1-oxidetriazin-2-yl)-aminocarbonyl]-1,2-benzenesulfonamide; and

N',N'-dimethyl-N-[(4-methoxy-6-methyl-1-oxidetriazin-2-yl)aminocarbonyl]-1,2-benzenesulfonamide.

13. A method for controlling the growth of undesired vegetation by applying to the locus to be protected an effective amount of a herbicidal compound, characterised in that said herbicidal compound comprises a compound of general formula I as defined in any of claims 1 - 12.

14. A method for regulating the growth of plants by applying to the locus of such plants an effective but substantially non-phytotoxic amount of a plant growth regulant,

characterised in

that said plant growth regulant comprises a compound of general formula I as defined in any of claims 1 - 12.

15. A process for the preparation of a compound selected from:

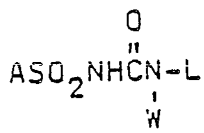

wherein

A is 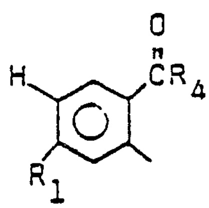 , 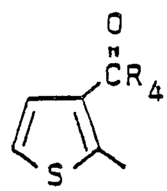 ,

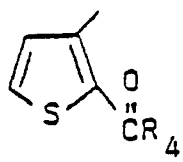 or 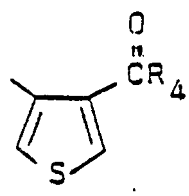

wherein $R_1$, W and L are as defined in claim 1 and $R_4$ is OH;

which comprises hydrolysing a corresponding ester wherein $R_4$ is as defined in claim 1.